# EUROPEAN PATENT SPECIFICATION

(11) **EP 2 861 599 B1**
(45) Date of publication and mention of the grant of the patent: **18.12.2019**
(21) Application number: 13731218.7
(22) Date of filing: 11.06.2013
(51) Int. Cl.: C07D 487/04, A61K 31/519, A61P 35/00, A61P 37/06

(54) **REVERSIBLE COVALENT PYRROLO- OR PYRAZOLOPYRIMIDINES USEFUL FOR THE TREATMENT OF CANCER AND AUTOIMMUNE DISEASES**
ZUR BEHANDLUNG VON KREBS UND AUTOIMMUNERKRANKUNGEN GEIGNETE REVERSIBEL KOVALENTE PYRROLO- ODER PYRAZOLOPYRIMIDINE
PYRROLO- OU PYRAZOLOPYRIMIDINES REVERSIBLEMENT COVALENTS UTILES POUR LE TRAITEMENT DU CANCER OU DE MALADIES AUTO-IMMUNES

(30) Priority: 18.06.2012 US 201261661203 P
(43) Date of publication of application: 22.04.2015
(73) Proprietor: Principia Biopharma Inc., S. San Francisco, CA 94080 (US)
(72) Inventor: GOLDSTEIN, David Michael, Redwood City, California 94061 (US); BERNER, Bret, Seattle WA 98116 (US)
(74) Representative: J A Kemp LLP
(86) International application number: PCT/US2013/045266
(87) International publication number: WO 2013/191965

(56) References cited:
- WO-A1-2012/158764
- WO-A2-2008/039218
- WO-A2-2012/158843

## Description

The present disclosure provides oral pharmaceutical formulations comprising reversible covalent compounds having a Michael acceptor moiety, in particular formulations to minimize exposure of these compounds to the stomach, a process of their production, and use of these formulations for the treatment of diseases treatable by such compounds such as cancer and autoimmune diseases.

Targeted therapy has received increased attention particularly in the oncology area due to the clinical success of kinase inhibitors as anti-cancer agents. The ongoing challenges to the development of targeted therapies include achieving high selectivity for the primary target and prolonged inhibition to maximize their therapeutic efficacy. Covalent drugs have become a highly attractive approach to designing next generation targeted therapies due to their enhanced ability to achieve high selectivity as well as prolonged inhibition even with significantly reduced systemic exposure of the drugs. Covalent drugs achieve their high selectivity and exceptional potency due to the covalent interaction with a specific cysteine residue in the active site of proteins for which the drug molecule binds. This covalent binding additionally provides prolonged efficacy with increased duration of action that outlasts the systemic exposure of the drug. Particular attention has been focused on the incorporation of Michael acceptors into drug molecules to enable the covalent binding of protein targets by interacting with specific cysteine residues. The highly electrophilic nature of Michael acceptors and their intrinsic reactivity towards cysteines and thiols has been well documented. Drugs containing Michael acceptors derived from an acrylamide moiety generally react irreversibly with thiols like glutathione and may also react irreversibly with proteins other than the desired target, especially proteins with hyper-reactive cysteines.

To avoid the potential toxicological risks of formation of irreversible covalent adducts, the design of reversible covalent drugs that retain the advantages of covalent cysteine targeting i.e., prolonged duration of action and high selectivity, without the potential liabilities associated with irreversible adduct formation have recently been reported (*see.* Serafimova I. et al. Nature Chem. Biol. Published online 1 April 2012; DOI: 10.1038/NCHEMBIO.925)). These reversible covalent drugs contain Michael acceptors with two electron withdrawing groups on the same carbon of the olefin. The presence of a second electron withdrawing group on the Michael acceptor, such as cyano, converts irreversible Michael acceptors, for example, acrylate or acrylamide, into electrophiles, for example cyanoacrylate or cyanoacrylamide, capable of binding to thiol and cysteine containing proteins or peptides reversibly i.e., reversible covalent Michael acceptors.

WO 2008/039218 discloses compounds that form covalent bonds with Bruton's tyrosine kinase (Btk), irreversible inhibitors of Btk, methods for preparing said compounds, pharmaceutical compositions comprising said compounds, and methods of using said Btk inhibitors, alone or in combination with other therapeutic agents, for the treatment of autoimmune diseases or conditions, heteroimmune diseases or conditions, cancer, and inflammatory diseases or conditions.

Applicants have discovered that reversible covalent drug molecules i.e., drugs which contain a Michael acceptor with a second electron withdrawing group can be poorly available or delayed for systemic absorption when the drug is administered orally. The oral bioavailability of such drugs can be low, and this can be manifested by low plasma AUC and/or Cmax values which restrict the drugs ability to achieve optimal efficacy in-vivo. The poor bioavailability of this new class of drugs can be attributed, in part, to the electrophilic property of reversible covalent Michael acceptors moiety in these drugs. Without wishing to be bound to any particular theory, Applicants believe that the electrophilic property of reversible covalent Michael acceptors can make these types of drugs more prone to poor absorption or clearance in the low pH environment of the stomach. Additionally, the expression of metabolizing enzymes, such as cysteine proteases, mucins, transporters and reactive thiol containing molecules in the stomach, such as glutathione, can also contribute to the low oral bioavailability of reversible covalent Michael acceptor- containing drugs (see Johnson D. S., et. al., Future Med Chem. 2010 June 1; 2(6):949-964 and Potashman M. H. et al. J. Med. Chem., Vol 52, No. 5. Pgs. 1231-1246). For example, the combination of digestive enzymes, such as the cysteine protease, pepsin, transporters and metabolizing enzymes such as CYP enzymes in the gastric mucosa, can result at low pH in high chemical and/or metabolic transformation of the reversible covalent Michael acceptors. Accordingly, by avoidance of exposure of the reversible covalent drugs to the stomach where the combination of low pH and digestive or metabolic enzymes and other sources of thiols occur, a significant increase in systemic exposure of the drug can be obtained. Additionally, avoidance of exposure to the stomach may reduce or altogether eliminate potential adverse side effects of these drugs such as diahrrea and emesis, commonly called vomiting.

Accordingly, in one aspect, the present invention provides a solid oral formulation comprising (i) a therapeutically effective amount of a compound, (ii) at least one coating chosen from an enteric coating and/or a non-enteric time-delayed release coating, and (iii) at least one pharmaceutically acceptable excipient,
wherein said compound is:
(a) a compound of Formula (Id) or a pharmaceutically acceptable salt thereof: 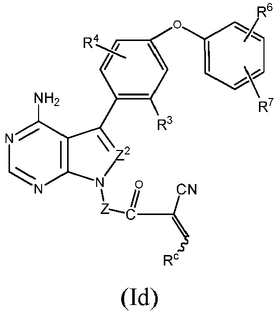 wherein:
   Z² is -N- or -CR²- where R² is hydrogen or alkyl;
   R³ and R⁴ are independently hydrogen, methyl, chloro, fluoro, cyclopropyl, hydroxy, methoxy, cyano, trifluoromethyl or trifluoromethoxy;
   R⁶ and R⁷ are independently hydrogen, methyl, methoxy, fluoro, chloro, trifluoromethyl, trifluoromethoxy, or cyano;
   Z is -(alkylene)-NR^{a}-, 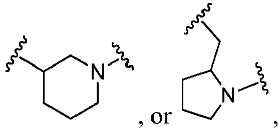 each ring optionally substituted with one or two substituents independently selected from alkyl, hydroxy, and halo, and where R^{a} is independently hydrogen or alkyl; and
   R^{c} is alkyl, haloalkoxy, substituted alkyl, cycloalkyl, cycloalkyleneNR^{d}R^{e} or cycloalkylene(alkylene)NR^{d}R^{e} (where R^{d} and R^{e} are independently hydrogen, alkyl, or cycloalkyl), or R^{c} is 3 to 6 membered saturated monocyclic heterocyclyl containing one or two heteroatoms selected from N, O, and S and is optionally substituted with one or two substituents selected from hydroxy, alkyl and fluoro; or
(b) a compound chosen from:
   N-(2-(4-amino-3-(2-fluoro-4-phenoxyphenyl)-1H-pyrazolo[3,4-d]pyrimidin-1-yl)ethyl)-1-cyano-2-cyclopropylethenesulfonamide;
   N-(2-(4-amino-3-(2-fluoro-4-phenoxyphenyl)-1H-pyrazolo[3,4-d]pyrimidin-1-yl)ethyl)-1-cyano-2-cyclopropyl-N-methylethenesulfonamide;
   2-(4-amino-3-(2-fluoro-4-phenoxyphenyl)-1H-pyrazolo[3,4-d]pyrimidin-1-yl)ethyl 2-cyano-3-cyclopropylacrylate;
   1-(4-amino-3-(2-fluoro-4-phenoxyphenyl)-1H-pyrazolo[3,4-d]pyrimidin-1-yl)-2-methylpropan-2-yl 2-cyano-3-cyclopropylacrylate;
   2-((2-(4-amino-3-(2-fluoro-4-phenoxyphenyl)-1H-pyrazolo[3,4-d]pyrimidin-1-yl)ethyl)sulfonyl)-3-cyclopropylacrylonitrile; and
   2-(5-((4-amino-3-(2-fluoro-4-phenoxyphenyl)-1H-pyrazolo[3,4-d]pyrimidin-1-yl)methyl)oxazol-2-yl)-3-cyclopropylacrylonitrile;
and pharmaceutically acceptable salts thereof.

In one embodiment, the formulation according to the invention comprises means for release of said compound in the intestine.

In one embodiment, a therapeutically effective amount of the said compound (or embodiments thereof disclosed herein); and/or a pharmaceutically acceptable salt thereof is released in the small intestine.

In another embodiment, the formulation comprises means for release of a therapeutically effective amount of said compound from said oral formulation in the intestine. In another embodiment, the said compound (or embodiments thereof disclosed herein); and/or a pharmaceutically acceptable salt thereof is released to a region of the intestine in which the pH is greater than about pH 5.5. In another embodiment, the said compound (or embodiments thereof disclosed herein); and/or a pharmaceutically acceptable salt thereof is released to a region of the intestine in which the pH is from about 5.5 to about 6.5. In yet another embodiment, the said compound (or embodiments thereof disclosed herein); and/or a pharmaceutically acceptable salt thereof is released to a region of the intestine in which the pH is from about 4.5 or about 5.5 to about 7.

In yet another embodiment, the said compound (or embodiments thereof disclosed herein); and/or a pharmaceutically acceptable salt thereof is released in one or more of the duodenum, jejunum or ileum. The release to the above regions of the intestine is achieved by coating the said compound (or embodiments thereof disclosed herein); and/or a pharmaceutically acceptable salt thereof or the dosage form comprising the said compound (or embodiments thereof disclosed herein); and/or a pharmaceutically acceptable salt thereof with at least one coating chosen from an enteric coating and/or a non-enteric time-delayed release coating. In one embodiment, when the compound and/or a pharmaceutically acceptable salt thereof or the dosage form comprising the compound and/or a pharmaceutically acceptable salt thereof is coated with an enteric coating, the enteric coating is a polymer. In another embodiment, when the compound and/or a pharmaceutically acceptable salt thereof or the dosage form comprising the compound and/or a pharmaceutically acceptable salt thereof is coated with an enteric coating, the enteric coating is an anionic polymer such as chosen from polymethacrylates (e.g., methacrylic acid ethacrylate poly, methacrylic acid methyl methacrylate poly); cellulose-based polymers (e.g., cellulose acetate phthalate CAP, cellulose acetate trimellitate CAT, cellulose acetate succinate CAS, hydroxypropylmethyl-cellulose phthalate HPMCP, hydroxypropylmethylcellulose acetate succinate HPMCAS) or polyvinyl derivatives such as polyvinyl acetate phthalate PVAP. In one embodiment, when a non-enteric coating is employed, the non-enteric time-delayed release dosage forms can be administered in fasted state and the time-delayed release coating can be designed to erode, burst, or become hightly permeable in about 0.3 to about 3 hours or in about 0.5 to about 2 hours after administration to release the said compound (or embodiments thereof disclosed herein); and/or a pharmaceutically acceptable salt thereof.

In another embodiment, the formulation according to the invention comprises means for increasing the oral bioavailability of said compound.

In one embodiment, the increase in the oral bioavailability of the said compound (or embodiments thereof disclosed herein); and/or a pharmaceutically acceptable salt thereof is due to the release of said compound and/or pharmaceutically acceptable salt thereof in the intestine.

In another embodiment, the increase in the oral bioavailability of the said compound (or embodiments thereof disclosed herein); and/or a pharmaceutically acceptable salt thereof is due to the release of said compound and/or pharmaceutically acceptable salt thereof in the small intestine.

In yet another embodiment, the increase in the oral bioavailability of the said compound (or embodiments thereof disclosed herein); and/or a pharmaceutically acceptable salt thereof is due to the release of said at least one compound and/or pharmaceutically acceptable salt thereof to a region of the gastrointestinal tract in which the pH is greater than pH 4.5. In one embodiment, the pH is greater than about 5. In another embodiment the pH is from about 5.5 to about 6.5. In yet another embodiment the pH is from about 4.5 or about 5.5 to about 7. In one embodiment, the release is in one or more of the duodenum, jejunum or ileum. The release to the above regions of the intestine is achieved by coating the said compound (or embodiments thereof disclosed herein); and/or a pharmaceutically acceptable salt thereof or the dosage form comprised of the said compound (or embodiments thereof disclosed herein); and/or a pharmaceutically acceptable salt thereof with at least one coating chosen from an enteric coating or a non-enteric, time-delayed release coating. In one embodiment, when the compound and/or a pharmaceutically acceptable salt thereof or the dosage form comprising the compound and/or a pharmaceutically acceptable salt thereof is coated with an enteric coating, the enteric coating is a polymer. In another embodiment, when the compound and/or a pharmaceutically acceptable salt thereof or the dosage form comprising a the compound and/or a pharmaceutically acceptable salt thereof is coated with an enteric coating, the enteric coating is an anionic polymer such as chosen from polymethacrylates (e.g., methacrylic acid ethacrylate poly, methacrylic acid methyl methacrylate poly); cellulose-based polymers (e.g., cellulose acetate phthalate CAP, cellulose acetate trimellitate CAT, cellulose acetate succinate CAS, hydroxypropylmethylcellulose phthalate HPMCP, hydroxypropylmethylcellulose acetate succinate HPMCAS) or polyvinyl derivatives such as polyvinyl acetate phthalate PVAP.

In one embodiment, the non-enteric delayed release dosage forms can be administered in fasted state and the time-delayed release coating can be designed to erode, burst, or become very permeable in about 0.3 to about 3 hours or in about 0.5 to about 2 hours after administration to release the said compound (or embodiments thereof disclosed herein); and/or a pharmaceutically acceptable salt thereof. When the dosage form comprised of the said compound is enteric-coated, it is generally administered in the fasted state to avoid variability in gastric emptying and the resulting variability in the initiation of efficacious plasma levels.
In yet another embodiment the enteric coating dissolves at a pH from about 4.5 to about 7 or about 5.5 to about 7.

In one embodiment, the enteric coating is a polymer which erodes at about pH 5.5 and above, or from about pH 5.5 to about 6.5. In another embodiment, the enteric coating is a polymer which erodes at about pH 4.5 and above, or from about pH 4.5 to about 7.

In one embodiment, the solid oral formulations disclosed herein, are enteric coated tablets or capsules comprising the said compound (or embodiments thereof disclosed herein) and/or a pharmaceutically acceptable salt thereof and at least one pharmaceutically acceptable excipient; or a compound of the present invention (or embodiments thereof disclosed herein) and/or a pharmaceutically acceptable salt thereof; and at least one pharmaceutically acceptable excipient.

In another embodiment, the solid oral formulations disclosed herein is a tablet or capsule comprising enteric coated said compound (or embodiments thereof disclosed herein) and/or a pharmaceutically acceptable salt thereof and at least one pharmaceutically acceptable excipient; or an enteric coated compound of the present invention(or embodiments thereof disclosed herein) and/or a pharmaceutically acceptable salt thereof; and at least one pharmaceutically acceptable excipient. In one embodiment, the enteric coating is a polymer. In another embodiment, the enteric coating is an anionic polymer such as chosen from polymethacrylates (e.g., methacrylic acid ethacrylate poly, methacrylic acid methyl methacrylate poly); cellulose-based polymers (e.g., cellulose acetate phthalate CAP, cellulose acetate trimellitate CAT, cellulose acetate succinate CAS, hydroxypropylmethylcellulose phthalate HPMCP, hydroxypropylmethylcellulose acetate succinate HPMCAS) or polyvinyl derivatives such as polyvinyl acetate phthalate PVAP. In one embodiment, any of the formulations disclosed herein contain a therapeutically effective amount of a compound of the present invention(or embodiments thereof disclosed herein) and/or a pharmaceutically acceptable salt thereof and at least one pharmaceutically acceptable excipient.

In one embodiment, any of the formulations disclosed herein contain, unless stated otherwise, one or more pharmaceutically acceptable excipient(s) such as binders, surfactants, diluents, buffering agents, antiadherents, glidants, hydrophilic or hydrophobic polymers, retardants, stabilizing agents or stabilizers, disintegrants or superdisintegrants, antioxidants, antifoaming agents, fillers, flavors, colors, lubricants, sorbents, preservatives, plasticizers, or sweeteners, or mixtures thereof, which facilitate processing of the compound of the present invention (or embodiments thereof disclosed herein) or a pharmaceutically acceptable salt thereof into preparations which can be used pharmaceutically. Any of the well-known techniques and excipients may be used as suitable and as understood in the art, *see* for example, Remington: The Science and Practice of Pharmacy, Twenty-first Ed., (Pharmaceutical Press, 2005); Liberman, H. A., Lachman, L., and Schwartz, J.B. Eds., Pharmaceutical Dosage Forms, Vol. 1-2 Taylor & Francis 1990; and R.I. Mahato, Ansel's Pharmaceutical Dosage Forms and Drug Delivery Systems, Second Ed. (Taylor & Francis, 2012).

In certain embodiments, the formulations may include one or more pH adjusting agents or buffering agents, for example, acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate, ammonium chloride, and the like. Such acids, bases and buffers are included in an amount required to maintain pH of the composition in an acceptable range.

In certain embodiments, the formulations may also include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

In certain embodiments, the formulations may also include one or more antifoaming agents to reduce foaming during processing which can result in coagulation of aqueous dispersions, bubbles in the finished film, or generally impair processing. Exemplary anti-foaming agents include silicon emulsions or sorbitan sesquoleate.

In certain embodiments, the formulations may also include one or more antioxidants, such as non-thiol antioxidants, for example, butylated hydroxytoluene (BHT), sodium ascorbate, ascorbic acid, and tocopherol. In certain embodiments, antioxidants enhance chemical stability where required.

In certain embodiments, the formulations may also include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

In certain embodiments, the formulations may also include one or more binders. Binders impart cohesive qualities and include, e.g., alginic acid and salts thereof; cellulose derivatives such as carboxymethylcellulose, methylcellulose (e.g., Methocel®), hydroxypropylmethylcellulose, hydroxyethylcellulose, hydroxypropylcellulose (e.g., Klucel®), ethylcellulose (e.g., Ethocel®), and microcrystalline cellulose (e.g., Avicel®); microcrystalline dextrose; amylose; magnesium aluminum silicate; polysaccharide acids; bentonites; gelatin; polyvinyl-pyrrolidone/vinyl acetate copolymer; crosspovidone; povidone; starch; pregelatinized starch; tragacanth, dextrin, a sugar, such as sucrose (e.g., Dipac®), glucose, dextrose, molasses, mannitol, sorbitol, xylitol (e.g., Xylitab®), and lactose; a natural or synthetic gum such as acacia, tragacanth, ghatti gum mucilage of isapol husks, polyvinylpyrrolidone (e.g., Polyvidone® CL, Kollidon® CL, Polyplasdone® XL-10), larch arabogalactan, Veegum®, polyethylene glycol, polyethylene oxide, waxes, sodium alginate, and the like.

In certain embodiments, the formulations may also include dispersing agents and/or viscosity modulating agents. Dispersing agents and/or viscosity modulating agents include materials that control the diffusion and homogeneity of a drug through liquid media or a granulation method or blend method. In some embodiments, these agents also facilitate the effectiveness of a coating or eroding matrix. Exemplary diffusion facilitators/dispersing agents include, e.g., hydrophilic polymers, electrolytes, Tween®60 or 80, PEG, polyvinylpyrrolidone (PVP; commercially known as Plasdone®), and the carbohydrate-based dispersing agents such as, for example, hydroxypropyl celluloses (e.g., HPC, H--PC-SL, and HPC-L), hydroxypropyl methylcelluloses (e.g., HPMC K100, RPMC K4M, HPMC K15M, and HPMC K100M), carboxymethylcellulose sodium, methylcellulose, hydroxyethylcellulose, hydroxypropyl-cellulose, hydroxypropylmethylcellulose phthalate, hydroxypropylmethylcellulose acetate stearate (HPMCAS), noncrystalline cellulose, magnesium aluminum silicate, triethanolamine, polyvinyl alcohol (PVA), vinyl pyrrolidone/vinyl acetate copolymer (S630), 4-(1,1,3,3-tetramethylbutyl)-phenol polymer with ethylene oxide and formaldehyde (also known as tyloxapol), poloxamers (e.g., Pluronics F68®, F88®., and F10®8, which are block copolymers of ethylene oxide and propylene oxide); and poloxamines (e.g., Tetronic 908®, also known as Poloxamine 908®, which is a tetrafonctional block copolymer derived from sequential addition of propylene oxide and ethylene oxide to ethylenediamine (BASF Corporation, Parsippany, N.J.)), polyvinylpyrrolidone K12, polyvinylpyrrolidone K17, polyvinylpyrrolidone K25, or polyvinylpyrrolidone K30, polyvinylpyrrolidone/vinyl acetate copolymer (S-630), polyethylene glycol, e.g., the polyethylene glycol can have a molecular weight of about 300 to about 6000, or about 3350 to about 4000, or about 7000 to 5400, sodium carboxymethylcellulose, methylcellulose, polysorbate-80, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, polysorbate-80, sodium alginate, polyethoxylated sorbitan monolaurate, polyethoxylated sorbitan monolaurate, povidone, carbomers, polyvinyl alcohol (PVA), alginates, chitosans and combinations thereof. Plasticizcers such as cellulose or triethyl cellulose can also be used as dispersing agents. Dispersing agents particularly useful in liposomal dispersions and self-emulsifying dispersions are dimyristoyl phosphatidyl choline, natural phosphatidyl choline from eggs, natural phosphatidyl glycerol from eggs, cholesterol and isopropyl myristate. In general, binder levels of about 10 to about 70% are used in powder-filled gelatin capsule formulations. Binder usage level in tablet formulations varies whether direct compression, wet granulation, roller compaction, or usage of other excipients such as fillers which itself can act as moderate binder. Formulators skilled in art can determine the binder level for the formulations, but binder usage level of up to 70% in tablet formulations is common.

In certain embodiments, the formulations may also include one or more "diluents" which refers to chemical compounds that are used to dilute the compound of interest prior to delivery. Diluents can also be used to stabilize compounds because they can provide a more stable environment Salts dissolved in buffered solutions (which also can provide pH control or maintenance) are utilized as diluents in the art, including, but not limited to a phosphate buffered saline solution. In certain embodiments, diluents increase bulk of the composition to facilitate compression or create sufficient bulk for homogenous blend for capsule filling. Such compounds include e.g., lactose, starch, mannitol, sorbitol, dextrose, microcrystalline cellulose such as Avicel®.; dibasic calcium phosphate, dicalcium phosphate dihydrate; tricalcium phosphate, calcium phosphate; anhydrous lactose, spray-dried lactose; pregelatinized starch, compressible sugar, such as Di-Pac® (Amstar); hydroxypropylmethylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylose; powdered cellulose, calcium carbonate; glycine, kaolin; mannitol, sodium chloride; inositol, bentonite, and the like.

In certain embodiments, the formulations may also include one or more "disintegrant" which includes both the dissolution and dispersion of the dosage form when contacted with gastrointestinal fluid. "Disintegration agents or disintegrants" facilitate the breakup or disintegration of a substance. Examples of disintegration agents include a starch, e.g., a natural starch such as corn starch or potato starch, a pregelatinized starch such as National 1551 or sodium starch glycolate such as Promogel® or Explotab®, a cellulose such as a wood product, methylcrystalline cellulose, e.g., Avicel®, Avicel® PH101, Avicel® PH 102, Avicel® PHI05, Elceme® P100, Emcocel®, Vivacel®, and Solka-Floc®, methylcellulose, croscarmellose, or a cross-linked cellulose, such as cross-linked sodium carboxymethylcellulose (Ac-Di-Sol®), cross-linked carboxymethylcellulose, or cross-linked croscarmellose, a cross-linked starch such as sodium starch glycolate, a cross-linked polymer such as crosspovidone, a cross-linked polyvinylpyrrolidone, alginate such as alginic acid or a salt of alginic acid such as sodium alginate, a clay such as Veegum® HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and the like.

In certain embodiments, the formulations may also include erosion facilitators. "Erosion facilitators" include materials that control the erosion of a particular material in gastrointestinal fluid. Erosion facilitators are generally known to those of ordinary skill in the art. Exemplary erosion facilitators include, e.g., hydrophilic polymers, electrolytes, proteins, peptides, and amino acids.

In certain embodiments, the formulations may also include one or more filling agents which include compounds such as lactose, calcium carbonate, calcium phosphate, dibasic calcium phosphate, calcium sulfate, microcrystalline cellulose, cellulose powder, dextrose, dextrates, dextran, starches, pregelatinized starch, sucrose, xylitol, lactitol, mannitol, sorbitol, sodium chloride, polyethylene glycol, and the like.

In certain embodiments, the formulations may also include one or more flavoring agentsand/or "sweeteners" e.g., acacia syrup, acesulfame K, alitame, anise, apple, aspartame, banana, Bavarian cream berry, black currant, butterscotch, calcium citrate, camphor, caramel, cherry, cherry cream chocolate, cinnamon, bubble gum, citrus, citrus punch, citrus cream, cotton candy, cocoa, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, monoammonium glyrrhizinate, maltol, mannitol, maple, marshmallow, menthol, mint cream, mixed berry, neohesperidine DC, neotame, orange, pear, peach, peppermint, peppermint cream, Powder, raspberry, root beer, rum, saccharin, safrole, sorbitol, spearmint, spearmint cream, strawberry, strawberry cream, stevia, sucralose, sucrose, sodium saccharin, saccharin, aspartame, acesulfame potassium, mannitol, talin, sylitol, sucralose, sorbitol, Swiss cream, tagatose, tangerine, thaumatin, tutti fruitti, vanilla, walnut, watermelon, wild cherry, wintergreen, xylitol, or any combination of these flavoring ingredients, e.g., anise-menthol, cherry-anise, cinnamon-orange, cherry-cinnamon, chocolate-mint, honey-lemon, lemon-lime, lemon-mint, menthol-eucalyptus, orange-cream, vanilla-mint, and mixtures thereof.

In certain embodiments, the formulations may also include one or more lubricants and glidants which are compounds that prevent, reduce or inhibit adhesion or friction of materials. Exemplary lubricants include, e.g., stearic acid, calcium hydroxide, talc, sodium stearyl lumerate, a hydrocarbon such as mineral oil, or hydrogenated vegetable oil such as hydrogenated soybean oil, higher fatty acids and their alkali-metal and alkaline earth metal salts, such as aluminum, calcium, magnesium, zinc, stearic acid, sodium stearates, glycerol, talc, waxes, boric acid, sodium benzoate, sodium acetate, sodium chloride, leucine, a polyethylene glycol (e.g., PEG4000) or a methoxypolyethylene glycol such as Carbowax®, sodium oleate, sodium benzoate, glyceryl behenate, polyethylene glycol, magnesium or sodium lauryl sulfate, colloidal silica such as Syloid®, Cab-O-Sil®, a starch such as corn starch, silicone oil, a surfactant, and the like.

In certain embodiments, the formulations may also include one or more plasticizers which are compounds used to soften the enteric or delayed release coatings to make them less brittle. Suitable plasticizers include, e.g., polyethylene glycols such as PEG 300, PEG 400, PEG 600, PEG 1450, PEG 3350, and PEG 800, stearic acid, propylene glycol, oleic acid, triethyl citrate, dibutyl sebacate, triethyl cellulose and triacetin. In some embodiments, plasticizers can also function as dispersing agents or wetting agents.

In certain embodiments, the formulations may also include one or more solubilizers which include compounds such as triacetin, triethylcitrate, ethyl oleate, ethyl caprylate, sodium lauryl sulfate, sodium doccusate, vitamin E TPGS, dimethylacetamide, N-methylpyrrolidone, N-hydroxyethylpyrrolidone, polyvinylpyrrolidone, hydroxypropylmethyl cellulose, hydroxypropyl cyclodextrins for example Captisol®, ethanol, n-butanol, isopropyl alcohol, cholesterol, bile salts, polyethylene glycol 200-600, glycofurol, transcutol, propylene glycol, and dimethyl isosorbide and the like. In one embodiment, the solubilizer is vitamin E TPGS and/or Captisol®.

In certain embodiments, the formulations may also include one or more suspending agents which include compounds such as polyvinylpyrrolidone, e.g., polyvinylpyrrolidone K112, polyvinylpyrrolidone K17, polyvinylpyrrolidone K25, or polyvinylpyrrolidone K30, vinyl pyrrolidone/vinyl acetate copolymer (S630), polyethylene glycol, e.g., the polyethylene glycol can have a molecular weight of about 300 to about 6000, or about 3350 to about 4000, or about 7000 to about 5400, sodium carboxymethylcellulose, methylcellulose, hydroxypropylmethylcellulose, hydroxymethylcellulose acetate stearate, polysorbate-80, hydroxyethylcellulose, sodium alginate, gums, such as, e.g., gum tragacanth and gum acacia, guar gum, xanthans, including xanthan gun, sugars, cellulosics, such as, e.g., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like.

In certain embodiments, the formulations may also include one or more surfactants which include compounds such as sodium lauryl sulfate, sodium docusate, Tween 60 or 80, triacetin, vitamin E TPGS, sorbitan monooleate, polyoxyethylene sorbitan monooleate, polysorbates, polaxomers, bile salts, glyceryl monostearate, copolymers of ethylene oxide and propylene oxide, e.g., Pluronic® (BASF), and the like. Some other surfactants include polyoxyethylene fatty acid glycerides and vegetable oils, e.g., polyoxyethylene (60) hydrogenated castor oil; and polyoxyethylene alkylethers and alkylphenyl ethers, e.g. octoxynol 10, octoxynol 40. In some embodiments, surfactants may be included to enhance physical stability or for other purposes.

In certain embodiments, the formulations may also include one or more viscosity enhancing agents which include, e.g., methyl cellulose, xanthan gum, carboxymethyl cellulose, hydroxypropyl cellulose, hydroxypropylmethyl cellulose, hydroxypropylmethyl cellulose acetate stearate, hydroxypropylmethyl cellulose phthalate, carbomer, polyvinyl alcohol alginates, acacia, chitosans and combinations thereof.

In certain embodiments, the formulations may also include one or more wetting agents which include compounds such as oleic acid, glyceryl monostearate, sorbitan monooleate, sorbitan monolaurate, triethanolamine oleate, polyoxyethylene sorbitan monooleate, polyoxyethylene sorbitan monolaurate, sodium docusate, sodium oleate, sodium lauryl sulfate, sodium doccusate, triacetin, Tween 80, vitamin E TPGS, ammonium salts and the like.

Pharmaceutical preparations disclosed herein can be obtained by mixing one or more solid excipient such as carrier, binder, filling agent, suspending agent, flavoring agent, sweetening agent, disintegrating agent, dispersing agent, surfactant, lubricant, colorant diluent, solubilizer, moistening agent, plasticizer, stabilizer, penetration enhancer, wetting agent, anti-foaming agent, antioxidant, preservative, or one or more combination thereof with one or more of the compounds described herein, optionally grinding the resulting mixture, and processing the mixture of granules, after adding suitable excipients, if desired, to obtain tablets.

Pharmaceutical preparations disclosed herein also include capsules made of gelatin, as well as soft, sealed capsules made of gelatin and a plasticizer, such as glycerol or sorbitol. Capsules may also be made of polymers such as hypromellose. The capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, lipids, solubilizers, or liquid polyethylene glycols. In addition, stabilizers may be added. All formulations for oral administration should be in dosages suitable for such administration.

These formulations can be manufactured by conventional pharmacological techniques. Conventional pharmacological techniques include, e.g., one or a combination of methods: (1) dry mixing, (2) direct compression, (3) milling, (4) dry or non-aqueous granulation, (5) wet granulation, (6) fusion, or (7) extrusion. *See,* e.g., Lachman et al., The Theory and Practice of Industrial Pharmacy, 3rd ed. (1986). Other methods include, e.g., spray drying, pan coating, melt granulation, granulation, fluidized bed spray drying or coating (e.g., wurster coating), tangential coating, top spraying, tableting, extruding, extrusion/ spheronization, and the like.

It should be appreciated that there is considerable overlap between excipients used in the solid dosage forms described herein. Thus, the above-listed additives should be taken as merely exemplary, and not limiting, of the types of excipient that can be included in solid dosage forms described herein. The type and amounts of such excipient can be readily determined by one skilled in the art, according to the particular properties desired.

In some embodiments, the solid dosage forms described herein are enteric coated oral dosage forms, i.e., as an oral dosage form of a pharmaceutical composition as described herein which utilizes an enteric coating to effect the release of the compound in the small intestine of the gastrointestinal tract. An "enterically coated" drug or tablet refers to a drug or tablet that is coated with a substance--i.e., with an "enteric coating"--that remains intact in the stomach but dissolves and releases the drug once the intestine (in one embodiment small intestine) is reached. As used herein "enteric coating", is a material, such as a polymer material or materials which encase the therapeutically active agent core either as a dosage form or as particles. Typically, a substantial amount or all of the enteric coating material is dissolved before the therapeutically active agent is released from the dosage form, so as to achieve delayed dissolution of the therapeutically active agent core or particles in the small intestine. Enteric coatings are discussed, for example, Loyd, V. Allen, Remington: The Science and Practice of Pharmacy, Twenty-first Ed., (Pharmaceutical Press, 2005; and P.J. Tarcha, Polymers for Controlled Drug Delivery, Chapter 3, CRC Press, 1991. Methods for applying enteric coatings to pharmaceutical compositions are well known in the art, and include for example, U.S. Patent Publication No. 2006/0045822.

The enteric coated dosage form may be a compressed or molded or extruded tablet (coated or uncoated) containing granules, powder, pellets, beads or particles of the compound of the present invention (or any embodiments thereof) and/or other excipients, which are themselves coated or uncoated provided at least one of them is coated. The enteric coated oral dosage form may also be a capsule (coated or uncoated) containing pellets, beads or granules of the compound of the present invention
(or any embodiments thereof) and/or other excipients, which are themselves coated or uncoated provided at least one of them is coated. Some examples of coatings that were originally used as enteric coatings are beeswax and glyceryl monostearate; beeswax, shellac and cellulose; and cetyl alcohol, mastic and shellac as well as shellac and stearic acid (U.S. Pat. No. 2,809,918); polyvinylacetate and ethyl cellulose (U.S. Pat. No. 3,835,221). More recently, the coatings used are neutral copolymers of polymethacrylic acid esters (Eudragit L30D). (F. W. Goodhart et al, Pharm. Tech., p. 64-71, April, 1984); copolymers of methacrylic acid and methacrylic acid methyl ester (Eudragit S), or a neutral copolymer of polymethacrylic acid esters containing metallic stearates (Mehta et al U.S. Pat. Nos. 4,728,512 and 4,794,001), cellulose acetate succinate, and hypromellose phthalate.

Any anionic polymer exhibiting a pH-dependent solubility profile can be used as an enteric coating in the methods and compositions described herein to achieve delivery to the intestine. In one embodiment, delivery to the small intestine. In another embodiment, delivery to the duodenum. In some embodiments the polymers described herein are anionic carboxylic polymers. In other embodiments, the polymers and compatible mixtures thereof, and some of their properties, include, but are not limited to:

### Shellac

Also called purified lac, it is a refined product obtained from the resinous secretion of an insect. This coating dissolves in media of pH>7;
Acrylic polymers.

The performance of acrylic polymers (primarily their solubility in biological fluids) can vary based on the degree and type of substitution. Examples of suitable acrylic polymers include methacrylic acid copolymers and ammonium methacrylate copolymers. The Eudragit series L, S, and RS (manufactured Rohm Pharma and known as Evonik®) are available as solubilized in organic solvent, aqueous dispersion, or dry powders. The Eudragit series RL, NE, and RS are insoluble in the gastrointestinal tract but are permeable and are used primarily for colonic targeting. The Eudragit series L, L-30D and S are insoluble in stomach and dissolve in the intestine;
Cellulose Derivatives.

Examples of suitable cellulose derivatives are: ethyl cellulose; reaction mixtures of partial acetate esters of cellulose with phthalic anhydride. The performance can vary based on the degree and type of substitution. Cellulose acetate phthalate (CAP) dissolves in pH>6. Aquateric (FMC) is an aqueous based system and is a spray dried CAP pseudolatex with particles <1 µm. Other components in Aquateric can include pluronics, Tweens, and acetylated monoglycerides. Other suitable cellulose derivatives include; cellulose acetate tritnellitate (Eastman); methylcellulose (Pharmacoat, Methocel); hydroxypropylmethyl cellulose phthalate (HPMCP); hydroxypropylmethyl cellulose succinate (HPMCS); and hydroxypropylmethylcellulose acetate succinate (HPMCAS e.g., AQOAT (Shin Etsu)). The performance can vary based on the degree and type of substitution. For example, HPMCP such as, HP-50, HP-55, HP-55S, HP-55F grades are suitable. The performance can vary based on the degree and type of substitution. For example, suitable grades of hydroxypropylmethylcellulose acetate succinate include, but are not limited to, AS-LG (LF), which dissolves at pH 5, AS-MG (MF), which dissolves at pH 5.5, and AS-HG (HF), which dissolves at higher pH. These polymers are offered as granules, or as fine powders for aqueous dispersions;
Poly Vinyl Acetate Phthalate (PVAP).

PVAP dissolves in pH>5, and it is much less permeable to water vapor and gastric fluids. Detailed description of above polymers and their pH-dependent solubility can be found at in the article titled "Enteric coated hard gelatin capsules" byProfessor Karl Thoma and Karoline Bechtold at http://pop.www.capsugel.com/media/library/enteric-coated-hard-gelatin-capsules.pdf In some embodiments, the coating can, and usually does, contain a plasticizer and possibly other coating excipients such as colorants, talc, and/or magnesium stearate, which are well known in the art. Suitable plasticizers include triethyl citrate (Citroflex 2), triacetin (glyceryl triacetate), acetyl triethyl citrate (Citroflec A2), Carbowax 400 (polyethylene glycol 400), diethyl phthalate, tributyl citrate, acetylated monoglycerides, glycerol, fatty acid esters, propylene glycol, and dibutyl phthalate. In particular, anionic carboxylic acrylic polymers usually will contain 10-25% by weight of a plasticizer, especially dibutyl phthalate, polyethylene glycol, triethyl citrate and triacetin. Conventional coating techniques such as fluid bed or Wurster coaters, or spray or pan coating are employed to apply coatings. The coating thickness must be sufficient to ensure that the oral dosage form remains intact until the desired site of topical delivery in the intestinal tract is reached.

Colorants, surfactants, anti-adhesion agents, antifoaming agents, lubricants (e.g., carnuba wax or PEG) and other additives may be added to the coatings besides plasticizers to solubilize or disperse the coating material, and to improve coating performance and the coated product.

To accelerate the dissolution of the enteric coat, a half-thickness, double coat of enteric polymer (for instance, Eudragit L30 D-55) may be applied, and the inner enteric coat may have a buffer up to pH 6.0 in the presence of 10% citric acid, followed by a final layer of standard Eudragit L 30 D-55. Applying two layers of enteric coat, each half the thickness of a typical enteric coat, Liu and Basit were able to accelerate enteric coating dissolution compared to a similar coating system applied, unbuffered, as a single layer (Liu, F. and Basit, A. Journal of Controlled Release. 147 (2010) 242-245.)

The intactness of the enteric coating may be measured, for example, by the degradation of the drug within the micropellets. The enteric coated dosage forms or pellets may be tested in dissolution testing first in gastric fluid and separately in intestinal fluid as described in USP to determine its function.

The enteric coated tablets and capsules formulation containing the disclosed compounds can be made by methods well known in the art. For example, tablets containing a compound disclosed herein can be enterically coated with a coating solution containing Eudragit®, diethylphthlate, isopropyl alcohol, talc, and water using a side vented coating pan (Freund Hi-Coater).

Alternatively, a multi-unit dosage form comprising enteric-coated pellets that can be incorporated into a tablet or into a capsule can be prepared as follows.

### Core material:

The core material for the individually enteric coating layered pellets can be constituted according to different principles. Seeds layered with the active agent, optionally mixed with alkaline substances or buffer, can be used as the core material for the further processing.

The seeds which are to be layered with the active agent can be water insoluble seeds comprising different oxides, celluloses, organic polymers and other materials, alone or in mixtures or water-soluble seeds comprising different inorganic salts, sugars, non-pareils and other materials, alone or in mixtures. Further, the seeds may comprise the active agent in the form of crystals, agglomerates, compacts etc. The size of the seeds is not essential for the present invention but may vary between approximately 0.1 and 2 mm. The seeds layered with the active agent are produced either by powder or solution/suspension layering using for instance granulation or spray coating layering equipment.

Before the seeds are layered, active agent may be mixed with further components. Such components can be binders, surfactants, fillers, disintegrating agents, alkaline additives or other and/or pharmaceutically acceptable ingredients alone or in mixtures. The binders are for example polymers such as hydroxypropyl methylcellulose (HPMC), hydroxypropylcellulose (HPC), carboxymethylcellulose sodium, polyvinyl pyrrolidone (PVP), or sugars, starches or other pharmaceutically acceptable substances with cohesive properties. Suitable surfactants are found in the groups of pharmaceutically acceptable non-ionic or ionic surfactants such as for instance sodium lauryl sulfate.

Alternatively, the active agent optionally mixed with with suitable constituents can be formulated into a core material. Said core material may be produced by extrusion/ spheronization, balling or compression utilizing conventional process equipment. The size of the formulated core material is approximately between 0.1 and 4 mm and for example, between 0.1 and 2 mm. The manufactured core material can further be layered with additional ingredients comprising the active agent and/or be used for further processing.

The active agent is mixed with pharmaceutical constituents to obtain preferred handling and processing properties and a suitable concentration of the active agent in the final preparation. Pharmaceutical constituents such as fillers, binders, lubricants, disintegrating agents, surfactants and other pharmaceutically acceptable additives may be used.

Alternatively, the aforementioned core material can be prepared by using spray drying or spray congealing technique.

### Enteric Coating Layer(s):

Before applying the enteric coating layer(s) onto the core material in the form of individual pellets, the pellets may optionally be covered with one or more separating layer(s) comprising pharmaceutical excipients optionally including alkaline compounds such as pH-buffering compounds. This/these separating layer(s), separate(s) the core material from the outer layers being enteric coating layer(s). This/these separating layer(s) protecting the core material of active agent should be water soluble or rapidly disintegrating in water.

A separating layer(s) can be optionally applied to the core material by coating or layering procedures in suitable equipments such as coating pan, coating granulator or in a fluidized bed apparatus using water and/or organic solvents for the coating process. As an alternative the separating layer(s) can be applied to the core material by using powder coating technique. The materials for the separating layers are pharmaceutically acceptable compounds such as, for instance, sugar, polyethylene glycol, polyvinylpyrrolidone, polyvinyl alcohol, polyvinyl acetate, hydroxypropyl cellulose, methylcellulose, ethylcellulose, hydroxypropyl methyl cellulose, carboxymethylcellulose sodium, water soluble salts of enteric coating polymers and others, used alone or in mixtures. Additives such as plasticizers, colorants, pigments, fillers anti-tacking and anti-static agents, such as for instance magnesium stearate, titanium dioxide, talc and other additives may also be included into the separating layer(s).

When the optional separating layer is applied to the core material it may constitute a variable thickness. The maximum thickness of the separating layer(s) is normally only limited by processing conditions. The separating layer may serve as a diffusion barrier and may act as a pH-buffering zone. The optionally applied separating layer(s) is not essential for the invention. However, the separating layer(s) may improve the chemical stability of the active substance and/or the physical properties of the novel multiple unit tableted dosage form.

Alternatively, the separating layer may be formed *in situ* by a reaction between an enteric coating polymer layer applied on the core material and an alkaline reacting compound in the core material. Thus, the separating layer formed comprises a water soluble salt formed between the enteric coating layer polymer(s) and an alkaline reacting compound which is in the position to form a salt

One or more enteric coating layers are applied onto the core material or onto the core material covered with separating layer(s) by using a suitable coating technique. The enteric coating layer material may be dispersed or dissolved in either water or in suitable organic solvents. As enteric coating layer polymers one or more, separately or in combination, of the following can be used, e.g. solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating polymer(s).

The enteric coating layers contain pharmaceutically acceptable plasticizers to obtain the desired mechanical properties, such as flexibility and hardness of the enteric coating layers. Such plasticizers are for instance, but not restricted to triacetin, citric acid esters, phthalic acid esters, dibutyl sebacate, cetyl alcohol, polyethylene glycols, polysorbates or other plasticizers.

The amount of plasticizer is optimized for each enteric coating layer formula, in relation to the selected enteric coating layer polymer(s), selected plasticizer(s) and the applied amount of said polymer(s), in such a way that the mechanical properties, i.e. flexibility and hardness of the enteric coating layer(s), for instance exemplified as Vickers hardness, are adjusted so that if a tablet is desired the acid resistance of the pellets covered with enteric coating layer(s) does not decrease significantly during compression of pellets into tablets. The amount of plasticizer is usually above 5% by weight of the enteric coating layer polymer(s), such as 15-50% and further such as 20-50%. Additives such as dispersants, colorants, pigments polymers e.g. poly(ethylacrylate, methylmethacrylate), anti-tacking and anti-foaming agents may also be included into the enteric coating layer(s). Other compounds may be added to increase film thickness and to decrease diffusion of acidic gastric juices into the acid susceptible material. The maximum thickness of the applied enteric coating is normally only limited by processing conditions and the desired dissolution profile.

### Over-Coating Layer:

Pellets covered with enteric coating layer(s) may optionally further be covered with one or more over-coating layer(s). The over-coating layer(s) should be water soluble or rapidly disintegrating in water. The over-coating layer(s) can be applied to the enteric coating layered pellets by coating or layering procedures in suitable equipments such as coating pan, coating granulator or in a fluidized bed apparatus using water and/or organic solvents for the coating or layering process. The materials for over-coating layers are chosen among pharmaceutically acceptable compounds such as, for instance sugar, polyethylene glycol, polyvinylpyrrolidone, polyvinyl alcohol, polyvinyl acetate, hydroxypropyl cellulose, methylcellulose, ethylcellulose, hydroxypropyl methyl cellulose, carboxymethylcellulose sodium and others, used alone or in mixtures. Additives such as plasticizers, colorants, pigments, fillers, anti-tacking and anti-static agents, such for instance magnesium stearate, titanium dioxide, talc and other additives may also be included into the over-coating layer(s). The over-coating layer may further prevent potential agglomeration of enteric coating layered pellets, further it may protect the enteric coating layer towards cracking during the compaction process and enhance the tableting process. The maximum thickness of the applied over-coating layer(s) is normally limited by processing conditions and the desired dissolution profile. The over-coating layer may also be used as a tablet film coating layer.

Enteric coating of soft gelatin capsules may contain an emulsion, oil, microemulsion, self-emulsifying system, lipid, triglycerides, polyethylene glycol, surfactants, other solubilizers and the like, and combinations thereof, to solubilize the active agent. The flexibility of the soft gelatin capsule is maintained by residual water and plasticizer. Moreover, for gelatin capsules the gelatin may be dissolved in water so that spraying must be accomplished at a rate with relatively low relative humidity such as can be accomplished in a fluid bed or Wurster. In addition, drying should be accomplished without removing the residual water or plasticizer causing cracking of the capsule shell. Commercially available blends optimized for enteric coating of soft gelatin capsules such as Instamodel EPD (Enteric Polymeric Dispersion), available from Ideal Cures, Pvt. Ltd. (Mumbai, India). On a laboratory scale enteric coated capsules may be prepared by: a) rotating capsules in a flask or dipping capsules in a solution of the gently heated enteric coating material with plasticizer at the lowest possible temperature or b) in a lab scale sprayer/fluid bed and then drying.

For aqueous active agents, it can be especially desirable to incorporate the drug in the water phase of an emulsion. Such "water-in-oil" emulsion provides a suitable biophysical environment for the drug and can provide an oil-water interface that can protect the drug from adverse effects of pH or enzymes that can degrade the drug. Additionally, such water-in-oil formulations can provide a lipid layer, which can interact favorably with lipids in cells of the body, and can increase the partition of the formulation onto the membranes of cells. Such partition can increase the absorption of drugs in such formulations into the circulation and therefore can increase the bioavailability of the drug.

In some embodiments the water-in-oil emulsion contains an oily phase composed of long chain carboxylic acids or esters or alcohols thereof, a surfactant or a surface active agent, and an aqueous phase containing primarily water and the active agent.

Long chain carboxylic acids are those ranging from C₈ to C₂₂ with up to three unsaturated bonds (also branching). Examples of saturated straight chain acids are n-dodecanoic acid, n-tetradecanoic acid, n-hexadecanoic acid, caproic acid, caprylic acid, capric acid, lauric acid, myristic acid, palmitic acid, stearic acid, arachidic acid, behenic acid, montanic acid and melissic acid. Also useful are unsaturated monoolefinic straight chain monocarboxylic acids. Examples of these are oleic acid, gadoleic acid and erucic acid. Also useful are unsaturated (polyolefinic) straight chain monocarboxylic acids. Examples of these are linoleic acid, ricinoleic acid, linolenic acid, arachidonic acid and behenolic acid. Useful branched acids include, for example, diacetyl tartaric acid.

Examples of long chain carboxylic acid esters include, but are not limited to, those from the group of: glyceryl monostearates; glyceryl monopalmitates; mixtures of glyceryl monostearate and glyceryl monopalmitate; glyceryl monolinoleate; glyceryl monooleate; mixtures of glyceryl monopalmitate, glyceryl monostearate, glyceryl monooleate and glyceryl monolinoleate; glyceryl monolinolenate; glyceryl monogadoleate; mixtures of glyceryl monopalmitate, glyceryl monostearate, glyceryl monooleate, glyceryl monolinoleate, glyceryl monolinolenate and glyceryl monogadoleate; acetylated glycerides such as distilled acetylated monoglycerides; mixtures of propylene glycol monoesters, distilled monoglycerides, sodium steroyl lactylate and silicon dioxide; d-alpha tocopherol polyethylene glycol 1000 succinate; mixtures of mono- and di-glyceride esters such as Atmul; calcium stearoyl lactylate; ethoxylated mono- and di-glycerides; lactated mono- and di-glycerides; lactylate carboxylic acid ester of glycerol and propylene glycol; lactylic esters of long chain carboxylic acids; polyglycerol esters of long chain carboxylic acids, propylene glycol mono- and di-esters of long chain carboxylic acids; sodium stearoyl lactylate; sorbitan monostearate; sorbitan monooleate; other sorbitan esters of long chain carboxylic acids; succinylated monoglycerides; stearyl monoglyceryl citrate; stearyl heptanoate; cetyl esters of waxes; stearyl octanoate; C₈-C₃₀ cholesterol/lavosterol esters; and sucrose long chain carboxylic acid esters. Examples of the self-emulsifying long chain carboxylic acid esters include those from the groups of stearates, pamitates, ricinoleates, oleates, behenates, ricinolenates, myristates, laurates, caprylates, and caproates. In some embodiments the oily phase may comprise a combination of 2 or more of the long chain carboxylic acids or esters or alcohols thereof. In some embodiments the oil phase may comprise a mixture of caprylic/capric triglyceride and C₈/C₁₀ mono-/di-glycerides of caprylic acid.

The alcohols that can be used are exemplified by the hydroxyl forms of the carboxylic acids exemplified above and also strearyl alcohol.

Surface active agents or surfactants are long chain molecules that can accumulate at hydrophilic/hydrophobic (water/oil) interfaces and lower the surface tension at the interface. As a result they can stabilise an emulsion. In some embodiments of this invention, the surfactant may comprise: Tween® (polyoxyethylene sorbate) family of surfactants, Span® (sorbitan long chain carboxylic acid esters) family of surfactants, Pluronic® (ethylene or propylene oxide block copolymers) family of surfactants, Labrasol®, Labrafil® and Labrafac®(each polyglycolyzed glycerides) families of surfactants, sorbitan esters of oleate, stearate, laurate or other long chain carboxylic acids, poloxamers (polyethylene-polypropylene glycol block copolymers or Pluronic®.), other sorbitan or sucrose long chain carboxylic acid esters, mono and diglycerides, PEG derivatives of caprylic/capric triglycerides and mixtures thereof or mixture of two or more of the above. In some embodiments the surfactant phase may comprise a mixture of Polyoxyethylene (20) sorbitan monooleate (Tween 80®) and sorbitan monooleate (Span 80®).

The aqueous phase may optionally comprise the active agent suspended in water and a buffer.

In some embodiments, such emulsions are coarse emulsions, microemulsions and liquid crystal emulsions. In other embodiments such emulsion may optionally comprise a permeation enhancer. In other embodiments, spray-dried dispersions or microparticles or nanoparticles containing encapsulated microemulsion, coarse emulsion or liquid crystal can be used.

In some embodiments, the solid dosage forms described herein are non-enteric time-delayed release dosage forms. The term "non-enteric time-delayed release" as used herein refers to the delivery so that the release of the drug can be accomplished at some generally predictable location in the intestinal tract more distal to that which would have been accomplished if there had been no delayed release alterations. In some embodiments the method for delay of release is a coating that becomes permeable, dissolves, ruptures, and/or is no longer intact after a designed duration. The coating in the time-delayed release dosage forms can have a fixed time to erode after which the drug is released (suitable coating include polymeric coating such as HPMC, and the like) or has a core comprised of a superdisinegrant(s) or osmotic agent(s) such as a salt, hydrophilic polymer, typically polyethylene oxide or an alkylcellulose, sugar, or the like, which draw water through a membrane or a gas generating agent such as citric acid and sodium bicarbonate. The membrane may rupture after the swelling pressure exceeds a certain threshold over a desired delay time. Alternatively, a membrane could become porous by leaching an aqueous extractable over a desired delay time. The time delayed dosage forms are for example administered in a fasted state to avoid the variability in gastric emptying in the fed state.

The time- delayed dosage form can be a mechanical pill such as an Enterion® capsule or Heidelberg® capsule (pH sensitive) which can release the drug when it receives a signal which can be transmitted once it leaves the stomach.

The compounds of the present invention, including embodiments thereof disclosed herein, are kinase inhibitors. Accordingly, in a further aspect, the present invention also provides a formulation for use in treating a disease which is an autoimmune or inflammatory disease, or cancer, wherein the formulation is a solid oral formulation according to the invention.
In one embodiment, the disease is treatable by inhibition of a tyrosine kinase such as BLK, BMX, EGFR, HER2, HER4, ITK, TEC, BTK, and TXK. In one embodiment, the tyrosine kinase is BTK.

In one embodiment the disease is inflammatory disease such as arthritis, kidney disease, or cancer such as B-cell non-Hodgkin lymphoma.

In one embodiment of this aspect, the subject in need is suffering from an autoimmune disease, e.g., inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjogren's syndrome, multiple sclerosis, Guillain-Barre syndrome, acute disseminated encephalomyelitis, Addison's disease, opsoclonus-myoclonus syndrome, ankylosing spondylitisis, antiphospholipid antibody syndrome, aplastic anemia, autoimmune hepatitis, coeliac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behcet's disease, chronic fatigue, dysautonomia, endometriosis, interstitial cystitis, neuromyotonia, scleroderma, or vulvodynia. For example, the disease is rheumatoid arthritis. For example, the autoimmune disease is lupus. In another embodiment of this aspect, the patient in need is suffering from a heteroimmune condition or disease, e.g., graft versus host disease, transplantation, transfusion, anaphylaxis, allergy, type I hypersensitivity, allergic conjunctivitis, allergic rhinitis, or atopic dermatitis.

In another embodiment of this aspect, the patient in need is suffering from an inflammatory disease, e.g., asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, proctitis, prostatitis, pyelonephritis, rhinitis, salpingitis, sinusitis, stomatitis, synovitis, tendonitis, tonsillitis, uveitis, vaginitis, vasculitis, or vulvitis. In another embodiment of this aspect, the patient is suffering from inflammatory skin disease which includes, by way of example, dermatitis, contact dermatitis, eczema, urticaria, rosacea, and scarring psoriatic lesions in the skin, joints, or other tissues or organs.

In yet another embodiment of this aspect, the subject in need is suffering from a cancer. In one embodiment, the cancer is a B-cell proliferative disorder, e.g., diffuse large B cell lymphoma, follicular lymphoma, chronic lymphocytic lymphoma, chronic lymphocytic leukemia, B-cell prolymphocytic leukemia, lymphoplamascytic lymphoma/Waldenstrom macroglobulinemia, splenic marginal zone lymphoma, plasma cell myeloma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, burkitt lymphoma/leukemia, or lymphomatoid granulomatosis. In some embodiments, the oral formulation comprising the enteric coated compound of Formula (IA) (or any of the embodiments thereof described herein),is administered in combination with another an anti-cancer agent e.g., the anti-cancer agent is an inhibitor of mitogen-activated protein kinase signaling, e.g., U0126, PD98059, PD184352, PD0325901, ARRY-142886, SB239063, SP600125, BAY 43-9006, wortmannin, Nexavar®, Tarceva®, Sutent®, Tykerb®, Sprycel®, Crizotinib, Xalkori®, or LY294002.

In yet another embodiment, the patient in need is suffering from a thromboembolic disorder, e.g., myocardial infarct, angina pectoris, reocclusion after angioplasty, restenosis after angioplasty, reocclusion after aortocoronary bypass, restenosis after aortocoronary bypass, stroke, transitory ischemia, a peripheral arterial occlusive disorder, pulmonary embolism, or deep venous thrombosis.

In a further aspect, the present invention also provides a solid oral formulation according to the invention for use in treating an inflammatory disease or proliferative disease in a patient in which the activity of a tyrosine kinase such as BLK, BMX, EGFR, HER2, HER4, ITK, TEC, BTK, and TXK contributes to the pathology and/or symptoms of the disease. In one embodiment of this aspect, the tyrosine kinase protein is BTK.

In any of the aforementioned aspects involving the treatment of proliferative disorders, including cancer, are further embodiments comprising administering the compound of the present invention (or any of the embodiments thereof described herein), in combination with at least one additional agent selected from the group consisting of alemtuzumab, arsenic trioxide, asparaginase (pegylated or non-), bevacizumab, cetuximab, platinum-based compounds such as cisplatin, cladribine, daunorubicin/doxorubicin /idarubicin, irinotecan, fludarabine, 5-fluorouracil, gemtuzamab, methotrexate, paclitaxel, Taxol™, temozolomide, thioguanine, or classes of drugs including hormones (an antiestrogen, an antiandrogen, or gonadotropin releasing hormone analogues, interferons such as alpha interferon, nitrogen mustards such as busulfan or melphalan or mechlorethamine, retinoids such as tretinoin, topoisomerase inhibitors such as irinotecan or topotecan, tyrosine kinase inhibitors such as gefinitinib or imatinib, or agents to treat signs or symptoms induced by such therapy including allopurinol, filgrastim, granisetron/ ondansetron/palonosetron, dronabinol. When combination therapy is used, the agents can be administered simultaneously or sequentially.

Also disclosed herein is a method of increasing oral bioavailability of a compound of the present invention
and/or a pharmaceutically acceptable salt thereof; comprising administering said compound in a pharmaceutical composition that releases said compound in the intestine, wherein the pharmaceutical composition is a solid oral dosage form.
In yet another embodiment of this method of increasing oral bioavailability, said compound (or embodiments thereof disclosed herein); and/or a pharmaceutically acceptable salt thereof is released to a region of the gastrointestinal tract in which the pH is greater than pH 4.5. In one embodiment, the pH is greater than about 5. In another embodiment the pH is greater than from about 5 or about 5.5 to about 7. In one embodiment, the release is in one or more of the duodenum, jejunum or ileum. In one embodiment, said compound (or embodiments thereof disclosed herein); and/or a pharmaceutically acceptable salt thereof or the dosage form comprised of a said compound (or embodiments thereof disclosed herein); and/or a pharmaceutically acceptable salt thereof is coated with at least one coating chosen from an enteric coating or a non-enteric, time-delayed release coating. In another embodiment, said compound (or embodiments thereof disclosed herein); and/or a pharmaceutically acceptable salt thereof or the dosage form comprised of said compound (or embodiments thereof disclosed herein); and/or a pharmaceutically acceptable salt thereof is coated with at least one coating chosen from an enteric coating. In one embodiment, when said compound (or embodiments thereof disclosed herein) and/or a pharmaceutically acceptable salt thereof or the dosage form comprising a said compound (or embodiments thereof disclosed herein) and/or a pharmaceutically acceptable salt thereof is coated with an enteric coating, the enteric coating is a polymer. In another embodiment, when the compound and/or a pharmaceutically acceptable salt thereof or the dosage form comprising the compound and/or a pharmaceutically acceptable salt thereof is coated with an enteric coating, the enteric coating is an anionic polymer such as polymethacrylates (e.g., methacrylic acid ethacrylate poly, - methacrylic acid methyl methacrylate poly); cellulose-based polymers (e.g., cellulose acetate phthalate CAP, cellulose acetate trimellitate CAT, cellulose acetate succinate CAS, hydroxypropylmethyl-cellulose phthalate HPMCP, hydroxypropylmethylcellulose acetate succinate HPMCAS) or polyvinyl derivatives such as polyvinyl acetate phthalate PVAP.

In another embodiment, the release is in one or more of the duodenum, jejunum or ileum. In the method of increasing oral bioavailability, including embodiments therein, in yet one embodiment, the non-enteric delayed release dosage forms can be administered in fasted state and the time-delayed release coating can be designed to erode, burst, or become very permeable in about 0.3 to about 3 hours or in about 0.5 to about 2 hours to release the said compound (or embodiments thereof disclosed herein); and/or a pharmaceutically acceptable salt thereof. When the dosage form comprised of the said at least one compound is enteric-coated, it is generally administered in the fasted state to avoid variability in gastric emptying and the resulting variability in the initiation of efficacious plasma levels.

### Embodiment H

In the formulations disclosed herein, the compound of may be a compound having the structure (Id): 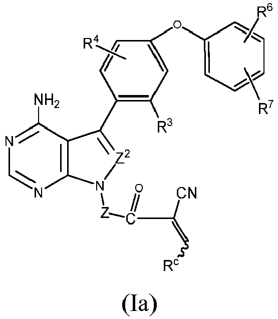 wherein:
Z² is -N- or CR² where R² is hydrogen or alkyl;
R³ and R⁴ are independently hydrogen, methyl, chloro, fluoro, cyclopropyl, hydroxy, methoxy, cyano, trifluoromethyl or trifluoromethoxy;
R⁶ and R⁷ are independently hydrogen, methyl, methoxy, fluoro, chloro, trifluoromethyl, trifluoromethoxy, or cyano;
Z is -(alkylene)-NR^{a}-, 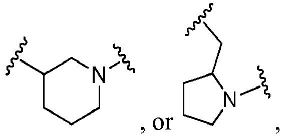 each ring optionally substituted with one or two substituents independently selected from alkyl, hydroxy, and halo, (for example alkyl or halo) where R^{a} is independently hydrogen or alkyl; and
R^{c} is alkyl, haloalkoxy, substituted alkyl, cycloalkyl, cycloalkyleneNR^{d}R^{e} or cycloalkylene(alkylene)NR^{d}R^{e} (where R^{d} and R^{e} are independently hydrogen, alkyl, or cycloalkyl), or 3 to 6 membered saturated monocyclic heterocyclyl containing one or two heteroatoms selected from N, O, and S and optionally substituted with one or two substituents independently selected from hydroxy, alkyl and fluoro.
   (a) Within embodiment (H), in one group of compounds R^{c} is alkyl, haloalkoxy, substituted alkyl, cycloalkyl, cycloalkyleneNR^{d}R^{e} where R^{d} and R^{e} are independently hydrogen, alkyl, or cycloalkyl or 3 to 6 membered saturated monocyclic heterocyclyl containing one or two heteroatoms selected from N, O, and S and optionally substituted with one or two substituents independently selected from hydroxy, alkyl and fluoro; and 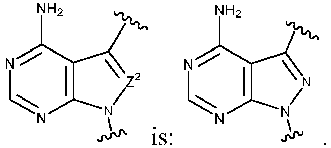
      (i) Within embodiment (H) and subpart (a) in one group of compounds 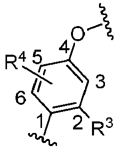 is a ring of formula: 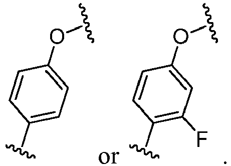
      (ii) Within embodiment (H) and subpart (a) in one group of compounds 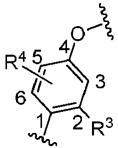 is a ring of formula: 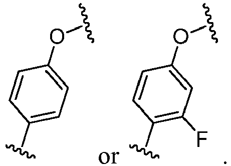 and 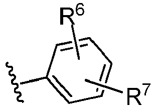 is a ring of formula: 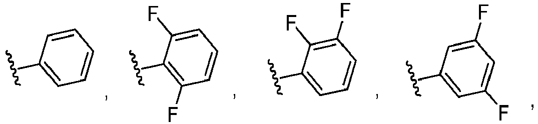 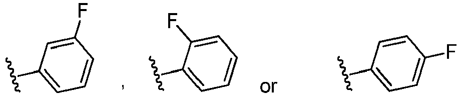
      (iii). Within embodiment (H), subparts (a), (i) and (ii) above, in one group of compounds, Z is 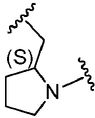 optionally substituted with one or two substituents independently selected from alkyl, hydroxy, and halo; In one group Z is 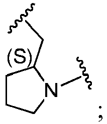 and R^{c} is alkyl, cycloalkyl, cycloalkyleneNR^{d}R^{e} (where R^{d} and R^{e} are independently hydrogen or alkyl), alkyl substituted with hydroxy, alkoxy, -NRR' (where R is alkyl, cycloalkyl, hydroxyalkyl, or alkoxyalkyl and R' is alkyl) or heterocycloamino which is optionally substituted with one or two groups independently selected from alkyl and hydroxyl, or 3 to 6 membered saturated monocyclic heterocyclyl containing one or two heteroatoms selected from N, O, and S and optionally substituted with one or two substituents independently selected from hydroxy, alkyl and fluoro. In one group, R^{c} is alkyl substituted with heterocycloamino which is optionally substituted with one or two groups independently selected from alkyl and hydroxyl, In another group, R^{c} is isopropyl, tert-butyl, -C(CH₃)₂OCH₂CH₃, -C(CH₃)₂N(CH₃)₂, -C(CH₃)₂morpholine-4-yl, cyclopropyl, 2-pyrrolidinyl, 3- or 4-piperidinyl, 1-methylpiperidin-4-yl, 1-methylpiperidin-3-yl, or 4-tetrahydropyranyl. For example, R^{c} is -C(CH₃)₂morpholine-4-yl, 2-pyrrolidinyl, 3- or 4-piperidinyl, 1-methylpiperidin-4-yl, 1-methylpiperidin-3-yl, or 4-tetrahydropyranyl.
      (iv). Within embodiment (H), subparts (a), (i) and (ii) above, in one group of compounds Z is 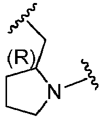 optionally substituted with one or two substituents independently selected from alkyl, hydroxy, and halo. In one group, Z is 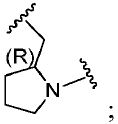 and R^{c} is alkyl substituted with -NRR' (where each R is hydrogen, alkyl, cycloalkyl, hydroxyalkyl, or alkoxyalkyl and R' is hydrogen) or heterocycloamino which is attached to alkyl via nitrogen ring atom and which is optionally substituted with one or two groups independently selected from alkyl and hydroxyl. In one group, R^{c} is -C(CH₃)₂NH₂, -C(CH₃)₂NHCH₃, -C(CH₃)₂NHCH₂CH₃, -C(CH₃)₂NHCH(CH₃)₂, -C(CH₃)₂NHcyclopropyl, -C(CH₃)₂NH(CH₂)₂OCH₃, or -C(CH₃)₂morpholine-4-yl.
      (v). Within embodiment (H), subparts (a), (b), (i) and (ii) above, in one group of compounds Z is 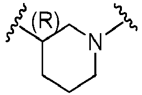 which is optionally substituted with one or two substituents independently selected from alkyl, hydroxy, and halo. In one group Z is 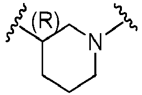 optionally substituted with alkyl or halo. In one group Z is 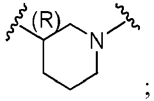 and
         R^{c} is alkyl, cycloalkyl, cycloalkyleneNR^{d}R^{e} (where R^{d} and R^{e} are independently hydrogen or alkyl), alkyl substituted with hydroxy, alkoxy, -NRR' (where each R is alkyl, cycloalkyl, hydroxyalkyl, or alkoxyalkyl and R' is hydrogen or alkyl) or heterocycloamino which is optionally substituted with one or two groups independently selected from alkyl and hydroxyl, or 3 to 6 membered saturated monocyclic heterocyclyl containing one or two heteroatoms selected from N, O, and S and optionally substituted with one or two substituents selected from hydroxy, alkyl and fluoro. In one group, R^{c} is isopropyl, tert-butyl,-C(CH₃)₂NH₂, -C(CH₃)₂NHCH₃, -C(CH₃)₂NHCH₂CH₃, -C(CH₃)₂NHCH(CH₃)₂,-C(CH₃)₂NHcyclopropyl, -C(CH₃)₂NH(CH₂)₂OCH₃, -C(CH₃)₂OCH₂CH₃,-C(CH₃)₂N(CH₃)₂, -C(CH₃)₂morpholine-4-yl, cyclopropyl, 2-pyrrolidinyl, 3- or 4-piperidinyl, 1-methylpiperidin-4-yl, 1-methylpiperidin-3-yl, or 4-tetrahydropyranyl. In another group, R^{c} is isopropyl, tert-butyl, or -C(CH₃)₂morpholine-4-yl.

### Embodiment L:

In yet another embodiment, in the formulations disclosed herein, the said least one compound or the compound is chosen from:
2-(3-(4-amino-3-(4-phenoxyphenyl)-1H-pyrazolo[3,4-d]pyrimidin-1-yl)piperidine-1-carbonyl)-3-cyclopropylacrylonitrile; **1**
2-(2-((4-amino-3-(4-phenoxyphenyl)-1H-pyrazolo[3,4-d]pyrimidin-1-yl)methyl)pyrrolidine-1-carbonyl)-3-cyclopropylacrylonitrile; **2**
2-(3-(4-amino-3-(4-(3,4-dichlorophenoxy)-3-methoxyphenyl)-1H-pyrazolo[3,4-d]pyrimidin-1-yl)piperidine-1-carbonyl)-3-cyclopropylacrylonitrile; 4
2-(2-((4-amino-3-(4-(3,4-dichlorophenoxy(3-methoxyphenyl)-1H-pyrazolo [3,4-d]pyrimidin-1-yl)methyl)pyrrolidine-1-carbonyl)-3-cyclopropylacrylonitrile; 5
2-(3-(4-amino-3-(4-phenoxyphenyl)-1H-pyrazolo[3,4-d]pyrimidin-1-yl)piperidine-1-carbonyl)-4,4-dimethylpent-2-enenitrile; 7
2-(2-((4-amino-3-(4-phenoxyphenyl)-1H-pyrazolo[3,4-d]pyrimidin-1-yl)methyl)pyrrolidine-1-carbonyl)-4,4-dimethylpent-2-enenitrile; 10
2-(2-((4-amino-3-(4-(3,4-dichlorophenoxy)-3-methoxyphenyl)-1H-pyrazolo[3,4-d]pyrimidin-1-yl)methyl)pyrrolidine-1-carbonyl)-4,4-dimethylpent-2-enenitrile; 12
2-(3-(4-amino-3-(4-(3,4-dichlorophenoxy)-3-methoxyphenyl)-1H-pyrazolo[3,4-d]pyrimidin-1-yl)piperidine-1-carbonyl)-4,4-dimethylpent-2-enenitrile; 19
(R)-2-(2-((4-amino-3-(4-phenoxyphenyl)-1H-pyrazolo[3,4-d]pyrimidin-1-yl)methyl)pyrrolidine-1-carbonyl)-3-cyclopropylacrylonitrile; 22
(R)-2-(2-((4-amino-3-(4-phenoxyphenyl)-1H-pyrazolo[3,4-d]pyrimidin-1-yl)methyl)pyrrolidine-1-carbonyl)-4,4-dimethylpent-2-enenitrile; 23A
(S)-2-(2-((4-amino-3-(4-phenoxyphenyl)-1H-pyrazolo[3,4-d]pyrimidin-1-yl)methyl)pyrrolidine-1-carbonyl)-4,4-dimethylpent-2-enenitrile; 23B
(R)-2-(2-((4-amino-3-(4-(3-fluorophenoxy)phenyl)-1H-pyrazolo[3,4-d]pyrimidin-1-yl)methyl)pyrrolidine-1-carbonyl)-3-cyclopropylacrylonitrile; 24A
(S)-2-(2-((4-amino-3-(4-(3-fluorophenoxy)phenyl)-1H-pyrazolo[3,4-d]pyrimidin-1-yl)methyl)pyrrolidine-1-carbonyl)-3-cyclopropylacrylonitrile; 24B
(R)-2-(2-((4-amino-3-(4-(3,5-difluorophenoxy)phenyl)-1H-pyrazolo[3,4-d]pyrimidin-1-yl)methyl)pyrrolidine-1-carbonyl)-3-cyclopropylacrylonitrile; 25A
(S)-2-(2-((4-amino-3-(4-(3,5-difluorophenoxy)phenyl)-1H-pyrazolo[3,4-d]pyrimidin-1-yl)methyl)pyrrolidine-1-carbonyl)-3-cyclopropylacrylonitrile; 25B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-cyclopropylacrylonitrile; 27A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-cyclopropylacrylonitrile; 27B
(R)-2-(3-(4-amino-3-(4-phenoxyphenyl)-1H-pyrazolo[3,4-d]pyrimidin-1-yl)piperidine-1-carbonyl)-4,4-dimethylpent-2-enenitrile; 29A
(S)-2-(3-(4-amino-3-(4-phenoxyphenyl)-1H-pyrazolo[3,4-d]pyrimidin-1-yl)piperidine-1-carbonyl)-4,4-dimethylpent-2-enenitrile; 29B
(R)-2-(2-((4-amino-3-(4-(2,3-difluorophenoxy)phenyl)-1H-pyrazolo[3,4-d]pyrimidin-1-yl)methyl)pyrrolidine-1-carbonyl)-3-cyclopropylacrylonitrile; 31A
(S)-2-(2-((4-amino-3-(4-(2,3-difluorophenoxy)phenyl)-1H-pyrazolo[3,4-d]pyrimidin-1-yl)methyl)pyrrolidine-1-carbonyl)-3-cyclopropylacrylonitrile; 31B
(R)-2-(2-((4-amino-3-(4-(2,6-difluorophenoxy)phenyl)-1H-pyrazolo[3,4-d]pyrimidin-1-yl)methyl)pyrrolidine-1-carbonyl)-3-cyclopropylacrylonitrile; 32A
(S)-2-(2-((4-amino-3-(4-(2,6-difluorophenoxy)phenyl)-1H-pyrazolo[3,4-d]pyrimidin-1-yl)methyl)pyrrolidine-1-carbonyl)-3-cyclopropylacrylonitrile; 32B
N-(2-(4-amino-3-(4-phenoxyphenyl)-1H-pyrazolo[3,4-d]pyrimidin-1-yl)ethyl)-2-cyano-3-cyclopropylacrylamide; 34
(R)-2-(2-((4-amino-3-(4-(2-fluorophenoxy)phenyl)-1H-pyrazolo[3,4-d]pyrimidin-1-yl)methyl)pyrrolidine-1-carbonyl)-3-cyclopropylacrylonitrile; 38A
(S)-2-(2-((4-amino-3-(4-(2-fluorophenoxy)phenyl)-1H-pyrazolo[3,4-d]pyrimidin-1-yl)methyl)pyrrolidine-1-carbonyl)-3-cyclopropylacrylonitrile; 38B
(R)-2-(2-((4-amino-3-(4-(2,5-difluorophenoxy)phenyl)-1H-pyrazolo[3,4-d]pyrimidin-1-yl)methyl)pyrrolidine-1-carbonyl)-3-cyclopropylacrylonitrile; 39A
(S)-2-(2-((4-amino-3-(4-(2,5-difluorophenoxy)phenyl)-1H-pyrazolo[3,4-d]pyrimidin-1-yl)methyl)pyrrolidine-1-carbonyl)-3-cyclopropylacrylonitrile; 39B
(R)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-3-cyclopropylacrylonitrile; 40A
(S)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-3-cyclopropylacrylonitrile; 40B
(R)-2-(2-((4-amino-3-(2-fluoro-4-(3,5-difluorophenoxy)phenyl)-1H-pyrazolo[3,4-d]pyrimidin-1-yl)methyl)pyrrolidine-1-carbonyl)-3-cyclopropylacrylonitrile; 41A
(S)-2-(2-((4-amino-3-(2-fluoro-4-(3,5-difluorophenoxy)phenyl)-1H-pyrazolo[3,4-d]pyrimidin-1-yl)methyl)pyrrolidine-1-carbonyl)-3-cyclopropylacrylonitrile; 41B
(R)-2-(2-((4-amino-3-(3-fluoro-4-(phenoxy)phenyl)-1H-pyrazolo[3,4-d]pyrimidin-1-yl)methyl)pyrrolidine-1-carbonyl)-3-cyclopropylacrylonitrile; 42A
(S)-2-(2-((4-amino-3-(3-fluoro-4-(phenoxy)phenyl)-1H-pyrazolo[3,4-d]pyrimidin-1-yl)methyl)pyrrolidine-1-carbonyl)-3-cyclopropylacrylonitrile; 42B
(R)-2-(2-((4-amino-3-(2-fluoro-4-(2,3-difluorophenoxy)phenyl)-1H-pyrazolo[3,4-d]pyrimidin-1-yl)methyl)pyrrolidine-1-carbonyl)-3-cyclopropylacrylonitrile; 43A
(S)-2-(2-((4-amino-3-(2-fluoro-4-(2,3-difluorophenoxy)phenyl)-1H-pyrazolo[3,4-d]pyrimidin-1-yl)methyl)pyrrolidine-1-carbonyl)-3-cyclopropylacrylonitrile; 43B
(R)-2-(2-((4-amino-3-(2-fluoro-4-(2,6-difluorophenoxy)phenyl)-1H-pyrazolo[3,4-d]pyrimidin-1-yl)methyl)pyrrolidine-1-carbonyl)-3-cyclopropylacrylonitrile; 44A
(S)-2-(2-((4-amino-3-(2-fluoro-4-(2,6-difluorophenoxy)phenyl)-1H-pyrazolo[3,4-d]pyrimidin-1-yl)methyl)pyrrolidine-1-carbonyl)-3-cyclopropylacrylonitrile; 44B
N-(2-(4-amino-3-(2-fluoro-4-phenoxyphenyl)-1H-pyrazolo[3,4-d]pyrimidin-1-yl)ethyl)-2-cyano-3-cyclopropylacrylamide; 45
N-(2-(4-amino-3-(2-fluoro-4-phenoxyphenyl)-1H-pyrazolo[3,4-d]pyrimidin-1-yl)-2-methylpropyl)-2-cyano-3-cyclopropylacrylamide; 46
N-(1-(4-amino-3-(2-fluoro-4-phenoxyphenyl)-1H-pyrazolo[3,4-d]pyrimidin-1-yl)-2-methylpropan-2-yl)-2-cyano-3-cyclopropylacrylamide; 47
N-(2-(4-amino-3-(2-fluoro-4-phenoxyphenyl)-1H-pyrazolo[3,4-d]pyrimidin-1-yl)ethyl)-1-cyano-2-cyclopropylethenesulfonamide; 48
N-(2-(4-amino-3-(2-fluoro-4-phenoxyphenyl)-1H-pyrazolo[3,4-d]pyrimidin-1-yl)ethyl)-1-cyano-2-cyclopropyl-N-methylethenesulfonamide; 49
2-(4-amino-3-(2-fluoro-4-phenoxyphenyl)-1H-pyrazolo[3,4-d]pyrimidin-1-yl)ethyl 2-cyano-3-cyclopropylacrylate; 50
1-(4-amino-3-(2-fluoro-4-phenoxyphenyl)-1H-pyrazolo[3,4-d]pyrimidin-1-yl)-2-methylpropan-2-yl 2-cyano-3-cyclopropylacrylate; 51
2-((2-(4-amino-3-(2-fluoro-4-phenoxyphenyl)-1H-pyrazolo[3,4-d]pyrimidin-1-yl)ethyl)sulfonyl)-3-cyclopropylacrylonitrile; 52
2-(5-((4-amino-3-(2-fluoro-4-phenoxyphenyl)-1H-pyrazolo[3,4-d]pyrimidin-1-yl)methyl)oxazol-2-yl)-3-cyclopropylacrylonitrile; 53
(R)-2-(3-(4-amino-5-(4-phenoxyphenyl)-7H-pyrrolo[2,3-d]pyrimidin-7-yl)piperidine-1-carbonyl)-3-cyclopropylacrylonitrile; 54A
(S)-2-(3-(4-amino-5-(4-phenoxyphenyl)-7H-pyrrolo[2,3-d]pyrimidin-7-yl)piperidine-1-carbonyl)-3-cyclopropylacrylonitrile; 54B
(R)-2-(3-(4-amino-5-(4-(3,5-difluorophenoxy)phenyl)-7H-pyrrolo[2,3-d]pyrimidin-7-yl)piperidine-1-carbonyl)-3-cyclopropylacrylonitrile; 55A
(S)-2-(3-(4-amino-5-(4-(3,5-difluorophenoxy)phenyl)-7H-pyrrolo[2,3-d]pyrimidin-7-yl)piperidine-1-carbonyl)-3-cyclopropylacrylonitrile; 55B
(R)-2-(3-(4-amino-5-(2-fluoro-4-phenoxyphenyl)-7H-pyrrolo[2,3-d]pyrimidin-7-yl)piperidine-1-carbonyl)-3-cyclopropylacrylonitrile; 56A
(S)-2-(3-(4-amino-5-(2-fluoro-4-phenoxyphenyl)-7H-pyrrolo[2,3-d]pyrimidin-7-yl)piperidine-1-carbonyl)-3-cyclopropylacrylonitrile; 56B
(R)-2-(2-((4-amino-5-(4-phenoxyphenyl)-7H-pyrrolo[2,3-d]pyrimidin-7-yl)methyl)pyrrolidine-1-carbonyl)-3-cyclopropylacrylonitrile; 57A
(S)-2-(2-((4-amino-5-(4-phenoxyphenyl)-7H-pyrrolo[2,3-d]pyrimidin-7-yl)methyl)pyrrolidine-1-carbonyl)-3-cyclopropylacrylonitrile; 57B
(R)-2-(2-((4-amino-5-(4-(3,5-difluorophenoxy)phenyl)-7H-pyrrolo[2,3-d]pyrimidin-7-yl)methyl)pyrrolidine-1-carbonyl)-3-cyclopropylacrylonitrile; 58A
(S)-2-(2-((4-amino-5-(4-(3,5-difluorophenoxy)phenyl)-7H-pyrrolo[2,3-d]pyrimidin-7-yl)methyl)pyrrolidine-1-carbonyl)-3-cyclopropylacrylonitrile; 58B
(R)-2-(2-((4-amino-5-(2-fluoro-4-phenoxyphenyl)-7H-pyrrolo[2,3-d]pyrimidin-7-yl)methyl)pyrrolidine-1-carbonyl)-3-cyclopropylacrylonitrile; 59A
(S)-2-(2-((4-amino-5-(2-fluoro-4-phenoxyphenyl)-7H-pyrrolo[2,3-d]pyrimidin-7-yl)methyl)pyrrolidine-1-carbonyl)-3-cyclopropylacrylonitrile; 59B
(R)-2-(3-(4-amino-6-methyl-5-(4-phenoxyphenyl)-7H-pyrrolo[2,3-d]pyrimidin-7-yl)piperidine-1-carbonyl)-3-cyclopropylacrylonitrile; 60A
(S)-2-(3-(4-amino-6-methyl-5-(4-phenoxyphenyl)-7H-pyrrolo[2,3-d]pyrimidin-7-yl)piperidine-1-carbonyl)-3-cyclopropylacrylonitrile; 60B
(R)-2-(3-(4-amino-5-(4-(3,5-difluorophenoxy)phenyl)-6-methyl-7H-pyrrolo[2,3-d]pyrimidin-7-yl)piperidine-1-carbonyl)-3-cyclopropylacrylonitrile; 61A
(S)-2-(3-(4-amino-5-(4-(3,5-difluorophenoxy)phenyl)-6-methyl-7H-pyrrolo[2,3-d]pyrimidin-7-yl)piperidine-1-carbonyl)-3-cyclopropylacrylonitrile; 61B
2-((3R)-3-(4-amino-5-(2-fluoro-4-phenoxyphenyl)-6-methyl-7H-pyrrolo[2,3-d]pyrimidin-7-yl)piperidine-1-carbonyl)-3-cyclopropylacrylonitrile; 62A
2-((3S)-3-(4-amino-5-(2-fluoro-4-phenoxyphenyl)-6-methyl-7H-pyrrolo[2,3-d]pyrimidin-7-yl)piperidine-1-carbonyl)-3-cyclopropylacrylonitrile; 62B
(R)-(2-(2-((4-amino-6-methyl-5-(4-phenoxyphenyl)-7H-pyrrolo[2,3-d]pyrimidin-7-yl)methyl)pyrrolidine-1-carbonyl)-3-cyclopropylacrylonitrile; 63A
(S)- (2-(2-((4-amino-6-methyl-5-(4-phenoxyphenyl)-7H-pyrrolo[2,3-d]pyrimidin-7-yl)methyl)pyrrolidine-1-carbonyl)-3-cyclopropylacrylonitrile; 63B
(R)-2-(2-((4-amino-5-(4-(3,5-difluorophenoxy)phenyl)-6-methyl-7H-pyrrolo[2,3-d]pyrimidin-7-yl)methyl)pyrrolidine-1-carbonyl)-3-cyclopropylacrylonitrile; 64A
(S)-2-(2-((4-amino-5-(4-(3,5-difluorophenoxy)phenyl)-6-methyl-7H-pyrrolo[2,3-d]pyrimidin-7-yl)methyl)pyrrolidine-1-carbonyl)-3-cyclopropylacrylonitrile; 64B
(R)-2-(2-((4-amino-5-(2-fluoro-4-phenoxyphenyl)-6-methyl-7H-pyrrolo[2,3-d]pyrimidin-7-yl)methyl)pyrrolidine-1-carbonyl)-3-cyclopropylacrylonitrile; 65A
(S)-2-(2-((4-amino-5-(2-fluoro-4-phenoxyphenyl)-6-methyl-7H-pyrrolo[2,3-d]pyrimidin-7-yl)methyl)pyrrolidine-1-carbonyl)-3-cyclopropylacrylonitrile; 65B
N-(3-(4-amino-3-(4-phenoxyphenyl)-1H-pyrazolo[3,4-d]pyrimidin-1-yl)-2,2-dimethylpropyl)-2-cyano-3-cyclopropylacrylamide; 67
(R)-2-(2-((4-amino-3-(2-methyl-4-phenoxyphenyl)-1H-pyrazolo[3,4-d]pyrimidin-1-yl)methyl)pyrrolidine-1-carbonyl)-3-cyclopropylacrylonitrile; 69A
(S)-2-(2-((4-amino-3-(2-methyl-4-phenoxyphenyl)-1H-pyrazolo[3,4-d]pyrimidin-1-yl)methyl)pyrrolidine-1-carbonyl)-3-cyclopropylacrylonitrile; 69B
(R)-2-(2-((4-amino-3-(2-chloro-4-phenoxyphenyl)-1H-pyrazolo[3,4-d]pyrimidin-1-yl)methyl)pyrrolidine-1-carbonyl)-3-cyclopropylacrylonitrile; 70A
(S)-2-(2-((4-amino-3-(2-chloro-4-phenoxyphenyl)-1H-pyrazolo[3,4-d]pyrimidin-1-yl)methyl)pyrrolidine-1-carbonyl)-3-cyclopropylacrylonitrile; 70B
(R)-2-(2-((4-amino-3-(4-(2,5-difluorophenoxy)phenyl)-1H-pyrazolo[3,4-d]pyrimidin-1-yl)methyl)pyrrolidine-1-carbonyl)-4-methylpent-2-enenitrile; 71A
(S)-2-(2-((4-amino-3-(4-(2,5-difluorophenoxy)phenyl)-1H-pyrazolo[3,4-d]pyrimidin-1-yl)methyl)pyrrolidine-1-carbonyl)-4-methylpent-2-enenitrile; 71B
(R)-4-amino-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile; 72A
(S)-4-amino-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile; 72B
(R)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile; 73A
(S)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile; 73B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4,4-dimethylpent-2-enenitrile; 74A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4,4-dimethylpent-2-enenitrile; 74B
(R)-2-(2-((4-amino-3-(4-(2,6-difluorophenoxy)phenyl)-1H-pyrazolo[3,4-d]pyrimidin-1-yl)methyl)pyrrolidine-1-carbonyl)-4-methylpent-2-enenitrile; 75A
(S)-2-(2-((4-amino-3-(4-(2,6-difluorophenoxy)phenyl)-1H-pyrazolo[3,4-d]pyrimidin-1-yl)methyl)pyrrolidine-1-carbonyl)-4-methylpent-2-enenitrile; 75B
(R)-2-(2-((4-amino-3-(4-(2,3-difluorophenoxy)phenyl)-1H-pyrazolo[3,4-d]pyrimidin-1-yl)methyl)pyrrolidine-1-carbonyl)-4-methylpent-2-enenitrile; 76A
(S)-2-(2-((4-amino-3-(4-(2,3-difluorophenoxy)phenyl)-1H-pyrazolo[3,4-d]pyrimidin-1-yl)methyl)pyrrolidine-1-carbonyl)-4-methylpent-2-enenitrile; 76B
(R)-2-(2-((4-amino-6-methyl-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile; 77A
(S)-2-(2-((4-amino-6-methyl-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile; 77B
(R)-2-(2-((4-amino-3-(4-(2,6-difluorophenoxy)-2-fluorophenyl)-1H-pyrazolo[3,4-d]pyrimidin-1-yl)methyl)pyrrolidine-1-carbonyl)-4-methylpent-2-enenitrile; 78A
(S)-2-(2-((4-amino-3-(4-(2,6-difluorophenoxy)-2-fluorophenyl)-1H-pyrazolo[3,4-d]pyrimidin-1-yl)methyl)pyrrolidine-1-carbonyl)-4-methylpent-2-enenitrile; 78B
(R)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-3-cyclopropylacrylonitrile; 79A
(S)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-3-cyclopropylacrylonitrile; 79B
(R)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-4-methylpent-2-enenitrile; 80A
(S)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-4-methylpent-2-enenitrile; 80B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile; 81A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile; 81B
(S)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile; 82B
(R)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile; 82A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(methylamino)pent-2-enenitrile; 83B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(methylamino)pent-2-enenitrile; 84A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(dimethylamino)-4-methylpent-2-enenitrile; 84B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(dimethylamino)-4-methylpent-2-enenitrile; 84A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(ethylamino)-4-methylpent-2-enenitrile; 85B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(ethylamino)-4-methylpent-2-enenitrile; 85A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(isopropylamino)-4-methylpent-2-enenitrile; 86B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(isopropylamino)-4-methylpent-2-enenitrile; 86A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(cyclopropylamino)-4-methylpent-2-enenitrile; 87B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(cyclopropylamino)-4-methylpent-2-enenitrile; 87A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-((2-methoxyethyl)amino)-4-methylpent-2-enenitrile; 88B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-((2-methoxyethyl)amino)-4-methylpent-2-enenitrile; 88A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-ethoxy-4-methylpent-2-enenitrile; 89B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-ethoxy-4-methylpent-2-enenitrile; 89A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-aminocyclopropyl)acrylonitrile; 90B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-aminocyclopropyl)acrylonitrile; 90A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(methylamino)cyclopropyl)acrylonitrile; 91B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(methylamino)cyclopropyl)acrylonitrile; 91A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(ethylamino)cyclopropyl)acrylonitrile; 92B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(ethylamino)cyclopropyl)acrylonitrile; 92A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(isopropylamino)cyclopropyl)acrylonitrile; 93B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(isopropylamino)cyclopropyl)acrylonitrile; 93A
2-((S)-2-((4-amino-3-(2-fluoro-4-phenoxyphenyl)-1H-pyrazolo[3,4-d]pyrimidin-1-yl)methyl)pyrrolidine-1-carbonyl)-3-(pyrrolidin-2-yl)acrylonitrile; 94B
2-((R)-2-((4-amino-3-(2-fluoro-4-phenoxyphenyl)-1H-pyrazolo[3,4-d]pyrimidin-1-yl)methyl)pyrrolidine-1-carbonyl)-3-(pyrrolidin-2-yl)acrylonitrile; 94A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile; 95B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile; 95A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-((dimethylamino)methyl)cyclopentyl)acrylonitrile; 97B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-((dimethylamino)methyl)cyclopentyl)acrylonitrile; 97A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-methylpiperidin-4-yl)acrylonitrile; 98B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-methylpiperidin-4-yl)acrylonitrile; 98A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(tetrahydro-2H-pyran-4-yl)acrylonitrile; 99B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(tetrahydro-2H-pyran-4-yl)acrylonitrile; 99A
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(piperidin-4-yl)acrylonitrile; 100B
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(piperidin-4-yl)acrylonitrile; 100A
2-((S)-2-((4-amino-3-(2-fluoro-4-phenoxyphenyl)-1H-pyrazolo[3,4-d]pyrimidin-1-yl)methyl)pyrrolidine-1-carbonyl)-3-(piperidin-3-yl)acrylonitrile; 101B
2-((R)-2-((4-amino-3-(2-fluoro-4-phenoxyphenyl)-1H-pyrazolo[3,4-d]pyrimidin-1-yl)methyl)pyrrolidine-1-carbonyl)-3-(piperidin-3-yl)acrylonitrile; 101A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile; 102B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile; 102A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4,4-dimethylpent-2-enenitrile; 103B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4,4-dimethylpent-2-enenitrile; 103A
(S)-4-amino-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile; 104B
(R)-4-amino-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile; 104A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-(methylamino)pent-2-enenitrile; 105B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-(methylamino)pent-2-enenitrile; 105A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(dimethylamino)-4-methylpent-2-enenitrile; 106B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(dimethylamino)-4-methylpent-2-enenitrile; 106A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(ethylamino)-4-methylpent-2-enenitrile; 107B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(ethylamino)-4-methylpent-2-enenitrile; 107A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(isopropylamino)-4-methylpent-2-enenitrile; 108B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(isopropylamino)-4-methylpent-2-enenitrile; 108A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(cyclopropylamino)-4-methylpent-2-enenitrile; 109B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(cyclopropylamino)-4-methylpent-2-enenitrile;109A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-((2-methoxyethyl)amino)-4-methylpent-2-enenitrile; 110B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-((2-methoxyethyl)amino)-4-methylpent-2-enenitrile; 110A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-ethoxy-4-methylpent-2-enenitrile; 111B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-ethoxy-4-methylpent-2-enenitrile; 111A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-aminocyclopropyl)acrylonitrile; 112B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-aminocyclopropyl)acrylonitrile; 112A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(methylamino)cyclopropyl)-acrylonitrile; 113B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(methylamino)cyclopropyl)-acrylonitrile; 113A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(ethylamino)cyclopropyl)acrylonitrile; 114B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(ethylamino)cyclopropyl)acrylonitrile; 114A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(isopropylamino)cyclopropyl)-acrylonitrile; 115B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(isopropylamino)cyclopropyl)-acrylonitrile; 115A
2-((S)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(pyrrolidin-2-yl)acrylonitrile; 116B
2-((R)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(pyrrolidin-2-yl)acrylonitrile; 116A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-morpholinopent-2-enenitrile; 117B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-morpholinopent-2-enenitrile; 117A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((diethylamino)methyl)cyclopentyl)-acrylonitrile; 118B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((diethylamino)methyl)cyclopentyl)-acrylonitrile; 118A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((dimethylamino)methyl)-cyclopentyl)acrylonitrile; 119B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((dimethylamino)-methyl)-cyclopentyl)acrylonitrile; 119A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-methylpiperidin-4-yl)acrylonitrile; 120B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-methylpiperidin-4-yl)acrylonitrile; 120A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(tetrahydro-2H-pyran-4-yl)acrylonitrile; 121B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(tetrahydro-2H-pyran-4-yl)acrylonitrile; 121A
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-4-yl)acrylonitrile; 122B
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-4-yl)acrylonitrile; 122A
2-((S)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-3-yl)acrylonitrile; 123B
2-((R)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-3-yl)acrylonitrile; 123A
(R)-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile; 124A
(S)-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile; 124B
(R)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile; 125A
(S)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile; 125B
(R)-4-amino-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile; 126A
(S)-4-amino-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile; 126B
(R)-2-(3-(4-amino-3-(2-fluoro-4-phenoxyphenyl)-1H-pyrazolo[3,4-d]pyrimidin-1-yl)piperidine-1-carbonyl)-4-methyl-4-(methylamino)pent-2-enenitrile; 127A
(S)-2-(3-(4-amino-3-(2-fluoro-4-phenoxyphenyl)-1H-pyrazolo[3,4-d]pyrimidin-1-yl)piperidine-1-carbonyl)-4-methyl-4-(methylamino)pent-2-enenitrile; 127B
(R)-2-(3-(4-amino-3-(2-fluoro-4-phenoxyphenyl)--1H-pyrazolo[3,4-d]pyrimidin-1-yl)piperidine-1-carbonyl)-4-(dimethylamino)-4-methylpent-2-enenitrile; 128A
(S)-2-(3-(4-amino-3-(2-fluoro-4-phenoxyphenyl)--1H-pyrazolo[3,4-d]pyrimidin-1-yl)piperidine-1-carbonyl)-4-(dimethylamino)-4-methylpent-2-enenitrile; 128B
(R)-2-(3-(4-amino-3-(2-fluoro-4-phenoxyphenyl)-1H-pyrazolo[3,4-d]pyrimidin-1-yl)piperidine-1-carbonyl)-4-(ethylamino)-4-methylpent-2-enenitrile; 129A
(S)-2-(3-(4-amino-3-(2-fluoro-4-phenoxyphenyl)-1H-pyrazolo[3,4-d]pyrimidin-1-yl)piperidine-1-carbonyl)-4-(ethylamino)-4-methylpent-2-enenitrile; 129B
(R)-2-(3-(4-amino-3-(2-fluoro-4-phenoxyphenyl)-1H-pyrazolo[3,4-d]pyrimidin-1-yl)piperidine-1-carbonyl)-4-(isopropylamino)-4-methylpent-2-enenitrile; 130A
(S)-2-(3-(4-amino-3-(2-fluoro-4-phenoxyphenyl)-1H-pyrazolo[3,4-d]pyrimidin-1-yl)piperidine-1-carbonyl)-4-(isopropylamino)-4-methylpent-2-enenitrile; 130B
(R)-2-(3-(4-amino-3-(2-fluoro-4-phenoxyphenyl)-1H-pyrazolo[3,4-d]pyrimidin-1-yl)piperidine-1-carbonyl)-4-(cyclopropylamino)-4-methylpent-2-enenitrile; 131A
(S)-2-(3-(4-amino-3-(2-fluoro-4-phenoxyphenyl)-1H-pyrazolo[3,4-d]pyrimidin-1-yl)piperidine-1-carbonyl)-4-(cyclopropylamino)-4-methylpent-2-enenitrile; 131B
(R)-2-(3-(4-amino-3-(2-fluoro-4-phenoxyphenyl)-1H-pyrazolo[3,4-d]pyrimidin-1-yl)piperidine-1-carbonyl)-4-((2-methoxyethyl)amino)-4-methylpent-2-enenitrile; 132A
(S)-2-(3-(4-amino-3-(2-fluoro-4-phenoxyphenyl)-1H-pyrazolo[3,4-d]pyrimidin-1-yl)piperidine-1-carbonyl)-4-((2-methoxyethyl)amino)-4-methylpent-2-enenitrile; 132B
(R)-2-(3-(4-amino-3-(2-fluoro-4-phenoxyphenyl)-1H-pyrazolo[3,4-d]pyrimidin-1-yl)piperidine-1-carbonyl)-4-ethoxy-4-methylpent-2-enenitrile; 133A
(S)-2-(3-(4-amino-3-(2-fluoro-4-phenoxyphenyl)-1H-pyrazolo[3,4-d]pyrimidin-1-yl)piperidine-1-carbonyl)-4-ethoxy-4-methylpent-2-enenitrile; 133B
(R)-2-(3-(4-amino-3-(2-fluoro-4-phenoxyphenyl)-1H-pyrazolo[3,4-d]pyrimidin-1-yl)piperidine-1-carbonyl)-3-(1-aminocyclopropyl)acrylonitrile; 134A
(S)-2-(3-(4-amino-3-(2-fluoro-4-phenoxyphenyl)-1H-pyrazolo[3,4-d]pyrimidin-1-yl)piperidine-1-carbonyl)-3-(1-aminocyclopropyl)acrylonitrile; 134B
(R)-2-(3-(4-amino-3-(2-fluoro-4-phenoxyphenyl)-1H-pyrazolo[3,4-d]pyrimidin-1-yl)piperidine-1-carbonyl)-3-(1-(methylamino)cyclopropyl)acrylonitrile; 135A
(S)-2-(3-(4-amino-3-(2-fluoro-4-phenoxyphenyl)-1H-pyrazolo[3,4-d]pyrimidin-1-yl)piperidine-1-carbonyl)-3-(1-(methylamino)cyclopropyl)acrylonitrile; 135B
(R)-2-(3-(4-amino-3-(2-fluoro-4-phenoxyphenyl)-1H-pyrazolo[3,4-d]pyrimidin-1-yl)piperidine-1-carbonyl)-3-(1-(ethylamino)cyclopropyl)acrylonitrile; 136A
(S)-2-(3-(4-amino-3-(2-fluoro-4-phenoxyphenyl)-1H-pyrazolo[3,4-d]pyrimidin-1-yl)piperidine-1-carbonyl)-3-(1-(ethylamino)cyclopropyl)acrylonitrile; 136B
(R)-2-(3-(4-amino-3-(2-fluoro-4-phenoxyphenyl)-1H-pyrazolo[3,4-d]pyrimidin-1-yl)piperidine-1-carbonyl)-3-(1-(isopropylamino)cyclopropyl)acrylonitrile; 137A
(S)-2-(3-(4-amino-3-(2-fluoro-4-phenoxyphenyl)-1H-pyrazolo[3,4-d]pyrimidin-1-yl)piperidine-1-carbonyl)-3-(1-(isopropylamino)cyclopropyl)acrylonitrile; 137B
2-((R)-3-(4-amino-3-(2-fluoro-4-phenoxyphenyl)-1H-pyrazolo[3,4-d]pyrimidin-1-yl)piperidine-1-carbonyl)-3-(pyrrolidin-2-yl)acrylonitrile; 138A
2-((S)-3-(4-amino-3-(2-fluoro-4-phenoxyphenyl)-1H-pyrazolo[3,4-d]pyrimidin-1-yl)piperidine-1-carbonyl)-3-(pyrrolidin-2-yl)acrylonitrile; 138B
(S)-2-(3-(4-amino-3-(2-fluoro-4-phenoxyphenyl)-1H-pyrazolo[3,4-d]pyrimidin-1-yl)piperidine-1-carbonyl)-4-methyl-4-morpholinopent-2-enenitrile; 139B
(R)-2-(3-(4-amino-3-(2-fluoro-4-phenoxyphenyl)-1H-pyrazolo[3,4-d]pyrimidin-1-yl)piperidine-1-carbonyl)-4-methyl-4-morpholinopent-2-enenitrile; 139A
(R)-2-(3-(4-amino-3-(2-fluoro-4-phenoxyphenyl)-1H-pyrazolo[3,4-d]pyrimidin-1-yl)piperidine-1-carbonyl)-3-(1-((diethylamino)methyl)-cyclopentyl)acrylonitrile; 140A
(S)-2-(3-(4-amino-3-(2-fluoro-4-phenoxyphenyl)-1H-pyrazolo[3,4-d]pyrimidin-1-yl)piperidine-1-carbonyl)-3-(1-((diethylamino)methyl)-cyclopentyl)acrylonitrile; 140B
(R)-2-(3-(4-amino-3-(2-fluoro-4-phenoxyphenyl)-1H-pyrazolo[3,4-d]pyrimidin-1-yl)piperidine-1-carbonyl)-3-(1-((dimethylamino)methyl)cyclopentyl)-acrylonitrile; 141A
(S)-2-(3-(4-amino-3-(2-fluoro-4-phenoxyphenyl)-1H-pyrazolo[3,4-d]pyrimidin-1-yl)piperidine-1-carbonyl)-3-(1-((dimethylamino)methyl)cyclopentyl)-acrylonitrile; 141B
(R)-2-(3-(4-amino-3-(2-fluoro-4-phenoxyphenyl)-1H-pyrazolo[3,4-d]pyrimidin-1-yl)piperidine-1-carbonyl)-3-(1-methylpiperidin-4-yl)acrylonitrile; 142A
(S)-2-(3-(4-amino-3-(2-fluoro-4-phenoxyphenyl)-1H-pyrazolo[3,4-d]pyrimidin-1-yl)piperidine-1-carbonyl)-3-(1-methylpiperidin-4-yl)acrylonitrile; 142B
(R)-2-(3-(4-amino-3-(2-fluoro-4-phenoxyphenyl)-1H-pyrazolo[3,4-d]pyrimidin-1-yl)piperidine-1-carbonyl)-3-(tetrahydro-2H-pyran-4-yl)acrylonitrile; 143A
(S)-2-(3-(4-amino-3-(2-fluoro-4-phenoxyphenyl)-1H-pyrazolo[3,4-d]pyrimidin-1-yl)piperidine-1-carbonyl)-3-(tetrahydro-2H-pyran-4-yl)acrylonitrile; 143B
(R)-2-(3-(4-amino-3-(2-fluoro-4-phenoxyphenyl)-1H-pyrazolo[3,4-d]pyrimidin-1-yl)piperidine-1-carbonyl)-3-(piperidin-4-yl)acrylonitrile; 144A
(S)-2-(3-(4-amino-3-(2-fluoro-4-phenoxyphenyl)-1H-pyrazolo[3,4-d]pyrimidin-1-yl)piperidine-1-carbonyl)-3-(piperidin-4-yl)acrylonitrile; 144B
2-((R)-3-(4-amino-3-(2-fluoro-4-phenoxyphenyl)-1H-pyrazolo[3,4-d]pyrimidin-1-yl)piperidine-1-carbonyl)-3-(piperidin-3-yl)acrylonitrile; 145A
2-((S)-3-(4-amino-3-(2-fluoro-4-phenoxyphenyl)-1H-pyrazolo[3,4-d]pyrimidin-1-yl)piperidine-1-carbonyl)-3-(piperidin-3-yl)acrylonitrile; 145B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-cyclopropylacrylonitrile; 146A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-cyclopropylacrylonitrile; 146B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile; 147A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile; 147B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4,4-dimethylpent-2-enenitrile; 148A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4,4-dimethylpent-2-enenitrile; 148B
(R)-4-amino-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile; 149A
(S)-4-amino-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile; 149B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-(methylamino)pent-2-enenitrile; 150A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-(methylamino)pent-2-enenitrile; 150B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(dimethylamino)-4-methylpent-2-enenitrile; 151A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(dimethylamino)-4-methylpent-2-enenitrile; 151B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(ethylamino)-4-methylpent-2-enenitrile; 152A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(ethylamino)-4-methylpent-2-enenitrile; 152B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(isopropylamino)-4-methylpent-2-enenitrile; 153A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(isopropylamino)-4-methylpent-2-enenitrile; 153B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(cyclopropylamino)-4-methylpent-2-enenitrile; 154A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(cyclopropylamino)-4-methylpent-2-enenitrile; 154B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-((2-methoxyethyl)amino)-4-methylpent-2-enenitrile; 155A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-((2-methoxyethyl)amino)-4-methylpent-2-enenitrile; 155B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-ethoxy-4-methylpent-2-enenitrile; 156A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-ethoxy-4-methylpent-2-enenitrile; 156B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-aminocyclopropyl)-acrylonitrile; 157A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-ammocyclopropyl)-acrylonitrile; 157B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(methylamino)cyclopropyl)-acrylonitrile; 158A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(methylamino)cyclopropyl)-acrylonitrile; 158B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(ethylamino)cyclopropyl)-acrylonitrile; 159A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(ethylamino)cyclopropyl)-acrylonitrile; 159B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3 -(1-(isopropylamino)-cyclopropyl)acrylonitrile; 160A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(isopropylamino)-cyclopropyl)acrylonitrile; 160B
2-((R)-3-(4-amino-3-(4-(2,3-difluorophenoxy)-2-fluorophenyl)-1H-pyrazolo[3,4-d]pyrimidin-1-yl)piperidine-1-carbonyl)-3-(pyrrolidin-2-yl)acrylonitrile; 161A
2-((S)-3-(4-amino-3-(4-(2,3-difluorophenoxy)-2-fluorophenyl)-1H-pyrazolo[3,4-d]pyrimidin-1-yl)piperidine-1-carbonyl)-3-(pyrrolidin-2-yl)acrylonitrile; 161B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-morpholinopent-2-enenitrile; 162A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-morpholinopent-2-enenitrile; 162B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((diethylamino)methyl)-cyclopentyl)-acrylonitrile; 163A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((diethylamino)methyl)-cyclopentyl)-acrylonitrile; 163B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((dimethylamino)-methyl)cyclopentyl)-acrylonitrile; 164A
(S)-2-(3-(4-amino-3-( 4-(2,3-difluorophenoxy)-2-fluorophenyl)-1H-pyrazolo[3,4-d]pyrimidin-1-yl)piperidine-1-carbonyl)-3-(1-((dimethylamino)-methyl)cyclopentyl)-acrylonitrile; 164B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-methylpiperidin-4-yl)acrylonitrile; 165A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-methylpiperidin-4-yl)acrylonitrile; 165B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(tetrahydro-2H-pyran-4-yl)acrylonitrile; 166A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(tetrahydro-2H-pyran-4-yl)acrylonitrile; 166B
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(piperidin-4-yl)acrylonitrile; 167A
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(piperidin-4-yl)acrylonitrile; 167B
2-((R)-3-(4-amino-3-(4-(2,3-difluorophenoxy)-2-fluorophenyl)-1H-pyrazolo[3,4-d]pyrimidin-1-yl)piperidine-1-carbonyl)-3-(piperidin-3-yl)acrylonitrile; 168A
2-((S)-3-(4-amino-3-(4-(2,3-difluorophenoxy)-2-fluorophenyl)-1H-pyrazolo[3,4-d]pyrimidin-1-yl)piperidine-1-carbonyl)-3-(piperidin-3-yl)acrylonitrile; 168B
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile; 169A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile; 169B
(R)-2-(2-((4-amino-5-(2-fluoro-4-phenoxyphenyl)-7H-pyrrolo[2,3-d]pyrimidin-7-yl)methyl)pyrrolidine-1-carbonyl)-4-(dimethylamino)-4-methylpent-2-enenitrile; 170A
(S)-2-(2-((4-amino-5-(2-fluoro-4-phenoxyphenyl)-7H-pyrrolo[2,3-d]pyrimidin-7-yl)methyl)pyrrolidine-1-carbonyl)-4-(dimethylamino)-4-methylpent-2-enenitrile; 170B
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-3-cyclopropylacrylonitrile; 171A
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-3-cyclopropylacrylonitrile; 171B
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-4-methylpent-2-enenitrile; 172A
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-4-methylpent-2-enenitrile; 172B
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-4,4-dimethylpent-2-enenitrile; 173A
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-4,4-dimethylpent-2-enenitrile; 173B
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-4-(dimethylamino)-4-methylpent-2-enenitrile; 174A
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-4-(dimethylamino)-4-methylpent-2-enenitrile; 174B
(R)-2-(2-((4-amino-5-(4-phenoxyphenyl)-7H-pyrrolo[2,3-d]pyrimidin-7-yl)methyl)-pyrrolidine-1-carbonyl)-4-(dimethylamino)-4-methylpent-2-enenitrile; 175A
(S)-2-(2-((4-amino-5-(4-phenoxyphenyl)-7H-pyrrolo[2,3-d]pyrimidin-7-yl)methyl)-pyrrolidine-1-carbonyl)-4-(dimethylamino)-4-methylpent-2-enenitrile; 175B
(R)-2-(2-((4-amino-5-(4-phenoxyphenyl)-7H-pyrrolo[2,3-d]pyrimidin-7-yl)methyl)-pyrrolidine-1-carbonyl)-4-methylpent-2-enenitrile; 176A
(S)-2-(2-((4-amino-5-(4-phenoxyphenyl)-7H-pyrrolo[2,3-d]pyrimidin-7-yl)methyl)-pyrrolidine-1-carbonyl)-4-methylpent-2-enenitrile; 176B
(R)-4-amino-2-(2-((4-amino-5-(2-fluoro-4-phenoxyphenyl)-7H-pyrrolo[2,3-d]-pyrimidin-7-yl)methyl)pyrrolidine-1-carbonyl)-4-methylpent-2-enenitrile; 177A
(S)-4-amino-2-(2-((4-amino-5-(2-fluoro-4-phenoxyphenyl)-7H-pyrrolo[2,3-d]-pyrimidin-7-yl)methyl)pyrrolidine-1-carbonyl)-4-methylpent-2-enenitrile; 177B
(R)-2-(2-((4-amino-5-(4-phenoxyphenyl)-7H-pyrrolo[2,3-d]pyrimidin-7-yl)-methyl)pyrrolidine-1-carbonyl)-4-(dimethylamino)-4-methylpent-2-enenitrile; 178A
(S)-2-(2-((4-amino-5-(4-phenoxyphenyl)-7H-pyrrolo[2,3-d]pyrimidin-7-yl)-methyl)pyrrolidine-1-carbonyl)-4-(dimethylamino)-4-methylpent-2-enenitrile; 178B
(R)-2-(2-((4-amino-5-(4-phenoxyphenyl)-7H-pyrrolo[2,3-d]pyrimidin-7-yl)-methyl)pyrrolidine-1-carbonyl)-4-methyl-4-morpholinopent-2-enenitrile 179A
(S)-2-(2-((4-amino-5-(4-phenoxyphenyl)-7H-pyrrolo[2,3-d]pyrimidin-7-yl)-methyl)pyrrolidine-1-carbonyl)-4-methyl-4-morpholmopent-2-enenitrile 179B
(R)-2-(2-((4-amino-5-(4-phenoxyphenyl)-7H-pyrrolo[2,3-d]pyrimidin-7-yl)-methyl)pyrrolidine-1-carbonyl)-4-(diethylamino)-4-methylpent-2-enenitrile; 180A
(S)-2-(2-((4-amino-5-(4-phenoxyphenyl)-7H-pyrrolo[2,3-d]pyrimidin-7-yl)-methyl)pyrrolidine-1-carbonyl)-4-(diethylamino)-4-methylpent-2-enenitrile; 180B
(R)-2-(2-((4-amino-5-(4-phenoxyphenyl)-7H-pyrrolo[2,3-d]pyrimidin-7-yl)-methyl)pyrrolidine-1-carbonyl)-4-ethoxy-4-methylpent-2-enenitrile; 181A
(S)-2-(2-((4-amino-5-(4-phenoxyphenyl)-7H-pyrrolo[2,3-d]pyrimidin-7-yl)-methyl)pyrrolidine-1-carbonyl)-4-ethoxy-4-methylpent-2-enenitrile; 181B
(R)-N-(1-(4-amino-3-(2-fluoro-4-phenoxyphenyl)-1H-pyrazolo[3,4-d]pyrimidin-1-yl)-propan-2-yl)-2-cyano-4,4-dimethylpent-2-enamide; 182A
(S)-N-(1-(4-amino-3-(2-fluoro-4-phenoxyphenyl)-1H-pyrazolo[3,4-d]pyrimidin-1-yl)propan-2-yl)-2-cyano-4,4-dimethylpent-2-enamide; 182B
(R)-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide; 183A
(S)-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide; 183B
(R)-N-(1-(4-amino-3-(2-fluoro-4-phenoxyphenyl)-1H-pyrazolo [3,4-d]pyrimidin-1-yl)propan-2-yl)-2-cyano-4-ethoxy-4-methylpent-2-enamide; 184A
(S)-N-(1-(4-amino-3-(2-fluoro-4-phenoxyphenyl)-1H-pyrazolo[3,4-d]pyrimidin-1-yl)propan-2-yl)-2-cyano-4-ethoxy-4-methylpent-2-enamide; 184B
(R)-N-(1-(4-amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamide; 185A
(S)-N-(1-(4-amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamide; 185B
2-((R)-2-((4-amino-3-(2-fluoro-4-phenoxyphenyl)-1H-pyrazolo[3,4-d]pyrimidin-1-yl)methyl)pyrrolidine-1-carbonyl)-3-((S)-pyrrolidin-2-yl)acrylonitrile; 186
2-((R)-2-((4-amino-3-(2-fluoro-4-phenoxyphenyl)-1H-pyrazolo[3,4-d]pyrimidin-1-yl)methyl)pyrrolidine-1-carbonyl)-3-((R)-pyrrolidin-2-yl)acrylonitrile; 187
(R)-4-amino-N-(1-(4-amino-3-(2-fluoro-4-phenoxyphenyl)-1H-pyrazolo[3,4-d]-pyrimidin-1-yl)propan-2-yl)-2-cyano-4-methylpent-2-enamide; 188A
(S)-4-amino-N-(1-(4-amino-3-(2-fluoro-4-phenoxyphenyl)-1H-pyrazolo[3,4-d]-pyrimidin-1-yl)propan-2-yl)-2-cyano-4-methylpent-2-enamide; 188B
N-(2-(4-amino-3-(4-phenoxyphenyl)-1H-pyrazolo[3,4-d]pyrimidin-1-yl)ethyl)-2-cyano-3-cyclopropyl-N-methylacrylamide; 189
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile; 190A
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile; 190B
(S)-N-(1-(4-amino-3-(2- fluoro-4-phenoxyphenyl)-1H-pyrazolo[3,4-d]pyrimidin-1-yl)propan-2-yl)-2-cyano-4,4-dimethylpent-2-enamide; 191
(S)-N-(1-( 4-amino-3-(2-fluoro-4-phenoxyphenyl)-1H-pyrazolo[3,4-d]pyrimidin-1-yl)propan-2-yl)-2-cyano-4-methylpent-2-enamide; 192
(S)-N-(1-(4-amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamide; 193
(S)-N-(1-(4-amino-3-(2-fluoro-4-phenoxyphenyl)-1H-pyrazolo[3,4-d]pyrimidin-1-yl)propan-2-yl)-2-cyano-4-ethoxy-4-methylpent-2-enamide; 194
(S)-4-amino-N-(1-(4-amino-3-(2-fluoro-4-phenoxyphenyl)-1H-pyrazolo[3,4-d]-pyrimidin-1-yl)propan-2-yl)-2-cyano-4-methylpent-2-enamide; 195 or
(S)-N-(1-(4-amino-3-(2-fluoro-4-phenoxyphenyl)-1H-pyrazolo[3,4-d]pyrimidin-1-yl)propan-2-yl)-2-cyano-3-cyclopropylacrylamide; 196
or R and S mixtures thereof;
or an individual (E) or (Z) isomer thereof;
and/or a pharmaceutically acceptable salt thereof.

For example, the said at least compound or the compound is chosen from:
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-cyclopropylacrylonitrile;
(R)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(R)-4-amino-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-ethoxy-4-methylpent-2-enenitrile;
(R)-2-(3-(4-amino-3-(2-fluoro-4-phenoxyphenyl)-1H-pyrazolo[3,4-d]pyrimidi-1-yl)piperidine-1-carbonyl)-4-ethoxy-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-methyl-4-morpholinopent-2-enenitrile; or
(R)-2-(3-(4-amino-3-(2,3-difluoro-4-phenoxyphenyl)-1H-pyrazolo[3,4-d]pyrimidin-1-yl)piperidine-1-carbonyl)-4-methyl-4-morpholinopent-2-enenitrile;
or an R and S mixtures thereof;
or an individual (E) or (Z) isomer thereof;
and/or a pharmaceutically acceptable salt thereof.

For example, the at least one compound or compound is chosen from :
(R)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-ethoxy-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
or R and S mixtures thereof;
or an individual (E) or (Z) isomer thereof;
and/or a pharmaceutically acceptable salt thereof.

For example, the formulations disclosed herein comprise a compound chosen from :
(R)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-ethoxy-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
or an R and S mixtures thereof;
or an individual (E) or (Z) isomer thereof;
and/or a pharmaceutically acceptable salt thereof.

### Definitions:

Unless otherwise stated, the following terms used in the specification and claims are defined for the purposes of this Application and have the following meaning:
"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, butyl (including all isomeric forms), pentyl (including all isomeric forms), and the like.

"Alkynyl" means a linear saturated monovalent hydrocarbon radical of two to six carbon atoms or a branched saturated monovalent hydrocarbon radical of three to six carbon atoms that contains a triple bond, e.g., ethynyl, propynyl, 2-propynyl, butynyl (including all isomeric forms), pentynyl (including all isomeric forms), and the like.

"Alkylene" means a linear saturated divalent hydrocarbon radical of one to six carbon atoms or a branched saturated divalent hydrocarbon radical of three to six carbon atoms unless otherwise stated e.g., methylene, ethylene, propylene, 1-methylpropylene, 2-methylpropylene, butylene, pentylene, and the like.

"Alkylthio" means a -SR radical where R is alkyl as defined above, e.g., methylthio, ethylthio, and the like.

"Alkylsulfonyl" means a -SO₂R radical where R is alkyl as defined above, e.g., methylsulfonyl, ethylsulfonyl, and the like.

"Amino" means a -NH₂.

"Alkylamino" means a -NHR radical where R is alkyl as defined above, e.g., methylamino, ethylamino, propylamino, or 2-propylamino, and the like.

"Alkoxy" means a -OR radical where R is alkyl as defined above, e.g., methoxy, ethoxy, propoxy, or 2-propoxy, *n-, iso-,* or *tert*-butoxy, and the like.

"Alkoxyalkyl" means a linear monovalent hydrocarbon radical of one to six carbon atoms or a branched monovalent hydrocarbon radical of three to six carbons substituted with at least one alkoxy group, for example one or two alkoxy groups, as defined above, e.g., 2-methoxyethyl, 1-, 2-, or 3-methoxypropyl, 2-ethoxyethyl, and the like.

"Alkoxycarbonyl" means a -C(O)OR radical where R is alkyl as defined above, e.g., methoxycarbonyl, ethoxycarbonyl, and the like.

"Aminocarbonyl" means a -CONRR' radical where R is independently hydrogen, alkyl, or substituted alkyl, each as defined herein and R' is hydrogen, alkyl, cycloalkyl, cycloalkylalkyl, aryl, aralkyl, heteroaryl, heteroaralkyl, heterocyclyl, heterocyclylalkyl, or substituted alkyl, each as defined herein and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, and substituted alkylaminocarbonyl, e.g., -CONH₂, methylaminocarbonyl, 2-dimethylaminocarbonyl, and the like. When R is hydrogen and R' is alkyl in -CONRR', the group is also referred to herein as alkylaminocarbonyl and when R and R' are both alkyl in -CONRR', the group is also referred to herein as dialkylaminocarbonyl. When R is hydrogen and R' is substituted alkyl in -CONRR', the group is also referred to herein as substituted alkylaminocarbonyl.

"Aminosulfonyl" means a -SO₂NRR' radical where R is independently hydrogen, alkyl, or substituted alkyl, each as defined herein and R' is hydrogen, alkyl, cycloalkyl, cycloalkylalkyl, aryl, aralkyl, heteroaryl, heteroaralkyl, heterocyclyl, heterocyclylalkyl, or substituted alkyl, each as defined herein and wherein the aryl, heteroaryl, or heterocyclyl ring either alone or part of another group e.g., aralkyl, is optionally substituted with one, two, or three substituents independently selected alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, substituted alkylaminocarbonyl, e.g., -SO₂NH₂, methylaminosulfonyl, dimethylaminosulfonyl, and the like. When R is hydrogen and R' is alkyl in -SO₂NRR', the group is also referred to herein as alkylaminosulfonyl and when R and R' are both alkyl in -SO₂NRR', the group is also referred to herein as dialkylaminosulfonyl.

"Acyl" means a -COR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, and substituted alkylaminocarbonyl, e.g., acetyl, propionyl, benzoyl, pyridinylcarbonyl, and the like. When R is alkyl, the radical is also referred to herein as alkylcarbonyl.

"Aryl" means a monovalent monocyclic or bicyclic aromatic hydrocarbon radical of 6 to 10 ring atoms e.g., phenyl or naphthyl. "Aralkyl" means a -(alkylene)-R radical where R is aryl as defined above.

"Cycloalkyl" means a cyclic saturated monovalent hydrocarbon radical of three to ten carbon atoms wherein one or two carbon atoms may be replaced by an oxo group, e.g., cyclopropyl, cyclobutyl, cyclopentyl, or cyclohexyl, and the like.

"Cycloalkylalkyl" means a -(alkylene)-R radical where R is cycloalkyl as defined above; e.g., cyclopropylmethyl, cyclobutylmethyl, cyclopentylethyl, or cyclohexylmethyl, and the like.

"Cycloalkylene" means a cyclic saturated divalent hydrocarbon radical of three to ten carbon atoms wherein one or two carbon atoms may be replaced by an oxo group, e.g., cyclopropylene, cyclobutylene, cyclopentylene, or cyclohexylene, and the like.

"Carboxy" means -COOH.

"Disubstituted amino" means a -NRR' radical where R and R' are independently alkyl, cycloalkyl, cycloalkylalkyl, acyl, aryl, aralkyl, heteroaryl, heteroaralkyl, heterocyclyl, heterocyclylalkyl, or substituted alkyl, each as defined herein, and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, and substituted alkylaminocarbonyl, e.g., dimethylamino, phenylmethylamino, and the like. When the R and R' groups are alkyl, the disubstituted amino group maybe referred to herein as dialkylamino.

"Halo" means fluoro, chloro, bromo, or iodo, for example fluoro or chloro.

"Haloalkyl" means alkyl radical as defined above, which is substituted with one or more halogen atoms, for example one to five halogen atoms, for example fluorine or chlorine, including those substituted with different halogens, e.g., -CH₂Cl, -CF₃, -CHF₂, -CH₂CF₃, -CF₂CF₃, -CF(CH₃)₂, and the like. When the alkyl is substituted with only fluoro, it is referred to in this Application as fluoroalkyl.

"Haloalkoxy" means a -OR radical where R is haloalkyl as defined above e.g., -OCF₃, -OCHF₂, and the like. When R is haloalkyl where the alkyl is substituted with only fluoro, it is referred to in this Application as fluoroalkoxy.

"Hydroxyalkyl" means a linear monovalent hydrocarbon radical of one to six carbon atoms or a branched monovalent hydrocarbon radical of three to six carbons substituted with one or two hydroxy groups, provided that if two hydroxy groups are present they are not both on the same carbon atom. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl and 2-(hydroxymethyl)-3-hydroxypropyl, for example 2-hydroxyethyl, 2,3-dihydroxypropyl, and 1-(hydroxymethyl)-2-hydroxyethyl.

"Heterocyclyl" means a saturated or unsaturated monovalent monocyclic group of 4 to 8 ring atoms in which one or two ring atoms are heteroatom selected from N, O, and S(O)ₙ, where n is an integer from 0 to 2, the remaining ring atoms being C. The heterocyclyl ring is optionally fused to a (one) aryl or heteroaryl ring as defined herein provided the aryl and heteroaryl rings are monocyclic. The heterocyclyl ring fused to monocyclic aryl or heteroaryl ring is also referred to in this Application as "bicyclic heterocyclyl" ring. Additionally, one or two ring carbon atoms in the heterocyclyl ring can optionally be replaced by a -CO- group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group. When the heterocyclyl group is a saturated ring and is not fused to aryl or heteroaryl ring as stated above, it is also referred to herein as saturated monocyclic heterocyclyl.

" Heterocyclylalkyl" means a -(alkylene)-R radical where R is heterocyclyl ring as defined above e.g., tetraydrofuranylmethyl, piperazinylmethyl, morpholinylethyl, and the like.

"Heterocycloamino" means a saturated or unsaturated monovalent monocyclic group of 4 to 8 ring atoms in which one or two ring atoms are heteroatom selected from N, O, and S(O)ₙ, where n is an integer from 0 to 2, the remaining ring atoms being C provided that at least one of the ring atoms is N. Additionally, one or two ring carbon atoms in the heterocycloamino ring can optionally be replaced by a -CO- group. When the heterocycloamino ring is unsaturated it can contain one or two ring double bonds provided that the ring is not aromatic.

"Heteroaryl" means a monovalent monocyclic or bicyclic aromatic radical of 5 to 10 ring atoms where one or more, for example one, two, or three, ring atoms are heteroatom selected from N, O, and S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like.

"Heteroaralkyl" means a -(alkylene)-R radical where R is heteroaryl as defined above.

"Heteroalkylene " means an -(alkylene)- radical where one, two or three carbons in the alkylene chain is replaced by -O-, N(H, alkyl, or substituted alkyl), S, SO, SO₂, or CO.

"Monosubstituted amino" means a -NHR radical where R is alkyl, cycloalkyl, cycloalkylalkyl, acyl, aryl, aralkyl, heteroaryl, heteroaralkyl, heterocyclyl, heterocyclylalkyl, or substituted alkyl, each as defined herein, and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, and substituted alkylaminocarbonyl, e.g., methylamino, phenylamino, hydroxyethylamino, and the like. When R is alkyl, the monosubstituted amino group maybe referred to herein as alkylamino.

The term "Michael acceptor moiety" refers to a functional group that can participate in a Michael reaction, wherein a new covalent bond is formed between a portion of the Michael acceptor moiety and the donor moiety. The "Z-CO-C(CN)=CHR^{c}" group present in the compound of Formula (Id) is a Michael acceptor moiety.

The present disclosure also includes protected derivatives of compounds of the present invention(or any of the embodiments thereof described herein),. For example, when said compounds (or any of the embodiments thereof described herein),contain groups such as hydroxy, carboxy, thiol or any group containing a nitrogen atom(s), these groups can be protected with a suitable protecting groups. A comprehensive list of suitable protective groups can be found in T.W. Greene, Protective Groups in Organic Synthesis, John Wiley & Sons, Inc. (1999). The protected derivatives of said compounds (or any of the embodiments thereof described herein), can be prepared by methods well known in the art.

The present disclosure also includes polymorphic forms (amorphous as well as crystalline) and deuterated forms of compounds of the present invention (or any of the embodiments thereof described herein).

A "pharmaceutically acceptable salt" of a compound means a salt that is pharmaceutically acceptable and that possesses the desired pharmacological activity of the parent compound. Such salts include:
acid addition salts, formed with inorganic acids such as hydrochloric acid, hydrobromic acid, sulfuric acid, nitric acid, phosphoric acid, and the like; or formed with organic acids such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or
salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, *N*-methylglucamine, and the like. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985.

The compounds of the present invention may have asymmetric centers. Compounds of the present invention containing an asymmetrically substituted atom may be isolated in optically active or racemic forms. It is well known in the art how to prepare optically active forms, such as by resolution of materials. All chiral, diastereomeric, racemic forms are within the scope of this disclosure, unless the specific stereochemistry or isomeric form is specifically indicated.

Compounds of the present invention (or any of the embodiments thereof described herein), can exist as tautomers and/or geometric isomers. All possible tautomers and *cis* and *trans* isomers, as individual forms and mixtures thereof are within the scope of this disclosure. Additionally, as used herein the term alkyl includes all the possible isomeric forms of said alkyl group albeit only a few examples are set forth. Furthermore, when the cyclic groups such as aryl, heteroaryl, heterocyclyl are substituted, they include all the positional isomers albeit only a few examples are set forth. Furthermore, all polymorphic forms and hydrates of a compound of the present invention (or any of the embodiments thereof described herein), are within the scope of this disclosure.

"Oxo" or "carbonyl" means C=(O) group.

"Optional" or "optionally" means that the subsequently described event or circumstance may but need not occur, and that the description includes instances where the event or circumstance occurs and instances in which it does not. For example, "heterocyclyl group optionally substituted with an alkyl group" means that the alkyl may but need not be present, and the description includes situations where the heterocyclyl group is substituted with an alkyl group and situations where the heterocyclyl group is not substituted with alkyl.

"Oral bioavailability" means the fraction of dose of unchanged drug that reaches the systemic circulation when the drug is administered orally versus when the drug is administered intravenuosly, the bioavailability of the drug being 100% when it is adminstered intravenously. Methods to determine the bioavailability of drugs are well known to those of ordinary skill in the art.

A "pharmaceutically acceptable carrier or excipient" means a carrier or an excipient that is useful in preparing a pharmaceutical composition that is generally safe, non-toxic and neither biologically nor otherwise undesirable, and includes a carrier or an excipient that is acceptable for veterinary use as well as human pharmaceutical use. "A pharmaceutically acceptable carrier/excipient" as used in the specification and claims includes both one and more than one such excipient.

"Substituted alkyl" means alkyl group as defined herein which is substituted with one, two, or three substituents independently selected from hydroxyl, alkoxy, carboxy, cyano, carboxy, alkoxycarbonyl, alkylthio, alkylsulfonyl, halo, -CONRR' or -NRR' (where each R is hydrogen, alkyl, cycloalkyl, hydroxyalkyl, and alkoxyalkyl, and each R' is hydrogen, alkyl, or cycloalkyl) or heterocyclyl (for example heterocycloamino) which is optionally substituted with one or two groups independently selected from alkyl, hydroxyl, alkoxy, alkylthio, alkylsulfonyl, halo, and -CONRR' where R and R' are as defined above.

"Solid dosage formulation" as used herein is typically a tablet, capsule, or the like, that is comprised of compounds disclosed herein (i.e., cpds of the present invention and/or the excipients that can be either solids or liquids e.g., the capsule can contain compounds disclosed herein (i.e., cpds of the present invention and/or the excipients that can be either be as solids or liquids.

"Substituted aryl or substituted heteroaryl" means aryl or heteroaryl as defined above, that is substituted with one, two, or three substituents independently selected from hydrogen, alkyl, substituted alkyl, cycloalkyl, cycloalkylalkyl, haloalkyl, haloalkoxy, halogen, amino, monosubstituted amino, disubstituted amino, acyl, aminocarbonyl, aminosulfonyl, -OR', -SR', -OC(O)R', -CO₂R', -NR"C(O)R', -NR"C(O)NR'R", -NR"C(O)₂R', -SO₂R', -NR"SO₂R', -CN, -NO₂, aryl, aralkyl, heteroaryl, heteroaralkyl, heterocyclyl, and heterocyclylalkyl where R' is hydrogen, alkyl, haloalkyl, substituted alkyl, cycloalkyl, cycloalkylalkyl, aryl, aralkyl, heteroaryl, heteroaralkyl, heterocyclyl or heterocyclylalkyl and R" is hydrogen, alkyl, or substituted alkyl; or R' and R' together with the nitrogen atom to which they are attached from heterocycloamino; provided at least one of the three substituent is not hydrogen is and furthermore wherein the aryl, heteroaryl, cycloalkyl, heterocycloamino, or heterocyclyl ring in any of the above groups, except as stated herein, is substituted with:
(i) one, two, or three substituents independently selected from hydrogen, alkyl, substituted alkyl, cycloalkyl, cycloalkylalkyl, haloalkyl, haloalkoxy, halogen, amino, monosubstituted amino, disubstituted amino, acyl, aminocarbonyl, aminosulfonyl, -OR', -SR', -OC(O)R', -CO₂R', -NR"C(O)R', -NR"C(O)NR'R", -NR"C(O)₂R', -SO₂R', -NR"SO₂R', -CN, -NO₂, aryl, aralkyl, heteroaryl, heteroaralkyl, heterocyclyl, and heterocyclylalkyl where R' is hydrogen, alkyl, haloalkyl, substituted alkyl, cycloalkyl, cycloalkylalkyl, aryl, aralkyl, heteroaryl, heteroaralkyl, heterocyclyl or heterocyclylalkyl and R" is hydrogen, alkyl, or substituted alkyl; or R' and R' together with the nitrogen atom to which they are attached from heterocycloamino; and furthermore wherein the aryl, heteroaryl, cycloalkyl, heterocycloamino, or heterocyclyl ring in any of the above groups in (i), except as stated herein, is substituted with one, two, or three substituents independently selected from hydrogen, alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, substituted alkylaminocarbonyl, amino, and monosubstituted or disubstituted amino.

"Treating" or "treatment" of a disease includes:
(1) preventing the disease, i.e. causing the clinical symptoms of the disease not to develop in a mammal that may be exposed to or predisposed to the disease but does not yet experience or display symptoms of the disease;
(2) inhibiting the disease, i.e., arresting or reducing the development of the disease or its clinical symptoms; or
(3) relieving the disease, i.e., causing regression of the disease or its clinical symptoms.

A "therapeutically effective amount" means the amount of a compound of the present invention (or any of the embodiments thereof described herein),that, when administered to a mammal for treating a disease, is sufficient to effect such treatment for the disease. The "therapeutically effective amount" will vary depending on the compound, the disease and its severity and the age, weight, etc., of the mammal to be treated.

### General Synthetic Scheme

Compounds of this invention can be made by the methods depicted in the reaction schemes shown below.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure over a temperature range from about -78 °C to about 150 °C, more for example from about 0 °C to about 125 °C and further for example at about room (or ambient) temperature, e.g., about 20 °C.
Representative methods for preparing compounds of Formula (IA), a general formula which encompasses the compounds of Formula (Id) and compounds chosen from N-(2-(4-amino-3-(2-fluoro-4-phenoxyphenyl)-1H-pyrazolo[3,4-d]pyrimidin-1-yl)ethyl)-1-cyano-2-cyclopropylethenesulfonamide, N-(2-(4-amino-3-(2-fluoro-4-phenoxyphenyl)-1H-pyrazolo[3,4-d]pyrimidin-1-yl)ethyl)-1-cyano- 2-cyclopropyl-N-methylethenesulfonamide, 2-(4-amino-3-(2-fluoro-4-phenoxyphenyl)-1H-pyrazolo[3,4-d]pyrimidin-1-yl)ethyl 2-cyano-3-cyclopropylacrylate, 1-(4-amino-3-(2-fluoro-4-phenoxyphenyl)-1H-pyrazolo[3,4-d]pyrimidin-1-yl)-2-methylpropan-2-yl 2-cyano-3-cyclopropylacrylate, 2-((2-(4-amino-3-(2-fluoro-4-phenoxyphenyl)-1H-pyrazolo[3,4-d]pyrimidin-1-yl)ethyl)sulfonyl)-3-cyclopropylacrylonitrile, and 2-(5-((4-amino-3-(2-fluoro-4-phenoxyphenyl)-1H-pyrazolo[3,4-d]pyrimidin-1-yl)methyl)oxazol-2-yl)-3-cyclopropylacrylonitrile, are disclosed below.

Compounds of Formula (IA) where: Z¹ is nitrogen, Z² is carbon or nitrogen and Z³ is carbon;
L is O, CO, CH₂, S, SO, SO₂, NR, NRCO, CONR, NR'SO₂, SO₂NR', or NRCONR, where each R and R' is independently hydrogen or alkyl;
Ar is aryl, heteroaryl, cycloalkyl or heterocyclyl;
R¹ is -Z-CO-C(CN)=CHR^{c} or -Z-SO₂-C(CN)=CHR^{c} where Z is bond, alkylene, cycloalkylene, 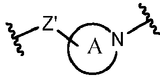 (where Z' is bond, alkylene, NR^{a}, or O and A is heterocycloamino optionally substituted with one or two substituents independently selected from alkyl, hydroxy, or fluoro), O, -alkylene-O-, NR^{a} and -(alkylene)-NR^{a}- (where each R^{a} is hydrogen, alkyl or cycloalkyl); and R^{c} is alkyl, haloalkoxy, substituted alkyl, cycloalkyl, cycloalkyleneNR^{d}R^{e} or cycloalkylene(alkylene)NR^{d}R^{e} (where R^{d} and R^{e} are independently hydrogen, alkyl, or cycloalkyl), or 3 to 6 membered saturated monocyclic heterocyclyl containing one or two heteroatoms selected from N, O, and S and optionally substituted with one or two substituents independently selected from hydroxy, alkyl and fluoro;
R³ is hydrogen, alkyl, cyclopropyl, hydroxy, alkoxy, cyano, halo, haloalkyl or haloalkoxy;
R⁴ is hydrogen, alkyl, alkynyl, cyclopropyl, alkylamino, dialkylamino, alkylthio, alkylsulfonyl, carboxy, alkoxycarbonyl, alkylaminosulfonyl, dialkylaminosulfonyl, -CONH₂, alkylaminocarbonyl, dialkylaminocarbonyl, 3, 4 or 5 membered heterocylyl, hydroxy, alkoxy, cyano, halo, haloalkyl or haloalkoxy; and
R⁶ and R⁷ are independently hydrogen, alkyl, hydroxy, alkoxy, halo, haloalkyl, haloalkoxy, carboxy, alkoxycarbonyl, cyano, -CONH₂, amino, or monosubstituted or disubstituted amino; and./or a pharmaceutically acceptable salt thereof; comprising at least one coating chosen from an enteric coating and/or a non-enteric, time-delayed release coating; and
R⁵ is hydrogen, alkyl, hydroxy, alkoxy, halo, haloalkyl, or haloalkoxy;
can be prepared as illustrated and described in Scheme A below.

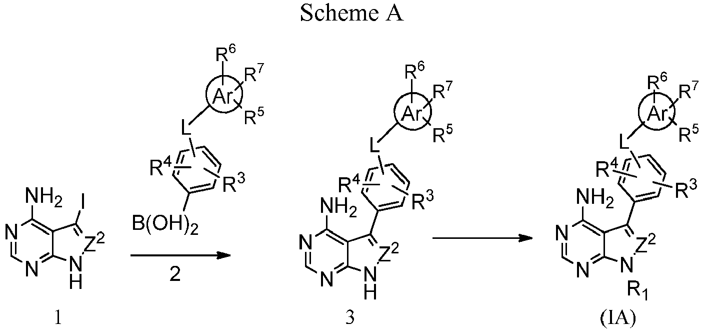

Coupling of an iodo compound of formula 1 where with a boronic acid compound of formula 2 or boronate esters thereof Ar, R¹, R³, R⁴, R⁵, R⁶, R⁷, L, and Ar are as defined above under Suzuki coupling reaction conditions provides a compound of formula 3. The Suzuki coupling reaction can be carried out in organic solvents (such as toluene, benzene, N,N-dimethylformamide (DMF), tetrahydrofuran, methanol, ethanol, acetonitrile, dimethoxyethane, acetone and the like) or water in the presence of base (such as sodium ethylate, sodium hydroxide, potassium hydroxide, sodium bicarbonate, potassium bicarbonate, potassium carbonate, sodium carbonate, triethylamine, and the like) and a palladium catalyst (such as tetrakis(triphenylphosphine)palladium, dichlorobis(triphenylphosphine)palladium, palladium acetate, and the like). The reaction is carried out at room temperature to 120° C. Compounds of formula 1 are either commercially available or can be readily prepared by methods well known in the art.

Treatment of a compound of formula 3 with a compound of formula R¹-LG where LG is a suitable leaving group such as halo, tosylate, mesylate, triflate, and the like provides a compound of Formula (IA). The alkylation or arylation reaction is typically carried out in the presence of a base such as sodium hydride or potassium tert-butoxide, potassium carbonate, and the like, and a catalyst such as 18-crown-6 in a suitable solvent such as N-methylpyrolidone, dimethylformamide, tetrahydrofuran, toluene, and the like.

It will be recognized by a person skilled in the art that precursors to R¹ group can be substituted at any step in the synthetic procedure illustrated in Scheme A above and converted to R¹ group as defined above at alternate stages in the synthetic process based on feasibility of the transformations. Some such examples are described below:
Substitution of precursors to R¹ in the synthesis of compounds of Formula (IA) when R¹ is -Z-(CO)-C(CN)=CHR^{c} where Z is 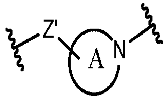 is illustrated and described in Method (a) below.

### Method (a):

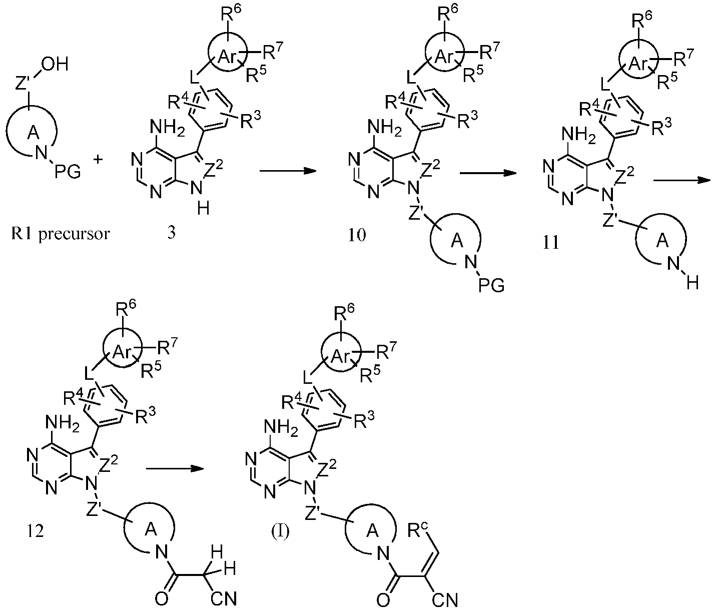

Treatment of a N-protected heterocycloamino R¹ precursor compound (suitable nitrogen protecting groups (PG) include t-butyloxycarbonyl (BOC), carbobenzyloxy (Cbz), or 2-trimethylsilyl-ethoxymethyl (SEM)) bearing an alcohol with a compound of formula 3 under Mitsunobu reaction conditions provides a compound of formula 10 where Ar, R³, R⁴, R⁵, R⁶, R⁷, L, Ar, and Z² are as defined above. Removal of the amino protecting group can be effected using strong acid (TFA or HCL in the case of a Boc group, hydrogenolysis in the case of Cbz, or fluoride anion to remove the SEM), to provide the amine of formula 11. Coupling of compound of formula 11 with a compound of formula CNCH₂CO₂H such as 2-cyanoacetic acid or 2-trifluoromethylacetic acid, under standard amide coupling conditions such as carbon diimidazole (CDI) and the like or an acid derivative thereof provides a compound of formula 12. Subsequent condensation of a compound of formula 12 with aldehydes of formula R^{c}CHO where R^{c} is as defined above under standard condensation reaction conditions such as using a base such as piperidine and the like, in the presence or absence of acetic acid and the like in solvents such as ethanol and the like at temperatures ranging from room temperature to reflux then provides a compound of Formula (IA). Aldehydes of formula R^{c}CHO are either commercially available or they can be prepared by methods know in the art. For example *tert*-butylaldehyde, isopropylaldehyde and cyclopropylaldehyde are commerically available. Compounds of Formula (IA) where R^{c} - C(CH₃)₂NH₂, and -C(CH₃)₂NHCH₃ can be prepared by reacting a compound of formula 12 with an aldehyde of formula BocNHC(CH₃)₂CHO and BocN(CH₃)C(CH₃)₂CHO respectively, followed by removal of the Boc group. Aldehydes of formula BocNHC(CH₃)₂CHO can be prepared as shown below:

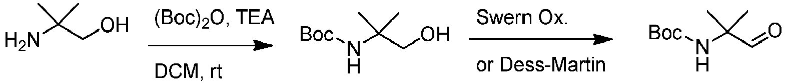

Treatment of 2-amino-2-methylpropan-1-ol with (Boc)₂O in the presence of organic amine provides the corresponding 2-BocNH-2-methylpropan-1-ol which upon reaction with a suitable oxidizing agent provide the desired aldehyde of formula 2-BocNH-2-methylpropanaldehyde.

Aldehydes of formula BocN(CH₃)C(CH₃)₂CHO can be prepared as shown below:

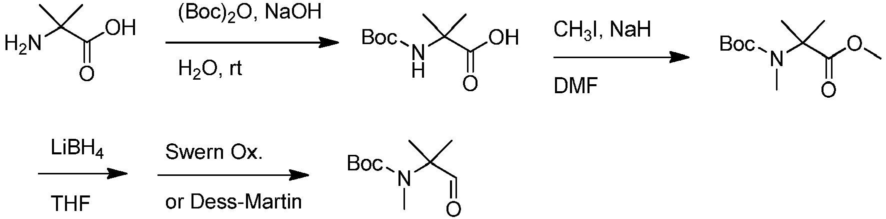

Treatment of 2-amino-2-methylpropanoic with (Boc)₂O in the presence of organic amine provides the corresponding 2-BocNH-2-methylpropanoic which upon reaction with an alkylating agent such as methyl iodide in the presence of sodium hydride provide 2-BocN(CH₃)-2-methylpropanoic ester. Reduction of the ester group in BocN(CH₃)-2-methylpropanoic ester with a suitable reducing agent provides the corresponding alcohol which is then covered to the desired aldehyde as described previously.

It will recognized by a person of ordinary skill in the art that the EWG moiety can be assembled at multiple points throughout the synthetic scheme and standard protecting group (PG) strategies can be employed as required.

Compounds of Formula (IA) where Z¹ and Z³ are nitrogen and Z² is carbon, Ar, R¹, R³, R⁴, R⁶, R⁷, and L, Ar are as defined above and R⁵ is hydrogen, alkyl, hydroxy, alkoxy, halo, haloalkyl, or haloalkoxy can be prepared as illustrated and described in Scheme B below.

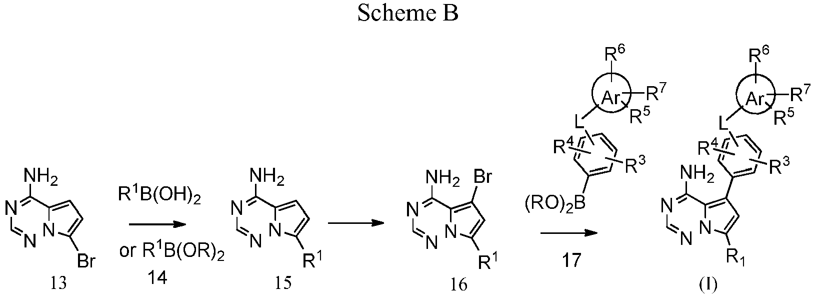

Cross coupling (Suzuki) of a compound of formula 13 (available commercially) with an appropriately substituted boronic acid or boronate esters of formula 13 (as described in Scheme A) provides a compound of formula 15 where R¹ is as defined above. Halogenation of compound 15 with a suitable halogenating agent such as N-bromosuccinamide, bromine, and the like, in an organic solvent (such as DMF, dichloromethane, tetrahydrofuran, toluene, acetic acid, water and the like) at temperatures ranging from -78° C to reflux temperature provides a compound of formula 16. Compound 16 is then coupled with a compound of formula 17 under Suzuki coupling reaction conditions to provide a compound of Formula (IA) where Ar, R¹, R³, R⁴, R⁵, R⁶, R⁷, L, and Ar are as definedabove.

It will be recognized by a person skilled in the art that precursors to R¹ can be substituted at any step in Scheme 2 above where R¹ exists and converted to R¹ at alternate stages in the synthetic process based on feasibility of the transformations. Some such transformations are described below:
Substitution of precursors to R¹ in the synthesis of compounds of Formula (IA) when R¹ is -Z-(CO)-C(CN)=CHR^{c} where Z is 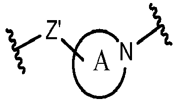 where Z' is bond, is illustrated and described in method (b) below. Standard protecting group (PG) strategies employed by those skilled in the art can be employed as required.

### Method (b):

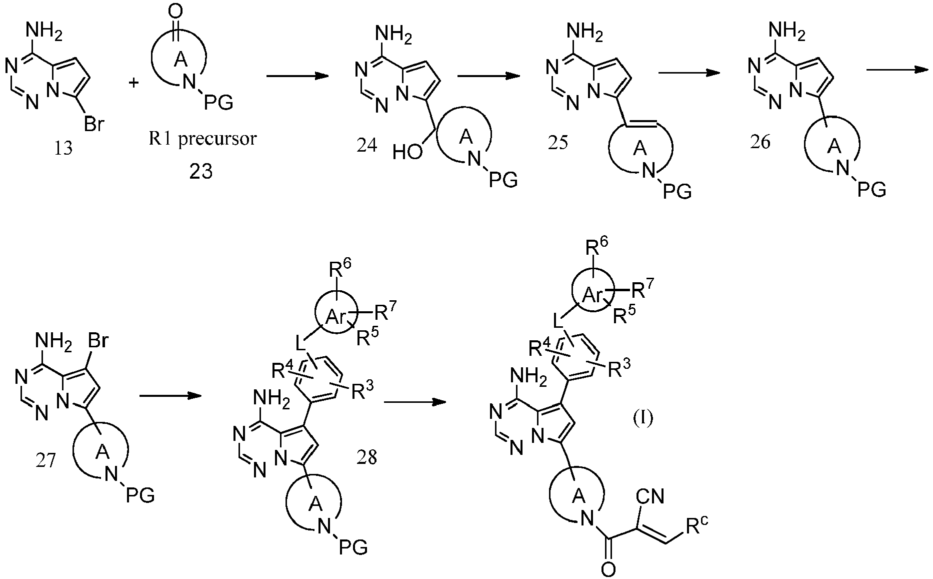

Treatment of compound 13 with trimethylsilyl chloride in solvents such as tetrahydrofuran (THF) at temperatures ranging from 0°C to room temperature prior to treatment by a Grignard reaction (for example by treatment with isopropyl magnesium chloride in THF at temperatures ranging from 0°C to room temperature) and subsequent addition of R¹ precursor compound of formula 23 bearing a ketone moiety where PG is a suitable protecting group such as tert-butoxycarbonyl (Boc), benzyl (Bn) or 2-trimethylsilyl-ethoxymethyl (SEM)), provides a compound of formula 24 which is converted to a compound of formula 25 under dehydration reaction conditions e.g., treatment of compound 24 with acids such as trifluoroacetic anhydride or trifluoroacetic acid, and the like, in solvents such as pyridine, toluene, methanol, and the like and temperatures ranging from -20°C to reflux. Reduction of the double bond in the compound of formula 25 with a suitable hydrogenation reaction conditions e.g., with platinum oxide or palladium hydroxide or palladium on carbon in alcoholic solvents such as methanol or ethanol, and the like in the presence or absence of acetic acid and under a hydrogen atmosphere provides a compound of formula 26.

Halogenation of a compound of formula 26 with a suitable halogenating agent as described in scheme B above provides a compound of formula 27 which can then be converted to a compound of Formula (IA) as described in method a above.

Compounds of Formula (IA) where Z¹ and Z² are nitrogen and Z³ is carbon and R¹, R³, R⁴, R⁵, R⁶, R⁷, L, and Ar are as defined above can be prepared as illustrated and described in Scheme C below.

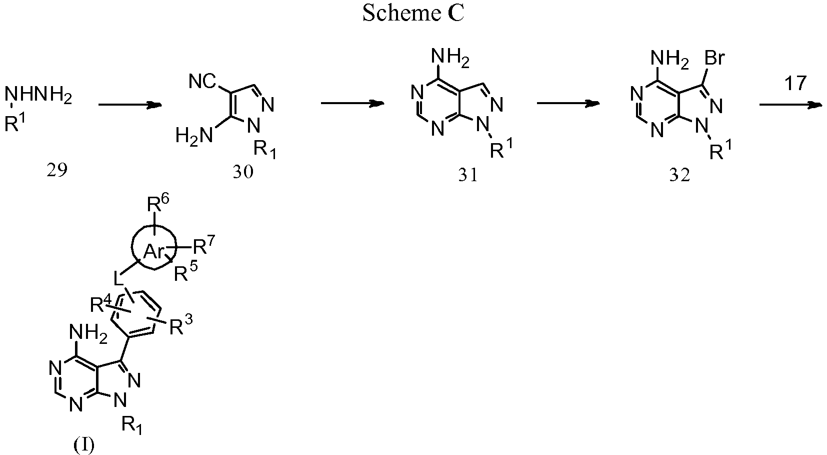

Reaction of a hydrazine compound of formula 1 where R¹ is as defined above with ethoxymethylene malonitrile in a suitable organic solvent such as ethanol and the like and at temperatures from 0° C to reflux provide a compound of formula 30. Compound of formula 1 that are either commercially available or readily synthesized by methods that are well known in the art.

Treatment of compound 30 with formamide or formamidine in the absence of solvent or in solvents such as ethanol and the like at temperatures from room temperature to 200 °C provides a compound of formula 31. Halogenation of 31 under halogenating conditions described above provides the compound of formula 32 which can then be converted to a compound of Formula (IA) as described in Scheme A above.

It will be recognized by a person skilled in the art that precursors to group R¹ can be substituted at any step in Scheme C above where R¹ exists and then converted to R¹ at alternate stages in the synthetic process based on feasibility of the transformations. Some such transformations are described below:
Substitution of precursors to R¹ in the synthesis of compounds of Formula (IA) when R¹ is -Z-(CO)-C(CN)=CHR^{c} where Z is 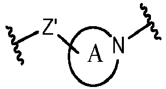 is illustrated and described in method (c) below. Standard protecting group (PG) strategies employed by those skilled in the art can be employed as required.

### Method (c):

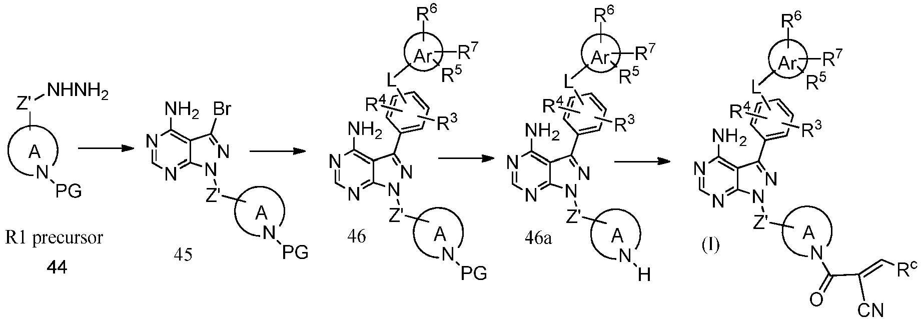

Substituting compound of formula 33 where Z' is a bond or alkylene and where PG is a suitable nitrogen protecting group such as tert-butoxycarbonyl (Boc), benzyl (Bn) or 2-trimethylsilyl-ethoxymethyl (SEM)) with a compound of formula 44 followed by steps 2-5 in Method (g) above provides a compound of formula 46. Removal of the amine protecting group under standard conditions such as HCl in ethyl acetate or trifluoroacetic acid in dichloromethane at 0° C to reflux for BOC and catalytic hydrogenation in ethyl alcohol for CBZ, provides a compound 46a that can then be converted to a compound of Formula (IA) by methods previously described in Method A .

Compounds of Formula (IA) where Z¹ is nitrogen and Z² and Z³ are carbon and R¹, R³, R⁴, R⁵, R⁶, R⁷, L, and Ar are as defined above can be prepared as illustrated and described in Scheme D below.

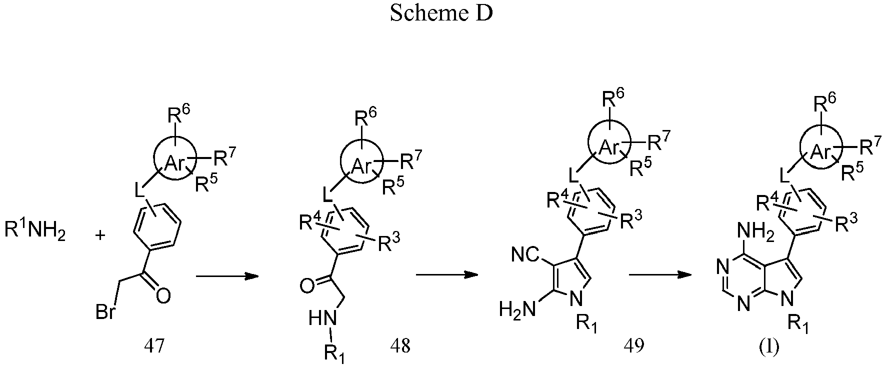

Alkylation of a compound of the formula R'NH₂ where R¹ is as defined above with a compound of formula 47 under standard alkylation reaction conditions (e.g., reacting in the presence of a base such as sodium hydride or potassium tert-butoxide, potassium carbonate, and the like, and a catalyst such as 18-crown-6 in a suitable solvent such as N-methylpyrolidone, dimethylformamide, tetrahydrofuran, toluene and the like) provides a compound of formula 48. Reaction of compound 48 with malonitrile and a base such as potassium hydroxide, sodium hydroxide, and the like in a suitable solvent such as methanol or ethanol and the like at temperatures from 0° C to reflux provides a compound of formula 49 which is then converted to a compound of Formula (IA) as described in Scheme C above.

As discussed previously, it will be recognized by a person skilled in the art that precursors to group R¹ can be substituted at any step in Scheme D above where R¹ exists and then converted to R¹ at alternate stages in the synthetic process based on feasibility of the transformations. For example, an amine of formula 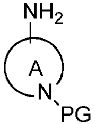 can be used instead of R¹NH₂ in Scheme D above to give a compound of 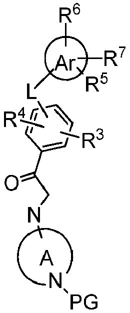 respectively, which is then converted to a compound of Formula (IA) where R¹ is -(heterocycloamino)-C(CN)=CHR^{c} following the procedures described above.

Substitution of precursors to R¹ in the synthesis of compounds of Formula (IA) when R¹ is -Z-(CO)-C(CN)=CHR^{c} where Z is -alkyleneNR^{a}- is illustrated and described in Scheme E below.

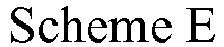

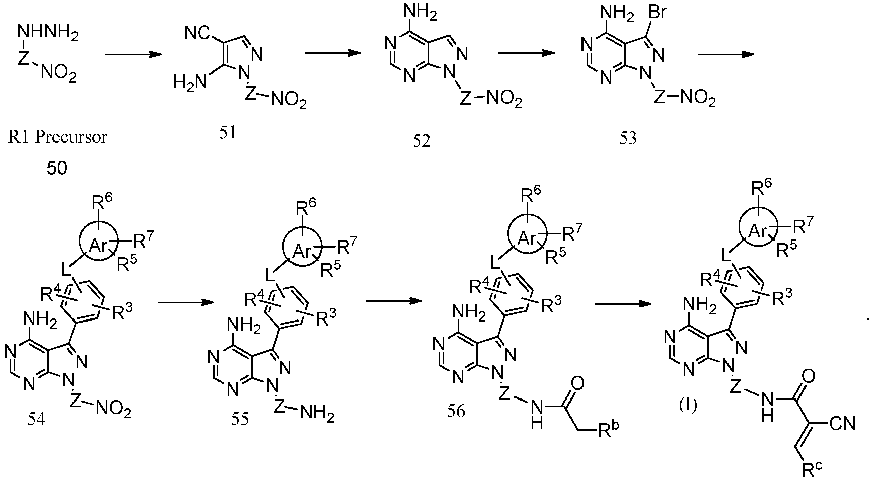

Treatment of a R¹ precursor containing hydrazines of formula 50 where Z is alkylene and R³-R⁷, L and Ar defined above with ethoxymethylene malonitrile as described in Scheme C provides a compound of formula 51 which is converted to a compound of formula 53 as described in Scheme C. Coupling of a bromo compound of formula 53 with a boronic acid compound or boronate esters thereof of formula 17 under Suzuki coupling reaction conditions as described in Scheme A provides a compound of formula 54. Reduction of nitro substituent of compound 54 may be accomplished by treatment with a reducing agent such as zinc powder and the like in a suitable solvent such as acetic acid and the like, or by catalytic hydrogenation to provide a compound of formula 55. Coupling of compounds of formula 55 with a compound of formula CNCH₂CO₂H such as 2-cyanoacetic acid or 2-trifluoromethylacetic acid, under standard amide coupling conditions such as carbon diimidazole (CDI) and the like or an acid derivative thereof provides a compound of formula 56. Subsequent condensation of a compound of formula 56 with aldehydes of formula R^{c}CHO where R^{c} is as defined above e.g., t-butyl or cyclopropyl aldehyde, under standard condensation reaction conditions such as using a base such as piperidine and the like, in the presence or absence of acetic acid and the like in solvents such as ethanol and the like at temperatures ranging from room temperature to reflux then provides a compound of Formula (IA). It will recognized by a person of ordinary skill in the art that the EWG' moiety can be assembled at multiple points throughout the synthetic scheme and standard protecting group (PG) strategies can be employed as required. Additionally, based on above synthetic teachings and the inforamation known in the art, a person of ordinary skill in the art can synthesize compounds of the present invention.

### Dosage

In general, the compounds of this invention will be administered in a therapeutically effective amount by any of the accepted modes of administration for agents that serve similar utilities. Therapeutically effective amounts of compounds of the present invention may range from about 0.01 to about 2000 mg per t per day, which can be administered in single or multiple doses. For example, the dosage level will be about 500 mg per day; further for example about 250-500 mg per day. For oral administration, the compositions are for example provided in the form of tablets containing about 1.0 to about 2000 milligrams of the active ingredient, particularly about 1.0, 5.0, 10, 15, 20, 25, 50, 75, 100, 150, 200, 250, 300, 400, 500, 600, 750, 800, 900, and 1000 milligrams of the active ingredient. The actual amount of the compound of this invention, i.e., the active ingredient, will depend upon numerous factors such as the severity of the disease to be treated, the age and relative health of the subject, the potency of the compound being utilized, the route and form of administration, and other factors.

The level of the compound in a formulation can vary within the full range employed by those skilled in the art. Typically, the formulation will contain, on a weight percent (wt %) basis, from about 0.01-99.99 wt % of a compound of the present invention based on the total formulation, with the balance being one or more suitable pharmaceutical excipients. For example, the compound is present at a level of about 1-80 wt %.

The compounds of the present invention may be used in combination with one or more other drugs in the treatment of diseases or conditions for which compounds of the present invention or the other drugs may have utility, where the combination of the drugs together are safer or more effective than either drug alone. Such other drug(s) may be administered, by a route and in an amount commonly used therefore, contemporaneously or sequentially with a compound of the present invention. When a compound of the present invention is used contemporaneously with one or more other drugs, a pharmaceutical composition in unit dosage form containing such other drugs and the compound of the present invention is preferred. However, the combination therapy may also include therapies in which the compound of the present invention and one or more other drugs are administered on different overlapping schedules. It is also contemplated that when used in combination with one or more other active ingredients, the compounds of the present invention and the other active ingredients may be used in lower doses than when each is used singly.

Accordingly, the pharmaceutical compositions of the present invention also include those that contain one or more other active ingredients, in addition to a compound of the present invention.

The above combinations include combinations of a compound of the present invention not only with one other active compound, but also with two or more other active compounds. Likewise, compounds of the present invention may be used in combination with other drugs that are used in the prevention, treatment, control, amelioration, or reduction of risk of the diseases or conditions for which compounds of the present invention are useful. Such other drugs may be administered, by a route and in an amount commonly used therefore, contemporaneously or sequentially with a compound of the present invention. When a compound of the present invention is used contemporaneously with one or more other drugs, a pharmaceutical composition containing such other drugs in addition to the compound of the present invention is preferred. Accordingly, the pharmaceutical compositions of the present invention also include those that also contain one or more other active ingredients, in addition to a compound of the present invention. The weight ratio of the compound of the present invention to the second active ingredient may be varied and will depend upon the effective dose of each ingredient. Generally, an effective dose of each will be used.

Where the subject is suffering from or at risk of suffering from an autoimmune disease, an inflammatory disease, or an allergy disease, a compound of the present invention can be used with one or more of the following therapeutic agents in any combination: immunosuppressants (e.g., tacrolimus, cyclosporin, rapamicin, methotrexate, cyclophosphamide, azathioprine, mercaptopurine, mycophenolate, or FTY720), glucocorticoids (e.g., prednisone, cortisone acetate, prednisolone, methylprednisolone, dexamethasone, betamethasone, triamcinolone, beclometasone, fludrocortisone acetate, deoxycorticosterone acetate, aldosterone), non-steroidal anti-inflammatory drugs (e.g., salicylates, arylalkanoic acids, 2-arylpropionic acids, N-arylanthranilic acids, oxicams, coxibs, or sulphonanilides), Cox-2-specific inhibitors (e.g., valdecoxib, celecoxib, or rofecoxib), leflunomide, gold thioglucose, gold thiomalate, aurofin, sulfasalazine, hydroxychloroquinine, minocycline, TNF-.alpha. binding proteins (e.g., infliximab, etanercept, or adalimumab), abatacept, anakinra, interferon-.beta., interferon-.gamma., interleukin-2, allergy vaccines, antihistamines, antileukotrienes, beta-agonists, theophylline, or anticholinergics.

Where the subject is suffering from or at risk of suffering from a B-cell proliferative disorder (e.g., plasma cell myeloma), the subject can be treated with a compound of the present invention in any combination with one or more other anti-cancer agents. In some embodiments, one or more of the anti-cancer agents are proapoptotic agents. Examples of anti-cancer agents include, but are not limited to, any of the following: gossyphol, genasense, polyphenol E, Chlorofusin, all trans-retinoic acid (ATRA), bryostatin, tumor necrosis factor-related apoptosis-inducing ligand (TRAIL), 5-aza-2'-deoxycytidine, all trans retinoic acid, doxorubicin, vincristine, etoposide, gemcitabine, imatinib (Gleevec™), geldanamycin, 17-N-Allylamino-17-Demethoxygeldanamycin (17-AAG), flavopiridol, LY294002, bortezomib, trastuzumab, BAY 11-7082, PKC412, or PD184352, Taxol™, also referred to as "paclitaxel", which is a well-known anti-cancer drug which acts by enhancing and stabilizing microtubule formation, and analogs of Taxol™., such as Taxotere™. Compounds that have the basic taxane skeleton as a common structure feature, have also been shown to have the ability to arrest cells in the G2-M phases due to stabilized microtubules and may be useful for treating cancer in combination with the compounds described herein.

Further examples of anti-cancer agents for use in combination with a compound of the present invention include inhibitors of mitogen-activated protein kinase signaling, e.g., U0126, PD98059, PD184352, PD0325901, ARRY-142886, SB239063, SP600125, BAY 43-9006, wortmannin, or LY294002; Syk inhibitors; mTOR inhibitors; and antibodies (e.g., rituxan).

Other anti-cancer agents that can be employed in combination with a compound of the present invention include Adriamycin, Dactinomycin, Bleomycin, Vinblastine, Cisplatin, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cladribine; crisnatol mesylate; cyclophosphamide; cytarabine; dacarbazine; daunorubicin hydrochloride; decitabine; dexormaplatin; dezaguanine; dezaguanine mesylate; diaziquone; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-nl; interferon alfa-n3; interferon beta-la; interferon gamma-1 b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other anti-cancer agents that can be employed in combination with a compound of the present invention include: 20-epi-1, 25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; 9-dioxamycin; diphenyl spiromustine; docosanol; dolasetron; doxifluridine; droloxifene; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylerie conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; R.sub.11 retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived 1; sense oligonucleotides; signal transduction inhibitors; signal transduction modulators; single chain antigen-binding protein; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

Yet other anticancer agents that can be employed in combination with a compound of the present invention include alkylating agents, antimetabolites, natural products, or hormones, e.g., nitrogen mustards (e.g., mechloroethamine, cyclophosphamide, chlorambucil, etc.), alkyl sulfonates (e.g., busulfan), nitrosoureas (e.g., carmustine, lomusitne, etc.), or triazenes (decarbazine, etc.). Examples of antimetabolites include but are not limited to folic acid analog (e.g., methotrexate), or pyrimidine analogs (e.g., Cytarabine), purine analogs (e.g., mercaptopurine, thioguanine, pentostatin).

Examples of natural products useful in combination with a compound of the present invention include but are not limited to vinca alkaloids (e.g., vinblastin, vincristine), epipodophyllotoxins (e.g., etoposide), antibiotics (e.g., daunorubicin, doxorubicin, bleomycin), enzymes (e.g., L-asparaginase), or biological response modifiers (e.g., interferon alpha).

Examples of alkylating agents that can be employed in combination with a compound of the present invention include, but are not limited to, nitrogen mustards (e.g., mechloroethamine, cyclophosphamide, chlorambucil, melphalan, etc.), ethylenimine and methylmelamines (e.g., hexamethlymelamine, thiotepa), alkyl sulfonates (e.g., busulfan), nitrosoureas (e.g., carmustine, lomusitne, semustine, streptozocin, etc.), or triazenes (decarbazine, etc.). Examples of antimetabolites include, but are not limited to folic acid analog (e.g., methotrexate), or pyrimidine analogs (e.g., fluorouracil, floxuridine, Cytarabine), purine analogs (e.g., mercaptopurine, thioguanine, pentostatin.

Examples of hormones and antagonists useful in combination with a compound the present invention include, but are not limited to, adrenocorticosteroids (e.g., prednisone), progestins (e.g., hydroxyprogesterone caproate, megestrol acetate, medroxyprogesterone acetate), estrogens (e.g., diethlystilbestrol, ethinyl estradiol), antiestrogen (e.g., tamoxifen), androgens (e.g., testosterone propionate, fluoxymesterone), antiandrogen (e.g., flutamide), gonadotropin releasing hormone analog (e.g., leuprolide). Other agents that can be used in the methods and compositions described herein for the treatment or prevention of cancer include platinum coordination complexes (e.g., cisplatin, carboblatin), anthracenedione (e.g., mitoxantrone), substituted urea (e.g., hydroxyurea), methyl hydrazine derivative (e.g., procarbazine), adrenocortical suppressant (e.g., mitotane, aminoglutethimide).

Examples of anti-cancer agents which act by arresting cells in the G2-M phases due to stabilized microtubules and which can be used in combination with an BTK inhibitor compound of the invention include without limitation the following marketed drugs and drugs in development: Erbulozole (also known as R-55104), Dolastatin 10 (also known as DLS-10 and NSC-376128), Mivobulin isethionate (also known as CI-980), Vincristine, NSC-639829, Discodermolide (also known as NVP-XX-A-296), ABT-751 (Abbott, also known as E-7010), Altorhyrtins (such as Altorhyrtin A and Altorhyrtin C), Spongistatins (such as Spongistatin 1, Spongistatin 2, Spongistatin 3, Spongistatin 4, Spongistatin 5, Spongistatin 6, Spongistatin 7, Spongistatin 8, and Spongistatin 9), Cemadotin hydrochloride (also known as LU-103793 and NSC-D-669356), Epothilones (such as Epothilone A, Epothilone B, Epothilone C (also known as desoxyepothilone A or dEpoA), Epothilone D (also referred to as KOS-862, dEpoB, and desoxyepothilone B), Epothilone E, Epothilone F, Epothilone B N-oxide, Epothilone A N-oxide, 16-aza-epothilone B, 21-aminoepothilone B (also known as BMS-310705), 21-hydroxyepothilone D (also known as Desoxyepothilone F and dEpoF), 26-fluoroepothilone), Auristatin PE (also known as NSC-654663), Soblidotin (also known as TZT-1027), LS-4559-P (Pharmacia, also known as LS-4577), LS-4578 (Pharmacia, also known as LS-477-P), LS-4477 (Pharmacia), LS-4559 (Pharmacia), RPR-112378 (Aventis), Vincristine sulfate, DZ-3358 (Daiichi), FR-182877 (Fujisawa, also known as WS-9885B), GS-164 (Takeda), GS-198 (Takeda), KAR-2 (Hungarian Academy of Sciences), BSF-223651 (BASF, also known as ILX-651 and LU-223651), SAH-49960 (Lilly/Novartis), SDZ-268970 (Lilly/Novartis), AM-97 (Armad/Kyowa Hakko), AM-132 (Armad), AM-138 (Armad/Kyowa Hakko), IDN-5005 (Indena), Cryptophycin 52 (also known as LY-355703), AC-7739 (Ajinomoto, also known as AVE-8063A and CS-39.HCl), AC-7700 (Ajinomoto, also known as AVE-8062, AVE-8062A, CS-39-L-Ser.HCl, and RPR-258062A), Vitilevuamide, Tubulysin A, Canadensol, Centaureidin (also known as NSC-106969), T-138067 (Tularik, also known as T-67, TL-138067 and TI-138067), COBRA-1 (Parker Hughes Institute, also known as DDE-261 and WHI-261), H10 (Kansas State University), H16 (Kansas State University), Oncocidin A1 (also known as BTO-956 and DIME), DDE-313 (Parker Hughes Institute), Fijianolide B. Laulimalide, SPA-2 (Parker Hughes Institute), SPA-1 (Parker Hughes Institute, also known as SPIKET-P), 3-IAABU (Cytoskeleton/Mt. Sinai School of Medicine, also known as MF-569), Narcosine (also known as NSC-5366), Nascapine, D-24851 (Asta Medica), A-105972 (Abbott), Hemiasterlin, 3-BAABU (Cytoskeleton/Mt. Sinai School of Medicine, also known as MF-191), TMPN (Arizona State University), Vanadocene acetylacetonate, T-138026 (Tularik), Monsatrol, Inanocine (also known as NSC-698666), 3-1AABE (Cytoskeleton/Mt. Sinai School of Medicine), A-204197 (Abbott), T-607 (Tuiarik, also known as T-900607), RPR-115781 (Aventis), Eleutherobins (such as Desmethyleleutherobin, Desaetyleleutherobin, Isoeleutherobin A, and Z-Eleutherobin), Caribaeoside, Caribaeolin, Halichondrin B, D-64131 (Asta Medica), D-68144 (Asta Medica), Diazonamide A, A-293620 (Abbott), NPI-2350 (Nereus), Taccalonolide A, TUB-245 (Aventis), A-259754 (Abbott), Diozostatin, (-)-Phenylahistin (also known as NSCL-96F037), D-68838 (Asta Medica), D-68836 (Asta Medica), Myoseverin B, D-43411 (Zentaris, also known as D-81862), A-289099 (Abbott), A-318315 (Abbott), HTI-286 (also known as SPA-110, trifluoroacetate salt) (Wyeth), D-82317 (Zentaris), D-82318 (Zentaris), SC-12983 (NCI), Resverastatin phosphate sodium, BPR-OY-007 (National Health Research Institutes), and SSR-250411 (Sanofi).

Where the subject is suffering from or at risk of suffering from a thromboembolic disorder (e.g., stroke), the subject can be treated with a compound of the present invention in any combination with one or more other anti-thromboembolic agents. Examples of anti-thromboembolic agents include, but are not limited any of the following: thrombolytic agents (e.g., alteplase anistreplase, streptokinase, urokinase, or tissue plasminogen activator), heparin, tinzaparin, warfarin, dabigatran (e.g., dabigatran etexilate), factor Xa inhibitors (e.g., fondaparinux, draparinux, rivaroxaban, DX-9065a, otamixaban, LY517717, or YM150), ticlopidine, clopidogrel, CS-747 (prasugrel, LY640315), ximelagatran, or BIBR 1048.

### Examples

The following preparations of compounds of Formula (IA) are given to enable those skilled in the art to more clearly understand and to practice the present invention. They should not be considered as limiting the scope of the invention, but merely as being illustrative and representative thereof. Those compounds which fall outside the scope of the present invention are labelled as "comparative examples". The 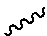 line at the alkene carbon, in the compounds below denotes that the compounds are isolated as an undefined mixture of (E) and (Z) isomers.

### Example 1

### Synthesis of (R)-2-(3-(4-amino-3-(4-phenoxyphenyl)-1H-pyrazolo[3,4-d]pyrimidin-1-yl)piperidine-1-carbonyl)-3 -cyclopropylacrylonitrile

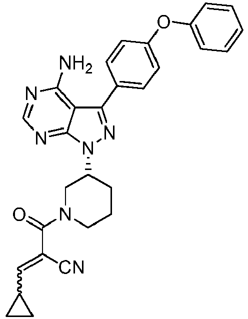

### Step 1

A solution of 5-amino-1H-pyrazole-4-carbonitrile (10 g, 92.51 mmol, 1.00 equiv) in formamide (80 mL) was stirred under nitrogen at 165°C for 5 h. The reaction mixture was cooled to room temperature and the solid was collected by filtration. The filter cake was washed first with 20 mL of water then 20 mL of methanol and dried to yield 9.5 g (76%) of 1H-pyrazolo[3,4-d]pyrimidin-4-amine as a white solid.

### Step 2

A mixture of 1H-pyrazolo[3,4-d]pyrimidin-4-amine (150 g, 1.11 mol, 1.00 equiv) and N-iodo-succinimide (375 g, 1.67 mol, 1.58 equiv) in N,N-dimethylformamide (2.5 L) was stirred at 80°C for 5 h. The reaction mixture was cooled to room temperature and then diluted with 10 L of water. The solid was collected by filtration, washed with 2x1L of saturated aqueous sodium sulfite and dried under vacuum to give 150 g (52%) of 3-iodo-1H-pyrazolo[3,4-d]pyrimidin-4-amine as a yellow solid.

### Step 3

To a stirred mixture of 3-iodo-1H-pyrazolo[3,4-d]pyrimidin-4-amine (5.9g, 22.6 mmol, 1.00 equiv), (S)-tert-butyl 3-hydroxypiperidine-1-carboxylate (10g, 50 mmol, 2.2 equiv) and triphenylphosphine (11.8g, 45 mmol, 2.0 equiv) in tetrahydrofuran (300 mL) at 10 °C was added a solution of diisopropyl azodicarboxylate in tetrahydrofuran (30 mL) dropwise in 30 min. The resulting mixture was stirred at room temperature for 12 h and then concentrated under vacuum. The residue was purified on a silica gel column eluted with dichloromethane/methanol (100/1) to give 3g (33%) of (R)-tert-butyl 3-(4-amino-3-iodo-1H-pyrazolo[3,4-d]pyrimidin-1-yl)piperidine-1-carboxylate as a yellow solid.

### Step 4

A mixture of (R)-tert-butyl 3-[4-amino-3-iodo-1H-pyrazolo[3,4-d]pyrimidin-1-yl]piperidine-1-carboxylate (1 g, 2.25 mmol, 1.00 equiv), (4-phenoxyphenyl)boronic acid (530 mg, 2.48 mmol, 1.10 equiv), sodium carbonate (480 mg, 4.53 mmol, 2.01 equiv) and tetrakis(triphenylphosphine)palladium (78 mg, 0.07 mmol, 0.03 equiv) in 1,4-dioxane (60 mL) and water (15 mL) was stirred under nitrogen at 90°C for 24 h. The reaction mixture was cooled to room temperature and then concentrated under vacuum. The residue was dissolved in 500 mL of dichloromethane. The resulting solution was washed with 200 mL of water, dried over anhydrous sodium sulfate and concentrated under vacuum. The residue was purified on a silica gel column eluted with dichloromethane/methanol (100/1) to give 700 mg (64%) of (R)-tert-butyl 3-[4-amino-3-(4-phenoxyphenyl)-1H-pyrazolo[3,4-d]pyrimidin-1-yl]piperidine-1-carboxylate as a yellow solid.

### Step 5

A mixture of (R)-tert-butyl 3-[4-amino-3-(4-phenoxyphenyl)-1H-pyrazolo[3,4-d]pyrimidin-1-yl]piperidine-1-carboxylate (700 mg, 1.44 mmol, 1.00 equiv) in dichloromethane (100 mL) and trifluoroacetic acid (20 mL) was stirred at room temperature for 12 h. The reaction mixture was concentrated under vacuum to yield 580 mg of crude (R)-3-(4-phenoxyphenyl)-1-(piperidin-3-yl)-1H-pyrazolo[3,4-d]pyrimidin-4-amine as a yellow oil.

### Step 6

A mixture of (R)-3-(4-phenoxyphenyl)-1-(piperidin-3-yl)-1H-pyrazolo[3,4-d]pyrimidin-4-amine (580 mg, 1.50 mmol, 1.00 equiv), carbonyldiimidazole (365 mg, 2.25 mmol, 1.50 equiv) and 2-cyanoacetic acid (190 mg, 2.24 mmol, 1.49 equiv) in dichloromethane (100 mL) was stirred at room temperature for 24 h. The reaction mixture was diluted with 100 mL of dichloromethane and washed with 3x100 mL of saturated aqueous ammonium chloride solution. The organic layer was dried over anhydrous sodium sulfate and concentrated under vacuum. The residue was purified on a silica gel column eluted with dichloromethane/ methanol (100 /1) to give 380 mg (56%) of (R)-3-[3-[4-amino-3-(4-phenoxyphenyl)-1H-pyrazolo[3,4-d]pyrimidin-1-yl]piperidin-1-yl]-3-oxopropanenitrile as a white solid.

### Step 7

A mixture of (R)-3-[3-[4-amino-3-(4-phenoxyphenyl)-1H-pyrazolo[3,4-d]pyrimidin-1-yl]piperidin-1-yl]-3-oxopropanenitrile (120 mg, 0.26 mmol, 1.00 equiv), piperidine (27 mg, 0.28 mmol, 1.07 equiv) and cyclopropanecarbaldehyde (28 mg, 0.40 mmol, 1.51 equiv) in methanol (8 mL) was stirred in sealed tube at room temperature for 24 h. The resulting mixture was concentrated under vacuum and the residue was purified on a silica gel column eluted with dichloromethane / methanol (100 /1) to give 85.4 mg (64%) of the title compound as a white solid. MS (ESI, pos. ion) m/z: 506 (M+1).

### Example 2

### Synthesis of 2-((R)-3-(4-amino-3-(4-(3,4-dichlorophenoxy)-3-methoxyphenyl)-1H-pyrazolo[3,4-d]pyrimidin-1-yl)piperidine-1-carbonyl)-3-cyclopropylacrylonitrile

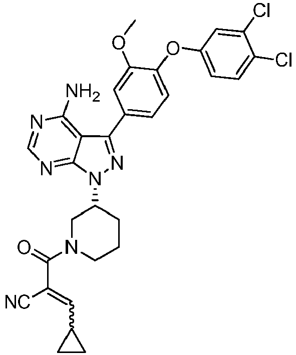

### Step 1

A mixture of 3,4-dichlorophenol (38 g, 233.13 mmol, 1.00 equiv), 1-fluoro-2-methoxy-4-nitrobenzene (40 g, 233.75 mmol, 1.00 equiv) and potassium carbonate (64 g, 463.77 mmol, 1.99 equiv) in N,N-dimethylformamide (250 mL) was stirred overnight at 60°C. The resulting solution was diluted with 1000 mL of water, extracted with 3 x 200 mL of ethyl acetate and the organic layers combined. The resulting mixture was washed with 3 x 500 mL of brine. The mixture was dried over anhydrous sodium sulfate and concentrated under vacuum to yield 60 g (82%) of 1,2-dichloro-4-(2-methoxy-4-nitrophenoxy)benzene as a brown solid.

### Step 2

A mixture of 1,2-dichloro-4-(2-methoxy-4-nitrophenoxy)benzene (60 g, 190.40 mmol, 1.00 equiv), Fe (53 g, 946.43 mmol, 4.97 equiv) and ammonium chloride (10 g, 188.68 mmol, 0.99 equiv) in tetrahydrofuran / water(1/2) (600 mL) was stirred overnight at 60°C under an inert atmosphere of nitrogen. The mixture was filtered through Celite and the filtrate was concentrated under vacuum. The resulting solution was extracted with 3 x 500 mL of dichloromethane and the organic layers combined. The resulting mixture was washed with 3 x 500 mL of brine. The mixture was dried over anhydrous magnesium sulfate and concentrated under vacuum to give 40 g (74%) of 4-(3,4-dichlorophenoxy)-3-methoxyaniline as a light gray solid.

### Step 3

A solution of sodium nitrite (14.4 g, 208.70 mmol, 1.98 equiv) in water (500 mL) was added dropwise into a solution of 4-(3,4-dichlorophenoxy)-3-methoxyaniline (30 g, 105.58 mmol, 1.00 equiv) in sulfuric acid (1000 mL) with stirring at 0°C and the mixture was stirred for 30 min at 0°C. The above mixture was added dropwise to a solution of potassium iodide (1000 mL, 5%) in water with stirring at 50°C. The reaction was completed immediately. The reaction mixture was cooled to room temperature, extracted with 3 x 500 mL of ethyl acetate and the organic layers combined. The resulting mixture was washed with 3 x 500 mL of saturated aqueous sodium bicarbonate and 3 x 500 mL of brine. The mixture was dried over anhydrous sodium sulfate and concentrated under vacuum to give 24 g (crude) of 1,2-dichloro-4-(4-iodo-2-methoxyphenoxy)benzene as red oil.

### Step 4

A mixture of 1,2-dichloro-4-(4-iodo-2-methoxyphenoxy)benzene (93 g, 235.43 mmol, 1.00 equiv) in 1,4-dioxane (500 mL), potassium acetate (46 g, 469.39 mmol, 1.99 equiv), 4,4,5,5-tetramethyl-2-(4,4,5,5-tetramethyl-1,3,2-dioxaborolan-2-yl)-1,3,2-dioxaborolane (89 g, 350.39 mmol, 1.49 equiv) and Pd(dppf)Cl₂ (4.65 g) was stirred overnight at 90°C under an inert atmosphere of nitrogen. The reaction mixture was cooled to room temperature and concentrated under vacuum. The residue was dissolved in 500 mL of ethyl acetate and washed with mL of water and brine. The mixture was dried over anhydrous sodium sulfate and concentrated under vacuum. The residue was applied onto a silica gel column with ethyl acetate / petroleum ether (1 / 100) to yield 10 g (11%) of 2-[4-(3,4-dichlorophenoxy)-3-methoxyphenyl]-4,4,5,5-tetramethyl-1,3,2-dioxaborolane as light yellow oil.

2-[4-(3,4-Dichlorophenoxy)-3-methoxyphenyl]-4,4,5,5-tetramethyl-1,3,2-dioxaborolane was then covered to the title compound following the procedures described in Example 1, steps 4-7 above.

### Example 3

### Synthesis of (R)-2-(3-(4-amino-3-(4-phenoxyphenyl)-1H-pyrazolo[3,4-d]pyrimidin-1-yl)piperidine-1-carbonyl)-4,4-dimethylpent-2-enenitrile

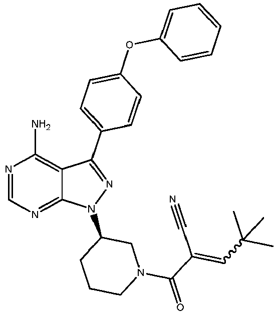

A mixture of (R)-3-[3-[4-amino-3-(4-phenoxyphenyl)-1H-pyrazolo[3,4-d]pyrimidin-1-yl]piperidin-1-yl]-3-oxopropanenitrile(150mg, 0.33 mmol, 1.00 equiv), methanol (15 mL), dichloromethane(5mL), piperidine (56mg, 0.66 mmol, 2 equiv) and pivalaldehyde (142 mg, 1.66 mmol, 5 equiv) was stirred for 48 h at 30 °C in a 25-mL sealed tube. The resulting mixture was concentrated under vacuum. The residue was applied onto a silica gel column with dichloromethane / methanol (100 / 1) to give 45mg (26%) of (R)-2-(3-(4-amino-3-(4-phenoxyphenyl)-1H-pyrazolo[3,4-d]pyrimidin-1-yl)piperidine-1-carbonyl)-4,4-dimethylpent-2-enenitrile as a white solid. MS (ESI, pos. ion) m/z: 522 (M+1).

### Example 4

### Synthesis of 2-(2-((4-amino-3-(4-phenoxyphenyl)-1H-pyrazolo[3,4-d]pyrimidin-1-yl)methyl)pyrrolidine-1-carbonyl)-4,4-dimethylpent-2-enenitrile

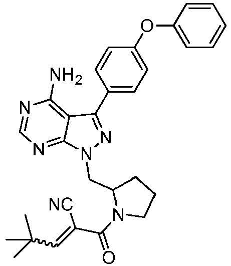

Synthesized as Examples 1 and 3 above but using tert-butyl 2-(hydroxymethyl)-pyrrolidine-1-carboxylate instead of (S)-tert-butyl-3-hydroxypiperidine-1-carboxylate. MS (ESI, pos. ion) m/z: 522 (M+1).

### Example 5 (Comparative Example)

### Synthesis of (N-((1r,4r)-4-(4-amino-3-(4-phenoxyphenyl)-1H-pyrazolo[3,4-d]pyrimidin-1-yl)cyclohexyl)-2-cyano-3-cyclopropylacrylamide

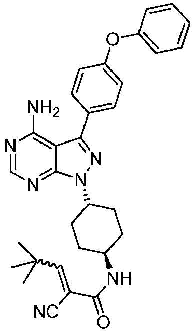

Synthesized as described in Examples 1 and 3 above except using tert-butyl(1r,4r)-4-hydroxycyclohexylcarbamate instead of (S)-tert-butyl3-hydroxypiperidine-1-carboxylate. MS (ESI, pos. ion) m/z: 536 (M+1).

### Example 6 (Comparative Example)

### Synthesis of (R)-N-(4-(4-amino-1-(1-(2-cyano-3-cyclopropylacryloyl)piperidin-3-yl)-1H-pyrazolo[3,4-d]pyrimidin-3-yl)phenyl)-4-(trifluoromethyl)benzamide

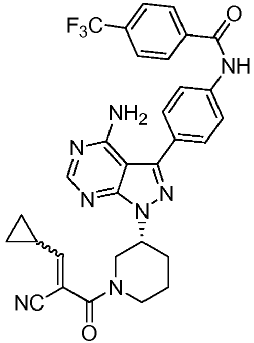

### Step 1

A mixture of3-iodo-1H-pyrazolo[3,4-d]pyrimidin-4-amine (5.9g, 22.6 mmol, 1.00 equiv), (S)-tert-butyl3-hydroxypiperidine-1-carboxylate (10g,50mmol, 2.2equiv), triphenylphosphine (11.8 g, 45 mmol, 2 equiv) in tetrahydrofuran (300 mL) was stirred at 10 °C. Diisopropyl azodicarboxylate in tetrahydrofuran (30 mL) was dropped in the mixture slowly in 30 min.The resulting mixture was stirred for 12 h at room temperature was and then concentrated under vacuum. The residue was loaded onto a silica gel column and eluted with dichloromethane/methanol (100/1) to give 3 g (33%) of (R)-tert-butyl 3-(4-amino-3-iodo-1H-pyrazolo[3,4-d]pyrimidin-1-yl)piperidine-1-carboxylate as yellow solid.

### Step 2

Into a 250-mL 3-necked round-bottom flask purged and maintained with an inert atmosphere of nitrogen, was placed (R)-tert-butyl 3-[4-amino-3-iodo-1H-pyrazolo[3,4-d]pyrimidin-1-yl]piperidine-1-carboxylate (2 g, 4.50 mmol, 1.00 equiv), 4-borono-benzenaminium chloride (0.934 g), Pd(PPh₃)₄ (0.312 g), ethylene glycol dimethyl ether (100 mL), sodium carbonate (1.194 g), and water(20 mL). The resulting solution was stirred for 12 h at 80 °C in an oil bath. The resulting mixture was concentrated under vacuum and residue was loaded onto a silica gel column and eluted with dichloromethane/methanol (50:1) to give 1.5 g (81%) of (R)-tert-butyl 3-(4-amino-3-(4-aminophenyl)-1H-pyrazolo[3,4-d]pyrimidin-1-yl)piperidine-1-carboxylate as a brown solid.

### Step 3

Into a 250-mL round-bottom flask, was placed (R)-tert-butyl 3-[4-amino-3-(4-aminophenyl)-1H-pyrazolo[3,4-d]pyrimidin-1-yl]piperidine-1-carboxylate (1.0 g, 2.44 mmol, 1.00 equiv), HATU (0.746 g), 4-(trifluoromethyl)benzoic acid (374 mg, 1.97 mmol, 0.81 equiv), triethylamine (500 mg, 4.94 mmol, 2.02 equiv), and N,N-dimethylformamide (50 mL). The resulting solution was stirred for 5 h at 25°C. The resulting mixture was quenched with water. The resulting solution was extracted with ethyl acetate and washed with sodium chloride (sat).The organic layers dried over anhydrous magnesium sulfate and concentrated under vacuum and residue was loaded onto a silica gel column and eluted with dichloromethane/methanol (50:1 to give 1.15 g (81%) of (R)-tert-butyl 3-[4-amino-3-(4-[[4-(trifluoromethyl)benzene]amido]phenyl)-1H-pyrazolo[3,4-d]pyrimidin-1-yl]piperidine-1-carboxylate as a brown solid.

### Step 4

Into a 250-mL round-bottom flask, was placed (R)-tert-butyl 3-[4-amino-3-(4-[[4-(trifluoromethyl)benzene]amido]phenyl)-1H-pyrazolo[3,4-d]pyrimidin-1-yl]piperidine-1-carboxylate (1.1 g, 1.89 mmol, 1.00 equiv), and dichloromethane (100 mL). This was followed by the addition of CF₃COOH (20 mL) dropwise with stirring at 25 °C over 10 min. The resulting solution was stirred for 3 h at 25 °C. The resulting mixture was concentrated under vacuum to give 0.829 g (91%) of (R)-N-[4-[4-amino-1-(piperidin-3-yl)-1H-pyrazolo[3,4-d]pyrimidin-3-yl]phenyl]-4-(trifluoromethyl)benzamide as brown oil.

### Step 5

Into a 250-mL round-bottom flask, was placed (R)-N-[4-[4-amino-1-(piperidin-4-yl)-1H-pyrazolo[3,4-d]pyrimidin-3-yl]phenyl]-4-(trifluoromethyl)benzamide (828 mg, 1.72 mmol, 1.00 equiv), 2-cyanoacetic acid (220 mg, 2.59 mmol, 1.50 equiv), CDI (420 mg, 2.59 mmol, 1.51 equiv), in dichloromethane (80 mL). The resulting solution was stirred for 12 h at 25 °C. The resulting mixture was washed with NH₄Cl and concentrated under vacuum. The residue was loaded onto a silica gel column and eluted with dichloromethane/methanol (50:1) to give 300 mg (32%) of (R)-N-(4-[4-amino-1-[1-(2-cyanoacetyl)piperidin-4-yl]-1H-pyrazolo[3,4-d]pyrimidin-3-yl]phenyl)-4-(trifluoromethyl)benzamide as a yellow solid.

### Step 6

Into a 10-mL round-bottom flask, was placed (R)-N-(4-[4-amino-1-[1-(2-cyanoacetyl)piperidin-3-yl]-1H-pyrazolo[3,4-d]pyrimidin-3-yl]phenyl)-4-(trifluoromethyl)-benzamide (65 mg, 0.12 mmol, 1.00 equiv), cyclopropanecarbaldehyde (16.6 mg, 0.24 mmol, 2.00 equiv), piperidine (10 mg, 0.12 mmol, 0.99 equiv), methanol (5 mL). The resulting solution was stirred for 12 h at 25 °C. The resulting mixture was concentrated under vacuum and the residue was loaded onto a silica gel column and eluted with dichloromethane/ methanol (50:1) to give 43 mg (60%) of (R)-N-[4-(4-amino-1-[1-[2-cyano-2-(cyclopropylmethylidene)acetyl]piperidin-3-yl]-1H-pyrazolo[3,4-d]pyrimidin-3-yl)phenyl]-4-(trifluoromethyl)benzamide as a white solid. MS (ESI, pos. ion) m/z: 601

### Example 7

### Synthesis of of (R)-2-(2-((4-amino-3-(4-(3-fluorophenoxy)phenyl)-1H-pyrazolo[3,4-d]pyrimidin-1-yl)methyl)pyrrolidine-1-carbonyl)-3-cyclopropylacrylonitrile

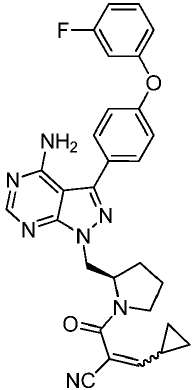

### Step 1

Into a 100-mL 3-necked round-bottom flask purged and maintained under an inert atmosphere of nitrogen, was placed tert-butyl (2R)-2-([4-amino-3-iodo-1H-pyrazolo[3,4-d]pyrimidin-1-yl]methyl)pyrrolidine-1-carboxylate (300 mg, 0.68 mmol, 1.00 equiv), prepared as described in Example 1 except in the Mitsunobu reaction using (R)-tert-butyl 2-(hydroxymethyl)pyrrolidine-1-carboxylate instead of (S)-tert-butyl 3-hydroxypiperidine-1-carboxylate, 2-[4-(3-fluorophenoxy)phenyl]-4,4,5,5-tetramethyl-1,3,2-dioxaborolane (254 mg, 0.81 mmol, 1.20 equiv), tetrakis(triphenylphosphane)palladium (47 mg, 0.04 mmol, 0.06 equiv), ethylene glycol dimethyl ether (50 mL), sodium carbonate (180 mg, 1.70 mmol, 2.50 equiv), and water(10 mL). The resulting solution was stirred for 12 h at 80 °C in an oil bath. The resulting mixture was concentrated under vacuum and extracted with dichloromethane. The organic layers were combined, dried and concentrated under vacuum. The residue was loaded on a silica gel column and eluted with dichloromethane/methanol (50/1) to give 0.27 g (79%) of tert-butyl (2R)-2-([4-amino-3-[4-(3-fluorophenoxy)phenyl]-1H-pyrazolo[3,4-d]-pyrimidin-1-yl]methyl)pyrrolidine-1-carboxylate as a brown solid.

### Step 2

Into a 100-mL round-bottom flask, was placed a solution of tert-butyl (2R)-2-([4-amino-3-[4-(3-fluorophenoxy)phenyl]-1H-pyrazolo[3,4-d]pyrimidin-1-yl]methyl)-pyrrolidine-1-carboxylate (270 mg, 0.54 mmol, 1.00 equiv) in dichloromethane (50 mL). This was followed by the addition of trifluoroacetic acid (10 mL) dropwise with stirring over 10 min. The resulting solution was stirred for 3 h at 25 °C. The resulting mixture was concentrated under vacuum to give 0.216 g (crude) of 3-[4-(3-fluorophenoxy)phenyl]-1-[(2R)-pyrrolidin-2-ylmethyl]-1H-pyrazolo[3,4-d]pyrimidin-4-amine as brown oil.

### Step 3

Into a 100-mL round-bottom flask, was placed 3-[4-(3-fluorophenoxy)phenyl]-1-[(2R)-pyrrolidin-2-ylmethyl]-1H-pyrazolo[3,4-d]pyrimidin-4-amine (216 mg, 0.53 mmol, 1.00 equiv), 2-cyanoacetic acid (36.8 mg, 0.43 mmol, 0.80 equiv), HATU (166 mg, 0.44 mmol, 0.80 equiv), triethylamine (109 mg, 1.08 mmol, 2.00 equiv), N,N-dimethylformamide (50 mL). The resulting solution was stirred for 3 h at 25 °C. The resulting solution was extracted with ethyl acetate and the organic layers combined and concentrated under vacuum. The residue was loaded on a silica gel column and eluted with dichloromethane methanol (50 1) to give 150 mg (60%) of 3-[(2R)-2-([4-amino-3-[4-(3-fluorophenoxy)phenyl]-1H-pyrazolo[3,4-d]pyrimidin-1-yl]methyl)pyrrolidin-1-yl]-3-oxopropanenitrile as a yellow solid.

### Step 4

Into a 10-mL round-bottom flask, was placed 3-[(2R)-2-([4-amino-3-[4-(3-fluorophenoxy)phenyl]-1H-pyrazolo[3,4-d]pyrimidin-1-yl]methyl)pyrrolidin-1-yl]-3-oxopropanenitrile (150 mg, 0.32 mmol, 1.00 equiv), piperidine (27 mg, 0.32 mmol, 1.00 equiv), cyclopropanecarbaldehyde (44.5 mg, 0.63 mmol, 2.00 equiv), methanol (5 mL). The resulting solution was stirred for 12 h at 25 °C and then concentrated under vacuum. The residue was loaded on a silica gel column and eluted with dichloromethane/methanol (50 /1) to give 48.5 mg (29%) of the title compound as a off-white solid. LC-MS: (ES, *m*/*z*): MS (ESI, pos. ion) m/z: 524 (M+1).

### Example 8

### Synthesis of (R)-2-(2-((4-amino-3-(4-(2,6-difluorophenoxy)phenyl)-1H-pyrazolo[3,4-d]-pyrimidin-1-yl)methyl)pyrrolidine-1-carbonyl)-3-cyclopropylacrylonitrile

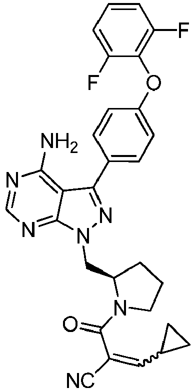

### Step 1

Into a 1L, 2-necked round-bottom flask purged and maintained under an inert atmosphere of nitrogen, was placed 3-iodo-1H-pyrazolo[3,4-d]pyrimidin-4-amine (10 g, 38.31 mmol, 1.00 equiv), tert-butyl (2R)-2-(hydroxymethyl)pyrrolidine-1-carboxylate (15.4 g, 76.52 mmol, 2.00 equiv), PPh₃ (20.1 g, 76.63 mmol, 2.00 equiv), and N,N-dimethylformamide (400 mL). DIAD (15.5 g, 76.65 mmol, 2.00 equiv) was added dropwise over 30 min. The resulting solution was stirred for 12 h at 25 °C and then diluted with 1 L of water. The resulting solution was extracted with ethyl acetate and the organic layers combined and washed with brine. The mixture was dried over anhydrous sodium sulfate and concentrated under vacuum and the residue was placed on a silica gel column and eluted with chloroform/methanol (100/1) to give 1.2 g (6%) of tert-butyl (2R)-2-([4-amino-3-iodo-1H-pyrazolo[3,4-d]pyrimidin-1-yl]methyl)pyrrolidine-1-carboxylate as a off-white solid.

### Step 2

Into a 500-mL 4-necked round-bottom flask, was placed a solution of sodium hydride (4.05 g, 168.75 mmol, 1.70 equiv) in N,N-dimethylformamide (200 mL). A solution of 1-fluoro-4-nitrobenzene (14 g, 99.22 mmol, 1.00 equiv) in N,N-dimethylformamide (50 mL) was added dropwise with stirring at 0 °C over 20 min. The resulting solution was stirred for 2 hr at room temperature. Cu₂Cl₂ (9.83 g, 100.31 mmol, 1.01 equiv) was added and a solution of 2,6-difluorophenol (15.5 g, 119.15 mmol, 1.20 equiv) in N,N-dimethylformamide (50 mL) was added dropwise with stirring at 25 °C over 10 min. The resulting solution was stirred for 12 h at 100 °C in an oil bath, diluted with 500 mL of water and extracted with ethyl acetate . The combined organic layers were washed with water and brine, dried over anhydrous sodium sulfate and concentrated under vacuum. The residue was placed on a silica gel column and eluted with ethyl acetate/petroleum ether (1/8) to give 20 g (80%) of 1,3-difluoro-2-(4-nitrophenoxy)benzene as brown oil.

### Step 3

Into a 500 mL, 3-necked round-bottom flask purged and maintained under an inert atmosphere of nitrogen, was placed a solution of 1,3-difluoro-2-(4-nitrophenoxy)benzene (20 g, 79.62 mmol, 1.00 equiv) in methanol (200 mL), Raney Nickel (2 g). A solution of hydrazine hydrate (12.67 g) in methanol (50 mL) was added dropwise with stirring in 15 min. The resulting solution was stirred for 12 h at 25 °C, then filtrated and the filtrate was concentrated under vacuum. The residue was diluted with f ethyl acetate, washed with water and brine, and dried over anhydrous sodium sulfate and concentrated under vacuum to give 16 g (91%) of 4-(2,6-difluorophenoxy)aniline as black oil.

### Step 4

Into a 250-mL 4-necked round-bottom flask, was placed 4-(2,6-difluorophenoxy)-aniline (8.84 g, 39.96 mmol, 1.00 equiv), hydrogen chloride (37%) (10.14 g, 277.81 mmol, 6.95 equiv) and water (20 mL). NaNO₂ (3.04 g, 44.06 mmol, 1.10 equiv) in water (10 mL) was added dropwise with stirring at 0 °C over 5 min., and the reaction mixture was stirred for 30 mins at 0 °C. The reaction mixture was added into a solution of NaI (18 g, 120.00 mmol, 3.00 equiv) in water (20 mL) at 25 °C in batches over 5 min. The resulting solution was stirred for 2 h at 25 °C and then extracted with of ethyl acetate and the organic layers were combined. The combined organic layers were washed with water and brine, dried over anhydrous sodium sulfate and concentrated under vacuum to give 10.2 g (77%) of 1,3-difluoro-2-(4-iodophenoxy)benzene as brown oil.

### Step 5

Into a 100 mL 3-necked round-bottom flask purged and maintained in an inert atmosphere of nitrogen, was placed a solution of 1,3-difluoro-2-(4-iodophenoxy)benzene (2 g, 6.02 mmol, 1.00 equiv) in N,N-dimethylformamide (50 mL), 4,4,5,5-tetramethyl-2-(tetramethyl-1,3,2-dioxaborolan-2-yl)-1,3,2-dioxaborolane (1.68 g, 6.62 mmol, 1.10 equiv), potassium acetate (1.76 g, 17.93 mmol, 3.0 equiv), and Pd(OAc)₂ (68 mg, 0.30 mmol, 0.05 equiv). The resulting solution was stirred for 12 h at 85 °C in an oil bath. The reaction mixture was then quenched with water. The resulting solution was extracted with ethyl acetate and the organic layers combined and washed with water and brine. The organics were dried over anhydrous sodium sulfate and concentrated under vacuum. The residue was loaded onto a silica gel column and eluted with ethyl acetate/petroleum ether (1/8) to give 1.5 g (75 %) of 2-[4-(2,6-difluorophenoxy)phenyl]-4,4,5,5-tetramethyl-1,3,2-dioxaborolane as a light yellow solid.

### Step 6

Into a 100 mL, 3-necked round-bottom flask purged and maintained in an inert atmosphere of nitrogen, was placed a solution of *tert*-butyl (2R)-2-([4-amino-3-iodo-1H-pyrazolo[3,4-d]pyrimidin-1-yl]methyl)pyrrolidine-1-carboxylate (300 mg, 0.68 mmol, 1.00 equiv) in 1,4-dioxane/water (60/15 mL), 2-[4-(2,6-difluorophenoxy)phenyl]-4,4,5,5-tetramethyl-1,3,2-dioxaborolane (290 mg, 0.87 mmol, 1.3 equiv), sodium carbonate (180 mg, 1.68 mmol, 2.5 equiv), and tetrakis(triphenylphosphane)palladium (40 mg, 0.03 mmol, 0.05 equiv). The resulting solution was stirred for 12 h at 90 °C in an oil bath and then concentrated under vacuum. The resulting solution was diluted with 50 mL of dichloromethane, washed with water and brine. The organics were dried over anhydrous sodium sulfate and concentrated under vacuum. The residue was loaded onto a silica gel column and eluted with dichloromethane/methanol (20/1) to give 280 mg (79%) of tert-butyl (2R)-2-([4-amino-3-[4-(2,6-difluorophenoxy)phenyl]-1H-pyrazolo[3,4-d]pyrimidin-1-yl]methyl)pyrrolidine-1-carboxylate as a white solid.

### Step 7

Into a 50 mL round-bottom flask, was placed a solution of tert-butyl (2R)-2-([4-amino-3-[4-(2,6-difluorophenoxy)phenyl]-1H-pyrazolo[3,4-d]pyrimidin-1-yl]methyl)-pyrrolidine-1-carboxylate (280 mg, 0.54 mmol, 1.00 equiv) in dichloromethane (10 mL). Trifluoroacetic acid (2 mg, 0.02 mmol, 0.03 equiv) was added dropwise with stirring at 25 °C. The resulting solution was stirred for 3 h at 25 °C and then concentrated under vacuum. The resulting solution was diluted with 50 mL of dichloromethane, washed with ethyl acetate and H₂O, brine and concentrated under vacuum to give 200 mg (88%) of 3-[4-(2,6-difluorophenoxy)phenyl]-1-[(2R)-pyrrolidin-2-ylmethyl]-1H-pyrazolo[3,4-d]pyrimidin-4-amine as a white solid.

### Step 8

Into a 100 mL round-bottom flask, was placed a solution of 3-[4-(2,6-difluoro-phenoxy)phenyl]-1-[(2R)-pyrrolidin-2-ylmethyl]-1H-pyrazolo[3,4-d]pyrimidin-4-amine (200 mg, 0.47 mmol, 1.00 equiv) in dichloromethane (10 mL), 2-cyanoacetic acid (121 mg, 1.42 mmol, 3.00 equiv), and 1,1-carbonyldiimidazole (230 mg, 1.42 mmol, 3.00 equiv). The resulting solution was stirred for 12 h at 25 °C and then washed with water and brine. The mixture was dried over anhydrous sodium sulfate and concentrated under vacuum. The residue was loaded onto a silica gel column and eluted with dichloromethane/methanol (50/1) to give 112 mg (48%) of 3-[(2R)-2-([4-amino-3-[4-(2,6-difluorophenoxy)phenyl]-1H-pyrazolo[3,4-d]pyrimidin-1-yl]methyl)pyrrolidin-1-yl]-3-oxopropanenitrile as a white solid.

### Step 9

Into a 10mL sealed tube, was placed a solution of 3-[(2R)-2-([4-amino-3-[4-(2,6-difluorophenoxy)phenyl]-1H-pyrazolo[3,4-d]pyrimidin-1-yl]methyl)pyrrolidin-1-yl]-3-oxopropanenitrile (100 mg, 0.20 mmol, 1.00 equiv) in methanol (3 mL), cyclopropanecarbaldehyde (1 mL), and piperidine (1 mL). The resulting solution was stirred for 12 h at 25 °C and then concentrated under vacuum. The resulting solution was diluted with 10 mL of dichloromethane, washed with saturated aqueous NH₄Cl, water and brine. The organics were dried over anhydrous sodium sulfate and concentrated under vacuum and the residue was purified via column chromatograpy using dichloromethane/methanol (20/1) to give 26 mg (23%) of the title compound as a white solid. LC-MS: (ES, *m*/*z*): 542 [M+H].

### Example 9

### Synthesis of (R)-2-(2-((4-amino-3-(2-fluoro-4-phenoxyphenyl)-1H-pyrazolo[3,4-d]-pyrimidin-1-yl)methyl)pyrrolidine-1-carbonyl)-3-cyclopropylacrylonitrile

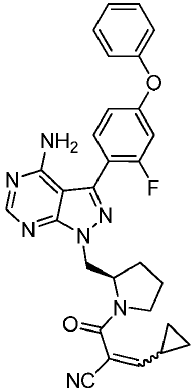

### Step 1

Into a 250 mL round-bottom flask, was placed a solution of 4-bromo-3-fluorophenol (5 g, 26.18 mmol, 1.00 equiv) in dichloromethane (100 mL), phenylboronic acid (3.5 g, 28.70 mmol, 1.10 equiv), Cu(AcO)₂ (5.7 g), triethylamine (5.3 g), and 4A molecular sieves (15 g). The resulting solution was stirred overnight at room temperature. The solids were filtered out. The filtrate was dried over anhydrous sodium sulfate and concentrated under vacuum. The residue was loaded onto a silica gel column and eluted with ethyl acetate/petroleum ether (1:100-1:50). This resulted in 2 g (29%) of 1-bromo-2-fluoro-4-phenoxybenzene as colorless oil.

### Step 2

Into a 100 mL 3-necked round-bottom flask purged and maintained under an inert atmosphere of nitrogen, was placed a solution of 1-bromo-2-fluoro-4-phenoxybenzene (2 g, 7.49 mmol, 1.00 equiv) in tetrahydrofuran (20 mL). BuLi (1M) (8 mL) was added dropwise with stirring at -70 to -80 °C. The resulting solution was stirred for 30 min at -70-80 °C in a liquid nitrogen bath. Tris(propan-2-yl)borate (1.7 g, 9.04 mmol, 1.21 equiv) was added dropwise with stirring at -70 to -80 °C. The resulting solution was allowed to react, with stirring, for an additional 2 h while the temperature was maintained at -70 to -80 °C. The reaction was then quenched by the addition of 100 mL of water, extracted with ethyl acetate and the organic layers were combined and dried over anhydrous sodium sulfate and concentrated under vacuum. The residue was loaded onto a silica gel column and eluted with ethyl acetate/petroleum ether (1:20) to give 1.6 g (92%) of (2-fluoro-4-phenoxyphenyl)-boronic acid as a white solid.

### Step 3

Into a 100 mL, 3-necked round-bottom flask purged and maintained under an inert atmosphere of nitrogen, was placed tert-butyl (2R)-2-([4-amino-3-iodo-1H-pyrazolo[3,4-d]pyrimidin-1-yl]methyl)pyrrolidine-1-carboxylate (380 mg, 0.86 mmol, 1.00 equiv), (2-fluoro-4-phenoxyphenyl)boronic acid (240 mg, 1.03 mmol, 1.20 equiv), tetrakis-(triphenylphosphane) palladium (60 mg, 0.05 mmol, 0.06 equiv), dioxane (50 mL), sodium carbonate (228 mg, 2.15 mmol, 2.50 equiv) and water (10 mL). The resulting solution was stirred for 12 h at 80 °C in an oil bath. The resulting mixture was concentrated under vacuum and the resulting solution was extracted with dichloromethane and the organic layers combined, dried and concentrated under vacuum. The residue was loaded onto a silica gel column and eluted with dichloromethane /methanol (50/1) to give 0.347 g (80%) of tert-butyl (2R)-2-[[4-amino-3-(2-fluoro-4-phenoxyphenyl)-1H-pyrazolo[3,4-d]pyrimidin-1-yl]methyl]pyrrolidine-1-carboxylate as a brown solid.

### Step 4

Into a 100 mL, round-bottom flask, was placed a solution of tert-butyl (2R)-2-[[4-amino-3-(2-fluoro-4-phenoxyphenyl)-1H-pyrazolo[3,4-d]pyrimidin-1-yl]methyl]pyrrolidine-1-carboxylate (347 mg, 0.69 mmol, 1.00 equiv) in dichloromethane (50 mL). Trifluoroacetic acid (10 mL) dropwise with stirring over 10 min and the resulting solution was stirred for 3 h at 25 °C. The resulting mixture was concentrated under vacuum to give 0.278 g (crude) of 3-(2-fluoro-4-phenoxyphenyl)-1-[(2R)-pyrrolidin-2-ylmethyl]-1H-pyrazolo[3,4-d]pyrimidin-4-amine as brown oil.

### Step 5

Into a 100 mL round-bottom flask, was placed 3-(2-fluoro-4-phenoxyphenyl)-1-[(2R)-pyrrolidin-2-ylmethyl]-1H-pyrazolo[3,4-d]pyrimidin-4-amine (278 mg, 0.69 mmol, 1.00 equiv), 2-cyanoacetic acid (36.8 mg, 0.43 mmol, 0.80 equiv), HATU (210 mg, 0.55 mmol, 0.80 equiv), triethylamine (109 mg, 1.08 mmol, 2.00 equiv), and N,N-dimethylformamide (50 mL). The resulting solution was stirred for 3 h at 25 °C, then diluted with 200 mL of water and extracted with ethyl acetate and the organic layers combined, dried and concentrated under vacuum. The residue was loaded onto a silica gel column and eluted with dichloromethane / methanol (50/1) to give 200 mg (62%) of 3-[(2R)-2-[[4-amino-3-(2-fluoro-4-phenoxyphenyl)-1H-pyrazolo[3,4-d]pyrimidin-1-yl]methyl]pyrrolidin-1-yl]-3-oxopropanenitrile as a yellow solid.

### Step 6

Into a 10 mL round-bottom flask, was placed 3-[(2R)-2-[[4-amino-3-(2-fluoro-4-phenoxyphenyl)-1H-pyrazolo[3,4-d]pyrimidin-1-yl]methyl]pyrrolidin-1-yl]-3-oxopropanenitrile (100 mg, 0.21 mmol, 1.00 equiv), piperidine (18 mg, 0.21 mmol, 1.00 equiv), cyclopropanecarbaldehyde (30 mg, 0.43 mmol, 2.00 equiv), and methanol (5 mL). The resulting solution was stirred for 12 h at 25 °C and then concentrated under vacuum. The residue was loaded onto a silica gel column and eluted with dichloromethane/methanol (50 / 1) to give 38 mg (33%) of the title compound as a off-white solid. LC-MS; (ES, *m*/*z*): MS (ESI, pos. ion) m/z: 524 (M+1).

### Example 10

### Synthesis of (R)-2-(2-((4-amino-3-(4-(3,5-difluorophenoxy)phenyl)-1H-pyrazolo[3,4-d]-pyrimidin-1-yl)methyl)pyrrolidine-1-carbonyl)-3-cyclopropylacrylonitrile

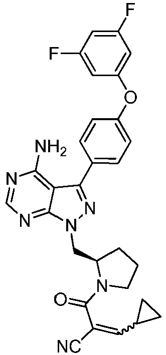

### Step 1

Into a 250-mL round-bottom flask, was placed a solution of 4-(tetramethyl-1,3,2-dioxaborolan-2-yl)phenol (5 g, 22.72 mmol, 1.00 equiv) in dichloromethane (100 mL), (3,5-difluorophenyl)boronic acid (4 g, 25.33 mmol, 1.11 equiv), Cu(AcO)₂ (5 g), 4A molecular sieves (15 g), triethylamine (4.6 g). The resulting solution was stirred for overnight at room temperature. The solids were filtered out. The filtrate was dried over anhydrous sodium sulfate and concentrated under vacuum. The residue was applied onto a silica gel column with ethyl acetate/petroleum ether (1:100-1:50). This resulted in 2 g (27%) of 2-[4-(3,5-difluorophenoxy)phenyl]-4,4,5,5-tetramethyl-1,3,2-dioxaborolane as a colorless oil.

### Step 2

Into a 100 mL, 3-necked round-bottom flask purged and maintained with an inert atmosphere of nitrogen, was placed tert-butyl (2R)-2-([4-amino-3-iodo-1H-pyrazolo[3,4-d]-pyrimidin-1-yl]methyl)pyrrolidine-1-carboxylate (250 mg, 0.56 mmol, 1.00 equiv), 2-[4-(3,5-difluorophenoxy)phenyl]-4,4,5,5-tetramethyl-1,3,2-dioxaborolane (226 mg, 0.68 mmol, 1.20 equiv), tetrakis(triphenylphosphane)palladium (39 mg, 0.03 mmol, 0.06 equiv), dioxane (50 mL), sodium carbonate (149 mg, 1.41 mmol, 2.50 equiv), and water (10 mL). The resulting solution was stirred for 12 h at 80 °C in an oil bath and then concentrated under vacuum. The resulting solution was extracted with dichloromethane and the organic layers combined, dried and concentrated under vacuum. The residue was loaded onto a silica gel column and eluted with dichloromethane/methanol (50/1) to give 0.237 g (81%) of tert-butyl (2R)-2-([4-amino-3-[4-(3,5-difluorophenoxy)phenyl]-1H-pyrazolo[3,4-d]pyrimidin-1-yl]methyl)pyrrolidine-1-carboxylate as a brown solid.

### Step 3

Into a 100 mL, round-bottom flask, was placed a solution of tert-butyl (2R)-2-([4-amino-3-[4-(3,5-difluorophenoxy)phenyl]-1H-pyrazolo[3,4-d]pyrimidin-1-yl]methyl)-pyrrolidine-1-carboxylate (230 mg, 0.44 mmol, 1.00 equiv) in dichloromethane (50 mL). This was followed by the addition of trifluoroacetic acid (10 mL) dropwise with stirring over 10 min. The resulting solution was stirred for 3 h at 25 °C. The resulting mixture was concentrated under vacuum to give 0.185 g (crude) of 3-[4-(3,5-difluorophenoxy)phenyl]-1-[(2R)-pyrrolidin-2-ylmethyl]-1H-pyrazolo[3,4-d]pyrimidin-4-amine as a brown oil.

### Step 4

Into a 100 mL, round-bottom flask, was placed 3-[4-(3,5-difluorophenoxy)phenyl]-1-[(2R)-pyrrolidin-2-ylmethyl]-1H-pyrazolo[3,4-d]pyrimidin-4-amine (185 mg, 0.44 mmol, 1.00 equiv), 2-cyanoacetic acid (30.7 mg, 0.36 mmol, 0.80 equiv), HATU (138 mg, 0.36 mmol, 0.80 equiv), triethylamine (91 mg, 0.90 mmol, 2.00 equiv), and N,N-dimethylformamide (50 mL). The resulting solution was stirred for 3 h at 25 °C and then extracted with ethyl acetate and the organic layers combined and concentrated under vacuum. The residue was loaded onto a silica gel column and eluted with dichloromethane/methanol (50/1) to give 101 mg (47%) of 3-[(2R)-2-([4-amino-3-[4-(3,5-difluorophenoxy)phenyl]-1H-pyrazolo[3,4-d]pyrimidin-1-yl]methyl)pyrrolidin-1-yl]-3-oxopropanenitrile as a off-white solid.

### Step 5

Into a 10-mL round-bottom flask, was placed cyclopropanecarbaldehyde (28.7 mg, 0.41 mmol, 2.00 equiv), piperidine (17.4 mg, 0.20 mmol, 1.00 equiv), 3-[(2R)-2-([4-amino-3-[4-(3,5-difluorophenoxy)phenyl]-1H-pyrazolo[3,4-d]pyrimidin-1-yl]methyl)pyrrolidin-1-yl]-3-oxopropanenitrile (100 mg, 0.20 mmol, 1.00 equiv), and methanol (5 mL). The resulting solution was stirred for 12 h at 25 °C and then concentrated under vacuum. The residue was loaded onto a silica gel column and eluted with dichloromethane/methanol (50 /1) to give 53.12 mg (45%) of the title compound as an off-white solid. LC-MS: (ES, m/z):MS (ESI, pos. ion) m/z: 541 (M+1).

### Example 11

### Synthesis of (R)-2-(3-(4-amino-3-(4-(2-fluorophenoxy)phenyl)-1H-pyrazolo[3,4-d]pyrimidin-1-yl)piperidine-1-carbonyl)-3-cyclopropylacrylonitrile

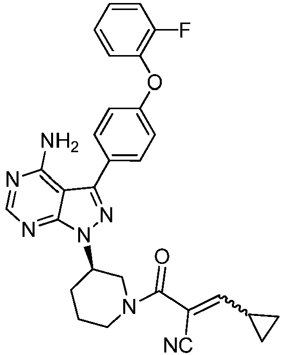

### Step 1

Into a 100 mL, 3-necked round-bottom flask purged and maintained with an inert atmosphere of nitrogen, was placed a solution of tert-butyl (3R)-3-[4-amino-3-iodo-1H-pyrazolo[3,4-d]pyrimidin-1-yl]piperidine-1-carboxylate (300 mg, 0.68 mmol, 1.00 equiv) in dioxane/H₂O(7/3=V/V) (30 mL), [4-(2-fluorophenoxy)phenyl]boronic acid (500 mg, 2.16 mmol, 6.99 equiv), sodium carbonate (200 mg, 1.89 mmol, 0.26 equiv), and Pd(PPh₃)₄ (500 mg, 0.43 mmol, 3.19 equiv). The resulting solution was stirred overnight at 100 °C in an oil bath an then concentrated under vacuum. The residue was loaded onto a silica gel column and eluted with dichloromethane/methanol (100:1) to give 0.2 g (59%) of tert-butyl (3R)-3-[4-amino-3-[4-(2-fluorophenoxy)phenyl]-1H-pyrazolo[3,4-d]pyrimidin-1-yl]piperidine-1-carboxylate as a light yellow solid.

### Step 2

Into a 100 mL, round-bottom flask purged and maintained under an inert atmosphere of nitrogen, was placed a solution of tert-butyl (3R)- 3-[4-amino-3-[4-(2-fluorophenoxy)-phenyl]-1H-pyrazolo[3,4-d]pyrimidin-1-yl]piperidine-1-carboxylate (200 mg, 0.40 mmol, 1.00 equiv) in dichloromethane (20 mL), and trifluoroacetic acid (10 g, 87.70 mmol, 221.25 equiv). The resulting solution was stirred overnight at room temperature and then concentrated under vacuum. The pH value of the solution was adjusted to 8-10 with 10% aqueous sodium carbonate. The solution was extracted with dichloromethane and the organic layers were combined and dried over anhydrous sodium sulfate and concentrated under vacuum to give 0.1 g (62%) of 3-[4-(2-fluorophenoxy)phenyl]-1-((3R)-piperidin-3-yl)-1H-pyrazolo[3,4-d]pyrimidin-4-amine as a light yellow solid.

### Step 3

Into a 50 mL round-bottom flask, was placed a solution of 3-[4-(2-fluorophenoxy)-phenyl]-1-[(3R)-piperidin-3-yl]-1H-pyrazolo[3,4-d]pyrimidin-4-amine (100 mg, 0.25 mmol, 1.00 equiv) in dichloromethane (10 mL), 1-[(1H-imidazol-1-yl)carbonyl]-1H-imidazole (60 mg, 0.37 mmol, 1.50 equiv), and 2-cyanoacetic acid (110 mg, 1.29 mmol, 5.23 equiv). The resulting solution was stirred for 60 min at room temperature and then concentrated under vacuum. The residue was loaded onto a silica gel column and eluted with dichloromethane /methanol (100:1) to give 0.06 g (51%) of 3-[(3R)-3-[4-amino-3-[4-(2-fluorophenoxy)-phenyl]-1H-pyrazolo[3,4-d]pyrimidin-1-yl]piperidin-1-yl]-3-oxopropanenitrile as a light yellow solid.

### Step 4

Into a 10 mL round-bottom flask, was placed a solution of 3-[(3R)-3-[4-amino-3-[4-(2-fluorophenoxy)phenyl]-1H-pyrazolo[3,4-d]pyrimidin-1-yl]piperidin-1-yl]-3-oxopropanenitrile (60 mg, 0.13 mmol, 1.00 equiv) in methanol (10 mL), cyclopropanecarbaldehyde (50 mg, 0.71 mmol, 5.61 equiv), and piperidine (70 mg, 0.82 mmol, 6.46 equiv). The resulting solution was stirred for 30 min at room temperature and then concentrated under vacuum. The residue was loaded onto a silica gel column and eluted with dichloromethane/methanol (100:1) to give 0.015 g (23%) of the title compound as an off-white solid. LC-MS0: (ES, m/z): 524 [M+H]⁺.

### Example 12

### Synthesis of (R)-2-(3-(4-amino-3-(4-(2,3-difluorophenoxy)phenyl)-1H-pyrazolo[3,4-d]pyrimidin-1-yl)piperidine-1-carbonyl)-3-cyclopropylacrylonitrile

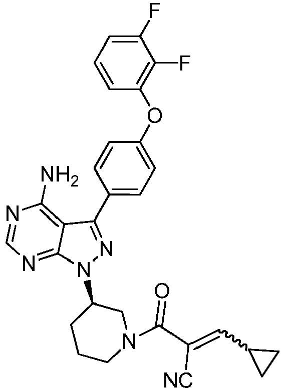

### Step 1

Into a 500 mL round-bottom flask, was placed a solution of (2,3-difluorophenyl)-boronic acid (30 g, 189.98 mmol, 1.00 equiv) in dichloromethane (250 mL). H₂O₂ (30 mL) was added dropwise with stirring. The resulting solution was stirred for 2 h at 25 °C. The resulting mixture was washed with water and brine, dried over anhydrous magnesium sulfate and concentrated under vacuum to give 23 g (93%) of 2,3-difluorophenol as brown oil.

### Step 2

Into a 500 mL, 4-necked round-bottom flask purged and maintained under an inert atmosphere of nitrogen, was placed a solution of sodium hydride (6.8 g, 170.00 mmol, 1.70 equiv, 60%) in N,N-dimethylformamide (200 mL). A solution of 1-fluoro-4-nitrobenzene (14.1 g, 99.93 mmol, 1.00 equiv) in N, N-dimethylformamide (50 mL) was added dropwise with stirring at 0 °C in 15 min. The resulting solution was stirred for 2 h at room temperature. CuCl (10 g, 101.01 mmol, 1.00 equiv) was added and a solution of 2,3-difluorophenol (15.6 g, 119.91 mmol, 1.20 equiv) in N,N-dimethylformamide (50 mL) was added dropwise with stirring. The resulting solution was allowed to react, with stirring, for an additional 12 h while the temperature was maintained at 100 °C in an oil bath. The resulting solution was extracted with ether and the organic layers combined. The organic layers was washed with water and brine, dried over anhydrous sodium sulfate and concentrated under vacuum. The residue was loaded onto a silica gel column and eluted with ethyl acetate/petroleum ether (1:8) to give 21.2 g (84%) of 1,2-difluoro-3-(4-nitrophenoxy)benzene as a brown solid.

### Step 3

Into a 500 mL, 3-necked round-bottom flask purged and maintained under an inert atmosphere of nitrogen, was placed a solution of 1,2-difluoro-3-(4-nitrophenoxy)benzene (21.2 g, 84.40 mmol, 1.00 equiv) in methanol (200 mL), and Raney Nickel (2 g). A solution of hydrazine hydrate (12.67 g, 3.00 equiv) in methanol (50 mL) was added dropwise with stirring in 15 min. The resulting solution was stirred for 12 h at 25 °C. The solids were filtered out and the filtrate was concentrated under vacuum. The residue was diluted with 200 mL of ethyl acetate and washed with water and brine, dried over anhydrous sodium sulfate and concentrated under vacuum to give 16.3 g (87%) of 4-(2,3-difluorophenoxy)aniline as black oil.

### Step 4

Into a 250-mL 4-necked round-bottom flask, was placed 4-(2,3-difluorophenoxy)-aniline (8.84 g, 39.96 mmol, 1.00 equiv), hydrogen chloride (10.14 g, 100.01 mmol, 2.50 equiv), and water (20 mL). A solution of NaNO₂ (3.04 g, 44.06 mmol, 1.10 equiv) in water (10 mL) was added dropwise with stirring in portions at 0 °C. The mixture was stirred at 0 °C for half an hour. To this was added urea (1 g, 16.65 mmol). The mixture was stirred at 0° C for 20 min and poured into the solution of NaI (18 g,120.00 mmol, 3.00 equiv) in water (20 mL) at room temperature. The resulting solution was stirred at room temperature for 1 h and then extracted with ethyl acetate. The organic layers combined and dried over anhydrous sodium sulfate and concentrated under vacuum to give 10.5 g (79%) of 1,2-difluoro-3-(4-iodophenoxy)benzene as brown oil.

### Step 5

Into a 100 mL, 3-necked round-bottom flask purged and maintained under an inert atmosphere of nitrogen, was placed a solution of 1,2-difluoro-3-(4-iodophenoxy)benzene (2 g, 6.02 mmol, 1.00 equiv) in N,N-dimethylformamide (50 mL), 4,4,5,5-tetramethyl-2-(tetramethyl-1,3,2-dioxaborolan-2-yl)-1,3,2-dioxaborolane (1.68 g, 6.62 mmol, 1.10 equiv), potassium acetate (68 mg, 0.69 mmol, 0.05 equiv), and Pd(OAc)₂(1.76 g, 7.84 mmol, 3.00 equiv). The resulting solution was stirred for 12 h at 85 °C in an oil bath. The reaction was then diluted with water, extracted with ethyl acetate and the organic layers were combined. The organics were washed with water and brine, dried over anhydrous sodium sulfate and concentrated under vacuum. The residue was loaded onto a silica gel column and eluted with ethyl acetate/petroleum ether (1/8) to give 1.5 g (75%) of 2-[4-(2,3-difluorophenoxy)phenyl]-4,4,5,5-tetramethyl-1,3,2-dioxaborolane as a light yellow solid.

### Step 6

Into a 500-mL round-bottom flask, was placed a solution of tert-butyl (3S)-3-hydroxypiperidine-1-carboxylate (10 g, 49.69 mmol, 1.00 equiv) in pyridine (200 mL). 4-Methylbenzene-1-sulfonyl chloride (28.5 g, 149.49 mmol, 3.0 equiv) was added dropwise with stirring at 0 °C in 30 min. The resulting solution was stirred for 5 h at 25 °C and then concentrated under vacuum. The residue was diluted with 200 mL of ethyl acetate. The pH value of the solution was adjusted to 3 with hydrogen chloride (1M) and the resulting mixture was washed with sodium bicarbonate and water. The organics were washed with brine, dried over anhydrous sodium sulfate and concentrated under vacuum to give 15 g (85%) of tert-butyl (3S)-3-[[(4-methylbenzene)sulfonyl]oxy]piperidine-1-carboxylate as a light yellow solid.

### Step 7

Into a 1000 mL 3-necked round-bottom flask, was placed a solution of 3-iodo-1H-pyrazolo[3,4-d]pyrimidin-4-amine (6 g, 22.99 mmol, 1.00 equiv) in N,N-dimethylformamide (500 mL), tert-butyl (3S)-3-[[(4-methylbenzene)sulfonyl]oxy]piperidine-1-carboxylate (9.8 g, 27.57 mmol, 1.20 equiv), and cesium carbonate (13.3 g, 40.82 mmol, 1.78 equiv). The resulting solution was stirred for 12 h at 60 °C in an oil bath and then quenched by the addition of 1500 mL of water. The resulting solution was extracted with dichloromethane and the organic layers combined. The organics were washed with brine, dried over anhydrous sodium sulfate and concentrated under vacuum. The residue was loaded onto a silica gel column and elution with ethyl acetate/petroleum ether (60%) gave 2.8 g (27%) of tert-butyl (3R)-3-[4-amino-3-iodo-1H-pyrazolo[3,4-d]pyrimidin-1-yl]piperidine-1-carboxylate (96.5%,e.e.) as a off-white solid.

### Step 8

Into a 250 mL 3-necked round-bottom flask purged and maintained under an inert atmosphere of nitrogen, was placed a solution of tert-butyl (3R)-3-[4-amino-3-iodo-1H-pyrazolo[3,4-d]pyrimidin-1-yl]piperidine-1-carboxylate (500 mg, 1.13 mmol, 1.00 equiv) in 1,4-dioxane/H₂O (100/30 mL), 2-[4-(2,3-difluorophenoxy)phenyl]-4,4,5,5-tetramethyl-1,3,2-dioxaborolane (420 mg, 1.26 mmol, 1.1 equiv), sodium carbonate (240 mg, 2.26 mmol, 2.0 equiv), and Pd(PPh₃)₄ (65 mg, 0.06 mmol, 0.05 equiv). The resulting solution was stirred for 12 h at 90 °C in an oil bath. The resulting mixture was concentrated under vacuum and the resiude was diluted with water. The resulting solution was extracted with dichloromethane and the organic layers were combined, washed with brine and filtered. The filtrate was dried over anhydrous sodium sulfate and concentrated under vacuum. The residue was loaded onto a silica gel column and elution with dichloromethane/methanol (10/1) gave 480 mg (82%) of tert-butyl (3R)-3-[4-amino-3-[4-(2,3-difluorophenoxy)phenyl]-1H-pyrazolo[3,4-d]pyrimidin-1-yl]piperidine-1-carboxylate as a white solid.

### Step 9

Into a 100 mL round-bottom flask, was placed a solution of tert-butyl (3R)-3-[4-amino-3-[4-(2,3-difluorophenoxy)phenyl]-1H-pyrazolo[3,4-d]pyrimidin-1-yl]piperidine-1-carboxylate (450 mg, 0.86 mmol, 1.00 equiv) in dichloromethane (40 mL) and CF₃COOH (10 mL). The resulting solution was stirred for 3 h at 25 °C and then concentrated under vacuum. The resulting solution was diluted with 50 mL of dichloromethane and washed with aqueous sodium bicarbonate and brine. The organics were dried over anhydrous sodium sulfate and concentrated under vacuum to give 400 mg (crude) of 3-[4-(2,3-difluorophenoxy)phenyl]-1-[(3R)-piperidin-3-yl]-1H-pyrazolo[3,4-d]pyrimidin-4-amine as a white solid.

### Step 10

Into a 100 mL round-bottom flask, was placed a solution of 3-[4-(2,3-difluorophenoxy)-phenyl]-1-[(3R)-piperidin-3-yl]-1H-pyrazolo[3,4-d]pyrimidin-4-amine (300 mg, 0.71 mmol, 1.00 equiv) in dichloromethane (30 mL), HATU (400 mg, 1.05 mmol, 1.5 equiv), triethylamine (220 mg, 2.17 mmol, 3.0 equiv), and 2-cyanoacetic acid (90 mg, 1.06 mmol, 1.5 equiv). The resulting solution was stirred for 10 h at 25 °C and then washed with water and brine. The organics were dried over anhydrous sodium sulfate and concentrated. The residue was loaded onto a silica gel column and elution with dichloromethane/methanol (10/1) gave 240 mg (69%) of 3-[(3R)-3-[4-amino-3-[4-(2,3-difluorophenoxy)phenyl]-1H-pyrazolo[3,4-d]pyrimidin-1-yl]piperidin-1-yl]-3-oxopropanenitrile as a white solid.

### Step 11

Into a 10 mL sealed tube, was placed a solution of 3-[(3R)-3-[4-amino-3-[4-(2,3-difluorophenoxy)phenyl]-1H-pyrazolo[3,4-d]pyrimidin-1-yl]piperidin-1-yl]-3-oxopropanenitrile (150 mg, 0.31 mmol, 1.00 equiv) in methanol (5 mL), cyclopropanecarbaldehyde (64 mg, 0.91 mmol, 3.0 equiv), and piperidine (78 mg). The resulting solution was stirred for 12 h at 25 °C and then concentrated under vacuum. The residue was diluted with 10 mL of dichloromethane and the resulting mixture was washed with saturated aqueous ammonium chloride, water and brine and dried over anhydrous sodium sulfate and concentrated. The residue was loaded onto a silica gel column and elution with dichloromethane/methanol (20/1) gave 28.5 mg (17%) of the title compound as a white solid.
LC-MS: (ES, *m*/*z*): 542 [M+H]⁺.

### Example 13

### Synthesis of (R)-2-(3-(4-amino-3-(4-(2,6-difluorophenoxy)phenyl)-1H-pyrazolo[3,4-d]pyrimidin-1-yl)piperidine-1-carbonyl)-3-cyclopropylacrylonitrile

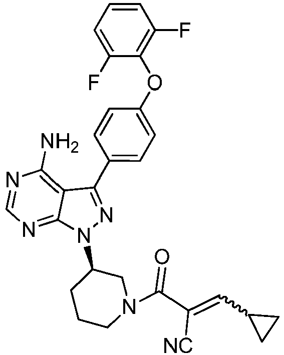

### Step 1

Into a 250 mL 3-necked round-bottom flask purged and maintained under an inert atmosphere of nitrogen, was placed a solution of tert-butyl (3R)-3-[4-amino-3-iodo-1H-pyrazolo[3,4-d]pyrimidin-1-yl]piperidine-1-carboxylate (500 mg, 1.13 mmol, 1.00 equiv) in 1,4-dioxane/H₂O (100/30 mL), 2-[4-(2,6-difluorophenoxy)phenyl]-4,4,5,5-tetramethyl-1,3,2-dioxaborolane (420 mg, 1.26 mmol, 1.1 equiv), sodium carbonate (240 mg, 2.26 mmol, 2.0 equiv), and Pd(PPh₃)₄ (65 mg, 0.06 mmol, 0.05 equiv). The resulting solution was stirred for 15 h at 90 °C in an oil bath and then concentrated under vacuum. The residue was diluted with water and extracted with dichloromethane and the organic layers combined. The organics were washed with brine, dried over anhydrous sodium sulfate and concentrated. The residue was loaded onto a silica gel column and eluted with dichloromethane/methanol (10/1) to give 500 mg (85%) of tert-butyl (3R)-3-[4-amino-3-[4-(2,6-difluorophenoxy)-phenyl]-1H-pyrazolo[3,4-d]pyrimidin-1-yl]piperidine-1-carboxylate as a white solid.

### Step 2

Into a 100 mL round-bottom flask, was placed a solution of tert-butyl (3R)-3-[4-amino-3-[4-(2,6-difluorophenoxy)phenyl]-1H-pyrazolo[3,4-d]pyrimidin-1-yl]piperidine-1-carboxylate (450 mg, 0.86 mmol, 1.00 equiv) in dichloromethane (40 mL). CF₃COOH (10 mL) to added dropwise with stirring at 25 °C over 10 min and the resulting solution was stirred for 3 h at 25 °C and then concentrated under vacuum. The resulting solution was diluted with dichloromethane and washed with aqueous sodium bicarbonate and brine. The organics were dried over anhydrous sodium sulfate and concentrated under vacuum to give 410 mg of 3-[4-(2,6-difluorophenoxy)phenyl]-1-[(3R)-piperidin-3-yl]-1H-pyrazolo[3,4-d]pyrimidin-4-amine as a white solid.

### Step 3

Into a 100 mL round-bottom flask, was placed a solution of 3-[4-(2,6-difluoro-phenoxy)phenyl]-1-[(3R)-piperidin-3-yl]-1H-pyrazolo[3,4-d]pyrimidin-4-amine (300 mg, 0.71 mmol, 1.00 equiv) in dichloromethane (30 mL), triethylamine (220 mg, 2.17 mmol, 3.0 equiv), HATU (400 mg, 1.05 mmol, 1.5 equiv), and 2-cyanoacetic acid (90 mg, 1.06 mmol, 1.5 equiv). The resulting solution was stirred for 10 h at 25 °C, thenwashed with water and brine. The organics were dried over anhydrous sodium sulfate and concentrated. The residue was loaded onto a silica gel column and eluted with dichloromethane/methanol (10/1) to give. 230 mg (60%) of 3-[(3R)-3-[4-amino-3-[4-(2,6-difluorophenoxy)phenyl]-1H-pyrazolo[3,4-d]pyrimidin-1-yl]piperidin-1-yl]-3-oxopropanenitrile as a white solid.

### Step 4

Into a 10 mL sealed tube, was placed a solution of 3-[(3R)-3-[4-amino-3-[4-(2,6-difluorophenoxy)phenyl]-1H-pyrazolo[3,4-d]pyrimidin-1-yl]piperidin-1-yl]-3-oxopropanenitrile (150 mg, 0.31 mmol, 1.00 equiv) in methanol (5 mL), piperidine (78 mg, 0.92 mmol, 3.0 equiv), and cyclopropanecarbaldehyde (64 mg, 0.91 mmol, 3.0 equiv). The resulting solution was stirred for 12 h at 25 °C and then concentrated under vacuum. The residue was diluted with 10 mL of dichloromethane, ans the solution was washed with saturated aqueous ammonium chloride, water and brine. The organics were dried over anhydrous sodium sulfate and concentrated. The residue was loaded onto a silica gel column and eluted with dichloromethane/methanol (20/1) to give 36 mg (21%) of 2-[[(3R)-3-[4-amino-3-[4-(2,6-difluorophenoxy)phenyl]-1H-pyrazolo[3,4-d]pyrimidin-1-yl]piperidin-1-yl]carbonyl]-3-cyclopropylprop-2-enenitrile as a white solid.
LC-MS: (ES, m/z): 542 [M+H].

### Example 14

### Synthesis of (R)-2-(3-(4-amino-3-(4-(2,5-difluorophenoxy)phenyl)-1H-pyrazolo[3,4-d]-pyrimidin-1-yl)piperidine-1-carbonyl)-3-cyclopropylacrylonitrile

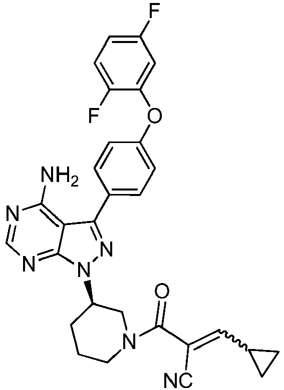

### Step 1

Into a 500 mL 3-necked round-bottom flask, was placed a solution of sodium hydride (3.9 g, 162.50 mmol, 1.7 equiv) in N,N-dimethylformamide (200 mL). This was followed by the addition of a solution of 1-fluoro-4-nitrobenzene (13.6 g, 96.39 mmol, 1.00 equiv) in N,N-dimethylformamide (50 mL) dropwise with stirring at 0 °C over 20 min. The reaction mixture was stirred for 2 hr at 25 °C and then CuCl (9.6 g, 96.97 mmol, 1.0 equiv) was added, followed by addition of a solution of 2,5-difluorophenol (15.5 g, 119.15 mmol, 1.2 equiv) in N,N-dimethylformamide (50 mL) dropwise with stirring at 25°C over 10 min. The resulting solution was stirred for 12 h at 100 °C in an oil bath and then diluted with water and washed with ether, water and brine. The reaction mixture was dried over anhydrous sodium sulfate and concentrated. The residue was loaded onto a silica gel column and eluted with ethyl acetate/petroleum ether (1/8) to give 19.5 g (81%) of 1,4-difluoro-2-(4-nitrophenoxy)-benzene as a brown solid.

### Step 2

Into a 500 mL 3-necked round-bottom flask purged and maintained under an inert atmosphere of nitrogen, was placed a solution of 1,4-difluoro-2-(4-nitrophenoxy)benzene (19.5 g, 77.63 mmol, 1.00 equiv) in methanol (200 mL), and Raney Nickel (2 g). This was followed by the addition of a solution of hydrazine hydrate (11.66 g) in methanol (50 mL) dropwise with stirring at 25 °C over 15 min. The resulting solution was stirred for 12 h at 25°C and then filtrated and the filtrate was concentrated under vacuum. The residue was diluted with ethyl acetate, washed with water and brine, dried over anhydrous sodium sulfate and concentrated under vacuum to give 16 g (93%) of 4-(2,5-difluorophenoxy)aniline as black oil.

### Step 3

Into a 250 mL 4-necked round-bottom flask, was placed 4-(2,5-difluorophenoxy)-aniline (9 g, 40.69 mmol, 1.00 equiv), hydrogen chloride (37%) (10.2 g, 100 mmol, 2.5 equiv) and water (20 mL). A solution of NaNO₂ (3.1 g, 44.93 mmol, 1.10 equiv) in water (10 mL) was added dropwise with stirring at 0 °C over 5 min. After stirring at 0 °C for 30 min., the mixture was added into a solution of NaI (18 g, 120.00 mmol, 3.0 equiv) in water (20 mL) dropwise with stirring at 25 °C . The resulting solution was stirred for 12 h at 25 °C and then extracted with ethyl acetate and the organic layers combined. The combined organics were washed with water and brine, dried over anhydrous sodium sulfate and concentrated under vacuum to give 10.5 g (78%) of 1,4-difluoro-2-(4-iodophenoxy)benzene as brown oil.

### Step 4

Into a 100 mL 3-necked round-bottom flask purged and maintained under an inert atmosphere of nitrogen, was placed a solution of 1,4-difluoro-2-(4-iodophenoxy)benzene (2 g, 6.02 mmol, 1.00 equiv) in N,N-dimethylformamide (50 mL), 4,4,5,5-tetramethyl-2-(tetramethyl-1,3,2-dioxaborolan-2-yl)-1,3,2-dioxaborolane (1.68 g, 6.62 mmol, 1.10 equiv), potassium acetate (1.76 g, 17.93 mmol, 3.0 equiv), and Pd(OAc)₂ (68 mg, 0.30 mmol, 0.05 equiv). The resulting solution was stirred for 12 h at 85 °C in an oil bath and then diluted with water. The resulting solution was extracted with ethyl acetate and the organic layers combined. The combined organics were washed with water and brine and dried over anhydrous sodium sulfate and concentrated. The residue was loaded onto a silica gel column and eluted wihtwith ethyl acetate/petroleum ether (1/8) to give 1.5 g (75%) of 2-[4-(2,5-difluorophenoxy)phenyl]-4,4,5,5-tetramethyl-1,3,2-dioxaborolane as a light yellow solid.

### Step 5

Into a 250-mL 3-necked round-bottom flask purged and maintained under an inert atmosphere of nitrogen, was placed a solution of tert-butyl (3R)-3-[4-amino-3-iodo-1H-pyrazolo[3,4-d]pyrimidin-1-yl]piperidine-1-carboxylate (500 mg, 1.13 mmol, 1.00 equiv) in 1,4-dioxane/H₂O (100/30 mL), 2-[4-(2,5-difluorophenoxy)phenyl]-4,4,5,5-tetramethyl-1,3,2-dioxaborolane (420 mg, 1.26 mmol, 1.1 equiv), sodium carbonate (240 mg, 2.26 mmol, 2.0 equiv), and Pd(PPh₃)₄ (65 mg, 0.06 mmol, 0.05 equiv). The resulting solution was stirred for 12 h at 90 °C in an oil bath and then concentrated under vacuum. The residue was diluted with water and the resulting solution was extracted with dichloromethane and the organic layers were combined. The combined organics were washed with brine, dried over anhydrous sodium sulfate and concentrated. The residue was loaded onto a silica gel and eluted with dichloromethane/methanol (10/1) to give 510 mg (87%) of tert-butyl (3R)-3-[4-amino-3-[4-(2,5-difluorophenoxy)phenyl]-1H-pyrazolo[3,4-d]pyrimidin-1-yl]piperidine-1-carboxylate as a white solid.

### Step 6

Into a 100 mL round-bottom flask, was placed a solution of tert-butyl (3R)-3-[4-amino-3-[4-(2,5-difluorophenoxy)phenyl]-1H-pyrazolo[3,4-d]pyrimidin-1-yl]piperidine-1-carboxylate (450 mg, 0.86 mmol, 1.00 equiv) in dichloromethane (40 mL). This was followed by the addition of CF₃COOH (10 mL) dropwise with stirring at 25°C over 5 min. The resulting solution was stirred for 3 h at 25 °C and then concentrated under vacuum. The residue was diluted with dichloromethane and the resulting mixture was washed with aqueous sodium bicarbonate and brine and dried over anhydrous sodium sulfate and concentrated under vacuum to give 400 mg (99%) of 3-[4-(2,5-difluorophenoxy)phenyl]-1-[(3R)-piperidin-3-yl]-1H-pyrazolo[3,4-d]pyrimidin-4-amine as a white solid.

### Step 7

Into a 100 mL round-bottom flask, was placed a solution of 3-[4-(2,5-difluoro-phenoxy)phenyl]-1-[(3R)-piperidin-3-yl]-1H-pyrazolo[3,4-d]pyrimidin-4-amine (300 mg, 0.71 mmol, 1.00 equiv) in dichloromethane (30 mL), HATU (400 mg, 1.05 mmol, 1.5 equiv), triethylamine (220 mg, 2.17 mmol, 3.0 equiv), and 2-cyanoacetic acid (90 mg, 1.06 mmol, 1.5 equiv). The resulting solution was stirred for 10 h at 25 °C and then washed with water and brine and dried over anhydrous sodium sulfate and concentrated. The residue was loaded onto a silica gel column and eluted with dichloromethane/methanol (10/1) to give 200 mg (58%) of 3-[(3R)-3-[4-amino-3-[4-(2,5-difluorophenoxy)phenyl]-1H-pyrazolo[3,4-d]pyrimidin-1-yl]piperidin-1-yl]-3-oxopropanenitrile as a white solid.

### Step 8

Into a 10 mL sealed tube, was placed a solution of 3-[(3R)-3-[4-amino-3-[4-(2,5-difluorophenoxy)phenyl]-1H-pyrazolo[3,4-d]pyrimidin-1-yl]piperidin-1-yl]-3-oxopropanenitrile (150 mg, 0.31 mmol, 1.00 equiv) in methanol (5 mL), piperidine (78 mg, 0.92 mmol, 3.0 equiv), and cyclopropanecarbaldehyde (64 mg, 0.91 mmol, 3.0 equiv). The resulting solution was stirred for 12 h at 25 °C and then concentrated under vacuum. The resulting solution was diluted with dichloromethane and washed with saturated aqueous ammonium chloride, water and brine. The organics were dried over anhydrous sodium sulfate and concentrated. The residue was loaded onto a silica gel column and eluted with dichloromethane/methanol (20/1) to give 38 mg (23%) of 2-[[(3R)-3-[4-amino-3-[4-(2,5-difluorophenoxy)phenyl]-1H-pyrazolo[3,4-d]pyrimidin-1-yl]piperidin-1-yl]carbonyl]-3-cyclopropylprop-2-enenitrile as a white solid. LC-MS (ES, *m*/*z*): 542 [M+H],

### Example 15

### Synthesis of (R)-2-(3-(4-amino-3-(2-fluoro-4-(phenoxy)phenyl)-1H-pyrazolo[3,4-d]-pyrimidin-1-yl)piperidine-1-carbonyl)-3-cyclopropylacrylonitrile

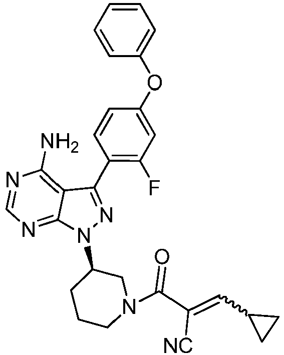

### Step 1

Into a 100 mL 3-necked round-bottom flask purged and maintained under an inert atmosphere of nitrogen, was placed tert-butyl (3R)-3-[4-amino-3-iodo-1H-pyrazolo[3,4-d]pyrimidin-1-yl]piperidine-1-carboxylate (400 mg, 0.90 mmol, 1.00 equiv), (2-fluoro-4-phenoxyphenyl)boronic acid (250 mg, 1.08 mmol, 1.20 equiv), sodium carbonate (190 mg, 1.79 mmol, 1.99 equiv), ethylene glycol dimethyl ether (50 mL), water (15 mL), and Pd(PPh₃)₄ (52 mg, 0.04 mmol, 0.05 equiv). The resulting solution was stirred for 12 h at 80 °C and then concentrated under vacuum. The residue was loaded onto a silica gel column and eluted with dichloromethane/methanol (100/1) to give 320 mg (70%) of tert-butyl (3R)-3-[4-amino-3-(2-fluoro-4-phenoxyphenyl)-1H-pyrazolo[3,4-d]pyrimidin-1-yl]piperidine-1-carboxylate as a yellow solid.

### Step 2

Into a 100 mL round-bottom flask, was placed a solution of tert-butyl (3R)-3-[4-amino-3-(2-fluoro-4-phenoxyphenyl)-1H-pyrazolo[3,4-d]pyrimidin-1-yl]piperidine-1-carboxylate (320 mg, 0.63 mmol, 1.00 equiv) in dichloromethane (50 mL). This was followed by the addition of trifluoroacetic acid (10 mL) dropwise with stirring. The resulting solution was stirred overnight at room temperature and then concentrated under vacuum. The residue was diluted with water and the pH value of the solution was adjusted to >7 with sodium carbonate. The resulting solution was extracted with dichloromethane and the organic layers combined and dried over anhydrous sodium sulfate and concentrated under vacuum to give 190 mg (74%) of 3-(2-fluoro-4-phenoxyphenyl)-1-[(3R)-piperidin-3-yl]-1H-pyrazolo[3,4-d]pyrimidin-4-amine as a brown solid.

### Step 3

Into a 100 mL round-bottom flask, was placed a solution of 3-(2-fluoro-4-phenoxyphenyl)-1-[(3R)-piperidin-3-yl]-1H-pyrazolo[3,4-d]pyrimidin-4-amine (190 mg, 0.47 mmol, 1.00 equiv) in dichloromethane (50 mL), 2-cyanoacetic acid (60 mg, 0.71 mmol, 1.50 equiv), and 1-[(1H-imidazol-1-yl)carbonyl]-1H-imidazole (114 mg, 0.70 mmol, 1.50 equiv). The resulting solution was stirred for 24 h at room temperature and then diluted with dichloromethane. The resulting mixture was washed with NH₄Cl and dried over anhydrous sodium sulfate and concentrated under vacuum. The residue was loaded onto a silica gel column and eluted with dichloromethane/methanol (100:1) to give 100 mg (45%) of 3-[(3R)-3-[4-amino-3-(2-fluoro-4-phenoxyphenyl)-1H-pyrazolo[3,4-d]pyrimidin-1-yl]piperidin-1-yl]-3-oxopropanenitrile as a white solid.

### Step 4

Into a 50 mL round-bottom flask, was placed a solution of 3-[(3R)-3-[4-amino-3-(2-fluoro-4-phenoxyphenyl)-1H-pyrazolo[3,4-d]pyrimidin-1-yl]piperidin-1-yl]-3-oxopropanenitrile (100 mg, 0.21 mmol, 1.00 equiv) in methanol (20 mL), cyclopropanecarbaldehyde (45 mg, 0.64 mmol, 3.00 equiv), piperidine (9 mg, 0.11 mmol, 0.50 equiv), and dichloromethane (5 mL). The resulting solution was stirred for 12 h at room temperature and then concentrated under vacuum. The residue was loaded onto a silica gel column and eluted with dichloromethane/methanol (100:1) to give 24 mg (24%) of the title compound as a white solid.

Proceeding as described above, but substituting cyclopropanecarbaldehyde with acetaldehyde, (R)-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile was synthesized.

### Example 16

### Synthesis of (S)-4-amino-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile bis(2,2,2-trifluoroacetate

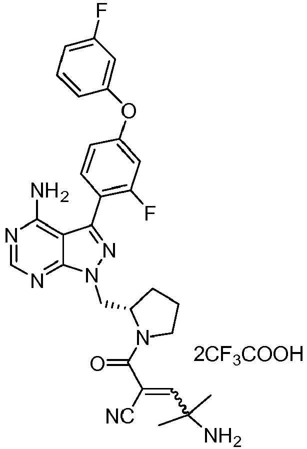

### Step 1

To a solution of 3-((S)-2-((4-amino-3-(2-fluoro-4-(3-fluorophenoxy)phenyl)-1H-pyrazolo[3,4-d]pyrimidin-1-yl)methyl)pyrrolidin-1-yl)-3-oxopropanenitrile (245 mg, 0.5 mmol, 1 equiv) and tert-butyl 2-methyl-1-oxopropan-2-ylcarbamate (935 mg, 5 mmol, 10 equiv) in dioxane (30 mL) was added 0.5 mL piperidine, 1 drop AcOH and 2 g of 4A molecular sieves. The resulting mixture was stirred for 6 h at 110 °C. The solids was filtered out, the filtrate was diluted with 200 mL of ethyl acetate, washed with brine, dried over Na₂SO₄, concentrated and purified with silica gel column (ethyl acetate/MeOH 10/1) to give 60 mg of tert-butyl 5-((S)-2-((4-amino-3-(2-fluoro-4-(3-fluorophenoxy)phenyl)-1H-pyrazolo[3,4-d]pyrimidin-1-yl)methyl)pyrrolidin-1-yl)-4-cyano-2-methyl-5-oxopent-3-en-2-ylcarbamate as white solid.

### Step2

To a solution of tert-butyl 5-((S)-2-((4-amino-3-(2-fluoro-4-(3-fluorophenoxy)phenyl) -1H-pyrazolo[3,4-d]pyrimidin-1-yl)methyl)pyrrolidin-1-yl)-4-cyano-2-methyl-5-oxopent-3-en-2-ylcarbamate (60 mg, 0.091 mmol) in DCM (20 mL) was added CF₃COOH (5 mL). The mixture was stirred for 2 h at room temperature and then concentrated and purified on Prep-HPLC. Conditions: (1#-Pre-HPLC-001(SHIMADZU)): Column, SunFire Prep C18, 19*150mm 5um; mobile phase, water with 0.05%TFA and CH₃CN (40% CH₃CN up to 100% in 20 min); Detector, 254 nm to give 12 mg of the title compound as light yellow solid.
LC-MS: *m*/*z* 559 (M+ H⁺).

### Example 17

### Synthesis of 2-((S)-2-((4-amino-3-(2-fluoro-4-phenoxyphenyl)-1H-pyrazolo[3,4-d]pyrimidin-1-yl)methyl)pyrrolidine-1-carbonyl)-4-methyl-4-(methylamino)pent-2-enenitrile bis (2,2,2-trifluoroacetate

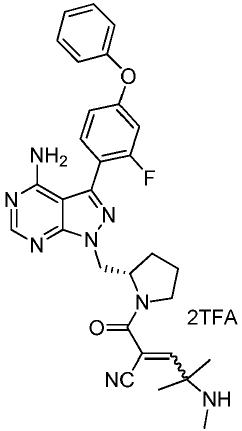

### Step 1

To a solution of 3-((S)-2-((4-amino-3-(2-fluoro-4-phenoxyphenyl)-1H-pyrazolo[3,4-d]-pyrimidin-1-yl)methyl)pyrrolidin-1-yl)-3-oxopropanenitrile (236 mg, 0.5 mmol, 1 equiv) and tert-butyl methyl(2-methyl-1-oxopropan-2-yl)carbamate (2.01 g, 10 mmol, 20 equiv) in dioxane (30 mL) was added 0.5 mL piperidine, 1 drop AcOH and 2 g of 4A molecular sieves. The resulting mixture was stirred for 6 h at 110 °C. The solids was filtered out, the filtrate was diluted with 50 mL of EA, washed with brine, dried over Na₂SO₄, concentrated. The residue was purified on silica gel column (EA to EA/MeOH 10/1) to give 60 mg of tert-butyl 5-((S)-2-((4-amino-3-(2-fluoro-4-phenoxyphenyl)-1H-pyrazolo[3,4-d]pyrimidin-1-yl)-methyl)pyrrolidin-1-yl)-4-cyano-2-methyl-5-oxopent-3-en-2-yl(methyl)carbamate as white solid.

### Step 2

To a solution of tert-butyl 5-((S)-2-((4-amino-3-(2-fluoro-4-phenoxyphenyl)-1H-pyrazolo[3 ,4-d]pyrimidin-1-yl)methyl)pyrrolidin-1-yl)-4-cyano-2-methyl-5-oxopent-3-en-2-yl(methyl)carbamate (60 mg, 0.092 mmol) in DCM (5 mL) was added 1.5 mL of CF₃COOH. The mixture was stirred for 2 h at room temperature, concentrated and the residue was purified on Prep-HPLC. Conditions: (1#-Pre-HPLC-001(SHIMADZU)): Column, SunFire Prep C18, 19*150mm 5um; mobile phase, water with 0.05%TFA and CH₃CN (40% CH₃CN up to 100% in 20 min); Detector, 254 nm to give 12 mg of the title compound salt as a white solid. LC-MS: *m*/*z* 555 (M+ H⁺).

### Example 18 (Comparative Example)

### Synthesis of (S)-2-(2-((4-amino-3-(2-fluoro-4-phenoxyphenyl)-1H-pyrazolο[3,4-d]pyrimidin-1-yl)methyl)-4,4-difluoropyrrolidine-1-carbonyl)-3-cyclopropylacrylonitrile trifluoroacetic acid salt

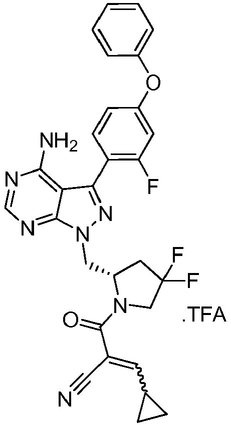

### Step 1

Into a solution of 1-tert-butyl 2-methyl (2S)-4, 4-difluoropyrrolidine-1, 2-dicarboxylate (900 mg, 3.39 mmol, 1.00 equiv) in tetrahydrofuran (15 mL) was added LiBH₄ (200 mg, 9.1 mmol, 2.7 equiv) in batches at 0° C. The resulting solution was stirred overnight at room temperature, then was diluted with EA and washed with water and brine, dried over anhydrous sodium sulfate and concentrated to give 0.8 g of tert-butyl (2S)-4, 4-difluoro-2-(hydroxymethyl) pyrrolidine-1-carboxylate as reddish oil.

### Step 2

Under nitrogen, to a solution of 3-iodo-10H-pyrazolo[3,4-d]pyrimidin-4-amine (2.61 g, 10.00 mmol, 1.00 equiv), tert-butyl (2S)-4,4-difluoro-2-(hydroxymethyl)pyrrolidine-1-carboxylate (2.37 g, 9.99 mmol, 1.00 equiv) and TPP (4 g, 15.2 mmol, 1.50 equiv) in THF was DIAD (3.00g, 15.0 mmol, 1.50 equiv) at 0° C in 30 min. The resulting solution was stirred overnight at room temperature. The mixture was then concentrated under vacuum and the residue was applied onto a silica gel column with dichloromethane/ethyl acetate (3/1) to give 1 g of tert-butyl (2S)-2-([4-amino-3-iodo-1H-pyrazolo[3,4-d]-pyrimidin-1-yl]methyl)-4,4-difluoropyrrolidine-1-carboxylate as reddish oil.

### Step 3

Under nitrogen atmosphere, a suspension of tert-butyl (2S)-2-([4-amino-3-iodo-1H-pyrazolo[3,4-d]pyrimidin-1-yl]methyl)-4,4-difluoropyrrolidine-1-carboxylate (800 mg, 1.67 mmol, 1.00 equiv), (2-fluoro-5-phenoxyphenyl)boronic acid (480 mg, 2.07 mmol, 1.20 equiv), Pd(dppf)Cl₂ (140 mg, 0.17 mmol, 0.10 equiv), sodium carbonate (0.53 g, 5.00 mmol, 3.00 equiv) in 1,4-dioxane/water (40/10 mL) was stirred at 80° C overnight. The resulting mixture was concentrated under vacuum. The residue was loaded on a silica gel column and eluted with ethyl acetate/petroleum ether (1:2 to 3:1) to give 0.6 g (67%) of tert-butyl (2S)-2-[[4-amino-3-(2-fluoro-4-phenoxyphenyl)-1H-pyrazolo[3,4-d]pyrimidin-1-yl]methyl]-4,4-difluoropyrrolidine-1-carboxylate as a reddish solid.

### Step 4

To a solution of tert-butyl (2S)-2-[[4-amino-3-(2-fluoro-4-phenoxyphenyl)-1H-pyrazolo[3,4-d]pyrimidin-1-yl]methyl]-4,4-difluoropyrrolidine-1-carboxylate (600 mg, 1.11 mmol, 1.00 equiv) in dichloromethane (10 mL) was added trifluoroacetic acid (2 mL) dropwise. The resulting solution was stirred at room temperature for 2h. The mixture was concentrated under vacuum to give 0.85 g (crude) of 1-[[(2S)-4,4-difluoropyrrolidin-2-yl]methyl]-3-(2-fluoro-4-phenoxyphenyl)-1H-pyrazolo[3,4-d]pyrimidin-4-amine trifluoroacetic acid salt as a brown semi-solid.

### Step5

To a solution of 1-[[(2S)-4,4-difluoropyrrolidin-2-yl]methyl]-3-(2-fluoro-4-phenoxyphenyl)-1H-pyrazolo[3,4-d]pyrimidin-4-amine (850 mg, crude), 2-cyanoacetic acid (120 mg, 1.31 mmol, 1.29 equiv) and TEA (650 mg, 6.45 mmol, 5.00 equiv) in dichloromethane (30 mL), was added HATU (500 mg, 1.32 mmol, 1.29 equiv). The resulting solution was stirred at room temperature overnight. The mixture was diluted with DCM, washed with HCl (2N), sat. NaHCO₃, brine, dried over sodium sulfate and concentrated. The residue was submitted to chromatography (SiO₂, DCM: MeOH = 30:1) to give 0.4 g (77%) of 3-[(2S)-2-[[4-amino-3-(2-fluoro-4-phenoxyphenyl)-1H-pyrazolo[3,4-d]pyrimidin-1-yl]methyl]-4,4-difluoropyrrolidin-1-yl]-3-oxopropanenitrile as a pale yellow solid.

### Step 6

A solution of 3-[(2S)-2-[[4-amino-3-(2-fluoro-4-phenoxyphenyl)-1H-pyrazolo [3,4-d]pyrimidin-1-yl]methyl]-4,4-difluoropyrrolidin-1-yl]-3-oxopropanenitrile (120 mg, 0.24 mmol, 1.00 equiv), cyclopropanecarbaldehyde (80 mg, 1.14 mmol, 5.00 equiv), piperidine (41 mg, 0.48 mmol, 2.00 equiv) in ethanol (10 mL) was stirred at 70° C for 2h. The resulting mixture was concentrated under vacuum. The residue was purified on Prep-HPLC. Conditions: (1#-Pre-HPLC-001(SHIMADZU)): Column, SunFire Prep C18, 19^{∗}150mm 5um; mobile phase, water with 0.05%TFA and CH₃CN (40% CH₃CN up to 100% in 20 min); Detector, 254 nm, to give 24 mg (18%) of the title compound as a white solid.
LC-MS m/z: 560 (M+1).

### Example 19

### Synthesis of (R)-2-(3-(4-amino-5-(4-phenoxyphenyl)-7H-pyrrolo[2,3-d]pyrimidin-7-yl)piperidine-1-carbonyl)-3-cyclopropylacrylonitrile

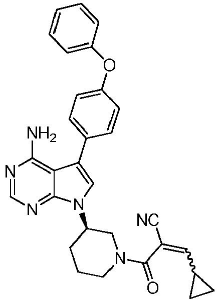

### Step 1

To the solution of 4-chloro-7H-pyrrolo[2,3-d]pyrimidine (10 g, 65.12 mmol, 1.0 eq) and (S)-tert-butyl 3-hydroxypiperidine-1-carboxylate (13.0 g, 65.12 mmol, 1.0 eq) and PPh₃ (34.20 g, 130.24 mmol, 2.0 eq) in THF (400 mL), DEAD (22.68 g, 130.24 mmol, 2.0 eq) was added at 0 °C. The resulted mixture was stirred and warmed to RT for 12 h. The reaction mixture was purified by column (10 % EtOAc in petroleum ether) to afford (R)-tert-butyl 3-(4-chloro-7H-pyrrolo[2,3-d]pyrimidin-7-yl)piperidine-1-carboxylate (2.1 g, 10 % in yield) as colorless oil.

### Step 2

A mixture of (R)-tert-butyl 3-(4-chloro-7H-pyrrolo[2,3-d]pyrimidin-7-yl)piperidine-1-carboxylate (1.7 g , 5.05 mmol) and NIS (1.25 g, 5.55 mmol) in DMF (20 mL) was stirred for 12 h at room temperature. Water was added to the mixture, which was extracted with EA , the combined organic layers were dried and purified by column to give (R)-tert-butyl 3-(4-chloro-5-iodo-7H-pyrrolo[2,3-d]pyrimidin-7-yl)piperidine-1-carboxylate (2.0 g, 86 % in yield).

### Step 3

A solution of (R)-tert-butyl 3-(4-chloro-5-iodo-7H-pyrrolo[2,3-d]pyrimidin-7-yl) piperidine-1-carboxylate (2.0 g, 4.32 mmol) in IPA saturated with NH₃ (20 mL) was stirred at 100° C for 12 h in a 100 mL of autoclave. The organic layer was concentrated and purified on silica gel chromatography (eluted with PE : EtOAc = 1 : 1) to afford (R)-tert-butyl 3-(4-amino-5-iodo-7H-pyrrolo[2,3-d]pyrimidin-7-yl)piperidine-1-carboxylate (1.5 g, 78 % in yield).

### Step 4

A mixture of (R)-tert-butyl 3-(4-amino-5-iodo-7H-pyrrolo[2,3-d]pyrimidin-7-yl)piperidine-1-carboxylate (250 mg, 0.56 mmol), 4-phenoxyphenylboronic acid (133 mg, 0.62 mmol), Pd(PPh₃)₄ (100 mg) and Na₂CO₃ (150 mg, 1.41mmol) in dioxane/H₂O(40/10ml) was stirred at 100° C for 4 h. The reaction mixture was concentrated and purified by Pre-TLC to obtain (R)-tert-butyl 3-(4-amino-5-(4-phenoxyphenyl)-7H-pyrrolo[2,3-d]pyrimidin-7-yl)piperidine-1-carboxylate (150 mg, 55 % in yield).

### Step 5

To a mixture of (R)-tert-butyl 3-(4-amino-5-(4-phenoxyphenyl)-7H-pyrrolo[2,3-d]pyrimidin-7-yl)piperidine-1-carboxylate (150 mg, 0.31 mmol) in 10 ml DCM was added TFA (10 ml). The reaction mixture was stirred at RT for 2 h. Solvent was removed and sat. NaHCO₃ (10 mL) was added. The resulting mixture was extracted with DCM. The organic layer was dried and concentrated to afford (R)-5-(4-phenoxyphenyl)-7-(piperidin-3-yl)-7H-pyrrolo[2,3-d]pyrimidin-4-amine (100 mg, 83 % in yield), which was subjected to the next step without any further purification.

### Step 6

To a mixture of (R)-5-(4-phenoxyphenyl)-7-(piperidin-3-yl)-7H-pyrrolo[2,3-d]pyrimidin-4-amine (100 mg, 0.26 mmol, 1.0 eq), 2-cyano-3-cyclopropylacrylic acid (45 mg, 0.32 mmol, 1.2 eq) and DIPEA (102 mg, 0.78 mmol, 3 eq) in 10 mL DCM was added HATU (150 mg, 0.40 mmol, 1.5 eq) and the reaction mixture was stirred for 4 h at RT under N₂. The reaction mixture was purified by Pre-TLC to give the title compound (60 mg, 54 % in yield). LCMS: m/z (505.0) (M+H)^{+ 1}HNMR (400 MHz, CDCl₃): δ 0.826∼0.837 (m, 2H), 1.147∼1.183 (m, 6H), 1.744∼2.210 (m, 5H), 4.661∼4.699 (m, 1H), 5.212∼5.226 (m, 2H), 6.499∼6.524 (m, 1H), 6.921∼7.367 (m, 10H) and 8.223(S, 1H).

Proceeding as described above but substituting 4-phenoxyphenylboronic acid with 2-(2-fluoro-4-phenoxyphenyl)-4,4,5,5-tetramethyl-1,3,2-dioxaborolane and 2-(4-(3,5-difluorophenoxy)phenyl)-4,4,5,5-tetramethyl-1,3,2-dioxaborolane, (R)-2-(3-(4-amino-5-(2-fluoro-4-phenoxyphenyl)-7H-pyrrolo[2,3-d]pyrimidin-7-yl)piperidine-1-carbonyl)-3-cyclopropylacrylonitrile LCMS m/z 523.1 (M+H)⁺ and (R)-2-(3-(4-amino-5-(4-(3,5-difluorophenoxy)phenyl)-7H-pyrrolo[2,3-d]pyrimidin-7-yl)piperidine-1-carbonyl)-3-cyclopropylacrylonitrile LCMS m/z 541.1 (M+H)⁺were prepared respectively.

### Example 20

### Synthesis of (S)-2-(2-((4-amino-6-methyl-5-(4-phenoxyphenyl)-7H-pyrrolo[2,3-d]pyrimidin-7-yl)methyl)pyrrolidine-1-carbonyl)-3-cyclopropylacrylonitrile

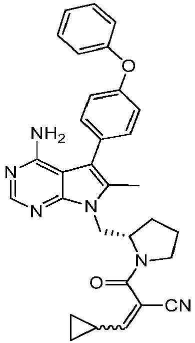

### Step 1

To a solution of 4-chloro-7H-pyrrolo[2,3-d]pyrimidine (10 g, 65.12 mmol, 1.0 eq) in THF (300 mL), NaH (5.30 g, 130.24 mmol, 2 eq) was added at 0° C. After 3 h, benzenesulfonyl chloride (22.53 g, 130.24 mmol, 2 eq) was added. The temperature was warmed to RT and continued for 1 h. The reaction mixture was poured into sat. NH₄Cl and extracted with EtOAc. The organic layers were dried, concentrated and purified by column chromatography (eluting with 10 % EtOAc in PE) to afford 4-chloro-7-(phenylsulfonyl)-7H-pyrrolo[2,3-d]pyrimidine as brown solid (4.5 g, 24 % in yield)

### Step 2

To the solution of 4-chloro-7-(phenylsulfonyl)-7H-pyrrolo[2,3-d]pyrimidine (3 g, 12.6 mmol, 1.0 eq) and TMEDA (3.0 mL, 18.9 mmol, 1.5 eq) in THF (120 mL), n-BuLi (7.5 mL, 18.9 mmol, 1.5 eq) was added at -78 °C. After 3 min, CH₃I (3.7 mL, 59.2 mmol, 4.7 eq) was added. After 3 h, the reaction mixture was warmed to RT over 1 h. The reaction was quenched by addition of sat NH₄Cl (10 mL) at -78 °C. EtOAc (200 mL) and water (100 mL) was added. The organic layer was separated, dried and concentrated to afford 4-chloro-6-methyl-7-(phenylsulfonyl)-7H-pyrrolo[2,3-d]pyrimidine as a brown solid (6.7 g, 90 % in yield).

### Step 3

To the solution of 4-chloro-6-methyl-7-(phenylsulfonyl)-7H-pyrrolo[2,3-d]pyrimidine (10 g, 32.5. mmol, 1.0 eq) in THF (400 mL), t-BuOK (18.23 g, 163.0 mmol, 5 eq) was added and stirred at RT for 12 h. Sat. NaHCO₃ (50 mL) was added and extracted with EtOAc. The organic layers were separated, dried and concentrated to afford 4-chloro-6-methyl-7H-pyrrolo[2,3-d]pyrimidine as a brown solid (2.7 g, 50 % in yield).

### Step 4

To the solution of 4-chloro-6-methyl-7H-pyrrolo[2,3-d]pyrimidine (1.0g, 5.97 mmol, 1.0 eq) and (S)-tert-butyl 2-(hydroxymethyl)pyrrolidine-1-carboxylate (1.32 g, 6.57 mmol, 1.1 eq) and PPh₃ (3.03 g, 11.94 mmol, 2.0 eq) in THF (50 mL), DIEA (2.08 g, 11.94 mmol, 2.0 eq) was added at 0° C. The resulted mixture was stirred and warmed to RT for 12 h. Solvent was removed and purified by column chromatography (eluting with 10 % EtOAc in PE) to afford (S)-tert-butyl 2-((4-chloro-6-methyl-7H-pyrrolo[2,3-d]pyrimidin-7-yl)methyl )pyrrolidine-1-carboxylate as a white solid (2.08 g, 100 % in yield).

### Step 5

To the solution of (S)-tert-butyl 2-((4-chloro-6-methyl-7H-pyrrolo[2,3-d]pyrimidin-7-yl)methyl)pyrrolidine-1-carboxylate (1.0g, 2.86 mmol, 1.0 eq) in DMF (20 mL), NIS (0.675 g, 3.00 mmol, 1.05 eq) was added at 0° C. The resulted mixture was stirred and warmed to RT for 12 h. Solvent was removed and purified by column chromatography to afford (S)-tert-butyl 2-((4-chloro-5-iodo-6-methyl-7H-pyrrolo[2,3-d]pyrimidin-7-yl)methyl)pyrrolidine-1-carboxylate as white solid (1.0 g, 77% in yield) which was converted to the title compound as described in Examle 19, Step 6 above. LCMS m/z 519.1 (M+H)⁺

Proceeding as described above but substituting 4-phenoxyphenylboronic acid with 2-(4-(3,5-difluorophenoxy)phenyl)-4,4,5,5-tetramethyl-1,3,2-dioxaborolane and 2-(2-fluoro-4-phenoxyphenyl)-4,4,5,5-tetramethyl-1,3,2-dioxaborolane, (S)-2-(2-((4-amino-5-(4-(3,5-difluorophenoxy)phenyl)-6-methyl-7H-pyrrolo[2,3-d]pyrimidin-7-yl)methyl)pyrrolidine-1-carbonyl)-3-cyclopropylacrylonitrile LCMS m/z m/z 555.2 (M+H)⁺ and (S)-2-((4-amino-5-(2-fluoro-4-phenoxyphenyl)-6-methyl-7H-pyrrolo[2,3-d]pyrimidin-7-yl)methyl)pyrrolidine-1-carbonyl)-3-cyclopropylacrylonitrile LCMS m/z 536.6 (M+H)⁺were prepared respectively.

### Example 21

### Synthesis of (R)-4-amino-2-(2-((4-amino-3-(2-fluoro-4-phenoxyphenyl)-1H-pyrazolo[3,4-d]-pyrimidin-1-yl)methyl)pyrrolidine-1-carbonyl)-4-methylpent-2-enenitrile tris (2, 2, 2-trifluoroacetate) salt

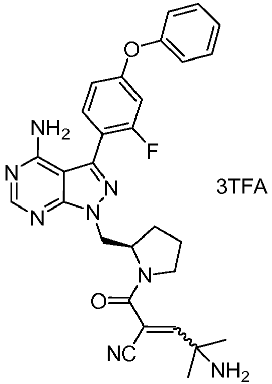

### Step 1

A solution of 3-((R)-2-((4-amino-3-(2-fluoro-4-phenoxyphenyl)-1H-pyrazolo[3,4-d] pyrimidin-1-yl)methyl)pyrrolidin-1-yl)-3-oxopropanenitrile (141 mg, 0.30 mmol, 1.0 equiv), tert-butyl 2-methyl-1-oxopropan-2-ylcarbamate (1.12 g, 6.00 mmol, 20.0 equiv), piperidine (255 mg, 3.0 mmol, 10.0 equiv) in 1,4-dioxane (15 mL) was refluxed for 2 h. The resulting mixture was concentrated under vacuum. The residue was submitted to flash chromatography eluting with ethyl acetate to give (R)-tert-butyl (5-(2-((4-amino-3-(2-fluoro-4-phenoxyphenyl)-1H-pyrazolo[3,4-d]pyrimidin-1-yl)methyl)pyrrolidin-1-yl)-4-cyano-2-methyl-5-oxopent-3-en-2-yl)carbamate 90 mg as a pale yellow solid.

### Step 2

To a solution of (R,E)-tert-butyl (5-(2-((4-amino-3-(2-fluoro-4-phenoxyphenyl)-1H-pyrazolo[3,4-d]pyrimidin-1-yl)methyl)pyrrolidin-1-yl)-4-cyano-2-methyl-5-oxopent-3-en-2-yl)carbamate (90 mg, 0.14 mmol, 1 equiv) in 16 mL DCM was added 4 mL trifluoroacetic acid dropwise. The resulting solution was stirred for 3h at room temperature. The solution was concentrated under reduced pressure. The residue was purified on Prep-HPLC. Conditions: (1#-Pre-HPLC-001(SHIMADZU)): Column, SunFire Prep C18, 19^{∗}150mm 5um; mobile phase, water with 0.05%TFA and CH3CN (40% CH₃CN up to 100% in 20 min); Detector, 254 nm to give the title compound as a pale light yellow solid. MS (ESI, pos. ion) m/z: 541 (M+1).

### Example 22

### Synthesis of (R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile

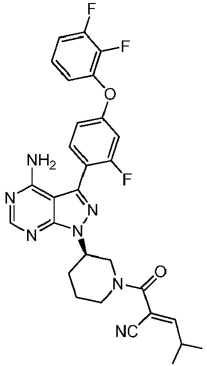

### Step 1

To a solution of 1H-pyrazolo[3,4-d]pyrimidin-4-ylamine (3.0 g, 22.20 mmol, 1.0 eq) in DMF (30 mL), NIS (6.7g , 24.42 mmol, 1.1 eq) was added at room temperature. The reaction mixture was stirred overnight at 60 °C. The reaction mixture was cooled to room temperature and 10 % aq. NaHCO₃ (150 mL) was added to the reaction mixture. The solid was filtered and re-crystallization from DMF solvent to give 3-iodo-1H-pyrazolo[3,4-d]pyrimidin-4-amine (4.0 g, 69 % in yield).

### Step 2

To a solution of 3-iodo-1H-pyrazolo[3,4-d]pyrimidin-4-ylamine (4.0 g, 15.32 mmol, 1.0 eq), (S)-3-hydroxypiperidine-1-carboxylic acid tert-butyl ester (4.313 g, 21.44 mmol, 1.4 eq), and PPh₃ (8.031 g, 30.64 mmol, 2.0 eq) in dry THF (200 mL), DIAD (4.658 g, 22.98 mmol, 1.5 eq) was added at room temperature. The reaction mixture was stirred at 70° C for 72 h. The reaction mixture was concentrated and purified by silica gel chromatography (eluted with PE: EtOAc =1:1) to afford (R)-tert-butyl 3-(4-amino-3-iodo-1H-pyrazolo[3,4-d]pyrimidin-1-yl)piperidine-1-carboxylate (2.8 g, 41.2% in yield).

### Step 3

A solution of (R)-tert-butyl 3-(4-amino-3-iodo-1H-pyrazolo[3,4-d]pyrimidin-1-yl)piperidine-1-carboxylate (2.8 g, 6.16 mmol, 1.0eq), 2-(4-(2,3-difluorophenoxy)-2-fluorophenyl)-4,4,5,5-tetramethyl-1,3,2-dioxaborolane (1.7 g, 6.16 mmol, 1.0 eq), Pd(PPh₃)₄ (0.28 g, 0.08 mmol, 0.07 eq) and Na₂CO₃ (1.7 g, 15.4 mmol, 2.5 eq) in dioxane/H₂O (40/10mL) was stirred at 90° C overnight. The reaction mixture was concentrated and purified by Pre-TLC to afford (R)-tert-butyl 3-(4-amino-3-(4-(2,3-difluorophenoxy)-2-fluorophenyl)-1H-pyrazolo[3,4-d]pyrimidin-1-yl)piperidine-1-carboxylate (1.7g, 51.1% yield).

### Step 4

To a solution of (R)-tert-butyl 3-(4-amino-3-(4-(2,3-difluorophenoxy)-2-fluorophenyl)-1H-pyrazolo[3,4-d]pyrimidin-1-yl)piperidine-1-carboxylate (1.7 g, 3.15 mmol) in 20 ml of DCM, TFA (20 ml) was added. The reaction mixture was stirred at RT for 4h. The mixture was washed with sat. NaHCO₃ (10 mL) and concentrated to give (R)-3-(4-(2,3-difluorophenoxy)-2-fluorophenyl)-1-(piperidin-3-yl)-1H-pyrazolo[3,4-d]pyrimidin-4-amine (1.1 g, 80% yield).

### Step 5

To a mixture of (R)-3-(4-(2,3-difluorophenoxy)-2-fluorophenyl)-1-(piperidin-3-yl)-1H-pyrazolo[3,4-d]pyrimidin-4-amine (100 mg, 0.23 mmol, 1.0 eq), 2-cyano-4-methyl-pent-2-enoic acid (38 mg, 0.27 mmol, 1.2 eq) and DIEA (88 mg, 0.68 mmol, 3.0 eq) in 10 ml DCM was added HATU (130 mg, 0.34 mmol, 1.5 eq). The reaction mixture was stirred for 4 h at RT under N₂. The mixture was purified by Pre-HPLC to give the title compound (25 mg40% yield). LCMS: m/z⁺ (562.2) (M+H)+.

### Example 23

### Synthesis of 2-((R)-2-((4-amino-3-(4-(2,3-difluorophenoxy)-2-fluorophenyl)-1H-pyrazolo [3,4-d]pyrimidin-1-yl)methyl)pyrrolidine-1-carbonyl)-4-ethoxy-4-methylpent-2-enenitrile 2, 2, 2-trifluoroacetate

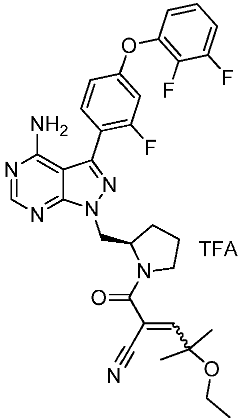

A solution of 3-((R)-2-((4-amino-3-(4-(2,3-difluorophenoxy)-2-fluorophenyl)-1H-pyrazolo[3, 4-d]pyrimidin-1-yl)methyl)pyrrolidin-1-yl)-3-oxopropanenitrile (202.8 mg, 0.40 mmol, 1.0 equiv), 2-ethoxy-2-methylpropanal (232 mg, 2.00 mmol, 5.0 equiv), piperidine (68 mg, 0.80 mmol, 2.0 equiv) in EtOH (20 mL) was stirred at room temperature overnight. The volatile phase was removed off under reduced pressure. The residue was purified on Prep-HPLC. Conditions: (1#-Pre-HPLC-001(SHIMADZU)): Column, SunFire Prep C18, 19*150mm 5um; mobile phase, water in 0.05%TFA and CH₃CN (40% CH₃CN up to 100% in 20 min); Detector, 254 nm. This resulted in 30 mg (10.43 %) of the title compound as a white solid. MS (ESI, pos. ion) m/z: 606 (M-TFA+1).

### Example 24

### Synthesis of N-(2-(4-amino-3-(4-phenoxyphenyl)-1H-pyrazolo[3,4-d]pyrimidin-1-yl)ethyl)-2-cyano-3-cyclopropylacrylamide

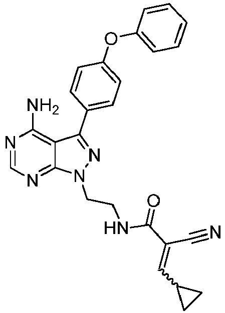

### Step 1

To a solution of 3-(4-phenoxyphenyl)-1H-pyrazolo[3,4-d]pyrimidin-4-amine (300 mg, 1.0 mmole), triphenylphosphine (1.04 g, 3.96 mmole) and tert-butyl (2-hydroxyethyl)-carbamate (238 mg, 1.5 mmoles) in THF (25 mL) was added DIAD (0.4 mL, 2 mmoles). The reaction was stirred for 5 hrs at room temperature and then water (30 mL) was added and extracted with ethyl acetate. The organic layers were combined, washed with aq. NaHCO₃ and brine, then dried (Na₂SO₄), filtered and concentrated. The resulting tert-butyl (2-(4-amino-3-(4-phenoxyphenyl)-1H-pyrazolo[3,4-d]pyrimidin-1-yl)ethyl)carbamate was used without further purification.

### Step 2

The tert-butyl (2-(4-amino-3-(4-phenoxyphenyl)-1H-pyrazolo[3,4-d]pyrimidin-1-yl)ethyl)carbamate was dissolved in TFA (5 mL). After 30 minutes of stirring at room temperature, the reaction was diluted with water and washed with ethyl acetate. The aqueous layer was basified to pH = 11-12 with NaOH and then washed with ethyl acetate. The organic layer was dried (Na₂SO₄), filtered and concentrated to collect 320 mg of 1-(2-aminoethyl)-3-(4-phenoxyphenyl)-1H-pyrazolo[3,4-d]pyrimidin-4-amine.

### Step 3

To a solution of 1-(2-aminoethyl)-3-(4-phenoxyphenyl)-1H-pyrazolo[3,4-d]pyrimidin-4-amine (287 mg, 0.829 mmole), 2-cyanoacetic acid (85 mg, 1.0 mmole) and TEA (0.14 ml, 1.0 mmole) in DMF (10 mL) was added HATU (347 mg, 0.912 mmole). After stirring 3 hr at room temperature, water was added and extracted with ethyl acetate. The organic layer was washed with aq. NaHCO₃ and brine, then dried (Na₂SO₄), filtered and concentrated. The resulting residue was subjected to column chromatography (3% MeOH/DCM) to provide 90 mg (22% yield from step 1) of N-(2-(4-amino-3-(4-phenoxyphenyl)-1H-pyrazolo[3,4-d]pyrimidin-1-yl)ethyl)-2-cyanoacetamide.

### Step 4

A solution of N-(2-(4-amino-3-(4-phenoxyphenyl)-1H-pyrazolo[3,4-d]pyrimidin-1-yl)ethyl)-2-cyanoacetamide (90 mg, 0.22 mole), cyclopropylcarboxaldehyde (18 mg, 0.26 mmole) and piperidine (22 mg, 0.26 mmole) in MeOH (5 mL) was stirred for 3 hr at room temperature. Then water was added and extracted with ethyl acetate. The organic layers were combined and washed with aq. NaHCO₃ and brine, then dried (Na₂SO₄), filtered and concentrated. The residue was purified by column chromatography (3% MeOH/DCM) to provide 39 mg (38% yield) of the title compound as a white solid. LCMS m/z 466 (M+H)⁺.

### Example 25

### Synthesis of 2-((S)-2-((4-amino-3-(2-fluoro-4-phenoxyphenyl)-1H-pyrazolo[3,4-d]pyrimidin-1-yl)methyl)pyrrolidine-1-carbonyl)-4-(cyclopropylamino)-4-methylpent-2-enenitrile

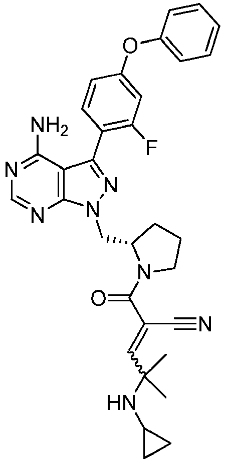

### Step 1

To a 250 mL of sealed tube was added ethyl 2-bromo-2-methylpropanoate (19.4 g, 0.1 mol, 1.0 equiv), cyclopropanamine (11.4 g, 0.2 mol, 2.0 equiv), K₂CO₃ (27.6 g, 0.2 mol, 2.0 equiv), KI (1.66 g, 0.01 mol, 0.1 equiv) and 200 mL of MeCN. The mixture was stirred at 100 °C for 12 h then cooled to room temperature and the solids were filtered. The filtrate was concentrated and purified on silica gel column eluting with pet. ether/ethyl acetate =4/1 to give 8.0 g (46%) of ethyl 2-(cyclopropylamino)-2-methylpropanoate.

### Step 2

To a solution of LiAlH₄ (760 mg, 20 mmol, 1.0 equiv) in THF (50 mL) was added ethyl 2-(cyclopropylamino)-2-methylpropanoate (3.42 g, 20 mmol, 1.0 equiv) in THF (10 mL) at 0 °C under N₂. The resulting suspension was stirred at 0°C for 2 h. The reaction was quenched with Na₂SO₄·10H₂O (3.0 g) at 0 °C. The solid was filtered off and the filtrate was concentrated under reduced pressure. This resulted in 2-(cyclopropylamino)-2-methylpropan-1-ol 1.3 g (50%) as a white solid.

### Step 3

To a solution of oxalyl chloride (11.43 g, 90 mmol, 1.5 equiv) in DCM (300 mL) was added DMSO (11.7 g, 150 mmol, 2.5 equiv) at -78 °C under N₂ atmosphere. The resulting mixture was stirred for 0.5 h then a solution of 2-(cyclopropylamino)-2-methylpropan-1-ol (7.74 g, 60 mmol, 1.0 equiv) in DCM (20 mL) was added dropwise at -78°C and then stirred for another 1 h. Then TEA (36.4 g, 0.36 mol, 6.0 equiv) was added and stirring was continued for 20 min at room temperature. The reaction was then diluted with DCM (200mL) and washed with aq. NaHCO₃ and brine, dried over Na₂SO₄, concentrated to give the crude product, which was purified with distillation under reduced pressure. This resulted in 1.0 g (13%) of 2-(cyclopropylamino)-2-methylpropanal was obtained as a colorless oil.

### Step 4

A solution of 3-((S)-2-((4-amino-3-(2-fluoro-4-phenoxyphenyl)-1H-pyrazolo[3,4-d]-pyrimidin-1-yl)methyl)pyrrolidin-1-yl)-3-oxopropanenitrile (118 mg, 0.25 mmol, 1.0 equiv), 2-(cyclopropylamino)-2-methylpropanal (0.16 g, 1.25 mmol, 5.0 equiv) and one drop of piperidine in MeCN (10 mL) was stirred overnight at 40 °C. The solvent was removed and the residue was purified on Prep-HPLC. Conditions: (1#-Pre-HPLC-001(SHIMADZU)): Column, SunFire Prep C18, 19^{∗}150mm 5um; mobile phase, water with 0.05%TFA and CH₃CN (40% CH₃CN up to 100% in 20 min); Detector, 254 nm. This resulted in 40 mg (27%) of the title compound as a white solid and bis TFA salt. LC-MS: *m*/*z* 581 (M+ H⁺).

### Example 26

### Synthesis of 2-((S)-2-((4-amino-3-(2-fluoro-4-phenoxyphenyl)-1H-pyrazolo[3,4-d]pyrimidin-1-yl)methyl)pyrrolidine-1-carbonyl)-4-(2-methoxyethylamino)-4-methylpent-2-enenitrile

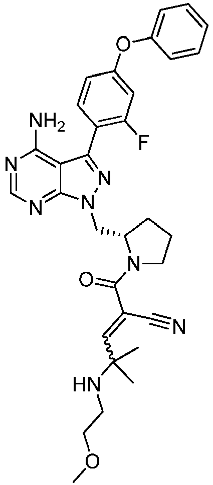

To a suspension of 4-amino-2-((S)-2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile (210 mg, 0.39 mmol, 1.0 equiv), KI (130 mg, 0.78 mmol, 2.0 equiv) and potassium carbonate (166 mg, 1.17 mmol, 3.0 equiv) in CH₃CN (15 mL) was added 1-bromo-2-methoxyethane (160 mg, 1.17 mmol, 3.0 equiv). The resulting suspension was stirred at 50°C overnight. The solvent was removed under reduced pressure and then water (20mL) was added to the residue. The resulting mixture was extracted with ethyl acetate (30 mL^{∗}3). The organic layers were combined, washed with brine, dried over sodium sulfate and concentrated. The residue was purified on Prep-HPLC. Conditions: (1#-Pre-HPLC-001(SHIMADZU)): Column, SunFire Prep C18, 19^{∗}150mm 5um; mobile phase, WATER WITH 0.5%NH4OH and CH3CN (40% CH3CN up to 100% in 20 min); Detector, 254 nm. This resulted in 8.7 mg (3.7 %) of the title compound as an off-white solid. MS (ESI, pos. ion) m/z: 599 (M+1).

### Example 27

### Synthesis of (R)-2-(2-((4-amino-3-(2-fluoro-4-phenoxyphenyl)-1H-pyrazolo[3,4-d]-pyrimidin-1-yl)methyl)pyrrolidine-1-carbonyl)-3-(1-aminocyclopropyl)acrylonitrile

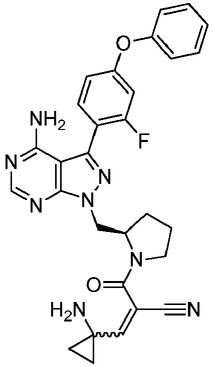

### Step 1

To a solution of tert-butyl (1-(hydroxymethyl)cyclopropyl)carbamate (Bioorg. Med. Chem. Lett., 2008, 18(6), 2188) (135 mg, 0.72 mmoles) in DCM (8 mL) was added Dess-Martin periodinane (277 mg, 0.65 mmole). After stirring 1 hr, the reaction was filtered through celite and concentrated to a yellow oil which was further purified by Isolera (7%-70% ethyl acetate/hexanes) to provide 84 mg (87 %) of tert-butyl (1-formylcyclopropyl)-carbamate as a white solid.

### Step 2

To a solution of 3-[(2R)-2-[[4-amino-3-(2-fluoro-4-phenoxy-phenyl)pyrazolo[3,4-d]pyrimidin-1-yl]methyl]pyrrolidin-1-yl]-3-oxo-propanenitrile (100.mg, 0.2100mmol) dissolved in methanol (4mL) and DCM (4mL) was added piperidine (0.1mL, 0.8500mmol) and tert-butyl N-(1-formylcyclopropyl)carbamate (58.9mg, 0.3200mmol). The reaction was heated to reflux for 6 hrs and then cooled and concentrated. The residue was and dissolved in ethyl acetate (50 mL) and washed with water (50mL) and then brine. The organic layer was dried (MgSO₄), filtered and concentrated. The residue was purified by Isolera (1%-8% MeOH/DCM) to provide 39 mg (13% yield) of tert-butyl N-[1-[3-[(2R)-2-[[4-amino-3-(2-fluoro-4-phenoxy-phenyl)pyrazolo[3,4-d]pyrimidin-1-yl]methyl]pyrrolidin-1-yl]-2-cyano-3-oxo-prop-1-enyl]cyclopropyl]carbamate.

### Step 3

To a solution of tert-butyl N-[1-[3-[(2R)-2-[[4-amino-3-(2-fluoro-4-phenoxyphenyl)pyrazolo[3,4-d]pyrimidin-1-yl]methyl]pyrrolidin-1-yl]-2-cyano-3-oxo-prop-1-enyl]cyclopropyl]carbamate (27 mg, 0.04 mmol) in DCM (3 mL) was added TFA (1 mL). The solution was stirred for 5 hrs. and then concentrated. The residue was purified by prep-TLC (5% MeOH/DCM) to provide 2.68 mg (12%) of the title compound. MS (pos. ion) m/z: 539 (M+1).

### Example 28

### Synthesis of 2-[(2S)-2-[[4-amino-3-(2-fluoro-4-phenoxy-phenyl)pyrazolo[3,4-d]pyrimidin-1-yl]methyl]pyrrolidine-1-carbonyl]-4-methyl-4-morpholino-pent-2-enenitrile.

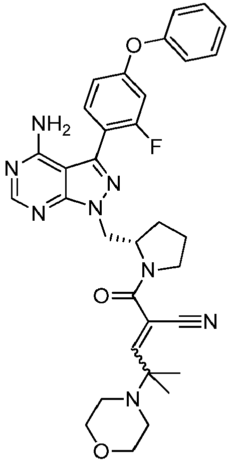

To a sealed tube was added 3-[(2S)-2-[[4-amino-3-(2-fluoro-4-phenoxyphenyl)pyrazolo[3,4-d]pyrimidin-1-yl]methyl]pyrrolidin-1-yl]-3-oxo-propanenitrile (900mg, 1.91mmol), ethanol (12mL), piperidine (0.23mL, 2.29mmol) and 2-methyl-2-morpholino-propanal (0.49mL, 2.86mmol). The tube was sealed and heated to 105°C for 24hrs. The mixture was then cooled, concentrated and then dissolved in ethyl acetate (100 mL) and washed with 5% citric acid (100 ml) and then brine. The organic layer was dried (MgSO₄), filtered and concentrated. The crude material was purified by Isolera (column size 100g. Solvent system 4%-8% MeOH/EtOAc) to obtain 245 mg (21% yield) of the title compound. MS (pos. ion) m/z: 611 (M+1).

Proceeding as described above by substituting 3-[(2S)-2-[[4-amino-3-(2-fluoro-4-phenoxy-phenyl)pyrazolo[3,4-d]pyrimidin-1-yl]methyl]pyrrolidin-1-yl]-3-oxo-propanenitrile with (R)-3-(3-(4-amino-3-(2-fluoro-4-phenoxyphenyl)-1H-pyrazolo[3,4-d]pyrimidin-1-yl)piperidin-1-yl)-3-oxopropanenitrile, (R)-2-(3-(4-amino-3-(2-fluoro-4-phenoxyphenyl)-1H-pyrazolo[3,4-d]pyrimidin-1-yl)piperidine-1-carbonyl)-4-methyl-4-morpholinopent-2-enenitrile was prepared.

### Example 29

### Synthesis of 2-[(2S)-2-[[4-amino-3-(2-fluoro-4-phenoxy-phenyl)pyrazolo[3,4-d]pyrimidin-1-yl]methyl]pyrrolidine-1-carbonyl]-4-methyl-4-morpholino-pent-2-enenitrile.

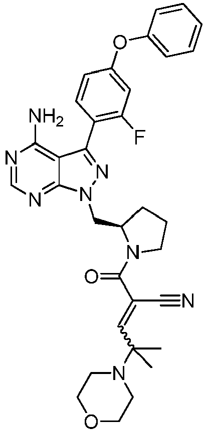

To a sealed tube was added 3-[(2R)-2-[[4-amino-3-(2-fluoro-4-phenoxy-phenyl)-pyrazolo[3,4-d]pyrimidin-1-yl]methyl]pyrrolidin-1-yl]-3-oxo-propanenitrile (762.6mg, 1.62mmol), 2-methyl-2-morpholino-propanal (508.54mg, 3.23mmol), piperidine (0.08mL, 0.81 mmol) and ethanol (6mL). The tube was sealed and heated at 100°C. After 22 hrs, the reaction was cooled and evaporated. The residue was purified by Isolera (column size 100g, 3%-7% MeOH/EtOAc) to obtain 550mg (56% yield) of the title compound. MS (pos. ion) m/z: 611 (M+1).

### Example 30

### Synthesis of 2-[(2R)-2-[[4-amino-3-(2-fluoro-4-phenoxy-phenyl)pyrazolo[3,4-d]pyrimidin-1-yl]methyl]pyrrolidine-1-carbonyl]-3-[(2S)-pyrrolidin-2-yl]prop-2-enenitrileHCl

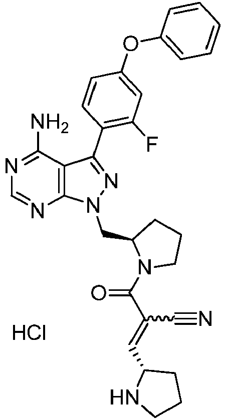

### Step 1

To a sealed tube was added 3-[(2R)-2-[[4-amino-3-(2-fluoro-4-phenoxyphenyl)pyrazolo[3,4-d]pyrimidin-1-yl]methyl]pyrrolidin-1-yl]-3-oxo-propanenitrile (507 mg, 1.07 mmol), tert-butyl (2S)-2-formylpyrrolidine-1-carboxylate (0.2,mL, 1.1 mmol), piperidine (0.05mL, 0.54 mmol) and ethanol (3mL). The tube was capped and heated to 100 °C for 16 hrs. The reaction was not complete so an additional amount of tert-butyl (2S)-2-formylpyrrolidine-1-carboxylate (732 mg, 3eq) was added and heating was continued for 4 hrs at 110 °C. The reaction was cooled and concentrated then dissolved in DCM (50mL) and washed with water (50mL) and brine. The organic layer was dried (MgSO₄), filtered and concentrated. The resulting material was purified by Isolera (250g column; 2% - 3% MeOH/DCM) to provide 403 mg (57%) of tert-butyl (2S)-2-[3-[(2R)-2-[[4-amino-3-(2-fluoro-4-phenoxy-phenyl)pyrazolo[3,4-d]pyrimidin-1-yl]methyl]pyrrolidin-1-yl]-2-cyano-3-oxo-prop-1-enyl]pyrrolidine-1-carboxylate as a solid.

### Step 2

To a solution of tert-butyl (2S)-2-[3-[(2R)-2-[[4-amino-3-(2-fluoro-4-phenoxyphenyl)pyrazolo[3,4-d]pyrimidin-1-yl]methyl]pyrrolidin-1-yl]-2-cyano-3-oxo-prop-1-enyl]pyrrolidine-1-carboxylate (84 mg, 0.13 mmol) in 1,4-dioxane (2mL) and added 4N HCl in dioxane (0.16 mL). The solution was stirred for 16hr at room temperature then concentrated. The residue was dissolved in methanol (∼1 mL) and added dropwise to ethyl ether (20mL) while stirring. The resulting solid was collected by filtration to provide 42 mg (59 %) of the title compound as an HCl salt. MS (pos. ion) m/z: 553 (M+1).

### Example 31

### 2-[(2S)-2-[[4-amino-3-(2-fluoro-4-phenoxy-phenyl)pyrazolo[3,4-d]pyrimidin-1-yl]methyl]-pyrrolidine-1-carbonyl]-3-(3-methyloxetan-3-yl)prop-2-enenitrile

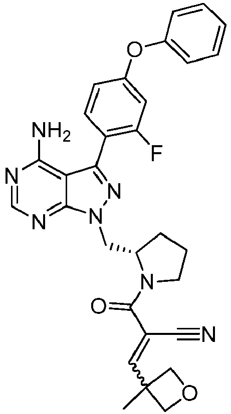

To a slurry of 3-[(2S)-2-[[4-amino-3-(2-fluoro-4-phenoxy-phenyl)pyrazolo[3,4-d]pyrimidin-1-yl]methyl]pyrrolidin-1-yl]-3-oxo-propanenitrile (74.mg, 0.16 mmol) in ethanol (3mL) was added 3-methyloxetane-3-carbaldehyde (78.54 mg, 0.78 mmol) and then piperidine (0.02 mL, 0.16 mmol) and the mixture heated to 80 °C with stirring. After 3 h, the mixture was cooled and partitioned between ethyl acetate and water. The organic phase was washed with brine, dried over sodium sulfate, and the filtered. Solvents were removed to afford an oil which was purified by column chromatography (gradient from neat methylene chloride to 95-5 methylene chloride:MeOH). The pure fractions were concentrated, then taken up in acetonitrile/water, frozen and lyophilized to afford 2-[(2S)-2-[[4-amino-3-(2-fluoro-4-phenoxy-phenyl)pyrazolo[3,4-d]pyrimidin-1-yl]methyl]pyrrolidine-1-carbonyl]-3-(3-methyloxetan-3-yl)prop-2-enenitrile as a colorless solid weighing 14 mg.

Compounds of Formula (III) can be prepared as described in PCT application publication No. WO 2011/060440.

### Biological Examples

### Example 1Tyrosine Kinase TR-FRET Assay

The inhibition of a kinase by a compound of the present invention can be measured by methods well known the the art. For example, inhibition of a kinase enzymatic activity by compounds of the present invention can be measured using time-resolved fluorescence resonance energy transfer (TR-FRET) (Invitrogen pamphlet: Optimization of a LanthaScreen Kinase assay for BTK). Here, a signal is observed only when a Europium-coupled phophotyrosine antibody binds the phosphorylated peptide. Compounds are first prepared in 100% DMSO and serially diluted 10 times via 3-fold dilution. 2.5 µl of inhibitor at 4-fold the final assay concentration is next transferred to the 384-well assay plate (Corning Catalog # 3676). A solution of 2-fold the final concentration of appropriate kinase enzyme and Alexafluor 647-coupled peptide substrate (Invitrogen Catalog # 5693) is next prepared in advance in a kinase buffer of 50 mM Hepes pH 7.5, 10 mM MgCl₂, and 1 mM EGTA. For this solution, the final concentration of the appropriate kinase and peptide is typically 1 nM and 100 nM, respectively. 5 µL of this 2-fold mix of kinase and peptide is added as the second step of the procedure to the 384-well assay plate. To initiate the enzymatic reaction, 2.5 µl of a 4-fold excess ATP solution in kinase buffer is added to the 384-well assay plate. Final ATP concentration is typically set to the Km for ATP. The reaction is allowed to proceed for 60 minutes. During the kinase reaction, a stop solution consisting of EDTA and a Europium-containing phosphotyrosine antibody (Invtrogen Catalog # 5692) is prepared in TR-FRET dilution buffer (Invitrogen Catalog # 3574). The stop solution contains an EDTA concentration of 20 mM and an antibody concentration of 4 nM. After the 60 minute reaction, 10 µl of the stop solution is added to all wells. Each well is mixed and incubated for 30 minutes at room temperature. Plates are read on a Perkin Elmer Envision TR-FRET plate reader under LanthaScreen settings. Excitation wavelength is 337 nm and Emission wavelengths are 620 nm and 665 nm. Data are acquired as the ratio of emission at 665 nm/emission at 620 nm and plotted as a function of compound concentration to ascertain compound potency. Here, a signal is observed only when a Europium-coupled phophotyrosine antibody binds the phosphorylated peptide.

### Example 2

### Btk enzymatic activity assay

A Caliper-based kinase assay (Caliper Life Sciences, Hopkinton, MA) was used to measure inhibition of Btk kinase activity of a compound of Formula (I). Serial dilutions of test compounds were incubated with human recombinant Btk (2 nM), ATP (40 uM) and a phosphoacceptor peptide substrate FAM-GEEPLYWSFPAKKK-NH₂ (1 uM) at room temperature for 3 h. The reaction was then terminated with EDTA, final concentration 20 mM and the phosphorylated reaction product was quantified on a Caliper Desktop Profiler (Caliper LabChip 3000). Percent inhibition was calculated for each compound dilution and the concentration that produced 50% inhibition was calculated. This value is presented as the IC₅₀. The IC₅₀ for a representative no. of compounds of the invention are provided below (*= Comparative Example).

| Cpd # (see Embodiment L) | IC₅₀ (um) | Cpd# | IC₅₀ (um) |
|---|---|---|---|
| Cpd Table 1 | | | |
| 1 | 0.0031 | 7 | .0013 |
| 2 | .0037 | 8* | .13 |
| 3* | .175 | 9* | .98 |
| 4 | .061 | 10 | .0054 |
| 5 | .001 | 11* | .014 |
| 6* | .365 | 15A* | 0.0017 |
| 17A* | .0021 | 24A | .0062 |
| 18A* | .0023 | 25A | .0096 |
| 22A | .0018 | 27A | .004 |
| 28* | .017 | 30A* | .0017 |
| 31A | .002 | 36A* | .0043 |
| 32A | .0017 | 37A* | .0042 |
| 34 | .0048 | 39A | .0071 |
| 35A* | .0044 | 22B | .0026 |
| 25B | 0.14 | 44A | 0.005 |
| 27B | 0.0006 | 44B | 0.003 |
| 39B | 0.0038 | 54A | 0.002 |
| 41A | 0.0032 | 56A | 0.0033 |
| 43A | 0.0018 | 57B | 0.01 |
| 63B | 0.033 | 69A | 0.005 |
| 65B | 0.056 | 70A | 0.011 |
| 67 | 0.027 | 72A | 0.0016 |
| 42A | .0028 | 72B | 0.028 |
| 73B | 0.011 | 79B | 0.003 |
| 77B | 0.007 | 59B | 0.026 |
| 71A | 0.007 | 80B | 0.004 |
| 74B | 0.008 | 81A | 0.0044 |
| 75B | 0.10 | 81B | 0.0059 |
| 76B | 0.007 | 82A | 0.0022 |
| 78B | 0.0075 | 82B | 0.113 |
| 87B | 0.012 | 83A | 0.0014 |
| 84A | 0.0036 | 83B | 0.016 |
| 84B | 0.0004 | 85A | 0.0004 |
| 85B | 0.0172 | 89A | 3.1 |
| 87B | 0.012 | 89B | 6.6 |
| 88B | 0.029 | 90A | 0.052 |
| 95A | 0.0265 | 95B | 0.0032 |
| 102A | 0.002 | 102B | 0.006 |
| 104A | 0.001 | 104B | 0.020 |
| 105A | 0.0013 | 105B | 0.0255 |
| 106A | 0.006 | 124A | 0.002 |
| 106B | 0.0015 | 126A | 0.003 |
| 133A | 0.007 | 139A | 0.0007 |
| 156A | 0.0073 | 171B | 0.007 |
| 173B | 0.034 | 175A | 0.003 |
| 175B | 0.001 | 182B | 0.002 |
| 183B | 0.003 | 184B | 0.005 |
| 185B | 0.011 | 186 | 0.0037 |
| 188 | 0.0007 | 195 | 0.006 |
| 180A | 0.037 | 180B | 0.004 |
| 162A | 0.0009 | 197B | 0.0325 |
| 29 A | 0.0027 | 125 A | 0.002 |

### Example 3

### Reversibility of Binding

The following approach was developed to differentiate compounds that form irreversible bonds with their targets, such as acrylamide compounds, from compound that bind reversibly. Reactions were prepared with the protein target at a higher concentration than the compounds of interest. Both irreversible and reversible compounds bound the target and became depleted from solution. The reactions were then treated with perturbations including both denaturation with 5 M guanidine hydrochloride and digestion with trypsin, disrupting proper folding of the target. It was found that the perturbation returned reversible compounds to solution due to dissociation from the target while irreversible compounds remained bound to the target. The concentration of compound in solution was assessed both preceding and following perturbation using high performance liquid chromatography (HPLC) coupled to tandem mass spectrometry. Using this technique, it was demonstrated that an acrylamide-containing compound 1 (shown in table below; comparative example) was depleted from solution in both the native and perturbed state, while reversible compounds 1 and 27 were depleted in the folded state but returned to solution following perturbation of the target (See table below; * = Comparative Example).

| Cpd | Compound in solution in the native state? | Compound in solution in the denatured or digested state? |
|---|---|---|
| 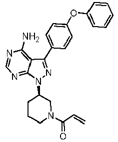 | no | no |
| Irreversible inhibitor* | | |
| 1 | no | yes |
| 27A | no | yes |

### Formulation Examples

### Example 1

### Sparingly soluble compounds dissolved in lipids contained in enteric-coated hard gelatin capsules

24 G of a sparingly soluble compounds is dissolved as a 12% (w/w) solution of glyceryl tricaprylate/tricaprate (Captex® 355) by adding first to a 0.5 kg glass mixing vessel, 88 g of Captex, followed by 24 g of the drug and gentle stirring for 5 minutes, and then followed by the remaining 88 g of the Captex and stirring overnight until dissolved. 200 mg of the drug solution is dispensed into each of 900 hard gelatin capsules (size 1). The Coni-Snap hard gelatin Licap capsules (Capsugel) cap and body joints are then sprayed with 50% aqueous ethanol for about 1 second to lower the sealing temperature of the gelatin. The capsules are then sealed by heating the joint to 55°C for about 1 minute.

The filled gelatin capsules are placed in a fluid-bed coater to apply the enteric coating. The coating solution is 82.89% Eudragit L30D mixed with 10% aqueous solution of PEG 6000 (8.29% w/w), talc (8.29% w/w), and 0.51% simethicone. A peristaltic pump (6-10 rpm) is used to deliver the spraying solution to the nozzle. The dispersion is slowly stirred. The fluid bed is operated with inlet and outlet temperatures, respectively of 55 and 45 °C, with an atomization pressure of 1.2-1.5 kg/cm², and an exhaust air velocity of 42 ft³/min. The enteric-coated capsules are sprayed until 5% weight gain, and then dried at 45 °C. The capsules are packaged in capped and sealed HDPE bottles and stored at ambient temperature.

### Example 2

### Drug in Non-Enteric, Delayed Time Released Tablet Made by Coating

To make immediate release tablet cores of a compound disclosed herein, a high shear granulation is prepared by blending 10 kg of the drug of interest, 1 kg microcrystalline cellulose or lactose or a combination of the two excipients, and 900 g of starch in a granulating bowl. After forming the wet mass by granulating with water, the granules are dried in a fluid bed dryer until the water content is less than 3%. After milling the dried granulation, it is sieved through 16 to 20 mesh. This granulate is then blended with 400 g sodium starch glycolate, 40 g magnesium stearate and 20 g silicon dioxide. This powder blend is then tableted using conventional tablet press equipped with B tooling to give a tablet weight between 100 and 600 mg.

To prepare a delayed release coating on these tablets, a coating solution is made comprised of 35% Eudragit RS 30D, 4.97% Eudragit RL 30D, 7% talc, 8% triethylcitrate, 0.03% simethicone, and 45% water. The talc and water are charged in a stainless steel vessel, and then mixed slowly until suspended. In a second stainless vessel, the Eudragit RS 30D and the simethicone are mixed followed by the addition of the Eudragit RL 30D and the plasticizer. Finally, the talc suspension is added to form the coating solution. The tablets are then placed in a fluid bed coater and coated with about half to 1.5 times the weight of the tablets to the desired weight gain. Talc may be added to dust the tablets during the coating. After coating, the coated tablets are dried.

### Example 3

### Compound in Non-Enteric Delayed Time Released Tablet Made by Press Coating

As in example 2, to make immediate release tablet cores of a compounds disclosed herein, a high shear granulation is prepared by blending 10 kg of the drug, 1 kg microcrystalline cellulose or lactose or a combination of the two excipients, and 900 g of starch in a granulating bowl. After forming the wet mass by granulating with water, the granules are dried in a fluid bed dryer until the water content was less than 3%. After milling the dried granulation, it is sieved through 16 to 20 mesh. This granulate sis then blended om a small V-blender with 400 g sodium starch glycolate, 40 g magnesium stearate and 20 g silicon dioxide. This powder blend is then tableted using a Manesty Dry-Cota tablet press with a flat face, round 0.2031" die and punch. The table hardness is controlled to 4±2 kp.

To apply the press coating, 2 kg hypromellose 2208 and 2 kg microcrystalline cellulose are blended in a V-blender for 10 minutes. 0.04 kg of magnesium stearate is then added and mixed for another 10 minutes. The cores from above are then press coated in a Dry-Cota press with a 0.3600" round shallow, concave punch and die.

### Example 4

### Drug in Enteric Coated Beads

1 kg of the drug and 0.1 kg talc are blended for 15 minutes in a V-blender. Then milled and screened to yield a fine powder. A binder solution is prepared with 10% (w/v) PVP in water. A coating pan is then charged with 1 kg of inert sugar spheres (20 to 50 mesh). The sugar spheres are then sprayed with the binder solution and the drug blend is applied to the spheres until all of the drug was consumed. The drug-loaded beads are then dried in a fluid bed dryer.

A fluidized bed coater is loaded with 1 kg of the above drug-loaded beads. The beads are then coated with 1 kg of the coating solution from Example 1, and then dried. Talc may be used to reduce tackiness during the coating process.

### Example 5

### Determination of Bioavailability... Stomach v. Intraduodenal Adminstration

Rats with surgically implanted intra-duodenal catheters were obtained commercially. Rats were dosed with solution or suspension of (S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile; via bolus injection: oral gavage was done to measure exposure after oral dosing, or dosing via administration through the intra-duodenal catheter to measure exposure bypassing the stomach. Compound was dosed at 20 mg/kg at a dose volume of 2 ml/kg. Blood was withdrawn at time points out to 24 hours and compound quantitated via LC/MS/MS to obtain plasma concentrations. PK parameters were calculated via commercially available software and measures of exposure were used to assess differences in dosing routes. The table below summarizes exposures as measured by area under the curve (AUC), concentration at its maximum (Cmax), and bioavailability.

| | Dosed at 20 mg/kg in 15% cyclodextrin | | | | Dosed in 0.5% xanthum gum suspension | |
|---|---|---|---|---|---|---|
| | Experiment 1 | | Excperiment 2 | | | |
| PK Parameter | PO | ID | PO | ID | PO 100 mg/kg | ID 100 mg/kg |
| AUC (ng-hr/ml) | 153.9 | 629.8 | 647.0 | 1505.0 | 858.3 | 1896.2 |
| Cmax (ng/ml) | 145 | 1405.0 | 405.8 | 1646.7 | 148.7 | 870.3 |
| F (%) | 6.6 | 27.0 | 27.8 | 64.6 | 7.4 | 16.3 |
| % increase | 409 | | 232 | | 220 | |

### Example 6

### In vitro assay for Release

The ability of a dosage form to release a compound disclosed herein from a dosage form at a particular pH can be determined by methods well known in the art. For example, effectiveness of the enteric coated formulation to release compound of the invention at desired pH can be determined by conducting disintegration and dissolution study in a calibrated USP Apparatus 1 or 2, with and without surfactant set at appropriate stirring rate and temperature. Solubility is determined initially at lower pH, for example in 0.1 N HCl, for a period of time (for example 2 h) at 37 °C to determine if any drug has been released. The enteric coating is considered acceptable if < 10% of drug is released in low pH medium. The pH of the medium is then adjusted to the desired pH (for example to pH 5.5) with a buffer at 37 °C at which point the enteric coating should disintegrate and release the drug in the medium. Samples are removed and analyzed for concentration of the drug at a predetermined time points (for example, 15, 30, 45 minutes, etc.). Solubility (concentration) of compound of invention can be determined using UV spectroscopy or by HPLC equipped with a UV detector against a predetermined concentration curve using the Reference Standard. To enhance the solubility of the drug, a surfactant (for example Tween 80, TPGS, SLS) can be added to the medium.

## Claims

1. A solid oral formulation comprising (i) a therapeutically effective amount of a compound, (ii) at least one coating chosen from an enteric coating and/or a non-enteric time-delayed release coating, and (iii) at least one pharmaceutically acceptable excipient,
wherein said compound is:
(a) a compound of Formula (Id) or a pharmaceutically acceptable salt thereof: 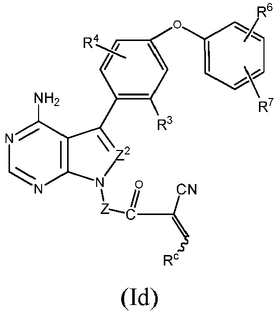 wherein:
Z² is -N- or -CR²- where R² is hydrogen or alkyl;
R³ and R⁴ are independently hydrogen, methyl, chloro, fluoro, cyclopropyl, hydroxy, methoxy, cyano, trifluoromethyl or trifluoromethoxy;
R⁶ and R⁷ are independently hydrogen, methyl, methoxy, fluoro, chloro, trifluoromethyl, trifluoromethoxy, or cyano;
Z is -(alkylene)-NR^{a}-, 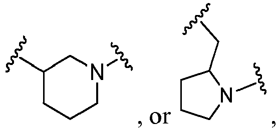 each ring optionally substituted with one or two substituents independently selected from alkyl, hydroxy, and halo, and where R^{a} is independently hydrogen or alkyl; and
R^{c} is alkyl, haloalkoxy, substituted alkyl, cycloalkyl, cycloalkyleneNR^{d}R^{e} or cycloalkylene(alkylene)NR^{d}R^{e} (where R^{d} and R^{e} are independently hydrogen, alkyl, or cycloalkyl), or R^{c} is 3 to 6 membered saturated monocyclic heterocyclyl containing one or two heteroatoms selected from N, O, and S and is optionally substituted with one or two substituents independently selected from hydroxy, alkyl and fluoro; or
(b) a compound chosen from:
N-(2-(4-amino-3-(2-fluoro-4-phenoxyphenyl)-1H-pyrazolo[3,4-d]pyrimidin-1-yl)ethyl)-1-cyano-2-cyclopropylethenesulfonamide;
N-(2-(4-amino-3-(2-fluoro-4-phenoxyphenyl)-1H-pyrazolo[3,4-d]pyrimidin-1-yl)ethyl)-1-cyano-2-cyclopropyl-N-methylethenesulfonamide;
2-(4-amino-3-(2-fluoro-4-phenoxyphenyl)-1H-pyrazolo[3,4-d]pyrimidin-1-yl)ethyl 2-cyano-3-cyclopropylacrylate;
1-(4-amino-3-(2-fluoro-4-phenoxyphenyl)-1H-pyrazolo[3,4-d]pyrimidin-1-yl)-2-methylpropan-2-yl 2-cyano-3-cyclopropylacrylate;
2-((2-(4-amino-3-(2-fluoro-4-phenoxyphenyl)-1H-pyrazolo[3,4-d]pyrimidin-1-yl)ethyl)sulfonyl)-3-cyclopropylacrylonitrile; and
2-(5-((4-amino-3-(2-fluoro-4-phenoxyphenyl)-1H-pyrazolo[3,4-d]pyrimidin-1-yl)methyl)oxazol-2-yl)-3-cyclopropylacrylonitrile;
and pharmaceutically acceptable salts thereof.

2. A solid oral formulation according to Claim 1 wherein:
R^{c} is alkyl, haloalkoxy, substituted alkyl, cycloalkyl, cycloalkyleneNR^{d}R^{e} (where R^{d} and R^{e} are independently hydrogen, alkyl, or cycloalkyl), or 3 to 6 membered saturated monocyclic heterocyclyl containing one or two heteroatoms selected from N, O, and S and optionally substituted with one or two substituents independently selected from hydroxy, alkyl and fluoro; and

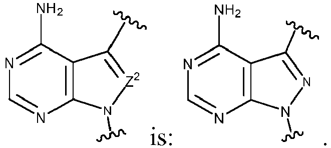

3. A solid oral formulation according to Claim 2 wherein: 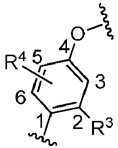 is a ring of formula: 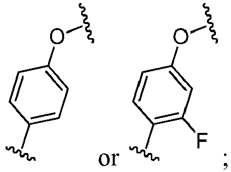 and 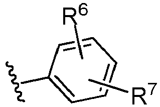 is a ring of formula:

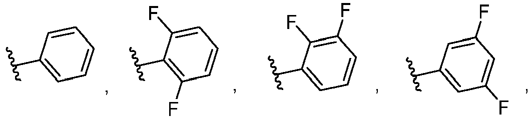

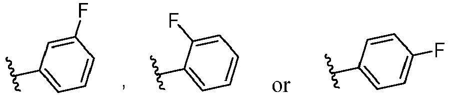

4. A solid oral formulation according to Claim 3 wherein:
Z is 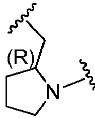 optionally substituted with one or two substituents independently selected from alkyl, hydroxy, and halo; and
R^{c} is -C(CH₃)₂NH₂, -C(CH₃)₂NHCH₃, -C(CH₃)₂NHCH₂CH₃, - C(CH₃)₂NHCH(CH₃)₂, -C(CH₃)₂NHcyclopropyl, -C(CH₃)₂NH(CH₂)₂OCH₃, or-C(CH₃)₂morpholine-4-yl.

5. A solid oral formulation according to Claim 3 wherein:
Z is 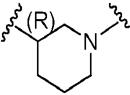 which is optionally substituted with one or two substituents independently selected from alkyl, hydroxy, and halo; and
R^{c} is isopropyl, tert-butyl, -C(CH₃)₂NH₂, -C(CH₃)₂NHCH₃, -C(CH₃)₂NHCH₂CH₃, - C(CH₃)₂NHCH(CH₃)₂, -C(CH₃)₂NHcyclopropyl, -C(CH₃)₂NH(CH₂)₂OCH₃, - C(CH₃)₂OCH₂CH₃, -C(CH₃)₂N(CH₃)₂, -C(CH₃)₂morpholine-4-yl, cyclopropyl, 2-pyrrolidinyl, 3- or 4-piperidinyl, 1-methylpiperidin-4-yl, 1-methylpiperidin-3-yl, or 4-tetrahydropyranyl.

6. A solid oral formulation according to Claim 3 wherein: 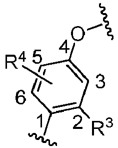 is a ring of formula: 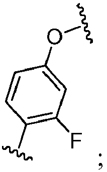 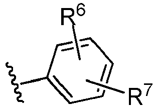 is a ring of formula: 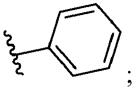 Z is 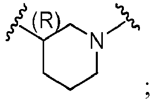 and
R^{c} is a C₁-C₆ linear alkyl or a C₃-C₆ branched alkyl, wherein the C₁-C₆ linear alkyl or C₃-C₆ branched alkyl is substituted with heterocycloamino.

7. A solid oral formulation according to Claim 1 wherein the compound is:
(R)-2-(2-((4-amino-3-(4-phenoxyphenyl)-1H-pyrazolo[3,4-d]pyrimidin-1-yl)methyl)pyrrolidine-1-carbonyl)-4,4-dimethylpent-2-enenitrile;
(S)-2-(2-((4-amino-3-(4-phenoxyphenyl)-1H-pyrazolo[3,4-d]pyrimidin-1-yl)methyl)pyrrolidine-1-carbonyl)-4,4-dimethylpent-2-enenitrile;
(R)-2-(2-((4-amino-3-(4-(3-fluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(4-(3-fluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(4-(3,5-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(4-(3,5-difluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-fluoro-4-phenoxyphenyl)-1H-pyrazolo[3,4-d]pyrimidin-l-yl)methyl)pyrrolidine-1-carbonyl)-3-cyclopropylacrylonitrile;
(R)-2-(2-((4-amino-3-(4-(2,3-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(4-(2,3-difluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(4-(2,6-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(4-(2,6-difluorophenoxy)phenyl)-1H-pyrazolo[3,4-d]pyrimidin-1-yl)methyl)pyrrolidine-1-carbonyl)-3-cyclopropylacrylonitrile;
N-(2-(4-amino-3-(4-phenoxyphenyl)-1H-pyrazolo[3,4-d]pyrimidin-1-yl)ethyl)-2-cyano-3-cyclopropylacrylamide;
(R)-2-(2-((4-amino-3-(4-(2-fluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(4-(2-fluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(4-(2,5-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(4-(2,5-difluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(2-fluoro-4-(3,5-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-fluoro-4-(3,5-difluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(3-fluoro-4-(phenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(3-fluoro-4-(phenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(2-fluoro-4-(2,3-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-fluoro-4-(2,3-difluorophenoxy)phenyl)-1H-pyrazolo[3,4-d]pyrimidin-1-yl)methyl)pyrrolidine-1-carbonyl)-3-cyclopropylacrylonitrile;
(R)-2-(2-((4-amino-3-(2-fluoro-4-(2,6-difluorophenoxy)phenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-fluoro-4-(2,6-difluorophenoxy)phenyl)-1H-pyrazolo[3,4-d]pyrimidin-1-yl)methyl)pyrrolidine-1-carbonyl)-3-cyclopropylacrylonitrile;
N-(2-(4-amino-3-(2-fluoro-4-phenoxyphenyl)-1H-pyrazolo[3,4-d]pyrimidin-1-yl)ethyl)-2-cyano-3-cyclopropylacrylamide;
N-(2-(4-amino-3-(2-fluoro-4-phenoxyphenyl)-1H-pyrazolo[3,4-d]pyrimidin-1-yl)-2-methylpropyl)-2-cyano-3-cyclopropylacrylamide;
N-(1-(4-amino-3-(2-fluoro-4-phenoxyphenyl)-1H-pyrazolo[3,4-d]pyrimidin-1-yl)-2-methylpropan-2-yl)-2-cyano-3-cyclopropylacrylamide;
N-(2-(4-amino-3-(2-fluoro-4-phenoxyphenyl)-1H-pyrazolo[3,4-d]pyrimidin-1-yl)ethyl)-1-cyano-2-cyclopropylethenesulfonamide;
N-(2-(4-amino-3-(2-fluoro-4-phenoxyphenyl)-1H-pyrazolo[3,4-d]pyrimidin-1-yl)ethyl)-1-cyano-2-cyclopropyl-N-methylethenesulfonamide;
2-(4-amino-3-(2-fluoro-4-phenoxyphenyl)-1H-pyrazolo[3,4-d]pyrimidin-1-yl)ethyl 2-cyano-3-cyclopropylacrylate;
1-(4-amino-3-(2-fluoro-4-phenoxyphenyl)-1H[3,4-d]pyrimidin-1-yl)-2-methylpropan-2-yl 2-cyano-3-cyclopropylacrylate;
2-((2-(4-amino-3-(2-fluoro-4-phenoxyphenyl)-1H-pyrazolo[3,4-d]pyrimidin-1-yl)ethyl)sulfonyl)-3-cyclopropylacrylonitrile;
2-(5-((4-amino-3-(2-fluoro-4-phenoxyphenyl)-1H-pyrazolo[3,4-d]pyrimidin-1-yl)methyl)oxazol-2-yl)-3-cyclopropylacrylonitrile;
(R)-2-(3-(4-amino-5-(4-(3,5-difluorophenoxy)phenyl)-7H-pyrrolo[2,3-d]pyrimidin-7-yl)piperidine-1-carbonyl)-3-cyclopropylacrylonitrile;
(S)-2-(3-(4-amino-5-(4-(3,5-difluorophenoxy)phenyl)-7H-pyrrolo[2,3-d]pyrimidin-7-yl)piperidine-1-carbonyl)-3-cyclopropylacrylonitrile;
(R)-2-(3-(4-amino-5-(2-fluoro-4-phenoxyphenyl)-7H-pyrrolo[2,3-d]pyrimidin-7-yl)piperidine-1-carbonyl)-3-cyclopropylacrylonitrile;
(S)-2-(3-(4-amino-5-(2-fluoro-4-phenoxyphenyl)-7H-pyrrolo[2,3-d]pyrimidin-7-yl)piperidine-1-carbonyl)-3-cyclopropylacrylonitrile;
(R)-2-(2-((4-amino-5-(4-phenoxyphenyl)-7H-pyrrolo[2,3-d]pyrimidin-7-yl)methyl)pyrrolidine-1-carbonyl)-3-cyclopropylacrylonitrile;
(S)-2-(2-((4-amino-5-(4-phenoxyphenyl)-7H-pyrrolo[2,3-d]pyrimidin-7-yl)methyl)pyrrolidine-1-carbonyl)-3-cyclopropylacrylonitrile;
(R)-2-(2-((4-amino-5-(4-(3,5-difluorophenoxy)phenyl)-7H-pyrrolo[2,3-d]pyrimidin-7-yl)methyl)pyrrolidine-1-carbonyl)-3-cyclopropylacrylonitrile;
(S)-2-(2-((4-amino-5-(4-(3,5-difluorophenoxy)phenyl)-7H-pyrrolo[2,3-d]pyrimidin-7-yl)methyl)pyrrolidine-1-carbonyl)-3-cyclopropylacrylonitrile;
(R)-2-(2-((4-amino-5-(2-fluoro-4-phenoxyphenyl)-7H-pyrrolo[2,3-d]pyrimidin-7-yl)methyl)pyrrolidine-1-carbonyl)-3-cyclopropylacrylonitrile;
(S)-2-(2-((4-amino-5-(2-fluoro-4-phenoxyphenyl)-7H-pyrrolo[2,3-d]pyrimidin-7-yl)methyl)pyrrolidine-1-carbonyl)-3-cyclopropylacrylonitrile;
(R)-2-(3-(4-amino-6-methyl-5-(4-phenoxyphenyl)-7H-pyrrolo[2,3-d]pyrimidin-7-yl)piperidine-1-carbonyl)-3-cyclopropylacrylonitrile;
(S)-2-(3-(4-amino-6-methyl-5-(4-phenoxyphenyl)-7H-pyrrolo[2,3-d]pyrimidin-7-yl)piperidine-1-carbonyl)-3-cyclopropylacrylonitrile;
(R)-2-(3-(4-amino-5-(4-(3,5-difluorophenoxy)phenyl)-6-methyl-7H-pyrrolo[2,3-d]pyrimidin-7-yl)piperidine-1-carbonyl)-3-cyclopropylacrylonitrile;
(S)-2-(3-(4-amino-5-(4-(3,5-difluorophenoxy)phenyl)-6-methyl-7H-pyrrolo[2,3-d]pyrimidin-7-yl)piperidine-1-carbonyl)-3-cyclopropylacrylonitrile;
2-((3R)-3-(4-amino-5-(2-fluoro-4-phenoxyphenyl)-6-methyl-7H-pyrrolo[2,3-d]pyrimidin-7-yl)piperidine-1-carbonyl)-3-cyclopropylacrylonitrile;
2-((3S)-3-(4-amino-5-(2-fluoro-4-phenoxyphenyl)-6-methyl-7H-pyrrolo[2,3-d]pyrimidin-7-yl)piperidine-1-carbonyl)-3-cyclopropylacrylonitrile;
(R)-(2-(2-((4-amino-6-methyl-5-(4-phenoxyphenyl)-7H-pyrrolo[2,3-d]pyrimidin-7-yl)methyl)pyrrolidine-1-carbonyl)-3-cyclopropylacrylonitrile;
(S)-(2-(2-((4-amino-6-methyl-5-(4-phenoxyphenyl)-7H-pyrrolo[2,3-d]pyrimidin-7-yl)methyl)pyrrolidine-1-carbonyl)-3-cyclopropylacrylonitrile;
(R)-2-(2-((4-amino-5-(4-(3,5-difluorophenoxy)phenyl)-6-methyl-7H-pyrrolo[2,3-d]pyrimidin-7-yl)methyl)pyrrolidine-1-carbonyl)-3-cyclopropylacrylonitrile;
(S)-2-(2-((4-amino-5-(4-(3,5-difluorophenoxy)phenyl)-6-methyl-7H-pyrrolo[2,3-d]pyrimidin-7-yl)methyl)pyrrolidine-1-carbonyl)-3-cyclopropylacrylonitrile;
(R)-2-(2-((4-amino-5-(2-fluoro-4-phenoxyphenyl)-6-methyl-7H-pyrrolo[2,3-d]pyrimidin-7-yl)methyl)pyrrolidine-1-carbonyl)-3-cyclopropylacrylonitrile; or
(S)-2-(2-((4-amino-5-(2-fluoro-4-phenoxyphenyl)-6-methyl-7H-pyrrolo[2,3-d]pyrimidin-7-yl)methyl)pyrrolidine-1-carbonyl)-3-cyclopropylacrylonitrile;
N-(3-(4-amino-3-(4-phenoxyphenyl)-1H-pyrazolo[3,4-d]pyrimidin-1-yl)-2,2-dimethylpropyl)-2-cyano-3-cyclopropylacrylamide;
(R)-2-(2-((4-amino-3-(2-methyl-4-phenoxyphenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-methyl-4-phenoxyphenyl)-1H-pyrazolo[3,4-d]pyrimidin-1-yl)methyl)pyrrolidine-1-carbonyl)-3-cyclopropylacrylonitrile;
(R)-2-(2-((4-amino-3-(2-chloro-4-phenoxyphenyl)-1H-pyrazolo[3,4-d]pyrimidin-1-yl)methyl)pyrrolidine-1-carbonyl)-3-cyclopropylacrylonitrile;
(S)-2-(2-((4-amino-3-(2-chloro-4-phenoxyphenyl)-1H-pyrazolo[3,4-d]pyrimidin-1-yl)methyl)pyrrolidine-1-carbonyl)-3-cyclopropylacrylonitrile;
(R)-2-(2-((4-amino-3-(4-(2,5-difluorophenoxy)phenyl)-1H-pyrazolo[3,4-d]pyrimidin-1-yl)methyl)pyrrolidine-1-carbonyl)-4-methylpent-2-enenitrile;
(S)-2-(2-((4-amino-3-(4-(2,5-difluorophenoxy)phenyl)-1H-pyrazolo[3,4-d]pyrimidin-1-yl)methyl)pyrrolidine-1-carbonyl)-4-methylpent-2-enenitrile;
(R)-4-amino-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile;
(S)-4-amino-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile;
(R)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile;
(S)-2-(2-((4-amino-3-(2-fluoro-4-(3-fluorophenoxy)phenyl)-1H-pyrazolo[3,4-d]pyrimidin-1-yl)methyl)pyrrolidine-1-carbonyl)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4,4-dimethylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4,4-dimethylpent-2-enenitrile;
(R)-2-(2-((4-amino-3-(4-(2,6-difluorophenoxy)phenyl)-1H-pyrazolo[3,4-d]pyrimidin-1-yl)methyl)pyrrolidine-1-carbonyl)-4-methylpent-2-enenitrile;
(S)-2-(2-((4-amino-3-(4-(2,6-difluorophenoxy)phenyl)-1H-pyrazolo[3,4-d]pyrimidin-1-yl)methyl)pyrrolidine-1-carbonyl)-4-methylpent-2-enenitrile;
(R)-2-(2-((4-amino-3-(4-(2,3-difluorophenoxy)phenyl)-1H-pyrazolo[3,4-d]pyrimidin-1-yl)methyl)pyrrolidine-1-carbonyl)-4-methylpent-2-enenitrile;
(S)-2-(2-((4-amino-3-(4-(2,3-difluorophenoxy)phenyl)-1H-pyrazolo[3,4-d]pyrimidin-1-yl)methyl)pyrrolidine-1-carbonyl)-4-methylpent-2-enenitrile;
(R)-2-(2-((4-amino-6-methyl-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(S)-2-(2-((4-amino-6-methyl-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(R)-2-(2-((4-amino-3-(4-(2,6-difluorophenoxy)-2-fluorophenyl)-1H-pyrazolo[3,4-d]pyrimidi-1-yl)methyl)pyrrolidine-1-carbonyl)-4-methylpent-2-enenitrile;
(S)-2-(2-((4-amino-3-(4-(2,6-difluorophenoxy)-2-fluorophenyl)-1H-pyrazolo[3,4-d]pyrimidin-1-yl)methyl)pyrrolidine-1-carbonyl)-4-methylpent-2-enenitrile;
(R)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-3-cyclopropylacrylonitrile;
(S)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-3-cyclopropylacrylonitrile;
(R)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-4-methylpent-2-enenitrile;
(S)-2-(2-((4-amino-3-(2-fluoro-4-phenoxyphenyl)-1H-pyrazolo[3,4-d]pyrimidin-1-yl)methyl)-4,4-difluoropyrrolidine-1-carbonyl)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenirile;
(R)-4-amino-2-(2-((4-amino-3-(2-fluoro-4-phenoxyphenyl)-1H-pyrazolo[3,4-d]pyrimidin-1-yl)methyl)pyrrolidine-1-carbonyl)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(methylamino)pent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(methylamino)pent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(dimethylamino)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(dimethylamino)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(ethylamino)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(ethylamino)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(isopropylamino)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(isopropylamino)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-(cyclopropylamino)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-(cyclopropylamino)-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-((2-methoxyethyl)amino)-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-((2-methoxyethyl)amino)-4-methylpent-2-enenitrile
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-ethoxy-4-methylpent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-ethoxy-4-methylpent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-aminocyclopropyl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-aminocyclopropyl)acrylonitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(methylamino)cyclopropyl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(methyl amino)cyclopropyl)acrylonitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(ethylamino)cyclopropyl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(ethylamino)cyclopropyl)acrylonitrile;
(S)-2-(2-((4-ammo-3-(2-fluoro-4-phenoxyphenyl)-1H-pyrazolo[3,4-d]pyrimidin-1-yl)methyl)pyrrolidine-1-carbonyl)-3-(1-(isopropylamino)cyclopropyl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-(isopropylamino)cyclopropyl)acrylonitrile;
2-((S)-2-((4-amino-3-(2-fluoro-4-phenoxyphenyl)-1H-pyrazolo[3,4-d]pyrimidin-1-yl)methyl)pyrrolidine-1-carbonyl)-3-(pyrrolidin-2-yl)acrylonitrile;
2-((R)-2-((4-amino-3-(2-fluoro-4-phenoxyphenyl)-1H-pyrazolo[3,4-d]pyrimidin-1-yl)methyl)pyrrolidine-1-carbonyl)-3-(pyrrolidin-2-yl)acrylonitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-morpholinopent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-((dimethylamino)methyl)cyclopentyl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-((dimethylamino)methyl)cyclopentyl)acrylonitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-methylpiperidin-4-yl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(1-methylpiperidin-4-yl)acrylonitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(tetrahydro-2H-pyran-4-yl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(tetrahydro-2H-pyran-4-yl)acrylonitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-3-(piperidin-4-yl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-3-(piperidin-4-yl)acrylonitrile;
2-((S)-2-((4-amino-3-(2-fluoro-4-phenoxyphenyl)-1H-pyrazolo[3,4-d]pyrimidin-1-yl)methyl)pyrrolidine-1-carbonyl)-3-(piperidin-3-yl)acrylonitrile;
2-((R)-2-((4-amino-3-(2-fluoro-4-phenoxyphenyl)-1H-pyrazolo[3,4-d]pyrimidin-1-yl)methyl)pyrrolidine-1-carbonyl)-3-(piperidin-3-yl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4,4-dimethylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4,4-dimethylpent-2-enenitrile;
(S)-4-amino-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile;
(R)-4-amino-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-(methylamino)pent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-(methylamino)pent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(dimethylamino)-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(dimethylamino)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(ethylamino)-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(ethylamino)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(isopropylamino)-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(isopropylamino)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(cyclopropylamino)-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-(cyclopropylamino)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-((2-methoxyethyl)amino)-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-((2-methoxyethyl)amino)-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-ethoxy-4-methylpent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-ethoxy-4-methylpent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-aminocyclopropyl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-aminocyclopropyl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(methylamino)cyclopropyl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(methylamino)cyclopropyl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(ethylamino)cyclopropyl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(ethylamino)cyclopropyl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(isopropylamino)cyclopropyl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-(isopropylamino)cyclopropyl)acrylonitrile;
2-((S)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(pyrrolidin-2-yl)acrylonitrile;
2-((R)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(pyrrolidin-2-yl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-morpholinopent-2-enenitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-4-methyl-4-morpholinopent-2-enenitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((diethylamino)methyl)cyclopentyl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((diethylamino)methyl)cyclopentyl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((dimethylamino)methyl)cyclopentyl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-((dimethylamino)-methyl)cyclopentyl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-methylpiperidin-4-yl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(1-methylpiperidin-4-yl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(tetrahydro-2H-pyran-4-yl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(tetrahydro-2H-pyran-4-yl)acrylonitrile;
(S)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-4-yl)acrylonitrile;
(R)-2-(2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-4-yl)acrylonitrile;
2-((S)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-3-yl)acrylonitrile;
2-((R)-2-((4-amino-3-(4-(2,3-difluorophenoxy)-2-fluorophenyl)-1H-pyrazolo[3,4-d]pyrimidin-1-yl)methyl)pyrrolidine-1-carbonyl)-3-(piperidin-3-yl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
(R)-4-amino-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile;
(S)-4-amino-2-(3-(4-amino-3-(2-fluoro-4-phenoxyphenyl)-1H-pyrazolo[3,4-d]pyrimidin-1-yl)piperidine-1-carbonyl)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-methyl-4-(methylamino)pent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-methyl-4-(methylamino)pent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(dimethylamino)-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(dimethylamino)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-(ethylamino)-4-methylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-(ethylamino)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-(isopropylamino)-4-methylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-(isopropylamino)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-(cyclopropylamino)-4-methylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-(cyclopropylamino)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-((2-methoxyethyl)amino)-4-methylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-((2-methoxyethyl)amino)-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-4-ethoxy-4-methylpent-2-enenitrile;
(S)-2-(3-(4-amino-3-(2-fluoro-4-phenoxyphenyl)-1H-pyrazolo[3,4-d]pyrimidin-1-yl)piperidine-1-carbonyl)-4-ethoxy-4-methylpent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-aminocyclopropyl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(1-aminocyclopropyl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-(methylamino)cyclopropyl)acrylomtrile;
(S)-2-(3-(4-amino-3-(2-fluoro-4-phenoxyphenyl)-1H-pyrazolo[3,4-d]pyrimidin-1-yl)piperidine-1-carbonyl)-3-(1-(methylamino)cyclopropyl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-(ethylamino)cyclopropyl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(1-(ethylamino)cyclopropyl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-(isopropylamino)cyclopropyl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(1-(isopropylamino)cyclopropyl)acrylonitrile;
2-((R)-3-(4-amino-3-(2-fluoro-4-phenoxyphenyl)-1H-pyrazolo[3,4-d]pyrimidin-1-yl)piperidine-1-carbonyl)-3-(pyrrolidin-2-yl)acrylonitrile;
2-((S)-3-(4-amino-3-(2-fluoro-4-phenoxyphenyl)-1H-pyrazolo[3,4-d]pyrimidin-1-yl)piperidine-1-carbonyl)-3-(pyrrolidin-2-yl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-4-methyl-4-morpholinopent-2-enenitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-((diethylamino)methyl)-cyc1opentyl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(1-((diethylamino)methyl)-cyclopentyl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-((dimethylamino)methyl)cyclopentyl)-acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(1-((dimethylamino)methyl)cyclopentyl)-acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(1-methylpiperidin-4-yl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(1-methylpiperidin-4-yl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(tetrahydro-2H-pyran-4-yl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(tetrahydro-2H-pyran-4-yl)acrylonitrile;
(R)-2-(3-(4-amino-3-(2-fluoro-4-phenoxyphenyl)-1H-pyrazolo[3,4-d]pyrimidin-1-yl)piperidine-1-carbonyl)-3-(piperidin-4-yl)acrylonitrile;
(S)-2-(3-(4-amino-3-(2-fluoro-4-phenoxyphenyl)-1H-pyrazolo[3,4-d]pyrimidin-1-yl)piperidine-1-carbonyl)-3-(piperidin-4-yl)acrylonitrile;
2-((R)-3-(4-amino-3-(2-fluoro-4-phenoxyphenyl)-1H-pyrazolo[3,4-d]pyrimidin-1-yl)piperidine-1-carbonyl)-3-(piperidin-3-yl)acrylonitrile;
2-((S)-3-(4-amino-3-(2-fluoro-4-phenoxyphenyl)-1H-pyrazolo[3,4-d]pyrimidin-1-yl)piperidine-1-carbonyl)-3-(piperidin-3-yl)acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-cyc1opropylacrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-cyc1opropylacrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile;
(S)-2-(3-(4-amino-3-(4-(2,3-difluorophenoxy)-2-fluorophenyl)-lH-pyrazolo[3,4-d]pyrimidin-1-yl)piperidine-1-carbonyl)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4,4-dimethylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4,4-dimethylpent-2-enenitrile;
(R)-4-amino-2-(3-(4-amino-3-(4-(2,3-difluorophenoxy)-2-fluorophenyl)-1H-pyrazolo[3,4-d]pyrimidin-1-yl)piperidine-1-carbonyl)-4-methylpent-2-enenitrile;
(S)-4-amino-2-(3-(4-amino-3-(4-(2,3-difluorophenoxy)-2-fluorophenyl)-1H-pyrazolo[3,4-d]pyrimidm-1-yl)piperidme-1-carbonyl)-4-methylpent-2-enemtrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-(methylamino)pent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-(methylamino)pent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(dimethylamino)-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(dimethylamino)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(ethylamino)-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(ethylamino)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(isopropylamino)-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(isopropylamino)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-(cyclopropylamino)-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-(cyclopropylamino)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-((2-methoxyethyl)amino)-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-((2-methoxyethyl)amino)-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-ethoxy-4-methylpent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-ethoxy-4-methylpent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-aminocyclopropyl)-acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-aminocyclopropyl)-acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(methylamino)cyclopropyl)-acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(methylamino)cyclopropyl)-acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(ethylamino)cyclopropyl)-acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(ethylamino)cyclopropyl)-acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(isopropylamino)-cyclopropyl)acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-(isopropylamino)-cyclopropyl)acrylonitrile;
2-((R)-3-(4-amino-3-(4-(2,3-difluorophenoxy)-2-fluorophenyl)-1H-pyrazolo[3,4-d]pyrimidin-1-yl)piperidine-1-carbonyl)-3-(pyrrolidin-2-yl)acrylonitrile;
2-((S)-3-(4-amino-3-(4-(2,3-difluorophenoxy)-2-fluorophenyl)-1H-pyrazolo[3,4-d]pyrimidin-1-yl)piperidine-1-carbonyl)-3-(pyrrolidin-2-yl)acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-morpholinopent-2-enenitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-4-methyl-4-morpholinopent-2-enenitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((diethylamino)methyl)-cyclopentyl)acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((diethylamino)methyl)-cyclopentyl)acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((dimethylamino)-methyl)cyclopentyl)acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-((dimethylamino)-methyl)cyclopentyl)acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(1-methylpiperidin-4-yl)acrylonitrile;
(S)-2-(3-(4-amino-3-(4-(2,3-difluorophenoxy)-2-fluorophenyl)-1H-pyrazolo[3,4-d]pyrimidin-1-yleridine-1-carbonyl)-3-(1-methylpiperidin-4-yl)acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(tetrahydro-2H-pyran-4-yl)acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(tetrahydro-2H-pyran-4-yl)acrylonitrile;
(R)-2-(3-(4-amino-3-(4-(2,3-difluorophenoxy)-2-fluorophenyl)-1H-pyrazolo[3,4-d]pyrimidin-1-yl)piperidine-1-carbonyl)-3-(piperidin-4-yl)acrylonitrile;
(S)-2-(3-(4-amino-3-(4-(2,3-difluorophenoxy)-2-fluorophenyl)-1H-pyrazolo[3,4-d]pyrimidin-1-yl)piperidine-1-carbonyl)-3-(piperidin-4-yl)acrylonitrile;
2-((R)-3-(4-amino-3-(4-(2,3-difluorophenoxy)-2-fluorophenyl)-1H-pyrazolo[3,4-d]pyrimidin-1-yl)piperidine-1-carbonyl)-3-(piperidin-3-yl)acrylonitrile;
2-((S)-3-(4-amino-3-(4-(2,3-difluorophenoxy)-2-fluorophenyl)-1H-pyrazolo[3,4-d]pyrimidin-1-yl)piperidine-1-carbonyl)-3-(piperidin-3-yl)acrylonitrile;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile;
(R)-2-(2-((4-amino-5-(2-fluoro-4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(S)-2-(2-((4-amino-5-(2-fluoro-4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-3-cyclopropylacrylonitrile;
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-3-cyclopropylacrylonitrile;
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-4-methylpent-2-enenitrile;
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-4-methylpent-2-enenitrile;
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-4,4-dimethylpent-2-enenitrile;
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-4,4-dimethylpent-2-enenitrile;
(R)-2-(2-((4-amino-5-(4-(2,3-difluorophenoxy)phenyl)-7H-pyrrolo[2,3-d]pyrimidin-7-yl)methyl)pyrrolidine-1-carbonyl)-4-(dimethylamino)-4-methylpent-2-enenitrile;
(S)-2-(2-((4-amino-5-(4-(2,3-difluorophenoxy)phenyl)-7H-pyrrolo[2,3-d]pyrimidin-7-yl)methyl)pyrrolidine-1-carbonyl)-4-(dimethylamino)-4-methylpent-2-enenitrile;
(R)-2-(2-((4-amino-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(S)-2-(2-((4-amino-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(R)-2-(2-((4-amino-5-(4-phenoxyphenyl)-7H-pyrrolo[2,3-d]pyrimidin-7-yl)methyl)pyrrolidine-1-carbonyl)-4-methylpent-2-enenitrile;
(S)-2-(2-((4-amino-5-(4-phenoxyphenyl)-7H-pyrrolo[2,3-d]pyrimidin-7-yl)methyl)pyrrolidine-1-carbonyl)-4-methylpent-2-enenitrile;
(R)-4-amino-2-(2-((4-amino-5-(2-fluoro-4-phenoxyphenyl)-7H-pyrrolo[2,3-d]pyrimidin-7-yl)methyl)pyrrolidine-1-carbonyl)-4-methylpent-2-enenitrile;
(S)-4-amino-2-(2-((4-amino-5-(2-fluoro-4-phenoxyphenyl)-7H-pyrrolo[2,3-d]pyrimidm-7-yl)methyl)pyrrolidine-1-carbonyl)-4-methylpent-2-enenitrile;
(R)-2-(2-((4-amino-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(S)-2-(2-((4-amino-5-(4-phenoxyphenyl)-7H-pyrrolo[2,3-d]pyrimidin-7-yl)methyl)pyrrolidine-1-carbonyl)-4-(dimethylamino)-4-methylpent-2-enenitrile;
(R)-2-(2-((4-amino-5-(4-phenoxyphenyl)-7H-pyrrolo[2,3-d]pyrimidin-7-yl)methyl)pyrrolidine-1-carbonyl)-4-methyl-4-morpholinopent-2-enenitrile
(S)-2-(2-((4-amino-5-(4-phenoxyphenyl)-7H-pyrrolo[2,3-d]pyrimidin-7-yl)methyl)pyrrolidine-1-carbonyl)-4-methyl-4-morpholinopent-2-enenitrile;
(R)-2-(2-((4-amino-5-(4-phenoxyphenyl)-7H-pyrrolo[2,3-d]pyrimidin-7-yl)methyl)pyrrolidine-1-carbonyl)-4-(diethylamino)-4-methylpent-2-enenitrile;
(S)-2-(2-((4-amino-5-(4-phenoxyphenyl)-7H-pyrrolo[2,3-d]pyrimidin-7-yl)methyl)pyrrolidine-1-carbonyl)-4-(diethylamino)-4-methylpent-2-enenitrile;
(R)-2-(2-((4-amino-5-(4-phenoxyphenyl)-7H-pyrrolo[2,3-d]pyrimidin-7-yl)methyl)pyrrolidine-1-carbonyl)-4-ethoxy-4-methylpent-2-enenitrile;
(S)-2-(2-((4-amino-5-(4-phenoxyphenyl)-7H-pyrrolo[2,3-d]pyrimidin-7-yl)methyl)pyrrolidine-1-carbonyl)-4-ethoxy-4-methylpent-2-enenitrile;
(R)-N-(1-(4-amino-3-(2-fluoro-4-phenoxyphenyl)-1H-pyrazolo[3,4-d]pyrimidin-1-yl)propan-2-yl)-2-cyano-4,4-dimethylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4,4-dimethylpent-2-enamide
(R)-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide;
(R)-N-(1-(4-amino-3-(2-fluoro-4-phenoxyphenyl)-1H-pyrazolo[3,4-d]pyrimidin-1-yl)propan-2-yl)-2-cyano-4-ethoxy-4-methylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4-ethoxy-4-methylpent-2-enamide;
(R)-N-(1-(4-amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamide;
2-((R)-2-((4-amino-3-(2-fluoro-4-phenoxyphenyl)-1H-pyrazolo[3,4-d]pyrimidin-1-yl)methyl)pyrrolidine-1-carbonyl)-3-((S)-pyrrolidin-2-yl)acrylonitrile;
2-((R)-2-((4-amino-3-(2-fluoro-4-phenoxyphenyl)-1H-pyrazolo[3,4-d]pyrimidin-1-yl)methyl)pyrrolidine-1-carbonyl)-3-((R)-pyrrolidin-2-yl)acrylonitrile;
(R)-4-amino-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide;
(S)-4-amino-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide;
N-(2-(4-amino-3-(4-phenoxyphenyl)-1H-pyrazolo[3,4-d]pyrimidin-1-yl)ethyl)-2-cyano-3-cyclopropyl-N-methylacrylamide;
(R)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile;
(S)-2-(2-((4-amino-3-(2-fluoro-4-phenoxyphenyl)-1H-pyrazolo[3,4-d]pyrimidin-1-yl)methyl)pyrrolidine-1-carbonyl)-4-methyl-4-(piperidin-1-yl)pent-2-enenitrile;
(S)-N-(1-(4-amino-3-(2-fluoro-4-phenoxyphenyl)-1H-pyrazolo[3,4-d]pyrimidin-1-yl)propan-2-yl)-2-cyano-4,4-dimethylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-4-ethoxy-4-methylpent-2-enamide;
(S)-4-amino-N-(1-(4-amino-3-(2-fluoro-4-phenoxyphenyl)-1H-pyrazolo[3,4-d]pyrimidin-1-yl)propan-2-yl)-2-cyano-4-methylpent-2-enamide;
(S)-N-(1-(4-amino-3-(2-fluoro-4-phenoxyphenyl)-1H-pyrazolo[3,4-d]pyrimidin-1-yl)propan-2-yl)-2-cyano-3-cyclopropylacrylamide;
2-[(2R)-2-[[4-amino-3-(2-fluoro-4-phenoxy-phenyl)pyrazolo[3,4-d]pyrimidin-1-yl]methyl]-pyrrolidine-1-carbonyl]-3-(3-methyloxetan-3-yl)prop-2-enenitrile; or
2-[(2S)-2-[[4-amino-3-(2-fluoro-4-phenoxy-phenyl)pyrazolo[3,4-d]pyrimidin-1-yl]methyl]-pyrrolidine-1-carbonyl]-3-(3-methyloxetan-3-yl)prop-2-enenitrile;
or a mixture of R and S isomers;
or an individual (E) or (Z) isomer thereof;
or a pharmaceutically acceptable salt of any of the foregoing.

8. A solid oral formulation according to Claim 1 wherein the compound is:
(R)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
or an individual (E) or (Z) isomer thereof;
or a pharmaceutically acceptable salt thereof.

9. A solid oral formulation according to Claim 1 wherein the compound is:
2-(2-((4-amino-3-(2-fluoro-4-phenoxyphenyl)-1H-pyrazolo[3,4-d]pyrimidin-1-yl)-methyl)pyrrolidine-1-carbonyl)-4,4-dimethylpent-2-enenitrile;
or an individual R or S isomer thereof;
or an individual (E) or (Z) isomer thereof;
or a pharmaceutically acceptable salt thereof.

10. A solid oral formulation according to any one of the preceding claims wherein the coating is an enteric coating that dissolves at a pH of from 4.5 to 7.

11. A solid oral formulation according to any one of the preceding claims wherein the coating is an enteric coating which is an anionic polymer.

12. A solid oral formulation according to Claim 11 wherein the anionic polymer is selected from polymethacrylates, cellulose-based polymers and polyvinyl derivatives.

13. A solid oral formulation according to any one of the preceding claims wherein the coating is an enteric coating that dissolves at a pH of from 5.5 to 7.

14. A solid oral formulation according to Claim 1 wherein the compound is 2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile;
or an individual R or S isomer thereof;
or an individual (E) or (Z) isomer thereof;
or a pharmaceutically acceptable salt of the foregoing compounds.

15. A formulation for use in treating a disease which is an autoimmune or inflammatory disease, or cancer, wherein the formulation is a solid oral formulation as defined in any one of Claims 1 to 14.

16. A formulation for use according to Claim 15, wherein the disease is inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjogren's syndrome, multiple sclerosis, Guillain-Barre syndrome, acute disseminated encephalomyelitis, Addison's disease, opsoclonus-myoclonus syndrome, ankylosing spondylitisis, antiphospholipid antibody syndrome, aplastic anemia, autoimmune hepatitis, coeliac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behcet's disease, chronic fatigue, dysautonomia, endometriosis, interstitial cystitis, neuromyotonia, scleroderma, or vulvodynia, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, proctitis, prostatitis, pyelonephritis, rhinitis, salpingitis, sinusitis, stomatitis, synovitis, tendonitis, tonsillitis, uveitis, vaginitis, vasculitis, or vulvitis, dermatitis, contact dermatitis, eczema, urticaria, rosacea, and scarring psoriatic lesions in the skin, joints, or other tissues or organs and the formulation is administered optionally in combination with one or more anti-inflammatory agents.

17. A formulation for use according to Claim 16, wherein the disease is lupus, rheumatoid arthritis, psoriatic arthritis, Sjogren's syndrome, Wegener's granulomatosis, psoriasis, asthma, or uveitis.

## Patentansprüche

1. Feste orale Formulierung umfassend (i) einen therapeutisch effektiven Betrag einer Verbindung, (ii) mindestens eine Beschichtung, ausgewählt aus einer enterischen Beschichtung und/oder einer nicht-enterischen zeitverzögerten Freisetzungsbeschichtung, und (iii) mindestens einen pharmazeutisch annehmbaren Hilfsstoff,
wobei die Verbindung ist:
(a) eine Verbindung der Formel (Id) oder ein pharmazeutisch annehmbares Salz davon: 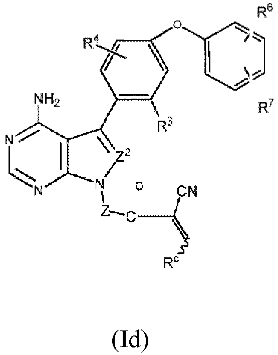 wobei:
Z² -N- oder -CR²- ist, wo R² Wasserstoff oder Alkyl ist;
R³ und R⁴ unabhängig Wasserstoff, Methyl, Chloro, Fluoro, Cyclopropyl, Hydroxy, Methoxy, Cyano, Trifluoromethyl oder Trifluoromethoxy sind;
R⁶ und R⁷ unabhängig Wasserstoff, Methyl, Methoxy, Fluoro, Chloro, Trifluoromethyl, Trifluoromethoxy, oder Cyano sind;
Z -(Alkylen)-NRa-, 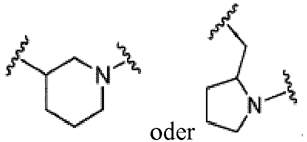 ist, jeder Ring optional substituiert mit einem oder zwei Substituenten unabhängig ausgewählt aus Alkyl, Hydroxy, und Halo, und wo R^{a} unabhängig Wasserstoff oder Alkyl ist; und
R^{c} Alkyl, Haloalkoxy, substituiertes Alkyl, Cycloalkyl, CycloalkylenNR^{d}R^{e} oder Cycloalkylen(alkylen)NR^{d}R^{e} (wo R^{d} und R^{e} unabhängig Wasserstoff, Alkyl, oder Cycloalkyl sind) ist, oder R^{c} 3 bis 6-gliedriges gesättigtes monocyclisches Heterocyclyl enthaltend ein oder zwei Heteroatome ausgewählt aus N, O, und S ist und optional mit einem oder zwei Substituenten unabhängig ausgewählt aus Hydroxy, Alkyl und Fluoro ist; oder
(b) eine Verbindung ausgewählt aus:
N-(2-(4-Amino-3-(2-fluoro-4-phenoxyphenyl)-1H-pyrazolo[3,4-d]pyrimidin-1-yl)ethyl)-1-cyano-2-cyclopropylethenesulfonamid;
N-(2-(4-Amino-3-(2-fluoro-4-phenoxyphenyl)-1H-pyrazolo[3,4-d]pyrimidin-1-yl)ethyl)-1-cyano-2-cyclopropyl-N-methylethenesulfonamid;
2-(4-Amino-3-(2-fluoro-4-phenoxyphenyl)-1H-pyrazolo[3,4-d]pyrimidin-1-yl)ethyl 2- cyano-3-cyclopropylacrylat;
1-(4-Amino-3-(2-fluoro-4-phenoxyphenyl)-1H-pyrazolo[3,4-d]pyrimidin-1-yl)-2-methylpropan-2-yl 2-cyano-3-cyclopropylacrylat;
2-((2-(4-Amino-3-(2-fluoro-4-phenoxyphenyl)-1H-pyrazolo[3,4-d]pyrimidin-1-yl)ethyl)sulfonyl)-3-cyclopropylacrylonitril; und
2-(5-((4-Amino-3-(2-fluoro-4-phenoxyphenyl)-1H-pyrazolo[3,4-d]pyrimidin-1-yl)methyl)oxazol-2-yl)-3-cyclopropylacrylonitril;
und pharmazeutisch annehmbare Salze davon.

2. Feste orale Formulierung nach Anspruch 1, wobei:
R^{c} Alkyl, Haloalkoxy, substituiertes Alkyl, Cycloalkyl, CycloalkylenNR^{d}R^{e} ist (wo R^{d} und R^{e} unabhängig Wasserstoff, Alkyl, oder Cycloalkyl sind), oder 3 bis 6-gliedriges gesättigtes monocyclisches Heterocyclyl enthaltend ein oder zwei Heteroatome ausgewählt aus N, O, und S und wahlweise substituiert mit einem oder zwei Substituenten unabhängig ausgewählt aus Hydroxy, Alkyl und Fluoro; und 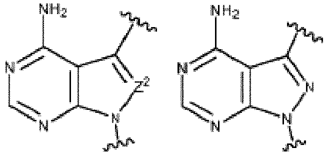 ist.

3. Feste orale Formulierung nach Anspruch 2, wobei: 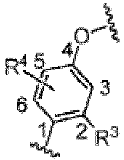 ein Ring der Formel 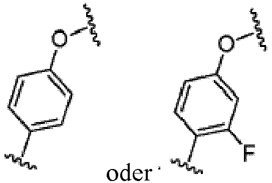 ist; und 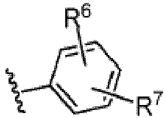 ein Ring der Formel: 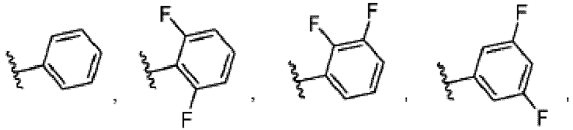 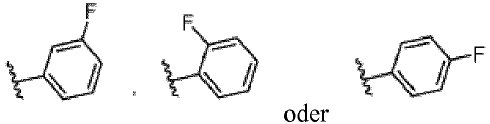 ist.

4. Feste orale Formulierung nach Anspruch 3, wobei: 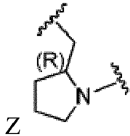 ist, optional substituiert mit einem oder zwei Substutenten unabhängig ausgewählt aus Alkyl, Hydroxy, und Halo; und
R^{c}-C(CH₃)₂NH₂, -C(CH₃)2NHCH₃, -C(CH₃)₂NHCH₂CH₃, -C(CH₃)₂NHCH(CH₃)₂, - C(CH₃)₂NHcyclopropyl, -C(CH₃)₂NH(CH₂)₂OCH₃, oder -C(CH₃)₂morpholin-4-yl ist.

5. Feste orale Formulierung nach Anspruch 3, wobei: 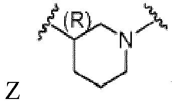 ist, das optional mit einem oder zwei Substituenten unabhängig ausgewählt aus Alkyl, Hydroxy, und Halo substuiert ist; und
R^{c} Isopropyl, Tert-butyl, -C(CH₃)₂NH₂, -C(CH₃)2NHCH₃, -C(CH₃)₂NHCH₂CH₃, - C(CH₃)₂NHCH(CH₃)₂, C(CH₃)₂NHcyclopropyl, C(CH₃)₂NH(CH₂)₂OCH₃, - C(CH₃)₂morpholin-4-yl, Cyclopropyl, 2-Pyrrolidinyl, 3- oder 4-Piperidinyl, 1-Methylpiperidin-4-yl, 1-Methylpiperidin-3-yl, oder 4-Tetrahydropyranyl ist.

6. Feste orale Formulierung nach Anspruch 3, wobei: 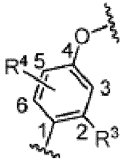 ein Ring der Formel: 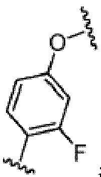 ist; 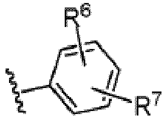 ein Ring der Formel: 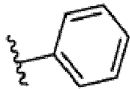 ist; 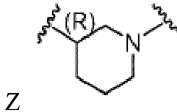 ist; und
R^{c} ein C₁-C₆ lineares Alkyl oder ein C₃-C₆ verzweigtes Alkyl ist, wobei das C₁-C₆ lineare Alkyl oder C₃-C₆ verzweigte Alkyl mit Heterocycloamino substituiert ist.

7. Feste orale Formulierung nach Anspruch 1, wobei die Verbindung ist:
(R)-2-(2-((4-Amino-3-(4-phenoxyphenyl)-1H-pyrazolo[3,4-d]pyrimidin-1-yl)methyl)pyrrolidin-1-carbonyl)-4,4-dimethylpent-2-enentril,
(S)-2-(2-((4-Amino-3-(4-phenoxyphenyl)-1H-pyrazolo[3,4-d]pyrimidin-1-yl)methyl)pyrrolidin-1-carbonyl)-4,4-dimethylpent-2-enentril,
(R)-2-(2-((4-Amino-3-(4-(3-fluorophenoxy)phenyl)-1H-pyrazolo[3,4-d]pyrimidin-1-yl)methyl)pyrrolidin-1-carbonyl)-3-cyclopropylacrylonitril,
(S)-2-(2-((4-Amino-3-(4-(3-fluorophenoxy)phenyl)-1H-pyrazolo[3,4-d]pyrimidin-1-yl)methyl)pyrrolidin-1-carbonyl)-3-cyclopropylacrylonitril,
(R)-2-(2-((4-Amino-3-(4-(3,5-difluorophenoxy)phenyl)-1H-pyrazolo[3,4-d]pyrimidin-1-yl)methyl)pyrrolidin-1-carbonyl)-3-cyclopropylacrylonitril,
(S)-2-(2-((4-Amino-3-(4-(3,5-difluorophenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-cyclopropylacrylonitril;
(R)-2-(2-((4-Amino-3-(4-(2,3-difluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(4-(2,3-difluorophenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(4-(2,6-difluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(4-(2,6-difluorophenoxy)phenyl)-1H-pyrazolo[3,4-d]pyrimidin-1-yl)methyl)pyrrolidin-1-carbonyl)-3-cyclopropylacrylonitril;
N-(2-(4-Amino-3-(4-phenoxyphenyl)-1H-pyrazolo[3,4-d]pyrimidin-1-yl)ethyl)-2-cyano-3-cyclopropylacrylamid;
(R)-2-(2-((4-Amino-3-(4-(2-fluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(4-(2-fluorophenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(4-(2,5-difluorophenoxy)phenyl)-1H-pyrazolo[3,4-d]pyrimidin-1-yl)methyl)pyrrolidin-1-carbonyl)-3-cyclopropylacrylonitril,
(S)-2-(2-((4-Amino-3-(4-(2,5-difluorophenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(2-fluoro-4-(3-fluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-fluoro-4-(3-fluorophenoxy)phenyl)-lH-pyrazolo[3,4-d]pyrimidin-1-yl)methyl)pyrrolidin-1-carbonyl)-3-cyclopropylacrylonitril
(R)-2-(2-((4-Amino-3-(2-fluoro-4-(3,5-difluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-fluoro-4-(3,5-difluorophenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(3-fluoro-4-(phenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(3-fluoro-4-(phenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(2-fluoro-4-(2,3-difluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-fluoro-4-(2,3-difluorophenoxy)phenyl)-1H-pyrazolo[3,4-d]pyrimidin-1-yl)methyl)pyrrolidin-1-carbonyl)-3-cyclopropylacrylonitril;
(R)-2-(2-((4-Amino-3-(2-fluoro-4-(2,6-difluorophenoxy)phenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-fluoro-4-(2,6-difluorophenoxy)phenyl)-1H-pyrazolo[3,4-d]pyrimidin-1-yl)methyl)pyrrolidin-1-carbonyl)-3-cyclopropylacrylonitril;
N-(2-(4-Amino-3-(2-fluoro-4-phenoxyphenyl)-1H-pyrazolo[3,4-d]pyrimidin-1-yl)ethyl)-2-cyano-3-cyclopropylacrylamid;
N-(2-(4-Amino-3-(2-fluoro-4-phenoxyphenyl)-1H-pyrazolo[3,4-d]pyrimidin-1-yl)-2-methylpropyl)-2-cyano-3-cyclopropylacrylamid;
N-(1-(4-Amino-3-(2-fluoro-4-phenoxyphenyl)-1H-pyrazolo[3,4-d]pyrimidin-1-yl)-2-methylpropan-2-yl)-2-cyano-3-cyclopropylacrylamid;
N-(2-(4-Amino-3-(2-fluoro-4-phenoxyphenyl)-1H-pyrazolo[3,4-d]pyrimidin-1-yl)ethyl)-1-cyano-2-cyclopropylethenesulfonamid;
N-(2-(4-Amino-3-(2-fluoro-4-phenoxyphenyl)-1H-pyrazolo[3,4-d]pyrimidin-1-yl)ethyl)-1-cyano-2-cyclopropyl-N-methylethenesulfonamid;
2-(4-Amino-3-(2-fluoro-4-phenoxyphenyl)-1H-pyrazolo[3,4-d]pyrimidin-1-yl)ethyl 2-cyano-3-cyclopropylacrylat;
1-(4-Amino-3-(2-fluoro-4-phenoxyphenyl)-1H-pyrazolo[3,4-d]pyrimidin-1-yl)-2-methylpropan-2-yl 2-cyano-3-cyclopropylacrylat;
2-((2-(4-Amino-3-(2-fluoro-4-phenoxyphenyl)-1H-pyrazolo[3,4-d]pyrimidin-1-yl)ethyl)sulfonyl)-3-cyclopropylacrylonitril;
2-(5-((4-Amino-3-(2-fluoro-4-phenoxyphenyl)-1H-pyrazolo[3,4-d]pyrimidin-1-yl)methyl)oxazol-2-yl)-3-cyclopropylacrylonitril;
(R)-2-(3-(4-Amino-5-(4-(3,5-difluorophenoxy)phenyl)-7H-pyrrolo[2,3-d]pyrimidin-7-yl)piperidin-1-carbonyl)-3-cyclopropylacrylonitril;
(S)-2-(3-(4-Amino-5-(4-(3,5-difluorophenoxy)phenyl)-7H-pyrrolo[2,3-d]pyrimidin-7-yl)piperidin-1-carbonyl)-3-cyclopropylacrylonitril;
(R)-2-(3-(4-Amino-5-(2-fluoro-4-phenoxyphenyl)-7H-pyrrolo[2,3-d]pyrimidin-7-yl)piperidin-1-carbonyl)-3-cyclopropylacrylonitril;
(S)-2-(3-(4-Amino-5-(2-fluoro-4-phenoxyphenyl)-7H-pyrrolo[2,3-d]pyrimidin-7-yl)piperidin-1-carbonyl)-3-cyclopropylacrylonitril;
(R)-2-(2-((4-Amino-5-(4-phenoxyphenyl)-7H-pyrrolo[2,3-d]pyrimidin-7-yl)methyl)pyrrolidin-1-carbonyl)-3-cyclopropylacrylonitril;
(S)-2-(2-((4-Amino-5-(4-phenoxyphenyl)-7H-pyrrolo[2,3-d]pyrimidin-7-yl)methyl)pyrrolidin-1-carbonyl)-3-cyclopropylacrylonitril;
(R)-2-(2-((4-Amino-5-(4-(3,5-difluorophenoxy)phenyl)-7H-pyrrolo[2,3-d]pyrimidin-7-yl)methyl)pyrrolidin-1-carbonyl)-3-cyclopropylacrylonitril;
(S)-2-(2-((4-Amino-5-(4-(3,5-difluorophenoxy)phenyl)-7H-pyrrolo[2,3-d]pyrimidin-7-yl)methyl)pyrrolidin-1-carbonyl)-3-cyclopropylacrylonitril,
(R)-2-(2-((4-Amino-5-(2-fluoro-4-phenoxyphenyl)-7H-pyrrolo[2,3-d]pyrimidin-7-yl)methyl)pyrrolidin-1-carbonyl)-3-cyclopropylacrylonitril;
(S)-2-(2-((4-Amino-5-(2-fluoro-4-phenoxyphenyl)-7H-pyrrolo[2,3-d]pyrimidin-7-yl)methyl)pyrrolidin-1-carbonyl)-3-cyclopropylacrylonitril;
(R)-2-(3-(4-Amino-6-methyl-5-(4-phenoxyphenyl)-7H-pyrrolo[2,3-d]pyrimidin-7-yl)piperidin-1-carbonyl)-3-cyclopropylacrylonitril;
(S)-2-(3-(4-Amino-6-methyl-5-(4-phenoxyphenyl)-7H-pyrrolo[2,3-d]pyrimidin-7-yl)piperidin-1-carbonyl)-3-cyclopropylacrylonitril;
(R)-2-(3-(4-Amino-5-(4-(3,5-difluorophenoxy)phenyl)-6-methyl-7H-pyrrolo[2,3-d]pyrimidin-7-yl)piperidin-1-carbonyl)-3-cyclopropylacrylonitril;
(S)-2-(3-(4-Amino-5-(4-(3,5-difluorophenoxy)phenyl)-6-methyl-7H-pyrrolo[2,3-d]pyrimidin-7-yl)piperidin-1-carbonyl)-3-cyclopropylacrylonitril;
2-((3R)-3-(4-Amino-5-(2-fluoro-4-phenoxyphenyl)-6-methyl-7H-pyrrolo[2,3-d]pyrimidin-7-yl)piperidin-1-carbonyl)-3-cyclopropylacrylonitril;
2-((3S)-3-(4-Amino-5-(2-fluoro-4-phenoxyphenyl)-6-methyl-7H-pyrrolo[2,3-d]pyrimidin-7-yl)piperidin-1-carbonyl)-3-cyclopropylacrylonitril;
(R)-(2-(2-((4-Amino-6-methyl-5-(4-phenoxyphenyl)-7H-pyrrolo[2,3-d]pyrimidin-7-yl)methyl)pyrrolidin-1-carbonyl)-3-cyclopropylacrylonitril;
(S)-(2-(2-((4-Amino-6-methyl-5-(4-phenoxyphenyl)-7H-pyrrolo[2,3-d]pyrimidin-7-yl)methyl)pyrrolidin-1-carbonyl)-3-cyclopropylacrylonitril;
(R)-2-(2-((4-Amino-5-(4-(3,5-difluorophenoxy)phenyl)-6-methyl-7H-pyrrolo[2,3-d]pyrimidin-7-yl)methyl)pyrrolidin-1-carbonyl)-3-cyclopropylacrylonitril,
(S)-2-(2-((4-Amino-5-(4-(3,5-difluorophenoxy)phenyl)-6-methyl-7H-pyrrolo[2,3-d]pyrimidin-7-yl)methyl)pyrrolidin-1-carbonyl)-3-cyclopropylacrylonitril;
(R)-2-(2-((4-Amino-5-(2-fluoro-4-phenoxyphenyl)-6-methyl-7H-pyrrolo[2,3-d]pyrimidin-7-yl)methyl)pyrrolidin-1-carbonyl)-3-cyclopropylacrylonitril; oder
(S)-2-(2-((4-Amino-5-(2-fluoro-4-phenoxyphenyl)-6-methyl-7H-pyrrolo[2,3-d]pyrimidin-7-yl)methyl)pyrrolidin-1-carbonyl)-3-cyclopropylacrylonitril;
N-(3-(4-Amino-3-(4-phenoxyphenyl)-1H-pyrazolo[3,4-d]pyrimidin-1-yl)-2,2-dimethylpropyl)-2-cyano-3-cyclopropylacrylamid;
(R)-2-(2-((4-Amino-3-(2-methyl-4-phenoxyphenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-methyl-4-phenoxyphenyl)-1H-pyrazolo[3,4-d]pyrimidin-1-yl)methyl)pyrrolidin-1-carbonyl)-3-cyclopropylacrylonitril;
(R)-2-(2-((4-Amino-3-(2-chloro-4-phenoxyphenyl)-1H-pyrazolo[3,4-d]pyrimidin-1-yl)methyl)pyrrolidin-1-carbonyl)-3-cyclopropylacrylonitril;
(S)-2-(2-((4-Amino-3-(2-chloro-4-phenoxyphenyl)-1H-pyrazolo[3,4-d]pyrimidin-1-yl)methyl)pyrrolidin-1-carbonyl)-3-cyclopropylacrylonitril;
(R)-2-(2-((4-Amino-3-(4-(2,5-difluorophenoxy)phenyl)-1H-pyrazolo[3,4-d]pyrimidin-1-yl)methyl)pyrrolidin-1-carbonyl)-4-methylpent-2-enentril,
(S)-2-(2-((4-Amino-3-(4-(2,5-difluorophenoxy)phenyl)-1H-pyrazolo[3,4-d]pyrimidin-1-yl)methyl)pyrrolidin-1-carbonyl)-4-methylpent-2-enentril;
(R)-4-Amino-2-(2-((4-Amino-3-(2-fluoro-4-(3-fluorophenoxy)phenyl)-1H-pyrazolo[3,4-d]pyrimidin-1-yl)methyl)pyrrolidin-1-carbonyl)-4-methylpent-2-enentril;
(S)-4-Amino-2-(2-((4-Amino-3-(2-fluoro-4-(3-fluorophenoxy)phenyl)-1H-pyrazolo[3,4-d]pyrimidin-1-yl)methyl)pyrrolidin-1-carbonyl)-4-methylpent-2-enentril;
(R)-2-(2-((4-Amino-3-(2-fluoro-4-(3-fluorophenoxy)phenyl)-1H-pyrazolo[3,4-d]pyrimidin-1-yl)methyl)pyrrolidin-1-carbonyl)-4-methylpent-2-enentril;
(S)-2-(2-((4-Amino-3-(2-fluoro-4-(3-fluorophenoxy)phenyl)-lH-pyrazolo[3,4-d]pyrimidin-1-yl)methyl)pyrrolidin-1-carbonyl)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4,4-dimethylpent-2-enentril,
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4,4-dimethylpent-2-enentril,
(R)-2-(2-((4-Amino-3-(4-(2,6-difluorophenoxy)phenyl)-1H-pyrazolo[3,4-d]pyrimidin-1-yl)methyl)pyrrolidin-1-carbonyl)-4-methylpent-2-enentril,
(S)-2-(2-((4-Amino-3-(4-(2,6-difluorophenoxy)phenyl)-1H-pyrazolo[3,4-d]pyrimidin-1-yl)methyl)pyrrolidin-1-carbonyl)-4-methylpent-2-enentril;
(R)-2-(2-((4-Amino-3-(4-(2,3-difluorophenoxy)phenyl)-1H-pyrazolo[3,4-d]pyrimidin-1-yl)methyl)pyrrolidin-1-carbonyl)-4-methylpent-2-enentril;
(S)-2-(2-((4-Amino-3-(4-(2,3-difluorophenoxy)phenyl)-1H-pyrazolo[3,4-d]pyrimidin-1-yl)methyl)pyrrolidin-1-carbonyl)-4-methylpent-2-enentril;
(R)-2-(2-((4-Amino-6-methyl-5-(4-phenoxyphenyl)-7H-pyrrolo[2,3-d]pyrimidin-7-yl)methyl)pyrrolidin-1-carbonyl)-4-(dimethylamino)-4-methylpent-2-enentril;
(S)-2-(2-((4-Amino-6-methyl-5-(4-phenoxyphenyl)-7H-pyrrolo[2,3-d]pyrimidin-7-yl)methyl)pyrrolidin-1-carbonyl)-4-(dimethylamino)-4-methylpent-2-enentril;
(R)-2-(2-((4-Amino-3-(4-(2,6-difluorophenoxy)-2-fluorophenyl)-1H-pyrazolo[3,4-d]pyrimidin-1-yl)methyl)pyrrolidin-1-carbonyl)-4-methylpent-2-enentril;
(S)-2-(2-((4-Amino-3-(4-(2,6-difluorophenoxy)-2-fluorophenyl)-1H-pyrazolo[3,4-d]pyrimidin-1-yl)methyl)pyrrolidin-1-carbonyl)-4-methylpent-2-enentril;
(R)-2-(2-((4-Amino-3-(2-fluoro-4-phenoxyphenyl)-1H-pyrazolo[3,4-d]pyrimidin-1-yl)methyl)-4,4-difluoropyrrolidin-1-carbonyl)-3-cyclopropylacrylonitril;
(S)-2-(2-((4-Amino-3-(2-fluoro-4-phenoxyphenyl)-1H-pyrazolo[3,4-d]pyrimidin-1-yl)methyl)-4,4-difluoropyrrolidin-1-carbonyl)-3-cyclopropylacrylonitril;
(R)-2-(2-((4-Amino-3-(2-fluoro-4-phenoxyphenyl)-1H-pyrazolo[3,4-d]pyrimidin-1-yl)methyl)-4,4-difluoropyrrolidin-1-carbonyl)-4-methylpent-2-enentril;
(S)-2-(2-((4-Amino-3-(2-fluoro-4-phenoxyphenyl)-1H-pyrazolo[3,4-d]pyrimidin-1-yl)methyl)-4,4-difluoropyrrolidin-1-carbonyl)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-methylpent-2-enentril;
(S)-4-Amino-2-(2-((4-Amino-3-(2-fluoro-4-phenoxyphenyl)-1H-pyrazolo[3,4-d]pyrimidin-1-yl)methyl)pyrrolidin-1-carbonyl)-4-methylpent-2-enentril;
(R)-4-Amino-2-(2-((4-Amino-3-(2-fluoro-4-phenoxyphenyl)-1H-pyrazolo[3,4-d]pyrimidin-1-yl)methyl)pyrrolidin-1-carbonyl)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-(methylamino)pent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-(methylamino)pent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-(dimethylamino)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-(dimethylamino)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-(ethylamino)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-(ethylamino)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-(isopropylamino)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-(isopropylamino)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-(cyclopropylamino)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-(cyclopropylamino)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-((2-methoxyethyl)Amino)-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-((2-methoxyethyl)Amino)-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-ethoxy-4-methylpent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-ethoxy-4-methylpent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-Aminocyclopropyl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-Aminocyclopropyl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-(methylamino)cyclopropyl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-(methylamino)cyclopropyl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-(ethylamino)cyclopropyl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-(ethylamino)cyclopropyl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-(isopropylamino)cyclopropyl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-(isopropylamino)cyclopropyl)acrylonitril;
2-((S)-2-((4-Amino-3-(2-fluoro-4-phenoxyphenyl)-1H-pyrazolo[3,4-d]pyrimidin-1-yl)methyl)pyrrolidin-1-carbonyl)-3-(pyrrolidin-2-yl)acrylonitril;
2-((R)-2-((4-Amino-3-(2-fluoro-4-phenoxyphenyl)-1H-pyrazolo[3,4-d]pyrimidin-1-yl)methyl)pyrrolidin-1-carbonyl)-3-(pyrrolidin-2-yl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-morpholinopent-2-enentril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-morpholinopent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-((dimethylamino)methyl)cyclopentyl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-((dimethylamino)methyl)cyclopentyl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-methylpiperidin-4-yl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(1-methylpiperidin-4-yl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(tetrahydro-2H-pyran-4-yl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(tetrahydro-2H-pyran-4-yl)acrylonitril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-3-(piperidin-4-yl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-3-(piperidin-4-yl)acrylonitril;
2-((S)-2-((4-Amino-3-(2-fluoro-4-phenoxyphenyl)-1H-pyrazolo[3,4-d]pyrimidin-1-yl)methyl)pyrrolidin-1-carbonyl)-3-(piperidin-3-yl)acrylonitril;
2-((R)-2-((4-Amino-3-(2-fluoro-4-phenoxyphenyl)-1H-pyrazolo[3,4-d]pyrimidin-1-yl)methyl)pyrrolidin-1-carbonyl)-3-(piperidin-3-yl)acrylonitril;
(S)-2-(2-((4-Amino-3-(4-(2,3-difluorophenoxy)-2-fluorophenyl)-1H-pyrazolo[3,4-djpyrimidin-1-yl)methyl)pyrrolidin-1-carbonyl)-4-methylpent-2-enentril;
(R)-2-(2-((4-Amino-3-(4-(2,3-difluorophenoxy)-2-fluorophenyl)-1H-pyrazolo[3,4-djpyrimidin-1-yl)methyl)pyrrolidin-1-carbonyl)-4-methylpent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4,4-dimethylpent-2-enentril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4,4-dimethylpent-2-enentril;
(S)-4-Amino-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-methylpent-2-enentril;
(R)-4-Amino-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-methylpent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-methyl-4-(methylamino)pent-2-enentril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-methyl-4-(methylamino)pent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(dimethylamino)-4-methylpent-2-enentril,
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(dimethylamino)-4-methylpent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(ethylamino)-4-methylpent-2-enentril,
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(ethylamino)-4-methylpent-2-enentril,
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(isopropylamino)-4-methylpent-2-enentril,
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(isopropylamino)-4-methylpent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(cyclopropylamino)-4-methylpent-2-enentril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-(cyclopropylamino)-4-methylpent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-((2-methoxyethyl)Amino)-4-methylpent-2-enentril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-((2-methoxyethyl)Amino)-4-methylpent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-ethoxy-4-methylpent-2-enentril;
(R)-2-(2-((4-Amino-3-(4-(2,3-difluorophenoxy)-2-fluorophenyl)-1H-pyrazolo[3,4-djpyrimidin-1-yl)methyl)pyrrolidin-1-carbonyl)-4-ethoxy-4-methylpent-2-enentril;
(S)-2-(2-((4-Amino-3-(4-(2,3-difluorophenoxy)-2-fluorophenyl)-1H-pyrazolo[3,4-djpyrimidin-1-yl)methyl)pyrrolidin-1-carbonyl)-3-(1-Aminocyclopropyl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-aminocyclopropyl)acrylonitril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-(methylamino)cyclopropyl)acrylonitril,
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-(methylamino)cyclopropyl)acrylonitril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-(ethylamino)cyclopropyl)acrylonitril,
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-(ethylamino)cyclopropyl)acrylonitril,
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-(isopropylamino)cyclopropyl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-(isopropylamino)cyclopropyl)acrylonitril;
2-((S)-2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(pyrrolidin-2-yl)acrylonitril;
2-((R)-2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(pyrrolidin-2-yl)acrylonitril;
(S)-2-(2-((4-Amino-3-(4-(2,3-difluorophenoxy)-2-fluorophenyl)-1H-pyrazolo[3,4-djpyrimidin-1-yl)methyl)pyrrolidin-1-carbonyl)-4-methyl-4-morpholinopent-2-enentril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-4-methyl-4-morpholinopent-2-enentril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-((diethylamino)methyl)cyclopentyl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-((diethylamino)methyl)cyclopentyl)acrylonitril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-((dimethylamino)methyl)cyclopentyl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-((dimethylamino)-methyl)cyclopentyl)acrylonitril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-methylpiperidin-4-yl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(1-methylpiperidin-4-yl)acrylonitril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(tetrahydro-2H-pyran-4-yl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(tetrahydro-2H-pyran-4-yl)acrylonitril;
(S)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(piperidin-4-yl)acrylonitril;
(R)-2-(2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(piperidin-4-yl)acrylonitril;
2-((S)-2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(piperidin-3-yl)acrylonitril;
2-((R)-2-((4-Amino-3-(4-(2,3-difluorophenoxy)-2-fluorophenyl)-1H-pyrazolo[3,4-d]pyrimidin-1-yl)methyl)pyrrolidin-1-carbonyl)-3-(piperidin-3-yl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-4-methylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4,4-dimethylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4,4-dimethylpent-2-enentril;
(R)-4-Amino-2-(3-(4-amino-3-(2-fluoro-4-phenoxyphenyl)-1H-pyrazolo[3,4-d]pyrimidin-1-yl)piperidin-1-carbonyl)-4-methylpent-2-enentril;
(S)-4-Amino-2-(3-(4-Amino-3-(2-fluoro-4-phenoxyphenyl)-1H-pyrazolo[3,4-d]pyrimidin-1-yl)piperidin-1-carbonyl)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4-methyl-4-(methylamino)pent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-methyl-4-(methylamino)pent-2-enentril;
(R)-2-(3-(4-Amino-3-(4-(2,3-difluorophenoxy)-2-fluorophenyl)-1H-pyrazolo[3,4-d]pyrimidin-1-yl)pipendin-1-carbonyl)-4-(dimethylamino)-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)pipendin-1-carbonyl)-4-(dimethylamino)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4-(ethylamino)-4-methylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-(ethylamino)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4-(isopropylamino)-4-methylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-(isopropylamino)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4-(cyclopropylamino)-4-methylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-(cyclopropylamino)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4-((2-methoxyethyl)Amino)-4-methylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-((2-methoxyethyl)Amino)-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-4-ethoxy-4-methylpent-2-enentril;
(S)-2-(3-(4-Amino-3-(2-fluoro-4-phenoxyphenyl)-1H-pyrazolo[3,4-d]pyrimidin-1-yl)piperidin-1-carbonyl)-4-ethoxy-4-methylpent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-3-(1-aminocyclopropyl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-aminocyclopropyl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(1-(methylamino)cyclopropyl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-(methylamino)cyclopropyl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(1-(ethylamino)cyclopropyl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-(ethylamino)cyclopropyl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(1-(isopropylamino)cyclopropyl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-(isopropylamino)cyclopropyl)acrylonitril;
2-((R)-3-(4-Amino-3-(2-fluoro-4-phenoxyphenyl)-1H-pyrazolo[3,4-d]pyrimidin-1-yl)piperidin-1-carbonyl)-3-(pyrrolidin-2-yl)acrylonitril;
2-((S)-3-(4-Amino-3-(2-fluoro-4-phenoxyphenyl)-1H-pyrazolo[3,4-d]pyrimidin-1-yl)piperidin-1-carbonyl)-3-(pyrrolidin-2-yl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-4-methyl-4-morpholinopent-2-enentril;
(R)-2-(3-(4-Amino-3-(2-fluoro-4-phenoxyphenyl)-1H-pyrazolo[3,4-d]pyrimidin-1-yl)piperidin-1-carbonyl)-3-(1-((diethylamino)methyl)-cyclopentyl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-((diethylamino)methyl)-cyclopentyl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(1-((dimethylamino)methyl)cyclopentyl)-acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-((dimethylamino)methyl)cyclopentyl)-acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(1-methylpiperidin-4-yl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(1-methylpiperidin-4-yl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(tetrahydro-2H-pyran-4-yl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(tetrahydro-2H-pyran-4-yl)acrylonitril;
(R)-2-(3-(4-Amino-3-(2-fluoro-4-phenoxyphenyl)-1H-pyrazolo[3,4-d]pyrimidin-1-yl)piperidin-1-carbonyl)-3-(piperidin-4-yl)acrylonitril;
(S)-2-(3-(4-Amino-3-(2-fluoro-4-phenoxyphenyl)-1H-pyrazolo[3,4-d]pyrimidin-1-yl)piperidin-1-carbonyl)-3-(piperidin-4-yl)acrylonitril;
2-((R)-3-(4-Amino-3-(2-fluoro-4-phenoxyphenyl)-1H-pyrazolo[3,4-d]pyrimidin-1-yl)piperidin-1-carbonyl)-3-(piperidin-3-yl)acrylonitril;
2-((S)-3-(4-Amino-3-(2-fluoro-4-phenoxyphenyl)-1H-pyrazolo[3,4-d]pyrimidin-1-yl)piperidin-1-carbonyl)-3-(piperidin-3-yl)acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-cyclopropylacrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-cyclopropylacrylonitril;
(R)-2-(3-(4-Amino-3-(4-(2,3-difluorophenoxy)-2-fluorophenyl)-1H-pyrazolo[3,4-d]pyrimidin-1-yl)pipendin-1-carbonyl)-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)pipendin-1-carbonyl)-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4,4-dimethylpent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4,4-dimethylpent-2-enentril;
(R)-4-Amino-2-(3-(4-Amino-3-(4-(2,3-difluorophenoxy)-2-fluorophenyl)-1H-pyrazolo[3,4-d]pyrimidin-1-yl)piperidin-1-carbonyl)-4-methylpent-2-enentril;
(S)-4-Amino-2-(3-(4-Amino-3-(4-(2,3-difluorophenoxy)-2-fluorophenyl)-1H-pyrazolo[3,4-d]pyrimidin-1-yl)piperidin-1-carbonyl)-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4-methyl-4-(methylamino)pent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-methyl-4-(methylamino)pent-2-enentril;
(R)-2-(3-(4-Amino-3-(4-(2,3-difluorophenoxy)-2-fluorophenyl)-1H-pyrazolo[3,4-d]pyrimidm-1-yl)piperidin-1-carbonyl)-4-(dimethylamino)-4-methylpent-2-enentril;
(S)-2-(3-(4-Amino-3-(4-(2,3-difluorophenoxy)-2-fluorophenyl)-1H-pyrazolo[3,4-d]pyrimidm-1-yl)piperidin-1-carbonyl)-4-(dimethylamino)-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4-(ethylamino)-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-(ethylamino)-4-methylpent-2-enentril;
(R)-2-(3-(4-Amino-3-(4-(2,3-difluorophenoxy)-2-fluorophenyl)-1H-pyrazolo[3,4-d]pyrimidm-1-yl)piperidin-1-carbonyl)-4-(isopropylamino)-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-(isopropylamino)-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4-(cyclopropylamino)-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-(cyclopropylamino)-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4-((2-methoxyethyl)Amino)-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-((2-methoxyethyl)Amino)-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4-ethoxy-4-methylpent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-ethoxy-4-methylpent-2-enentril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-Aminocyclopropyl)-acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-Aminocyclopropyl)-acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-(methylamino)cyclopropyl)-acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-(methylamino)cyclopropyl)-acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-(ethylamino)cyclopropyl)-acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-(ethylamino)cyclopropyl)-acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-(isopropylamino)-cyclopropyl)acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-(isopropylamino)-cyclopropyl)acrylonitril;
2-((R)-3-(4-Amino-3-(4-(2,3-difluorophenoxy)-2-fluorophenyl)-1H-pyrazolo[3,4-d]pyrimidin-1-yl)piperidin-1-carbonyl)-3-(pyrrolidin-2-yl)acrylonitril;
2-((S)-3-(4-Amino-3-(4-(2,3-difluorophenoxy)-2-fluorophenyl)-1H-pyrazolo[3,4-d]pyrimidin-1-yl)piperidin-1-carbonyl)-3-(pyrrolidin-2-yl)acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-4-methyl-4-morpholinopent-2-enentril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-4-methyl-4-morpholinopent-2-enentril;
(R)-2-(3-(4-Amino-3-(4-(2,3-difluorophenoxy)-2-fluorophenyl)-1H-pyrazolo[3,4-d]pyrimidin-1-yl)pipendin-1-carbonyl)-3-(1-((diethylamino)methyl)-cyclopentyl)acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-((diethylamino)methyl)-cyclopentyl)acrylonitril;
(R)-2-(3-(4-Amino-3-(4-(2,3-difluorophenoxy)-2-fluorophenyl)-1H-pyrazolo[3,4-d]pyrimidin-1-yl)pipendin-1-carbonyl)-3-(1-((dimethylamino)-methyl)cyclopentyl)acrylonitril;
(S)-2-(3-(4-Amino-3-(4-(2,3-difluorophenoxy)-2-fluorophenyl)-1H-pyrazolo[3,4-d]pyrimidin-1-yl)pipendin-1-carbonyl)-3-(1-((dimethylamino)-methyl)cyclopentyl)acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-methylpiperidin-4-yl)acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(1-methylpiperidin-4-yl)acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(tetrahydro-2H-pyran-4-yl)acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(tetrahydro-2H-pyran-4-yl)acrylonitril;
(R)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(piperidin-4-yl)acrylonitril;
(S)-2-(3-(4-Amino-3-(4-(2,3-difluorophenoxy)-2-fluorophenyl)-1H-pyrazolo[3,4-d]pyrimidin-1-yl)piperidin-1-carbonyl)-3-(piperidin-4-yl)acrylonitril;
2-((R)-3-(4-Amino-3-(4-(2,3-difluorophenoxy)-2-fluorophenyl)-1H-pyrazolo[3,4-d]pyrimidin-1-yl)piperidin-1-carbonyl)-3-(piperidin-3-yl)acrylonitril;
2-((S)-3-(4-Amino-3-(4-(2,3-difluorophenoxy)-2-fluorophenyl)-1H-pyrazolo[3,4-d]pyrimidin-1-yl)piperidin-1-carbonyl)-3-(piperidin-3-yl)acrylonitril;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-(piperidin-1-yl)pent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-(piperidin-1-yl)pent-2-enentril;
(R)-2-(2-((4-Amino-5-(2-fluoro-4-phenoxyphenyl)-7H-pyrrolo[2,3-d]pyrimidin-7-yl)methyl)pyrrolidin-1-carbonyl)-4-(dimethylamino)-4-methylpent-2-enentril;
(S)-2-(2-((4-Amino-5-(2-fluoro-4-phenoxyphenyl)-7H-pyrrolo[2,3-d]pyrimidin-7-yl)methyl)pyrrolidin-1-carbonyl)-4-(dimethylamino)-4-methylpent-2-enentril;
(R)-2-(2-((4-Amino-5-(4-(2,3-difluorophenoxy)phenyl)-7H-pyrrolo[2,3-d]pyrimidin-7-yl)methyl)pyrrolidin-1-carbonyl)-3-cyclopropylacrylonitril;
(S)-2-(2-((4-Amino-5-(4-(2,3-difluorophenoxy)phenyl)-7H-pyrrolo[2,3-d]pyrimidin-7-yl)methyl)pyrrolidin-1-carbonyl)-3-cyclopropylacrylonitril;
(R)-2-(2-((4-Amino-5-(4-(2,3-difluorophenoxy)phenyl)-7H-pyrrolo[2,3-d]pyrimidin-7-yl)methyl)pyrrolidin-1-carbonyl)-4-methylpent-2-enentril;
(S)-2-(2-((4-Amino-5-(4-(2,3-difluorophenoxy)phenyl)-7H-pyrrolo[2,3-d]pyrimidin-7-yl)methyl)pyrrolidin-1-carbonyl)-4-methylpent-2-enentril;
(R)-2-(2-((4-Amino-5-(4-(2,3-difluorophenoxy)phenyl)-7H-pyrrolo[2,3-d]pyrimidin-7-yl)methyl)pyrrolidin-1-carbonyl)-4,4-dimethylpent-2-enentril;
(S)-2-(2-((4-Amino-5-(4-(2,3-difluorophenoxy)phenyl)-7H-pyrrolo[2,3-d]pyrimidin-7-yl)methyl)pyrrolidin-1-carbonyl)-4,4-dimethylpent-2-enentril;
(R)-2-(2-((4-Amino-5-(4-(2,3-difluorophenoxy)phenyl)-7H-pyrrolo[2,3-d]pyrimidin-7-yl)methyl)pyrrolidin-1-carbonyl)-4-(dimethylamino)-4-methylpent-2-enentril;
(S)-2-(2-((4-Amino-5-(4-(2,3-difluorophenoxy)phenyl)-7H-pyrrolo[2,3-d]pyrimidin-7-yl)methyl)pyrrolidin-1-carbonyl)-4-(dimethylamino)-4-methylpent-2-enentril;
(R)-2-(2-((4-Amino-5-(4-phenoxyphenyl)-7H-pyrrolo[2,3-d]pyrimidin-7-yl)methyl)pyrrolidin-1-carbonyl)-4-(dimethylamino)-4-methylpent-2-enentril;
(S)-2-(2-((4-Amino-5-(4-phenoxyphenyl)-7H-pyrrolo[2,3-d]pyrimidin-7-yl)methyl)pyrrolidin-1-carbonyl)-4-(dimethylamino)-4-methylpent-2-enentril;
(R)-2-(2-((4-Amino-5-(4-phenoxyphenyl)-7H-pyrrolo[2,3-d]pyrimidin-7-yl)methyl)pyrrolidin-1-carbonyl)-4-methylpent-2-enentril;
(S)-2-(2-((4-Amino-5-(4-phenoxyphenyl)-7H-pyrrolo[2,3-d]pyrimidin-7-yl)methyl)pyrrolidin-1-carbonyl)-4-methylpent-2-enentril;
(R)-4-Amino-2-(2-((4-Amino-5-(2-fluoro-4-phenoxyphenyl)-7H-pyrrolo[2,3-d]pyrimidin-7-yl)methyl)pyrrolidin-1-carbonyl)-4-methylpent-2-enentril;
(S)-4-Amino-2-(2-((4-Amino-5-(2-fluoro-4-phenoxyphenyl)-7H-pyrrolo[2,3-d]pyrimidin-7-yl)methyl)pyrrolidin-1-carbonyl)-4-methylpent-2-enentril;
(R)-2-(2-((4-Amino-5-(4-phenoxyphenyl)-7H-pyrrolo[2,3-d]pyrimidin-7-yl)methyl)pyrrolidin-1-carbonyl)-4-(dimethylamino)-4-methylpent-2-enentril;
(S)-2-(2-(( 4-Amino-5-(4-phenoxyphenyl)-7H-pyrrolo[2,3-d]pyrimidin-7-yl)methyl)pyrrolidin-1-carbonyl)-4-(dimethylamino)-4-methylpent-2-enentril;
(R)-2-(2-((4-Amino-5-(4-phenoxyphenyl)-7H-pyrrolo[2,3-d]pyrimidin-7-yl)methyl)pyrrolidin-1-carbonyl)-4-methyl-4-morpholinopent-2-enentril;
(S)-2-(2-((4-Amino-5-(4-phenoxyphenyl)-7H-pyrrolo[2,3-d]pyrimidin-7-yl)methyl)pyrrolidin-1-carbonyl)-4-methyl-4-morpholinopent-2-enentril;
(R)-2-(2-((4-Amino-5-(4-phenoxyphenyl)-7H-pyrrolo[2,3-d]pyrimidin-7-yl)methyl)pyrrolidin-1-carbonyl)-4-(diethylamino)-4-methylpent-2-enentril;
(S)-2-(2-((4-Amino-5-(4-phenoxyphenyl)-7H-pyrrolo[2,3-d]pyrimidin-7-yl)methyl)pyrrolidin-1-carbonyl)-4-(diethylamino)-4-methylpent-2-enentril;
(R)-2-(2-((4-Amino-5-(4-phenoxyphenyl)-7H-pyrrolo[2,3-d]pyrimidin-7-yl)methyl)pyrrolidin-1-carbonyl)-4-ethoxy-4-methylpent-2-enentril;
(S)-2-(2-((4-Amino-5-(4-phenoxyphenyl)-7H-pyrrolo[2,3-d]pyrimidin-7-yl)methyl)pyrrolidin-1-carbonyl)-4-ethoxy-4-methylpent-2-enentril;
(R)-N-(1-(4-Amino-3-(2-fluoro-4-phenoxyphenyl)-1H-pyrazolo[3,4-d]pyrimidin-1-yl)propan-2-yl)-2-cyano-4,4-dimethylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4,4-dimethylpent-2-enamid;
(R)-N-(1-(4-Amino-3-(2-fluoro-4-phenoxyphenyl)-1H-pyrazolo[3,4-d]pyrimidin-1-yl)propan-2-yl)-2-cyano-4-methylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4-methylpent-2-enamid;
(R)-N-(1-(4-Amino-3-(2-fluoro-4-phenoxyphenyl)-1H-pyrazolo[3,4-d]pyrimidin-1-yl)propan-2-yl)-2-cyano-4-ethoxy-4-methylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4-ethoxy-4-methylpent-2-enamid;
(R)-N-(1-(4-Amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamid;
2-((R)-2-((4-Amino-3-(2-fluoro-4-phenoxyphenyl)-1H-pyrazolo[3,4-d]pyrimidin-1-yl)methyl)pyrrolidin-1-carbonyl)-3-((S)-pyrrolidin-2-yl)acrylonitril;
2-((R)-2-((4-Amino-3-(2-fluoro-4-phenoxyphenyl)-1H-pyrazolo[3,4-d]pyrimidin-1-yl)methyl)pyrrolidin-1-carbonyl)-3-((R)-pyrrolidin-2-yl)acrylonitril;
(R)-4-Amino-N-(1-(4-Amino-3-(2-fluoro-4-phenoxyphenyl)-1H-pyrazolo[3,4-d]pyrimidin-1-yl)propan-2-yl)-2-cyano-4-methylpent-2-enamid;
(S)-4-Amino-N-(1-(4-Amino-3-(2-fluoro-4-phenoxyphenyl)-1H-pyrazolo[3,4-d]pyrimidin-1-yl)propan-2-yl)-2-cyano-4-methylpent-2-enamid;
N-(2-(4-Amino-3-(4-phenoxyphenyl)-1H-pyrazolo[3,4-d]pyrimidin-1-yl)ethyl)-2-cyano-3-cyclopropyl-N-methylacrylamide;
(R)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-(piperidin-1-yl)pent-2-enentril;
(S)-2-(2-((4-Amino-3-(2-fluoro-4-phenoxyphenyl)-1H-pyrazolo[3,4-d]pyrimidin-1-yl)methyl)pyrrolidin-1-carbonyl)-4-methyl-4-(piperidin-1-yl)pent-2-enentril;
(S)-N-(1-(4-Amino-3-(2-fluoro-4-phenoxyphenyl)-1H-pyrazolo[3,4-d]pyrimidin-1-yl)propan-2-yl)-2-cyano-4,4-dimethylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4-methylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4-(dimethylamino)-4-methylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-4-ethoxy-4-methylpent-2-enamid;
(S)-4-Amino-N-(1-(4-Amino-3-(2-fluoro-4-phenoxyphenyl)-1H-pyrazolo[3,4-d]pyrimidin-1-yl)propan-2-yl)-2-cyano-4-methylpent-2-enamid;
(S)-N-(1-(4-Amino-3-(2-fluoro-4-phenoxyphenyl)-1H-pyrazolo[3,4-d]pyrimidin-1-yl)propan-2-yl)-2-cyano-3-cyclopropylacrylamid;
2-[(2R)-2-[[4-Amino-3-(2-fluoro-4-phenoxy-phenyl)pyrazolo[3,4-d]pyrimidin-1-yl]methyl]-pyrrolidin-1-carbonyl]-3-(3-methyloxetan-3-yl)prop-2-enentril; oder
2-[(2S)-2-[[4-Amino-3-(2-fluoro-4-phenoxy-phenyl)pyrazolo[3,4-d]pyrimidin-1-yl]methyl]-pyrrolidin-1-carbonyl]-3-(3-methyloxetan-3-yl)prop-2-enentril;
oder eine Mischung von R und S Isomeren;
oder ein individuelles (E) oder (Z) Isomer davon;
oder ein pharmazeutisch annehmbares Salz von einem der Vorhergehenden.

8. Feste orale Formulierung nach Anspruch 1, wobei die Verbindung ist:
(R)-2-(3-(4-Amino-3-(2-fluoro-4-phenoxyphenyl)-1H-pyrazolo[3,4-d]pyrimidin-1-yl)piperidin-1-carbonyl)-4,4-dimethylpent-2-enentril;
oder ein individuelles (E) oder (Z) Isomer davon;
oder ein pharmazeutisch annehmbares Salz davon.

9. Feste orale Formulierung nach Anspruch 1, wobei die Verbindung ist:
2-(2-((4-Amino-3-(2-fluoro-4-phenoxyphenyl)-1H-pyrazolo[3,4-d]pyrimidin-1-yl)-methyl)pyrrolidin-1-carbonyl)-4,4-dimethylpent-2-enentril;
oder ein individuelles R oder S Isomer davon;
oder ein individuelles (E) oder (Z) Isomer davon;
oder ein pharmazeutisch annehmbares Salz davon.

10. Feste orale Formulierung nach einem der vorhergehenden Ansprüche, wobei die Beschichtung eine enterische Beschichtung ist, die sich bei einem pH-Wert von 4,7 bis 7 auflöst.

11. Feste orale Formulierung nach einem der vorhergehenden Ansprüche, wobei die Beschichtung eine enterische Beschichtung ist, die ein anionisches Polymer ist.

12. Feste orale Formulierung nach Anspruch 11, wobei das anionische Polymer ausgewählt ist aus Polymethacrylaten, auf Cellulose basierenden Polymeren und Plyvinylderivaten.

13. Feste orale Formulierung nach einem der vorhergehenden Ansprüche, wobei die Beschichtung eine enterische Beschichtung ist, die sich bei einem pH-Wert von 5,5 bis 7 auflöst.

14. Feste orale Formulierung nach Anspruch 1, wobei die Verbindung ist 2-(3-(4-Amino-3-(2-fluoro-4-phenoxyphenyl)-1H-pyrazolo[3,4-d]pyrimidin-1-yl)piperidin-1-carbonyl)-4,4-dimethylpent-2-enentril;
oder ein individuelles R oder S Isomer davon;
oder ein individuelles (E) oder (Z) Isomer davon;
oder ein pharmazeutisch annehmbares Salz der vorhergehenden Verbindungen.

15. Formulierung zur Verwendung bei der Behandlung einer Krankheit, die eine Autoimmun- oder Entzündungskrankheit, oder Krebs ist, wobei die Formulierung eine feste orale Formulierung wie in einem der Ansprüche 1 bis 14 definiert ist.

16. Formulierung zur Verwendung nach Anspruch 15, wobei die Krankheit entzündliche Darmerkrankung, Arthritis, Lupus, rheumatoide Arthritis, Psoriasis-Arthritis, Osteoarthritis, Still-Krankheit, juvenile Arthritis, Diabetes, Myasthenia gravis, Hashimoto-Thyreoiditis, Ord-Thyreoiditis, Morbus Basedow, Sjögren-Syndrom, Multiple Sklerose, Guillain-Barre-Syndrom, akute disseminierte Enzephalomyelitis, Addison-Krankheit, Opsoklonus-Myoklonus-Syndrom, Spondylitis ankylosans, Antiphospholipid-Antikörper-Syndrom, aplastische Anämie, Autoimmunhepatitis, Zöliakie, Goodpasture-Syndrom, idiopathische Thrombozytopenie, optische Neuritis, Sklerodermie, primäre biliäre Zirrhose, Reiter-Syndrom, Takayasu-Arteritis, vorläufige Arteritis, warme autoimmune hämolytische Anämie, Wegener-Granulomatose, Psoriasis, Alopecia universalis, Behcet-Krankheit, chronische Müdigkeit, Dysautonomie, Endometriose, interstitielle Zystitis, Neuromyotonie, Sklerodermie oder Vulvodynie, Asthma, Appendizitis, Blepharitis, Bronchiolitis, Bronchitis, Bursitis, Cervicitis, Cholangitis, Cholezystitis, Colitis, Konjunktivitis, Zystitis, Dakryoadenitis, Dermatitis, Dermatomyositis, Enzephalitis, Endokarditis, Endometritis, Enteritis, Enterokolitis, Epicondylitis, Epididymitis, Fasziitis, Fibrositis, Gastritis, Gastroenteritis, Hepatitis, Hidradenitis Suppurativa, Laryngitis, Mastitis, Meningitis, Myelitis Myocarditis, Myocytis, Nephritis, Oophoritis, Orchitis, Osteitis, Otitis, Pancreatitis, Parotitis, Pericarditis, Peritonitis, Pharyngitis, Pleuritis, Phlebitis, Pneumonitis, Pneumonie, Proctitis, Prostatitis, Pyelonephritis, Rhinitis, Salpingitis, Sinusitis, Stomatitis, Synovitis, Tendonitis, Tonsillitis,Uveitis, Vaginitis, Vaskulitis oder Vulvitis, Dermatitis, Kontaktdermatitis, Ekzem, Urtikaria, Rosacea und narbenbildende Psoriasisläsionen in der Haut, Gelenken, oder anderen Geweben oder Organen ist und die Formulierung optional in Kombination mit einem oder mehreren entzündungshemmenden Mitteln verabreicht wird.

17. Formulierung zur Verwendung nach Anspruch 16, wobei die Krankheit Lupus, rheumatoide Arthritis, Psoriasis-Arthritis, Sjögren-Syndrom, Wegener's Granulomatose, Psoriasis, Asthma, oder Uveitis ist.

## Revendications

1. Formulation orale solide comprenant (i) une quantité thérapeutiquement efficace d'un composé, (ii) au moins un enrobage choisi parmi un enrobage entérique et/ou un enrobage non entérique à libération retardée, et (iii) au moins un excipient pharmaceutiquement acceptable,
ledit composé étant :
(a) un composé de formule (Id) ou un sel pharmaceutiquement acceptable de celui-ci : 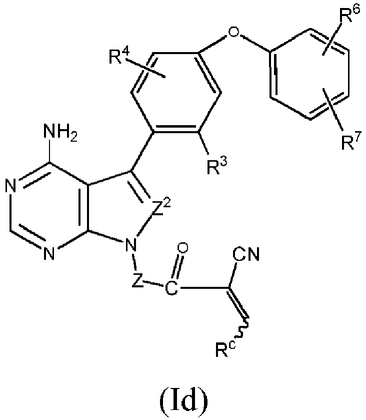 dans laquelle :
Z² est -N- ou -CR²- où R² est un atome d'hydrogène ou un groupe alkyle ;
R³ et R⁴ sont indépendamment un atome d'hydrogène, un groupe méthyle, chloro, fluoro, cyclopropyle, hydroxy, méthoxy, cyano, trifluorométhyle ou trifluorométhoxy ;
R⁶ et R⁷ sont indépendamment un atome d'hydrogène, un groupe méthyle, méthoxy, fluoro, chloro, trifluorométhyle, trifluorométhoxy ou cyano ;
Z est un groupe -(alkylène)-NR^{a}-, 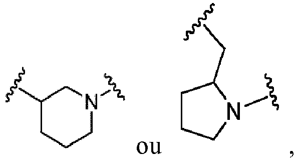 chaque cycle étant éventuellement substitué par un ou deux substituants indépendamment choisis parmi un groupe alkyle, hydroxy et halogéno, et où R^{a} est indépendamment un atome d'hydrogène ou un groupe alkyle ; et
R^{c} est un groupe alkyle, halogénoalcoxy, un groupe alkyle substitué, cycloalkyle, cycloalkylèneNR^{d}R^{e} ou cycloalkylène(alkylène)NR^{d}R^{e} (où R^{d} et R^{e} sont indépendamment un atome d'hydrogène, un groupe alkyle ou cycloalkyle), ou R^{c} est un cycle hétérocyclyle monocyclique saturé à 3 à 6 éléments contenant un ou deux hétéroatomes choisi parmi N, O et S et est éventuellement substitué par un ou deux substituants indépendamment choisis parmi un groupe hydroxy, alkyle et fluoro ; ou
(b) un composé choisi parmi :
le N-(2-(4-amino-3-(2-fluoro-4-phénoxyphényl)-1H-pyrazolo[3,4-d]pyrimidin-1-yl)éthyl)-1-cyano-2-cyclopropyléthènesulfonamide ;
le N-(2-(4-amino-3-(2-fluoro-4-phénoxyphényl)-1H-pyrazolo[3,4-d]pyrirnidin-1-yl)éthyl)-1-cyano-2-cyclopropyl-N-méthyléthènesulfonamide ;
le 2-cyano-3-cyclopropylacrylate de 2-(4-amino-3-(2-fluoro-4-phénoxyphényl)-1H-pyrazolo[3,4-d]pyrimidin-1-yl)éthyle ;
le 2-cyano-3-cyclopropylacrylate de 1-(4-amino-3-(2-fluoro-4-phénoxyphényl)-1H-pyrazolo[3,4-d]pyrimidin-1-yl)-2-méthylpropan-2-yle ;
le 2-((2-(4-amino-3-(2-fluoro-4-phénoxyphényl)-1H-pyrazolo[3,4-d]pyrimidin-1-yl)éthyl)sulfonyl)-3-cyclopropylacrylonitrile ; et
le 2-(5-((4-amino-3-(2-fluoro-4-phénoxyphényl)-1H-pyrazolo[3,4-d]pyrimidin-1-yl)méthyl)oxazol-2-yl)-3-cyclopropylacrylonitrile ;
et des sels pharmaceutiquement acceptables de ceux-ci.

2. Formulation orale solide selon la revendication 1, dans laquelle :
R^{c} est un groupe alkyle, halogénoalcoxy, alkyle substitué, cycloalkyle, cycloalkylèneNR^{d}R^{e} (où R^{d} et R^{e} sont indépendamment un atome d'hydrogène, un groupe alkyle ou cycloalkyle), ou un cycle hétérocyclyle monocyclique saturé à 3 à 6 éléments contenant un ou deux hétéroatomes choisis parmi N, O et S et éventuellement substitué par un ou deux substituants indépendamment choisis parmi un groupe hydroxy, alkyle et fluoro ; et

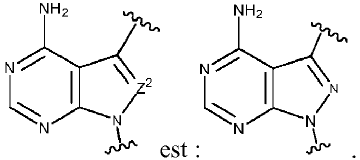

3. Formulation orale solide selon la revendication 2, dans laquelle : 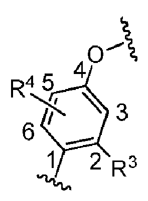 est un cycle de formule 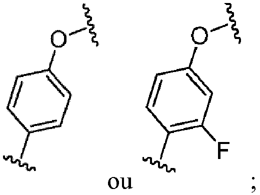 et 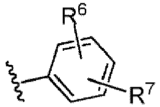 est un cycle de formule :

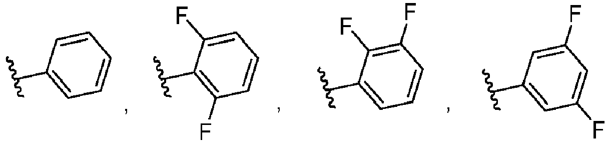

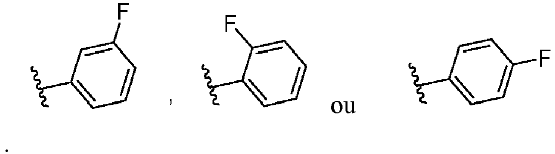

4. Formulation orale solide selon la revendication 3, dans laquelle :
Z est 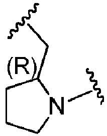 éventuellement substitué par un ou deux substituants indépendamment choisis parmi un groupe alkyle, hydroxy et halogéno ; et R^{c} est -C(CH₃)₂NH₂, -C(CH₃)₂NHCH₃, -C(CH₃)₂NHCH₃CH₃,-C(CH₃)₂NHCH(CH₃)₂, - C(CH₃)₂NHcyclopropyle, -C(CH₃)₂NH(CH₂)₂OCH₃ ou -C(CH3)₂morpholine-4-yle.

5. Formulation orale solide selon la revendication 3, dans laquelle :
Z est 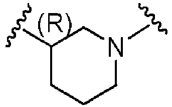 qui est éventuellement substitué par un ou deux substituants indépendamment choisis parmi un groupe alkyle, hydroxy et halogéno ; et R^{c} est un groupe isopropyle, tert-butyle, -C(CH₃)₂NH₂, -C(CH₃)₂NHCH₃, - C(CH₃)₂NHCH₂CH₃, -C(CH₃)₃NHCH(CH₃)₂, -C(CH₃)₂NHcyclopropyle, - C(CH₃)₂NH(CH₂)₂OCH₃, -C(CH₃)₂OCH₂CH₃, -C(CH₃)₂N(CH₃)₂, -C(CH₃)₂morpholine-4-yle, cyclopropyle, 2-pyrrolidinyle, 3- ou 4-pipéridinyle, 1-méthylpipéridin-4-yle, 1-méthylpipéridin-3-yle ou 4-tétrahydropyranyle.

6. Formulation orale solide selon la revendication 3, dans laquelle : 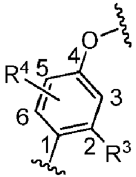 est un cycle de formule 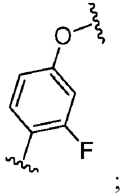 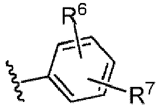 est un cycle de formule : 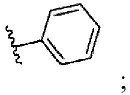 Z est 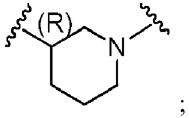 et
Rc est un groupe alkyle linéaire en C₁-C₆ ou un groupe alkyle ramifié en C₃-C₆, le groupe alkyle linéaire en C₁-C₆ ou le groupe alkyle ramifié en C₃-C₆ étant substitué par un groupe hétérocycloamino.

7. Formulation orale solide selon la revendication 1, dans laquelle le composé est :
le (R)-2-(2-((4-amino-3-(4-phénoxyphényl)-1H-pyrazolo[3,4-d]pyrimidin-1-yl)méthyl)pyrrolidine-1-carbonyl)-4,4-diméthylpent-2-ènenitrile ;
le (S)-2-(2-((4-amino-3-(4-phénoxyphényl)-1H-pyrazolo[3,4-d]pyrimidin-1-yl)méthyl)pyrrolidine-1-carbonyl)-4,4-diméthylpent-2-ènenitrile ;
le (R)-2-(2-((4-amino-3-(4-(3-fluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(4-(3-fluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(4-(3,5-difluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(4-(3,5-difluorophénoxy)phényl)-1H-pyrazolo[3,4-d]pyrimidin-1-yl)méthyl)pyrolidine-1-carbonyl)-3-cyclopropylacrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(4-(2,3-difluorophenoxy)phényl)-1H-pyrazolo[3,4-d]pyrimidin-1-yl)méthyl)pyrrolidine-1-carbonyl)-3-cyclopropylacrylonitrile;
le (S)-2-(2-((4-amino-3-(4-(2,3-difluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(4-(2,6-difluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(4-(2,6-difluorophénoxy)phényl)-1H-pyrazolo[3,4-d]pyrimidin-1-yl)méthyl)pyrrolidine-1-carbonyl)-3-cyclopropylacrylonitrile ;
le N-(2-(4-amino-3-(4-phénoxyphényl)-1H-pyrazolo[3,4-d]pyrimidin-1-yl)éthyl)-2-cyano-3-cyclopropylacrylamide ;
le (R)-2-(2-((4-amino-3-(4-(2-fluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(4-(2-fluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(4-(2,5-difluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(4-(2,5-difluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(2-fluoro-4-(3-fluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-fluoro-4-(3-fluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(2-fluoro-4-(3,5-difluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-fluoro-4-(3,5-difluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(3-fluoro-4-(phénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(3-fluoro-4-(phénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(2-fluoro-4-(2,3-difluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-fluoro-4-(2,3-difluorophénoxy)phényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(2-fluoro-4-(2,6-difluorophénoxy)phényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-fluoro-4-(2,6-difluorophénoxy)phényl)-1H-pyrazolo[3,4-d]pyrimidin-1-yl)méthyl)pyrrolidine-1-carbonyl)-3-cyclopropylacrylonitrile ;
le N-(2-(4-amino-3-(2-fluoro-4-phénoxyphényl)-1H-pyrazolo[3,4-d]pyrimidin-1-yl)éthyl)-2-cyano-3-cyclopropylacrylamide ;
le N-(2-(4-amino-3-(2-fluoro-4-phénoxyphényl)-1H-pyrazolo[3,4-d]pyrirnidin-1-yl)-2-méthylpropyl)-2-cyano-3-cyclopropylacrylamide ;
le N-(1-(4-amino-3-(2-fluoro-4-phénoxyphényl)-1H-pyrazolo[3,4-d]pyrimidin-1-yl)-2-méthylpropan-2-yl)-2-cyano-3-cyclopropylacrylamide ;
le N-(2-(4-amino-3-(2-fluoro-4-phénoxyphényl)-1H-pyrazolo[3,4-d]pyrimidin-1-yl)éthyl)-1-cyano-2-cyclopropyléthènesulfonamide ;
le N-(2-(4-amino-3-(2-fluoro-4-phénoxyphényl)-1H-pyrazolo[3,4-d]pyrimidin-1-yl)éthyl)-1-cyano-2-cyclopropyl-N-méthyléthènesulfonamide ;
le 2-cyano-3-cyclopropylacrylate de 2-(4-amino-3-(2-fluoro-4-phénoxyphényl)-1H-pyrazolo[3,4-d]pyrimidin-1-yl)éthyle ;
le 2-cyano-3-cyclopropylacrylate de 1-(4-amino-3-(2-fluoro-4-phénoxyphényl)-1H-pyrazolo[3,4-d]pyrimidin-1-yl)-2-méthylpropan-2-yle ;
le 2-((2-(4-amino-3-(2-fluoro-4-phénoxyphényl)-1H-pyrazolo[3,4-d]pyrimidin-1-yl)éthyl)sulfonyl)-3-cyclopropylacrylonitrile ;
le 2-(5-((4-amino-3-(2-fluoro-4-phénoxyphényl)-1H-pyrazolo[3,4-d]pyrimidin-1-yl)méthyl)oxazol-2-yl)-3-cyclopropylacrylonitrile ;
le (R)-2-(3-(4-amino-5-(4-(3,5-difluorophénoxy)phényl)-7H-pyrrolo[2,3-d]pyrimidin-7-yl)pipéridine-1-carbonyl)-3-cyclopropylacrylonitrile ;
le (S)-2-(3-(4-amino-5-(4-(3,5-difluorophénoxy)phényl)-7H-pyrrolo[2,3-d]pyrimidin-7-yl)pipéridine-1-carbonyl)-3-cyclopropylacrylonitrile ;
le (R)-2-(3-(4-amino-5-(2-fluoro-4-phénoxyphényl)-7H-pyrrolo[2,3-d]pyrimidin-7-yl)pipéridine-1-carbonyl)-3-cyclopropylacrylonitrile ;
le (S)-2-(3-(4-amino-5-(2-fluoro-4-phénoxyphényl)-7H-pyrrolo[2,3-d]pyrimidin-7-yl)pipéridine-1-carbonyl)-3-cyclopropylacrylonitrile ;
le (R)-2-(2-((4-amino-5-(4-phénoxyphényl)-7H-pyrrolo[2,3-d]pyrimidin-7-yl)méthyl)pyrrolidine-1-carbonyl)-3-cyclopropylacrylonitrile ;
le (S)-2-(2-((4-amino-5-(4-phénoxyphényl)-7H-pyrrolo[2,3-d]pyrimidin-7-yl)méthyl)pyrrolidine-1-carbonyl)-3-cyclopropylacrylonitrile ;
le (R)-2-(2-((4-amino-5-(4-(3,5-difluorophénoxy)phényl)-7H-pyrrolo[2,3-d]pyrimidin-7-yl)méthyl)pyrrolidine-1-carbonyl)-3-cyclopropylacrylonitrile ;
le (S)-2-(2-((4-amino-5-(4-(3,5-difluorophénoxy)phényl)-7H-pyrrolo[2,3-d]pyrimidin-7-yl)méthyl)pyrrolidine-1-carbonyl)-3-cyclopropylacrylonitrile ;
le (R)-2-(2-((4-amino-5-(2-fluoro-4-phénoxyphényl)-7H-pyrrolo[2,3-d]pyrimidin-7-yl)méthyl)pyrrolidine-1-carbonyl)-3-cyclopropylacrylonitrile ;
le (S)-2-(2-((4-amino-5-(2-fluoro-4-phénoxyphényl)-7H-pyrrolo[2,3-d]pyrimidin-7-yl)méthyl)pyrrolidine-1-carbonyl)-3-cyclopropylacrylonitrile ;
le (R)-2-(3-(4-amino-6-méthyl-5-(4-phénoxyphényl)-7H-pyrrolo[2,3-d]pyrimidin-7-yl)pipéridiné-1-carbonyl)-3-cyclopropylacrylonitrile ;
le (S)-2-(3-(4-amino-6-méthyl-5-(4-phénoxyphényl)-7H-pyrrolo[2,3-d]pyrimidin-7-yl)pipéridine-1-carbonyl)-3-cyclopropylacrylonitrile ;
le (R)-2-(3-(4-amino-5-(4-(3,5-difluorophénoxy)phényl)-6-méthyl-7H-pyrrolo[2,3-d]pyrimidin-7-yl)pipéridine-1-carbonyl)-3-cyclopropylacrylonitrile ;
le (S)-2-(3-(4-amino-5-(4-(3,5-difluorophénoxy)phényl)-6-méthyl-7H-pyrrolo[2,3-d]pyrimidin-7-yl)pipéridiné-1-carbonyl)-3-cyclopropylacrylonitrile ;
le 2-((3R)-3-(4-amino-5-(2-fluoro-4-phénoxyphényl)-6-méthyl-7H-pyrrolo[2,3-d]pyrirnidin-7-yl)pipéridine-1-carbonyl)-3-cyclopropylacrylonitrile ;
le 2-((3S)-3-(4-amino-5-(2-fluoro-4-phénoxyphényl)-6-méthyl-7H-pyrrolo[2,3-d]pyrimidin-7-yl)pipéridine-1-carbonyl)-3-cyclopropylacrylonitrile ;
le (R)-(2-(2-((4-amino-6-méthyl-5-(4-phénoxyphényl)-7H-pyrrolo[2,3-d]pyrimidin-7-yl)méthyl)pyrrolidinee-1-carbonyl)-3-cyclopropylacrylonitrile ;
le (S)-(2-(2-((4-amino-6-méthyl-5-(4-phénoxyphényl)-7H-pyrrolo[2,3-d]pyrimidin-7-yl)méthyl)pyrrolidine-1-carbonyl)-3-cyclopropylacrylonitrile ;
le (R)-2-(2-((4-amino-5-(4-(3,5-difluorophénoxy)phényl)-6-méthyl-7H-pyrrolo[2,3-d]pyrimidin-7-yl)méthyl)pyrrolidine-1-carbonyl)-3-cyclopropylacrylonitrile ;
le (S)-2-(2-((4-amino-5-(4-(3,5-difluorophénoxy)phényl)-6-méthyl-7H-pyrrolo[2,3-d]pyrimidin-7-yl)méthyl)pyrrolidine-1-carbonyl)-3-cyclopropylacrylonitrile ;
le (R)-2-(2-((4-amino-5-(2-fluoro-4-phénoxyphényl)-6-méthyl-7H-pyrrolo[2,3-d]pyrimidin-7-yl)méthyl)pyrrolidine-1-carbonyl)-3-cyclopropylacrylonitrile ; ou
le (S)-2-(2-((4-amino-5-(2-fluoro-4-phénoxyphényl)-6-méthyl-7H-pyrrolo[2,3-d]pyrimidin-7-yl)méthyl)pyrrolidine-1-carbonyl)-3-cyclopropylacrylonitrile ;
le N-(3-(4-amino-3-(4-phénoxyphényl)-1H-pyrazolo[3,4-d]pyrimidin-1-yl)-2,2-diméthylpropyl)-2-cyano-3-cyclopropylacrylamide ;
le (R)-2-(2-((4-amino-3-(2-méthyl-4-phénoxyphényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-méthyl-4-phénoxyphényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(2-chloro-4-phénoxyphényl)-1H-pyrazolo[3,4-d]pyrimidin-1-yl)méthyl)pyrrolidine-1-carbonyl)-3-cyclopropylacrylonitrile ;
le (S)-2-(2-((4-amino-3-(2-chloro-4-phénoxyphényl)-1H-pyrazolo[3,4-d]pyrimidin-1-yl)méthyl)pyrrolidine-1-carbonyl)-3-cyclopropylacrylonitrile ;
le (R)-2-(2-((4-amino-3-(4-(2,5-difluorophénoxy)phényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(4-(2,5-difluorophénoxy)phényl)-1H-pyrazolo[3,4-d]pyrimidin-1-yl)méthyl)pyrrolidine-1-carbonyl)-4-méthylpent-2-ènenitrile ;
le (R)-4-amino-2-(2-((4-amino-3-(2-fluoro-4-(3-fluorophénoxy)phényl)-1H-pyrazolo[3,4-d]pyrimidin-1-yl)méthyl)pyrrolidine-1-carbonyl)-4-méthylpent-2-ènenitrile ;
le (S)-4-amino-2-(2-((4-amino-3-(2-fluoro-4-(3-fluorophénoxy)phényl)-1H-pyrazolo[3,4-d]pyrimidin-1-yl)méthyl)pyrrolidine-1-carbonyl)-4-méthylpent-2-ènenitrile ;
le (R)-2-(2-((4-amino-3-(2-fluoro-4-(3-fluorophénoxy)phényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(2-fluoro-4-(3-fluorophénoxy)phényl)-1H-pyrazolo[3,4-d]pyrimidin-1-yl)méthyl)pyrrolidine-1-carbonyl)-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4,4-diméthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4,4-diméthylpent-2-ènenitrile ;
le (R)-2-(2-((4-amino-3-(4-(2,6-difluorophénoxy)phényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(4-(2,6-difluorophénoxy)phényl)-1H-pyrazolo[3,4-d]pyrimidin-1-yl)méthyl)pyrrolidine-1-carbonyl)-4-méthylpent-2-ènenitrile ;
le (R)-2-(2-((4-amino-3-(4-(2,3-difluorophénoxy)phényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(4-(2,3-difluorophénoxy)phényl)-1H-pyrazolo[3,4-d]pyrimidin-1-yl)méthyl)pyrrolidine-1-carbonyl)-4-méthylpent-2-ènenitrile ;
le (R)-2-(2-((4-amino-6-méthyl-5-(4-phénoxyphényl)-7H-pyrrolo[2,3-d]pyrimidin-7-yl)méthyl)pyrrolidine-1-carbonyl)-4-(diméthylamino)-4-méthylpent-2-ènenitrile ;
le (S)-2-(2-((4-amino-6-méthyl-5-(4-phénoxyphényl)-1H-pyrazolo[2,3-d]pyrimidin-7-yl)méthyl)pyrrolidine-1-carbonyl)-4-(diméthylamino)-4-méthylpent-2-ènenitrile ;
le (R)-2-(2-((4-amino-3-(4-(2,6-difluorophénoxy)-2-fluorophényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(4-(2,6-difluorophénoxy)-2-fluorophényl)-1H-pyrazolo[3,4-d]pyrimidin-1-yl)méthyl)pyrrolidine-1-carbonyl)-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)-4,4-difluoropyrrolidine-1-carbonyl)-3-cyclopropylacrylonitrile ;
le (S)-2-(2-((4-amino-3-(2-fluoro-4-phénoxyphényl)-1H-pyrazolo[3,4-d]pyrimidin-1-yl)méthyl)-4,4-difluoropyrrolidine-1-carbonyl)-3-cyclopropylacrylonitrile ;
le (R)-2-(2-((4-amino-3-(2-fluoro-4-phénoxyphényl)-1H-pyrazolo[3,4-d]pyrirnidin-1-yl)méthyl)-4,4-difluoropyrrolidine-1-carbonyl)-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)-4,4-difluoropyrrolidine-1-carbonyl)-4-méthylpent-2-ènenitrile ;
le (R)-2-(2-((4-amino-3-(2-fluoro-4-phénoxyphényl)-lH-pyrazolo[3,4-d]pyrimidin-1-yl)méthyl)pyrrolidine-1-carbonyl)-4-méhylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-méthylpent-2-ènenitrile ;
le (S)-4-amino-2-(2-((4-amino-3-(2-fluoro-4-phénoxyphényl)-1H-pyrazolo[3,4-d]pyrimidin-1-yl)méthyl)pyrrolidine-1-carbonyl)-4-méthylpent-2-ènenitrile ;
le (R)-4-amino-2-(2-((4-amino-3-(2-fluoro-4-phénoxyphényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-(méthylamino)pent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-(méthylamino)pent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-(diméthylamino)-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-(diméthylamino)-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-(éthylamino)-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-(éthylamino)-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-(isopropylamino)-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)pyrrolodine-1-carbonyl)-4-(isopropylamino)-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-(cyclopropylamino)-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-(cyclopropylamino)-4-méthylpent-2-ènenitrile ;
le (S)-2-(2-((4-amino-3-(2fluoro-4-phénoxyphényl)-1H-pyrazolo[3,4-d]pyrimidin-1-yl)méthyl)pyrrolidine-1-carbonyl)-4-((2-méthoxyéthyl)amino)-4-méthylpent-2-ènenitrile ;
le (R)-2-(2-((4-amino-3-(2-fluoro-4-phénoxyphenyl)-1H-pyrazolo[3,4-d]pyrimidin-1-yl)méthyl)pyrrolidine-1-carbonyl)-4-((2-méthoxyéthyl)amino)-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-éthoxy-4-méthylpent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-éthoxy-4-méthylpent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-aminocyclopropyl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-aminocyclopropyl)acrylonitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-(méthylamino)cyclopropyl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-(méthylamino)cyclopropyl)acrylonitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-(éthylamino)cyclopropyl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-(éthylamino)cyclopropyl)acrylonitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-(isopropylamino)cyclopropyl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-(isopropylamino)cyclopropyl)acrylonitrile ;
le 2-((S)-2-((4-amino-3-(2-fluoro-4-phénoxyphényl)-1H-pyrazolo[3,4-d]pyrimidin-1-yl)méthyl)pyrrolidine-1-carbonyl)-3-(pyrrolidin-2-yl)acrylonitrile ;
le 2-((R)-2-((4-amino-3-(2-fluoro-4-phénoxyphényl)-1H-pyrazolo[3,4-d]pyrimidin-1-yl)méthyl)pyrrolidine-1-carbonyl)-3-(pyrrolidin-2-yl)acrylonitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-morpholinopent-2-ènenitrile ;
le (R)-2-(2-((4-amino-3-(2-fluoro-4-phénoxyphényl)-1H-pyrazolo[3,4-d]pyrimidin-1-yl)méthyl)pyrrolidinr-1-carbonyl)-4-méthyl-4-morpholinopent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-((diméthylamino)méthyl)cyclopentylacrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-((diméthylamino)méthyl)cyclopentyl)acryloitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-méthylpipéridin-4-yl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(1-méthylpipéridin-4-yl)acrylonitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-3-(tétrahydro-2H-pyran-4-yl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(tétrahydro-2H-pyran-4-yl)acrylonitrile ;
le (S)-2-(2-((4-amino-3-(2-fluuoro-4-phénoxyphényl)-1H-pyrazolo[3,4-d]pyrimidin-1-yl)méthyl)pyrrolidine-1-carbonyl)-3-(pipéridin-4-yl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-3-(pipéridin-4-yl)acrylonitrile ;
le 2-((S)-2-((4-amino-3-(2-fluoro-4-phénoxyphényl)-1H-pyrazolo[3,4-d]pyrimidin-1-yl)méthyl)pyrrolidine-1-carbonyl)-3-(pipéridin-3-yl)acrylonitrile ;
le 2-((R)-2-((4-amino-3-(2-fluoro-4-phénoxyphényl)-1H-pyrazolo[3,4-d]pyrimidin-1-yl)méthyl)pyrrolidine-1-carbonyl)-3-(pipéridin-3-yl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(4-(2,3-difluorophénoxy)-2-fluorophènyl)-1H-pyrazolo[3,4-d]pyrimidin-1-yl)méthyl)pyrrolidine-1-carbonyl)-4,4-diméthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4,4-diméthylpent-2ènenitrile ;
le (S)-4-amino-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthylpent-2-ènenitrile ;
le (R)-4-amino-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthylpent-2-ènenitrile ;
le (S)-2-(2-((4-amino-3-(4-(2,3-difluorophénoxy)-2-fluorophènyl)-1H-pyrazolo[3,4-d]pyrimidin-1-yl)méthyl)pyrrolidine-1-carbonyl)-4-méthyl-4-(méthylamino)pent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthyl-4-(méthylamino)pent-2-ènenitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(diméthylamino)-4-méthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(diméthylamino)-4-méthylpent-2-ènenitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(éthylamino)-4-méthylpent-2-ènenitrile ;
le (R)-2-(2-((4-amino-3-(4-(2,3-difluorophénoxy)-2-fluorophényl)-lH-pyrazolo[3,4-d]pyrimidin-1-yl)méthyl)pyrrolidine-1-carbonyl)-4-(éthylamino)-4-méthylpent-2-ènenitrle ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(isopropylamino)-4-méthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(isopropylamino)-4-méthylpent-2-ènenitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(cyclopropylarnino)-4-méthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-(cyclopropylamino)-4-méthylpent-2-ènenitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-((2-méthoxyéthyl)amino)-4-méthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-((2-méthoxyéthyl)amino)-4-méthylpent-2-ènenitrile ;
le (S)-2-(2-((4-amino3-(4-(2,3-difluorophénoxy)-2-fluorophényl)-1H-pyrazolo[3,4-d]pyrimidin-1-yl)méthyl)pyrrolidine-1-carbonyl)-4-éthoxy-4-méthylpent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-éthoxy-4-méthylpent-2-ènenitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-aminocyclopropyl)acrylonitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-aminocyclopropyl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1(méthylamino)cyclopropyl)acrylonitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-(méthylamino)cyclopropyl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-(éthylamino)cyclopropyl)acrylonitrile ;
le (R)-2-(2-((4-amino-3-(4-(2,3-difluorophénoxy)-2-fluorophényl)-1H-pyrazolo[3,4-d]pyliimidin-1-yl)méthyl)pyrrolidine-1-carbonyl)-3-(1-(éthylamino)cyclopropyl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-(isopropylamino)cyclopropyl)acrylonitrile ;
le (R)-2-(2-((4-amino-3-(4-(2,3-difluorophénoxy)-2-fluorophényl)-lH-pyrazolo[3,4-d]pyrimidin-1-yl)méthyl)pyrrolidine-1-carbonyl)-3-(1-(isopropylamino)cyclopropyl)acrylonitrile ;
le 2-((S)-2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(pyrrolidin-2-yl)acrylonitrile ;
le 2-((R)-2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(pyrrolidin-2-yl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthyl-4-morpholinopent-2-ènenitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-4-méthyl-4-morpholinopent-2-ènenitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-((diéthylarnino)méthyl)cyclopentyl)acrylonitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-((diéthylamino)méthyl)cyclopentyl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-((diméthylamino)méthyl)cyclopentyl)acrylonitrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-((diméthylamino)-méthyl)cyclopentyl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(1-méthylpipéridin-4-yl)acrylonitrile ;
le (R)-2-(2-((4-amino-3-(4-(2,3-difluorophénoxy)-2-fluorophényl)-1H-pyrazolo[3,4-d] pyrimidin-1-yl)méthyl)pyrrolidine-1-carbonyl)-3-(1-méthylpipéridin-4-yl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(tétrahydro-2H-pyran-4-yl)acrylomtrile ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(tétrahydro-2H-pyran-4-yl)acrylonitrile ;
le (S)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(pipéridin-4-yl)acrylonitnle ;
le (R)-2-(2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(pipéridin-4-yl)acrylonitnle ;
le 2-((S)-2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(pipéridin-3-yl)acrylonitrile ;
le 2-((R)-2-((4-amino-3-(4-(2,3-difluorophénoxy)-2-fluorophényl)-1H-pyrazolo[3,4-d]pyrimidin-1-yl)méthyl)pyrrolidine-1-carbonyl)-3-(pipéridin-3-yl)acrylonitrile ;
le (R)-2-(3-(4-amino-3-(2-fluoro-4-phénoxyphényl)-1H-pyrazolo[3,4-d]pyrimidin-1-yl)pipéridine-1-carbonyl)-4-méthylpent-2-ènenitrile ;
le (S)-2-(3-(4-amino-3-(2-fluoro-4-phénoxyphényl)-1H-pyrazolo[3,4-d]pyrimidin-1-yl)pipéridine-1-carbonyl)-4-méthylpent-2-ènenitrile ;
le (R)-2-(3-(4-amino-3-(2-fluoro-4-phénoxyphényl)-1H-pyrazolo[3,4-d]pyrimidin-1-yl)pipéridine-1-carbonyl)-4,4-diméthylpent-2-ènenitrile ;
le (S)-2-(3-(4-amino-3-(2-fluoro-4-phénoxyphényl)-1H-pyrazolo[3,4-d]pyrimidin-1-yl)pipéridine-1-carbonyl)-4,4-diméthylpent-2-ènenitrile ;
le (R)-4-amino-2-(3-(4-amino-3-(2-fluoro-4-phénoxyphényl)-1H-pyrazolo[3,4-d]pyrimidin-1-yl)pipéridine-1-carbonyl)-4-méthylpent-2-ènenitrile ;
le (S)-4-amino-2-(3-(4-amino-3-(2-fluoro-4-phénoxyphényl)-1H-pyrazolo[3,4-d]pyrimidin-1-yl)pipéridine-1-carbonyl)-4-méthylpent-2-ènenitrile ;
le (R)-2-(3-(4-amino-3-(2-fluoro-4-phénoxyphényl)-1H-pyrazolo[3,4-d]pyrimidin-1-yl)pipéridine-1-carbonyl)-4-méthyl-4-(méthylamino)pent-2-ènenitrile ;
le (S)-2-(3-(4-amino-3-(2-fluoro-4-phénoxyphényl)-1H-pyrazolo[3,4-d]pyrimidin-1-yl)pipéridine-1-carbonyl)-4-méthyl-4-(méthylamino)pent-2-ènenitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-(diméthylamino)-4-méthylpent-2-ènenitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-(diméthylamino)-4-méthylpent-2-ènenitrile ;
le (R)-2-(3-(4-amino-3-(2-fluoro-4-phénoxyphényl)-1H-pyrazolo[3,4-d]pyrimidin-1-yl)pipéridine-1-carbonyl)-4-(éthylamino)-4-méthylpent-2-ènenitrile ;
le (S)-2-(3-(4-amino-3-(2-fluoro-4-phénoxyphényl)-1H-pyrazolo[3,4-d]pyrimidin-1-yl)pipéridine-1-carbonyl)-4-(éthylamino)-4-méthylpent-2-ènenitrile ;
le (R)-2-(3-(4-amino-3-(2-fluoro-4-phénoxyphényl)-1H-pyrazolo[3,4-d]pyrimidin-1-yl)pipéridine-1-carbonyl)-4-(isopropylamino)-4-méthylpent-2-ènenitrile ;
le (S)-2-(3-(4-amino-3-(2-fluoro-4-phénoxyphényl)-1H-pyrazolo[3,4-d]pyrimidin-1-yl)pipéridine-1-carbonyl)-4-(isopropylamino)-4-méthylpent-2-ènenitrile;
le (R)-2-(3-(4-amino-3-(2-fluoro-4-phénoxyphényl)-1H-pyrazolo[3,4-d]pyrimidin-1-yl)pipéridine-1-carbonyl)-4-(cyclopropylamino)-4-méthylpent-2-ènenitrile;
le (S)-2-(3-(4-amino-3-(2-fluoro-4-phénoxyphényl)-1H-pyrazolo[3,4-d]pyrimidin-1-yl)pipéridine-1-carbonyl)-4-(cyclopropylamino)-4-méthylpent-2-ènenitrile;
le (R)-2-(3-(4-amino-3-(2-fluoro-4-phénoxyphényl)-1H-pyrazolo[3,4-d]pyrimidin-1-yl)pipéridine-1-carbonyl)-4-((2-méthoxyéthyhamino)-4-méthylpent-2-ènenitrile ;
le (S)-2-(3-(4-amino-3-(2-fluoro-4-phénoxyphényl)-1H-pyrazolo[3,4-d]pyrimidin-1-yl)pipéridine-1-carbonyl)-4-((2-méthoxyéthyl)amino)-4-méthylpent-2-ènenitrile ;
le (R)-2-(3-(4-amino-3-(2-fluoro-4-phénoxyphényl)-1H-pyrazolo[3,4-d]pyrimidin-1-yl)pipéridine-1-carbonyl)-4-éthoxy-4-méthylpent-2-ènenitrile ;
le (S)-2-(3-(4-amino-3-(2-fluoro-4-phénoxyphényl)-1H-pyrazolo[3,4-d]pyrimidin-1-yl)pipéridⁱne-1-carbonyl)-4-éthoxy-4-méthylpent-2-ènenitrile ;
le (R)-2-(3-(4-amino-3-(2-fluoro-4-phénoxyphényl)-1H-pyrazolo[3,4-d]pyrimidin-1-yl)pipéridine-1-carbonyl)-3-(1-aminocyclopropyl)aclylonitrile;
le (S)-2-(3-(4-amino-3-(2-fluoro-4-phénoxyphényl)-1H-pyrazolo[3,4-d]pyrimidin-1-yl)pipéridine-1-carbonyl)-3-(1-aminocyclopropyl)acrylonitrile;
le (R)-2-(3-(4-amino-3-(2-fluoro-4-phénoxyphényl)-1H-pyrazolo[3,4-d]pyrimidin-1-yl)pipéridine-1-carbonyl)-3-(1-(méthylamino)cyclopropyl)acrylonitrile;
le (S)-2-(3-(4-amino-3-(2-fluoro-4-phénoxyphényl)-1H-pyrazolo[3,4-d]pyrimidin-1-yl)pipéridine-1-carbonyl)-3-(1-(méthylamino)cyclopropyl)acrylonitrile;
le (R)-2-(3-(4-amino-3-(2-fluoro-4-phénoxyphényl)-1H-pyrazolo[3,4-d]pyrimidin-1-yl)pipéridine-1-carbonyl)-3-(1-(éthylamino)cyclopropyl)acrylonitrile;
le (S)-2-(3-(4-amino-3-(2-fluoro-4-phénoxyphényl)-1H-pyrazolo[3,4-d]pyrimidin-1-yl)pipéridine-1-carbonyl)-3-(1-(éthylamino)cyclopropyl)acrylonitrile;
le (R)-2-(3-(4-amino-3-(2-fluoro-4-phénoxyphényl)-1H-pyrazolo[3,4-d]pyrimidin-1-yl)pipéridine-1-carbonyl)-3-(1-(isopropylamino)cyclopropyl)acrylonitrile;
le (S)-2-(3-(4-amino-3-(2-fluoro0-4-phénoxyphényl)-1H-pyrazolo[3,4-d]pyrimidin-1-yl)pipéridine-1-carbonyl)-3-(1-(isopropylamino)cyclopropyl)acrylonitrile;
le 2-((R)-3-(4-amino-3-(2-fluoro-4-phénoxyphényl)-1H-pyrazolo[3,4-d]pyrimidin-1-yl)pipéridⁱne-1-carbonyl)-3-(pyrrolidin-2-yl)acrylonitrile;
le 2-((S)-3-(4-amⁱno-3-(2-fluoro-4-phénoxyphényl)-1H-pyrazolo[3,4-d]pyrimidin-1-yl)pipéridine-1-carbonyl)-3-(pyrrolidin-2-yl)acrylonitrile;
le (S)-2-(3-(4-amino-3-(2-fluoro-4-phénoxyphényl)-1H-pyrazolo[3,4-d]pyrimidin-1-yl)pipéridⁱne-1-carbonyl)-4-méthyl-4-morpholinopent-2-ènenitrile;
le (R)-2-(3-(4-amino-3-(2-fluoro-4-phénoxyphényl)-1H-pyrazolo[3,4-d]pyrimidin-1-yl)pipéridine-1-carbonyl)-3-(1-((diéthylamino)méthyl)-cyclopentyl)acrylonitrile;
le (S)-2-(3-(4-amino-3-(2-fluoro-4-phénoxyphényl)-1H-pyrazolo[3,4-d]pyrimidin-1-yl)pipéridine-1-carbonyl)-3-(1-((diéthylamino)méthyl)-cyclopentyl)acrylonitrile;
le (R)-2-(3-(4-amino-3-(2-fluoro-4-phénoxyphényl)-1H-pyrazolo[3,4-d]pyrimidin-1-yl)pipéridine-1-carbonyl)-3-(1-((diméthylamino)méthyl)cyclopentyl)-acrylonitrile;
le (S)-2-(3-(4-amino-3-(2-fluoro-4-phénoxyphényl)-1H-pyrazolo[3,4-d]pyrimidin-1-yl)pipéridine-1-carbonyl)-3-(1-((diméthylamino)méthyl)cyclopentyl)-acrylonitrile;
le (R)-2-(3-(4-amino-3-(2-fluoro-4-phénoxyphényl)-1H-pyrazolo[3,4-d]pyrimidin-1-yl)pipéridine-1-carbonyl)-3-(1-méthylpipéridin-4-yl)acrylonitrile ;
le (S)-2-(3-(4-amino-3-(2-fluoro-4-phénoxyphényl)-1H-pyrazolo[3,4-d]pyrimidin-1-yl)pipéridine-1-carbonyl)-3-(1-méthylpipéridin-4-yl)acrylonitrile ;
le (R)-2-(3-(4-amino-3-(2-fluoro-4-phénoxyphényl)-1H-pyrazolo[3,4-d]pyrimidin-1-yl)pipéridine-1-carbonyl)-3-(tétrahydro-2H-pyran-4-yl)acrylonitrile;
le (S)-2-(3-(4-amino-3-(2-fluoro-4-phénoxyphényl)-1H-pyrazolo[3,4-d]pyrimidin-1-yl)pipéridine-1-carbonyl)-3-(tétrahydro-2H-pyran-4-yl)acrylonitrile;
le (R)-2-(3-(4-amino-3-(2-fluoro-4-phénoxyphényl)-1H-pyrazolo[3,4-d]pyrimidin-1-yl)pipéridⁱne-1-carbonyl)-3-(pipéridin-4-yl)acrylonitrile ;
le (S)-2-(3-(4-amino-3-(2-fluoro-4-phénoxyphenyl)-1H-pyrazolo[3,4-d]pyrimidin-1-yl)pipéridⁱne-1-carbonyl)-3-(pipéridin-4-yl)acrylonitrile ;
le 2-((R)-3-(4-amino-3-(2-fluoro-4-phénoxyphényl)-1H-pyrazolo[3,4-d]pyrimidin-1-yl)pipéridine-1-carbonyl)-3-(pipéridin-3-yl)acrylonitrile;
le 2-((S)-3-(4-amino-3-(2-fluoro-4-phénoxyphényl)-1H-pyrazolo[3,4-d]pyrimidin-1-yl)pipéridine-1-carbonyl)-3-(pipéridin-3-yl)acrylonitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-cyclopropylacrylonitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-cyclopropylacrylonitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-méthylpent-2-ènenitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-méthylpent-2-ènenitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4,4-diméthylpent-2-ènenitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4,4-diméthylpent-2-ènenitrile ;
le (R)-4-amino-2-(3-(4-amino-3-(4-(2,3-difluorophénoxy)-2-fluorophényl)-1H-pyrazolo[3,4-d]pyrimidin-1-yl)pipéridine-1-carbonyl)-4-méthylpent-2-ènenitrile ;
le (S)-4-amino-2-(3-(4-amino-3-(4-(2,3-difluorophénoxy)-2-fluorophényl)-1H-pyrazolo[3,4-d]pyrimidin-1-yl)pipéridine-1-carbonyl)-4-méthylpent-2-ènenitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-méthyl-4-(méthylamino)pent-2-ènenitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-méthyl-4-(méthylamino)pent-2-ènenitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-(diméthylamino)-4-méthylpent-2-ènenitrile;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-(diméthylamino)-4-méthylpent-2-ènenitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-(éthylamino)-4-méthylpent-2-ènenitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-(éthylamino)-4-méthylpent-2-ènenitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-(isopropylamino)-4-méthylpent-2-ènenitrile;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-(isopropylamino)-4-méthylpent-2-ènenitrile;
le (R)-2-(3-(4-amino-3-(4-(2,3-difluorophénoxy)-2-fluorophéenyl)-1H-pyrazolo[3,4-d]pyrimidin-1-yl)pipéridine-1-carbonyl)-4-(cyclopropylamino)-4-méthylpent-2-ènenitrile;
le (S)-2-(3-(4-amino-3-(4-(2,3-difluorophénoxy)-2-fluorophényl)-lH-pyrazolo[3,4-d]pyrirnidin-1-yl)pipéridine-1-carbonyl)-4-(cyclopropylamino)-4-méthylpent-2-ènenitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-((2-méthoxyéthylammo)-4-méthylpent-2-ènenitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-4-((2-méthoxyéthyl)amino)-4-méthylpent-2-ènenitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-éthoxy-4-méthylpent-2-ènenitrile ;
le (S)-2-(3-(4-ammo-3-(4-(2,3-difluorophénoxy)-2-fluorophényl)-1H-pyrazolo[3,4-d]pyrimidin-1-yl)pipéridine-1-carbonyl)-4-éthoxy-4-méthylpent-2-ènenitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-aminocyclopropyl)-acrylonitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-aminocyclopropyl)-acrylonitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-(méthylamino)cyclopropyl)-acrylonitrile;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-(méthylamino)cyclopropyl)-acrylonitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-(éthylamino)cyclopropyl)-acrylonitrile;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-(éthylamino)cyclopropyl)-acrylonitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-(isopropylamino)-cyclopropyl)acrylonitrile;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-(isopropylamino)-cyclopropyl)acrylonitrile;
le 2-((R)-3-(4-amino-3-(4-(2,3-difluorophénoxy)-2-fluorophényl)-1H-pyrazolo[3,4-d]pyrimidin-1-yl)pipéridine-1-carbonyl)-3-(pyrrolidin-2-yl)acrylonitrile ;
le 2-((S)-3-(4-amino-3-(4-(2,3-difluorophénoxy)-2-fluorophényl)-1H-pyrazolo[3,4-d]pyrimidin-1-yl)pipéridine-1-carbonyl)-3-(pyrrolidin-2-yl)acrylonitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-4-méthyl-4-morpholinopent-2-ènenitrile;
le (S)-2-(3-(4-amino-3-(4-(2,3-difluorophénoxy)-2-fluorophényl)-lH-pyrazolo[3,4-d]pyrirnidin-1-yl)pipéridine-1-carbonyl)-4-méthyl-4-morpholinopent-2-ènenitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-((diéthylamino)méthyl)-cyclopentyl)acrylonitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-((diéthylamino)méthyl)-cyclopentyl)acrylonitrile;
le (R)-2-(3-(4-amino-3-(4-(2,3-difluorophénoxy)-2-fluorophényl)-lH-pyrazolo[3,4-d]pyrimidin-1-yl)pipéridine-1-carbonyl)-3-(1-((diméthylamino)-méthyl)cyclopentyl)acrylonitrile ;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-((diméthylamino)-méthyl)cyclopentyl)acrylonitrile ;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(1-méthylpipéridin-4-yl)acrylonitrile;
le (S)-2-(3-(4-amino-3-(4-(2,3-difluorophénoxy)-2-fluorophényl)-lH-pyrazolo[3,4-d]pyrimidin-1-yl)pipéridine-1-carbonyl)-3-(1-méthylpipéridin-4-yl)acrylonitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(tétrahydro-2H-pyran-4-yl)acrylonitrile;
le (S)-2-(3-(4-amino-3-(4-(2,3-difluorophénoxy)-2-fluorophényl)-1H-pyrazolo[3,4-d]pyrimidin-1-yl)pipéridine-1-carbonyl)-3-(tétrahydro-2H-pyran-4-yl)acrylonitrile;
le (R)-2-(3-(4-amino-3-(4-(2,3-difluorophénoxy)-2-fluorophényl)-1H-pyrazolo[3,4-d]pyrimidin-1-yl)pipéridine-1-carbonyl)-3-(pipéridin-4-yl)acrylonitrile ;
le (S)-2-(3-(4-amino-3-(4-(2,3-difluorophénoxy)-2-fluorophényl)-lH-pyrazolo[3,4-d]pyrimidin-1-yl)pipéridine-1-carbonyl)-3-(pipéridin-4-yl)acrylonitrile ;
le 2-((R)-3-(4-amino-3-(4-(2,3-difluorophénoxy)-2-fluorophényl)-1H-pyrazolo[3,4-d]pyrimidin-1-yl)pipéridine-1-carbonyl)-3-(pipéridin-3-yl)acrylonitrile ;
le 2-((S)-3-(4-amino-3-(4-(2,3-difluorophénoxy)-2-fluorophényl)-1H-pyrazolo[3,4-d]pyrimidin-1-yl)pipéridine-1-carbonyl)-3-(pipéridin-3-yl)acrylonitrile ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-(pipéridin-1-yl)pent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-(pipéridin-1-yl)pent-2-ènenitrile;
le (R)-2-(2-((4-amino-5-(2-fluoro-4-phénoxyphényl)-7H-pyrrolo[2,3-d]pyrimidin-7-yl)méthl)pyrrolidine-1-carbonyl)-4-(diméthylamino)-4-méthylpent-2-ènenitrile;
le (S)-2-(2-((4-amino-5-(2-fluoro-4-phénoxyphényl)-7H-pyrrolo[2,3-d]pyrimidin-7-yl)méthyl)pyrrolidine-1-carbonyl)-4-(diméthylamino)-4-méthylpent-2-ènenitrile;
le (R)-2-(2-((4-amino-5-(4-(2,3-difluorophénoxy)phényl)-7H-pyrrolo[2,3-d]pyrimidin-7-yl)méthyl)pyrrolidine-1-carbonyl)-3-cyclopropylacrylonitrile ;
le (S)-2-(2-((4-amino-5-(4-(2,3-difluorophénoxy)phényl)-7H-pyrrolo[2,3-d]pyrimidin-7-yl)méthyl)pyrrolidine-1-carbonyl)-3-cyclopropylacrylonitrile;
le (R)-2-(2-((4-amino-5-(4-(2,3-difluorophénoxy)phényl)-7H-pyrrolo[2,3-d]pyrimidin-7-yl)méthyl)pyrrolidine-1-carbonyl)-4-méthylpent-2-ènenitrile ;
le (S)-2-(2-((4-amino-5-(4-(2,3-difluorophénoxy)phényl)-7H-pyrrolo[2,3-d]pyrimidin-7-yl)méthyl)pyrrolidine-1-carbonyl)-4-méthylpent-2-ènenitrile ;
le (R)-2-(2-((4-amino-5-(4-(2,3-difluorophénoxy)phényl)-7H-pyrrolo[2,3-d]pyrimidin-7-yl)méthyl)pyrrolidine-1-carbonyl)-4,4-diméthylpent-2-ènenitrile ;
le (S)-2-(2-((4-amino-5-(4-(2,3-difluorophénoxy)phényl)-7H-pyrrolo[2,3-d]pyrimidin-7-yl)méthyl)pyrrolidine-1-carbonyl)-4,4-diméthylpent-2-ènenitrile;
le (R)-2-(2-((4-amino-5-(4-(2,3-difluorophénoxy)phényl)-7H-pyrrolo[2,3-d]pyrimidin-7-yl)méthyl)pyrrolidine-1-carbonyl)-4-(diméthylamino)-4-méthylpent-2-ènenitrile;
le (S)-2-(2-((4-amino-5-(4-(2,3-difluorophénoxy)phényl)-7H-pyrrolo[2,3-d]pyrimidin-7-yl)méthyl)pyrrolidine-1-carbonyl)-4-(diméthylamino)-4-méthylpent-2-ènenitrile;
le (R)-2-(2-((4-amino-5-(4-phénoxyphényl)-7H-pyrrolo[2,3-d]pyrimidin-7-yl)méthyl)pyrrolidine-1-carbonyl)-4-(diméthylamino)-4-méthylpent-2-ènenitrile;
le (S)-2-(2-((4-amino-5-(4-phénoxyphényl)-7H-pyrrolo[2,3-d]pyrimidin-7-yl)méthyl)pyrrolidine-1-carbonyl)-4-(diméthylamino)-4-méthylpent-2-ènenitrile;
le (R)-2-(2-((4-amino-5-(4-phénoxyphényl)-7H-pyrrolo[2,3-d]pyrimidin-7-yl)méthyl)pyrrolidine-1-carbonyl)-4-méthylpent-2-ènenitrile;
le (S)-2-(2-((4-amino-5-(4-phénoxyphényl)-7H-pyrrolo[2,3-d]pyrimidin-7-yl)méthyl)pyrrolidine-1-carbonyl)-4-méthylpent-2-ènenitrile ;
le (R)-4-arnino-2-(2-((4-amino-5-(2-fluoro-4-phénoxyphényl)-7H-pyrrolo[2,3-d]pyrimidin-7-yl)méthyl)pyrrolidine-1-carbonyl)-4-méthylpent-2-ènenitrile ;
le (S)-4-amino-2-(2-((4-amino-5-(2-fluoro-4-phénoxyphényl)-7H-pyrrolo[2,3-d]pyrimidin-7-yl)méthyl)pyrrolidine-1-carbonyl)-4-méthylpent-2-ènenitrile ;
le (R)-2-(2-((4-amino-5-(4-phénoxyphényl)-7H-pyrrolo[2,3-d]pyrimidin-7-yl)méthyl)pyrrolidine-1-carbonyl)-4-(diméthylamino)-4-méthylpent-2-ènenitrile;
le (S)-2-(2-((4-amino-5-(4-phénoxyphényl)-7H-pyrrolo[2,3-d]pyrimidin-7-yl)méthyl)pyrrolidine-1-carbonyl)-4-(diméthylamino)-4-méthylpent-2-ènenitrile;
le (R)-2-(2-((4-amino-5-(4-phénoxyphényl)-7H-pyrrolo[2,3-d]pyrimidin-7-yl)méthyl)pyrrolidine-1-carbonyl)-4-méthyl-4-morpholinopent-2-ènenitrile;
le (S)-2-(2-((4-amino-5-(4-phénoxyphényl)-7H-pyrrolo[2,3-d]pyrimidin-7-yl)méthyl)pyrrolidine-1-carbonyl)-4-méthyl-4-morpholinopent-2-ènenitrile;
le (R)-2-(2-((4-amino-5-(4-phénoxyphényl)-7H-pyrrolo[2,3-d]pyrimidin-7-yl)méthyl)pyrrolidine-1-carbonyl)-4-(diéthylamino)-4-méthylpent-2-ènenitrile;
le (S)-2-(2-((4-amino-5-(4-phénoxyphényl)-7H-pyrrolo[2,3-d]pyrimidin-7-yl)méthyl)pyrrolidine-1-carbonyl)-4-(diéthylamino)-4-méthylpent-2-ènenitrile ;
le (R)-2-(2-((4-amino-5-(4-phénoxyphényl)-7H-pyrrolo[2,3-d]pyrimidin-7-yl)méthyl)pyrrolidine-1-carbonyl)-4-éthoxy-4-méthylpent-2-ènenitrile ;
le (S)-2-(2-((4-amino-5-(4-phénoxyphényl)-7H-pyrrolo[2,3-d]pyrimidin-7-yl)méthyl)pyrrolidine-1-carbonyl)-4-éthoxy-4-méthylpent-2-ènenitrile ;
le (R)-N-(1-(4-amino-3-(2-fluoro-4-phénoxyphényl)-1H-pyrazolo[3,4-d]pyrimidin-1-yl)propan-2-yl)-2-cyano-4,4-diméthylpent-2-énamide ;
le (S)-N-(1-(4-amino-3-(2-fluoro-4-phénoxyphényl)-1H-pyrazolo[3,4-d]pyrimidin-1-yl)propan-2-yl)-2-cyano-4,4-diméthylpent-2-énamide ;
le (R)-N-(1-(4-amino-3-(2-fluoro-4-phénoxyphényl)-1H-pyrazolo[3,4-d]pyrimidin-1-yl)propan-2-yl)-2-cyano-4-méthylpent-2-énamide ;
le (S)-N-(1-(4-amino-3-(2-fluoro-4-phénoxyphényl)-1H-pyrazolo[3,4-d]pyrimidin-1-yl)propan-2-yl)-2-cyano-4-méthylpent-2-énamide ;
le (R)-N-(1-(4-amino-3-(2-fluoro-4-phénoxyphényl)-1H-pyrazolo[3,4-d]pyrimidin-1-yl)propan-2-yl)-2-cyano-4-éthoxy-4-méthylpent-2-énamide ;
le (S)-N-(1-(4-amino-3-(2-fluoro-4-phénoxyphényl)-1H-pyrazolo[3,4-d]pyrimidin-1-yl)propan-2-yl)-2-cyano-4-éthoxy-4-méthylpent-2-énamide ;
le (R)-N-(1-(4-amino-3-(2fluoro-4-phénoxyphényl)-1H-pyrazolo[3,4-d]pyrimidin-1-yl)propan-2-yl)-2-cyano-4-(diméthylamino)-4-méthylpent-2-énamide ;
le (S)-N-(1-(4-amino-3-(2-fluoro-4-phénoxyphényl)-1H-pyrazolo[3,4-d]pyrirnidin-1-yl)propan-2-yl)-2-cyano-4-(diméthylamino)-4-méthylpent-2-énamide ;
le 2-((R)-2-((4-amino-3-(2-fluoro-4-phénoxyphényl)-1H-pyrazolo[3,4-d]pyrimidin-1-yl)méthyl)pyrrolidine-1-carbonyl)-3-((S)-pyrrolidin-2-yl)acrylonitrile;
le 2-((R)-2-((4-amino-3-(2-fluoro-4-phénoxyphényl)-1H-pyrazolo[3,4-d]pyrimidin-1-yl)méthyl)pyrrolidine-1-carbonyl)-3-((R)-pyrrolidin-2-yl)acrylonitrile ;
le (R)-4-amino-N-(1-(4-amino-3-(2-fluoro-4-phénoxyphényl)-1H-pyrazolo[3,4-d]pyrimidin-1-yl)propan-2-yl)-2-cyano-4-méthylpent-2-énamide ;
le (S)-4-amino-N-(1-(4-amino-3-(2-fluoro-4-phénoxyphényl)-1H-pyrazolo[3,4-d]pyrimidin-1-yl)propan-2-yl)-2-cyano-4-méthylpent-2-énamide ;
le N-(2-(4-amino-3-(4-phénoxyphényl)-1H-pyrazolo[3,4-d]pyrimidin-1-yl)éthyl)-2-cyano-3-cyclopropyl-N-méthylacrylamide ;
le (R)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-(pipéridin-1-yl)pent-2-ènenitrile ;
le (S)-2-(2-((4-amino-3-(2-fluoro-4-phénoxyphényl)-1H-pyrazolo[3,4-d]pyrimidin-1-yl)méthyl)pyrrolidine-1-carbonyl)-4-méthyl-4-(pipéridin-1-yl)pent-2-ènenitrile ;
le (S)-N-(1-(4-amino-3-(2-fluoro-4-phénoxyphényl)-1H-pyrazolo[3,4-d]pyrimidin-1-yl)propan-2-yl)-2-cyano-4,4-diméthylpent-2-énamide ;
le (S)-N-(1-(4-amino-3-(2-fluoro-4-phénoxyphényl)-1H-pyrazolo[3,4-d]pyrimidin-1-yl)propan-2-yl)-2-cyano-4-méthylpent-2-énamide ;
le (S)-N-(1-(4-amino-3-(2-fluoro-4-phénoxyphényl)-1H-pyrazolo[3,4-d]pyrimidin-1-yl)propan-2-yl)-2-cyano-4-(diméthylamino)-4-méthylpent-2-énamide ;
le (S)-N-(1 -(4-amino-3-(2-fluoro-4-phénoxyphényl)-1H-pyrazolo[3,4-d]pyrimidin-1-yl)propan-2-yl)-2-cyano-4-éthoxy-4-méthylpent-2-énamide ;
le (S)-4-amino-N-(1-(4-amino-3-(2-fluoro-4-phénoxyphényl)-1H-pyrazolo[3,4-d]pyrimidin-1-yl)propan-2-yl)-2-cyano-4-méthylpent-2-énamide ;
le (S)-N-(1-(4-amino-3-(2-fluoro-4-phénoxyphényl)-1H-pyrazolo[3,4-d]pyrimidin-1-yl)propan-2-yl)-2-cyano-3-cyclopropylacrylamide;
le 2-[(2R)-2-[[4-amino-3-(2-fluoro-4-phénoxy-phényl)pyrazolo[3,4-d]pyrimidin-1-yl]méthyl]-pyrrolidine-1-carbonyl]-3-(3-méthyloxétan-3-yl)prop-2-ènenitrile;ou
le 2-[(2S)-2-[[4-amino-3-(2-fluoro-4-phénoxy-phényl)pyrazolo[3,4-d]pyrimidin-1-yl]méthyl]-pyrrolidine-1-carbonyl]-3-(3-méthyloxétan-3-yl)prop-2-ènenitrile;
ou un mélange d'isomères R et S ;
ou un isomère (E) ou (Z) individuel de ceux-ci ;
ou un sel pharmaceutiquement acceptable de l'un quelconque des éléments précités.

8. Formulation orale solide selon la revendication 1, dans laquelle le composé est :
le (R)-2-(3-(4-amino-3-(2-fluoro-4-phénoxyphényl)-1H-pyrazolo[3,4-d]pyrimidin-1-yl)pipéridⁱne-1-carbonyl)-4,4-diméthylpent-2-ènenitrile ;
ou un isomère (E) ou (Z) individuel de celui-ci ;
ou un sel pharmaceutiquement acceptable de celui-ci.

9. Formulation orale solide selon la revendication 1, dans laquelle le composé est :
le 2-(2-((4-amino-3-(2-fluoro-4-phénoxyphényl)-1H-pyrazolo[3,4-d]pyrimidin-1-yl)-méthyl)pyrrolidine-1-carbonyl)-4,4-diméthylpent-2-ènenitrile ;
ou un isomère R ou S individuel de celui-ci ;
ou un isomère (E) ou (Z) individuel de celui-ci ;
ou un sel pharmaceutiquement acceptable de celui-ci.

10. Formulation orale solide selon l'une quelconque des revendications précédentes, dans laquelle l'enrobage est un enrobage entérique qui se dissout à un pH de 4,5 à 7.

11. Formulation orale solide selon l'une quelconque des revendications précédentes, dans laquelle l'enrobage est un enrobage entérique qui est un polymère anionique.

12. Formulation orale solide selon la revendication 11, dans laquelle le polymère anionique est choisi parmi des polyméthacrylates, des polymères à base de cellulose et des dérivés polyvinyliques.

13. Formulation orale solide selon l'une quelconque des revendications précédentes, dans laquelle l'enrobage est un enrobage entérique qui se dissout à un pH de 5,5 à 7.

14. Formulation orale solide selon la revendication 1, dans laquelle le composé est le 2-(3-(4-amino-3-(2-fluoro-4-phénoxyphényl)-1H-pyrazolo[3,4-d]pyrimidin-1-yl)pipéridine-1-carbonyl)-4,4-diméthylpent-2-ènenitrile ;
ou un isomère R ou S individuel de celui-ci ;
ou un isomère (E) ou (Z) individuel de celui-ci ;
ou un sel pharmaceutiquement acceptable des composés précités.

15. Formulation destinée à une utilisation dans le traitement d'une maladie qui est une maladie auto-immune ou inflammatoire ou un cancer, la formulation étant une formulation orale solide telle que définie dans l'une quelconque des revendications 1 à 14.

16. Formulation destinée à une utilisation selon la revendication 15, dans laquelle la maladie inflammatoire est une maladie intestinale inflammatoire, l'arthrite, le lupus, la polyarthrite rhumatoïde, l'arthrite psoriasique, l'arthrose, la maladie Still, la polyarthrite juvénile, le diabète, la myasthénie grave, la thyroïdite de Hashimoto, la thyroïdite de Ord, la maladie de Graves, le syndrome de Sjögren, la sclérose en plaques, le syndrome de Guillain-Barré, l'encéphalomyélite disséminée aiguë, la maladie d'Addison, le syndrome opsoclonus-myoclonus, la spondylarthrite ankylosante, le syndrome de l'anticorps antiphospholipide, l'anémie aplasique, l'hépatite auto-immune, la maladie cœliaque, le syndrome de Goodpasture, le purpura thrombocytopénique idiopathique, la névrite optique, la sclérodermie, la cirrhose biliaire primaire, le syndrome de Reiter, l'artérite de Takayasu, l'artérite temporale, l'anémie hémolytique auto-immune à anticorps chauds, la granulomatose de Wegener, le psoriasis, l'alopécie universelle, la maladie de Behçet, la fatigue chronique, la dysautonomie, l'endométriose, la cystite interstitielle, la neuromyotonie, la sclérodermie ou la vulvodynie, l'asthme, l'appendicite, la blépharite, la bronchiole, la bronchite, la bursite, la cervicite, la cholangite, la cholécystite, la colite, la conjonctivite, la cystite, la dacryoadénite, la dermatite, la dermatomyosite, l'encéphalite, l'endocardite, l'endométrite, l'entérite, l'entérocolite, l'épicondylite, l'épididymite, la fasciite, la fibrosite, la gastrite, la gastroentérite, l'hépatite, l'hidrosadénite suppurée, la laryngite, la mastite, la méningite, la myélite, la myocardite, la myosite, la néphrite, l'oophorite, l'orchite, l'ostéite, l'otite, la pancréatite, la parotidite, la péricardite, la péritonite, la pharyngite, la pleurite, la phlébite, la pneumonite, la pneumonie, la proctite, la prostatite, la pyélonéphrite, la rhinite, la salpingite, la sinusite, la stomatite, la synovite, la tendinite, la tonsillite, l'uvéite, la vaginite, la vascularite ou la vulvite, la dermatite, la dermatite de contact, l'eczéma, l'urticaire, la rosacée et les lésions psoriasiques cicatricielles de la peau, des articulation ou d'autres tissus ou organes, et la formulation est administrée éventuellement en combinaison avec au moins agent anti-inflammatoire.

17. Formulation destinée à une utilisation selon la revendication 16, dans laquelle la maladie est le lupus, la polyarthrite rhumatoïde, l'arthrite psoriasique, le syndrome de Sjögren, la granulomatose de Wegener, le psoriasis, l'asthme ou l'uvéite.
